(19) Europäisches Patentamt
European Patent Office
Office européen des brevets

(11) EP 2 308 880 A1

(12) **EUROPEAN PATENT APPLICATION**
published in accordance with Art. 153(4) EPC

(43) Date of publication:
**13.04.2011 Bulletin 2011/15**

(21) Application number: **09773568.2**

(22) Date of filing: **03.07.2009**

(51) Int Cl.:
*C07D 491/044* (2006.01) *A61K 31/4162* (2006.01)
*A61P 1/02* (2006.01) *A61P 1/04* (2006.01)
*A61P 1/16* (2006.01) *A61P 1/18* (2006.01)
*A61P 3/10* (2006.01) *A61P 5/14* (2006.01)
*A61P 5/38* (2006.01) *A61P 7/06* (2006.01)
*A61P 7/10* (2006.01) *A61P 9/10* (2006.01)
*A61P 9/14* (2006.01) *A61P 11/00* (2006.01)
*A61P 11/06* (2006.01) *A61P 13/12* (2006.01)
*A61P 17/00* (2006.01) *A61P 17/02* (2006.01)
*A61P 17/04* (2006.01) *A61P 17/06* (2006.01)
*A61P 17/08* (2006.01)

(86) International application number:
**PCT/JP2009/062184**

(87) International publication number:
**WO 2010/001990 (07.01.2010 Gazette 2010/01)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **03.07.2008 JP 2008174346**

(71) Applicant: **Kyowa Hakko Kirin Co., Ltd.**
**Tokyo 100-8185 (JP)**

(72) Inventors:
- **YAMAMOTO, Junichiro**
  **Sunto-gun,**
  **Shizuoka 411-8731 (JP)**
- **MORI, Kiyotoshi**
  **San Diego, CA 92128 (US)**
- **ERA, Tomohiro**
  **Sunto-gun,**
  **Shizuoka 411-8731 (JP)**
- **NAKASATO, Yoshisuke**
  **Sunto-gun,**
  **Shizuoka 411-8731 (JP)**
- **UCHIDA, Kenji**
  **Sunto-gun,**
  **Shizuoka 411-8731 (JP)**

(74) Representative: **Vossius & Partner**
**Siebertstrasse 4**
**81675 München (DE)**

(54) **TETRACYCLIC COMPOUND**

(57) Provided are a tetracyclic compound represented by the formula (I):

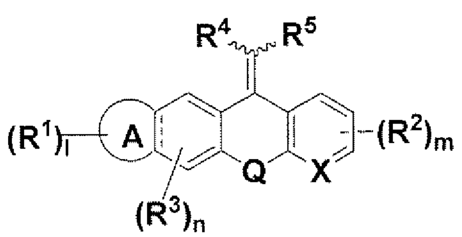
(I)

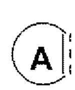
(A)

[in the formula(I),the formula (A) represents a benzene ring or the like,
X represents CH or a nitrogen atom,
Q represents -O- or the like,$R^1$, $R^2$, and $R^3$ may be the same or different and each represent a hydrogen atom or the like,
1, m, and n each represent an integer from one to the maximum substitutable number of substituents, and

EP 2 308 880 A1

$R^4$ and $R^5$ may be the same or different and each represent a hydrogen atom or the like], or a pharmaceutically acceptable salt thereof, and the like.

## Description

TECHNICAL FIELD

The present invention relates to a tetracyclic compound or a pharmaceutically acceptable salt thereof having a modulating effect to glucocorticoid receptors, and the like.

BACKGROUND ART

The glucocorticoid receptor, a member of the nuclear receptor family, is a ligand-dependent transcription factor activated or inactivated in response to binding of a ligand, such as endogenous cortisol. The glucocorticoid receptor usually resides in the cytoplasm in a form binding to a heat shock protein 90 (Hsp90). When a ligand binds to it, the glucocorticoid receptor dissociates from the Hsp90 and moves into the nucleus. After that, the glucocorticoid receptor forms a complex with a coactivator which mediates gene expression, and binds to a glucocorticoid response element (GRE) on the chromosome to regulate gene expression. In addition to the above-mentioned gene expression regulatory mechanism, other actions of regulating gene expression by capturing a coactivator necessary for another transcription factor have been reported.

Such regulation of the gene expression relates to various physiological effects, such as amino acid metabolism, lipid metabolism, bone calcium metabolism, water and electrolyte metabolism, gluconeogenesis, anti-inflammatory effects, immunosuppressive effects, central nervous system activation, and the like. In particular, the immunosuppressive effects and the anti-inflammatory effects of glucocorticoid receptor ligands are widely used for the therapy of acute and chronic diseases. For example, it is shown that the strong anti-inflammatory effects of glucocorticoid are useful for the therapy of many inflammatory diseases. However, since glucocorticoid has side effects, such as hyperglycemia, hypertension, hyperlipemia, osteoporosis, adrenal atrophy, glaucoma, cataract, loss of muscle strength, susceptibility to infections, moon face, atrophoderma, pigmentation, hypertrichosis, insomnia, and menstrual disorder, the amount used is restricted in some cases, which is a problem in clinical applications.

Examples of diseases relating to the glucocorticoid receptor include reversible obstructive airway disease, chronic obstructive pulmonary disease (COPD), chronic bronchial asthma, intrinsic asthma, extrinsic asthma, dust asthma, late-onset asthma and airway hyperreactivity, bronchitis, interstitial pneumonia, idiopathic interstitial pneumonia, pulmonary edema, toxic pulmonary edema, restrictive lung disease, allergic alveolitis, pulmonary fibrosis, pulmonary emphysema, adult respiratory distress syndrome (ARDS), rheumatic disease, reactive arthritis, inflammatory parenchyma disease, arthritis deformans, connective tissue disease, systemic lupus erythematosus, systemic lupus erythematosus, systemic sclerosis, dermatomyositis, polymyositis, polyarteritis nodosa, mixed connective tissue disease, Sjogren syndrome, microscopic polyangiitis, Wegener granulomatosis, allergic granulomatous angiitis, hypersensitivity angiitis, Behcet disease, Cogan syndrome, RS3PE (remitting seronegative symmetrical synovitis with pitting edema), Hashimoto thyroiditis, myasthenia gravis, atopic dermatitis, psoriasis, pityriasis rubra pilaris, erythematous disease caused by various kinds of noxa (lights, chemicals, burns, or the like), bullous skin disease, discoid lichenoid disease, pruritus, seborrheic eczema, roseola, pemphigus vulgaris, erythema exsudativum multiforme, balanitis, vulvitis, alopecia such as alopecia areata, cutaneous T-cell lymphoma, acne, scleroderma, cutaneous hypereosinophilic syndrome, male pattern alopecia, alopecia senilis, vitiligo vulgaris, photoallergic sensitivity, inflammatory and hyperproliferative skin disease, erythema multiforme, pyoderma, keratoconjunctivitis, conjunctivitis, uveitis, keratitis, panophthalmitis, optic neuritis, choroiditis, sympathetic ophthalmia, keratoconus, keratoleukoma, Mooren ulcer, scleritis, Graves ophthalmopathy, Vogt-Koyanagi-Harada syndrome, sarcoidosis, cataract, glaucoma, siderosis, retinitis pigmentosa, senile macular degeneration, rhinitis, sinusitis, pollinosis, nasal polyp, otitis externa, otitis media, cerebral edema, multiple sclerosis, acute encephalomyelitis, meningitis, various kinds of convulsive disorders (BNS convulsions, or the like), Guillain-Barre syndrome, Meniere disease, polyneuritis, mononeuritis, radiculopathy, allergic encephalomyelitis, cerebral infarction, inflammatory bowel disease, local enterocolitis (Crohn disease), ulcerative colitis, ischemic bowel disease, necrotizing enterocolitis, abdominal cavity disease, reflux esophagitis, gastroenteritis caused by other causes (eosinophilic gastroenteritis, regional sprue, or the like), anal eczema, anal fissure, hemorrhoids, idiopathic proctitis, mastocytosis, pseudomembranous colitis, acute hepatocyte disruption, acute hepatitis, chronic progressive hepatitis, chronic intermittent hepatitis, autoimmune hepatitis, virus B hepatitis, cirrhosis, alcoholic cirrhosis, hepatic failure, fulminant hepatic failure, late-onset hepatic failure, hepatic fibrosis, nephrotic syndrome, ischemic acute renal failure, chronic renal failure, interstitial nephritis, lupus nephritis, Goodpasture syndrome, hemolytic uremic syndrome, diabetic nephritis, glomerulonephritis, post-infectious autoimmune disease such as rheumatic fever and post-infectious glomerulonephritis, renal fibrosis, acquired hemolytic anemia, idiopathic thrombocytopenia, erythroid aplasia, aplastic anemia, hypoplastic anemia, idiopathic thrombocytopenic purpura, autoimmune hemolytic anemia, agranulocytosis, pernicious anemia, megaloblastic anemia, anerythroplasia, acute lymphocytic leukemia, malignant lymphoma, lymphogranuloma, lymphosarcoma, extensive metastasis (in particular, of breast cancer, bronchial cancer, prostate cancer, or the like), cutaneous T-cell

lymphoma, Wegener granuloma, endocrine ophthalmopathy, thyrotoxicosis, Quervain thyroiditis, Hashimoto disease, Basedow disease, hyperthyroidism, adiposis, Addison disease, angioedema, vasculitis, vascular injury caused by ischemic disease or thrombosis, angiitis, myocardial infarction, cardiomyopathy, edematous disease, varix, dissecting aneurysm of the aorta, angina pectoris, atherosclerosis, aortitis syndrome, arteriosclerosis obliterans, arteriosclerosis, thromboangiitis obliterans, polyarteritis nodosa, ischemia-repertusian injury, polymyositis, eosinophilic fasciitis, muscular dystrophy, diabetes mellitus type I and type II, osteoporosis, sepsis, a lesion of the gingiva, the periodontium, the alveolar bone, or the cementum of the tooth, transplant rejection, graft-versus-host disease, endotoxin shock, gingivitis, periodontitis, pancreatitis, and the like. Applications to all these diseases are described in detail in Hatz, HJ, Glucocoriticoid: Immunologische, Pharmakologie und Therapie richtlinien, Wissenshafliche Verlagssellschat Wissenshaftliche Verlagsgesellschaft mbH, Stuttgart (1998).

Therefore, compounds having a modulating effect to glucocorticoid receptors are considered to be capable of treating and/or preventing these diseases.

Meanwhile, glucocorticoid receptor modulators having a tricyclic structure are known (Patent Literatures 1 to 5).

PRIOR ART

Patent Literatures

[Patent Literature 1] WO 2004/052847
[Patent Literature 2] WO 2005/066153
[Patent Literature 3] WO 2005/066161
[Patent Literature 4] WO 2008/008882
[Patent Literature 5] WO 2008/021926

SUMMARY OF INVENTION

PROBLEMS TO BE SOLVED BY INVENTION

An object of the present invention is to provide a tetracyclic compound or a pharmaceutically acceptable salt thereof having a modulating effect to glucocorticoid receptors, and the like.

MEANS FOR SOLVING PROBLEMS

The present invention relates to the following (1) to (34).

(1) A tetracyclic compound represented by the formula (I) :

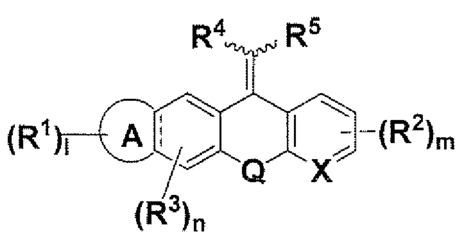

(I)

[in the formula (I)

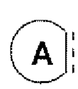

represents a benzene ring or a monocyclic aromatic heterocycle;
X represents CH or a nitrogen atom;

Q represents -O-, -S-, $-NR^6-$ (wherein $R^6$ represents optionally substituted lower alkyl, optionally substituted cycloalkyl, optionally substituted aryl, an optionally substituted aliphatic heterocyclic group, or an optionally substituted aromatic heterocyclic group), $-CH_2-CH_2-$, -CH=CH-, $-CH_2-O-$, $-O-CH_2-$, $-CH_2-S-$, $-S-CH_2-$, $-NR^6-CO-$ (wherein $R^6$ has the same meaning as defined above), $-CO-NR^6-$ (wherein $R^6$ has the same meaning as defined above), $-NR^6-CH_2-$ (wherein $R^6$ has the same meaning as defined above), $-CH_2-NR_6-$ (wherein $R^6$ has the same meaning as defined above), -CH=N-, or -N=CH-;

$R^1$, $R^2$, and $R^3$ may be the same or different and each represent a hydrogen atom, hydroxy, cyano, carboxy, halogen, optionally substituted lower alkyl, optionally substituted lower alkenyl, optionally substituted lower alkynyl, optionally substituted cycloalkyl, optionally substituted lower alkanoyl, optionally substituted lower alkoxycarbonyl, optionally substituted aryl, optionally substituted aroyl, optionally substituted arylsulfonyl, optionally substituted lower alkylsulfonyl, an optionally substituted aliphatic heterocyclic group, an optionally substituted aromatic heterocyclic group, $-NR^7R^8$ (wherein $R^7$ and $R^8$ may be the same or different and each represent a hydrogen atom, optionally substituted lower alkylsulfonyl, optionally substituted lower alkyl, optionally substituted lower alkanoyl, optionally substituted aryl, an optionally substituted aliphatic heterocyclic group, or an optionally substituted aromatic heterocyclic group, or $R^7$ and $R^8$ are combined together with the adjacent nitrogen atom to form an optionally substituted nitrogen-containing heterocyclic group), or $-OR^9$ (wherein $R^9$ represents optionally substituted lower alkyl, optionally substituted lower cycloalkyl, optionally substituted lower alkanoyl, optionally substituted aryl, an optionally substituted aliphatic heterocyclic group, or an optionally substituted aromatic heterocyclic group); l, m, and n may be the same or different and each represent an integer from one to the maximum substitutable number; and $R^4$ and $R^5$ may be the same or different and each represent a hydrogen atom, cyano, carboxy, halogen, optionally substituted lower alkyl, optionally substituted lower alkenyl, optionally substituted lower alkynyl, optionally substituted cycloalkyl, optionally substituted lower alkanoyl, optionally substituted lower alkoxycarbonyl, optionally substituted aroyl, optionally substituted arylsulfonyl, optionally substituted lower alkylsulfonyl, optionally substituted aryl, an optionally substituted aromatic heterocyclic group, or $-CONR^{10}R^{11}$ (wherein $R^{10}$ and $R^{11}$ may be the same or different and each represent a hydrogen atom, optionally substituted lower alkyl, optionally substituted cycloalkyl, optionally substituted aryl, or an optionally substituted aromatic heterocyclic group)],
or a pharmaceutically acceptable salt thereof.

(2) The tetracyclic compound or a pharmaceutically acceptable salt thereof according to (1), wherein $R^4$ is a hydrogen atom, cyano, carboxy, halogen, optionally substituted lower alkyl, optionally substituted lower alkenyl, optionally substituted lower alkynyl, optionally substituted cycloalkyl, optionally substituted lower alkanoyl, optionally substituted lower alkoxycarbonyl, optionally substituted aroyl, optionally substituted arylsulfonyl, or optionally substituted lower alkylsulfonyl, and $R^5$ is carboxy, optionally substituted aryl, an optionally substituted aromatic heterocyclic group, optionally substituted lower alkoxycarbonyl, or $-CONR^{10}R^{11}$ (wherein $R^{10}$ and $R^{11}$ have the same meanings as defined above, respectively).

(3) The tetracyclic compound or a pharmaceutically acceptable salt thereof according to (2), wherein $R^5$ is optionally substituted aryl, or an optionally substituted aromatic heterocyclic group.

(4) The tetracyclic compound or a pharmaceutically acceptable salt thereof according to any one of (1) to (3), wherein each of l, m, and n is 1.

(5) The tetracyclic compound or a pharmaceutically acceptable salt thereof according to any one of (1) to (4), wherein Q is $-CH_2-O-$ or $-O-CH_2-$.

(6) The tetracyclic compound or a pharmaceutically acceptable salt thereof according to any one of (1) to (5), wherein $R^4$ is lower alkyl.

(7) The tetracyclic compound or a pharmaceutically acceptable salt thereof according to any one of (1) to (5), wherein $R^4$ is ethyl.

(8) The tetracyclic compound or a pharmaceutically acceptable salt thereof according to any one of (1), (2), and (4) to (7), wherein $R^5$ is (lower alkylsulfonamido)phenyl.

(9) The tetracyclic compound or a pharmaceutically acceptable salt thereof according to any one of (1), (2), and (4) to (7), wherein $R^5$ is 3-(methanesulfonamido)phenyl.

(10) The tetracyclic compound or a pharmaceutically acceptable salt thereof according to any one of (1), (2), and

(4) to (7), wherein $R^5$ is $-CONR^{10}R^{11}$ (wherein $R^{10}$ and $R^{11}$ have the same meanings as defined above).

(11) The tetracyclic compound or a pharmaceutically acceptable salt thereof according to any one of (1) to (10), wherein

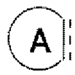

is a monocyclic aromatic heterocycle.

(12) A tetracyclic compound represented by the formula (Ia) or (Ib):

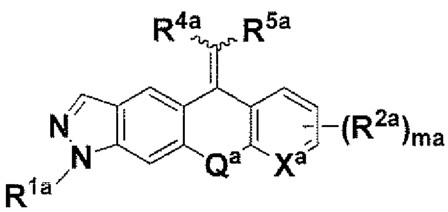 (Ia) 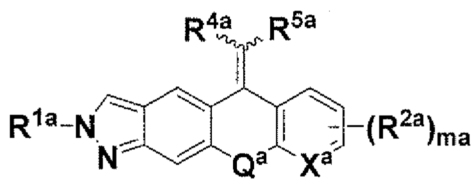 (Ib) ,

(wherein $X^a$, $Q^a$, $R^{1a}$, $R^{2a}$, $R^{4a}$, and $R^{5a}$ have the same meanings as those of X, Q, $R^1$, $R^2$, $R^4$, and $R^5$ described above, respectively; and ma represents an integer of 1 to 3), or a pharmaceutically acceptable salt thereof.

(13) The tetracyclic compound or a pharmaceutically acceptable salt thereof according to (12), which is represented by the formula (Ia).

(14) The tetracyclic compound or a pharmaceutically acceptable salt thereof according to (12) or (13), wherein $Q^a$ is $-CH_2-O-$ or $-O-CH_2-$.

(15) The tetracyclic compound or a pharmaceutically acceptable salt thereof according to any one of (12) to (14), wherein $R^{4a}$ is lower alkyl.

(16) The tetracyclic compound or a pharmaceutically acceptable salt thereof according to any one of (12) to (14), wherein $R^{4a}$ is ethyl.

(17) The tetracyclic compound or a pharmaceutically acceptable salt thereof according to any one of (12) to (16), wherein $R^{5a}$ is (lower alkylsulfonamido)phenyl.

(18) The tetracyclic compound or a pharmaceutically acceptable salt thereof according to any one of (12) to (16), wherein $R^{5a}$ is 3-(methanesulfonamido)phenyl.

(19) The tetracyclic compound or a pharmaceutically acceptable salt thereof according to any one of (12) to (16), wherein $R^{5a}$ is $-CONR^{10a}R^{11a}$ (wherein $R^{10a}$ and $R^{11a}$ have the same meanings as those of $R^{10}$ and $R^{11}$ described above, respectively) .

(20) A tetracyclic compound represented by the formula (Ic) or (Id):

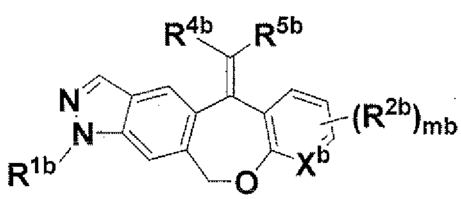 (Ic) 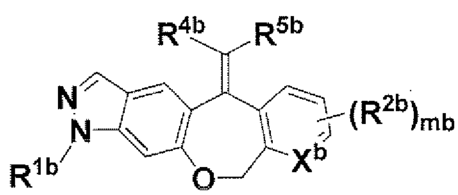 (Id) ,

(wherein $X^b$, $R^{1b}$, $R^{2b}$, $R^{4b}$, and $R^{5b}$ have the same meanings as those of X, $R^1$, $R^2$, $R^4$, and $R^5$ described above, respectively; and mb represents an integer of 1 to 3), or a pharmaceutically acceptable salt thereof.

(21) The tetracyclic compound or a pharmaceutically acceptable salt thereof according to (20), wherein $R^{4b}$ is lower alkyl.

(22) The tetracyclic compound or a pharmaceutically acceptable salt thereof according to (20), wherein $R^{4b}$ is ethyl.

(23) The tetracyclic compound or a pharmaceutically acceptable salt thereof according to any one of (20) to (22), wherein $R^{5b}$ is (lower alkylsulfonamido) phenyl.

(24) The tetracyclic compound or a pharmaceutically acceptable salt thereof according to any one of (20) to (22), wherein $R^{5b}$ is 3-(methanesulfonamido) phenyl.

(25) The tetracyclic compound or a pharmaceutically acceptable salt thereof according to any one of (20) to (22), wherein $R^{5b}$ is $-CONR^{10b}R^{11b}$ (wherein $R^{10b}$ and $R^{11b}$ have the same meanings as those of $R^{10}$ and $R^{11}$ described above, respectively) .

(26) A pharmaceutical composition comprising, as an active ingredient, the tetracyclic compound or a pharmaceutically acceptable salt thereof according to any one of (1) to (25).

(27) A glucocorticoid receptor modulator comprising, as an active ingredient, the tetracyclic compound or a pharmaceutically acceptable salt thereof according to any one of (1) to (25).

(28) A therapeutic and/or preventive agent for a disease in which glucocorticoid receptors are involved, comprising, as an active ingredient, the tetracyclic compound or a pharmaceutically acceptable salt thereof according to any one of (1) to (25).

(29) A method for modulating glucocorticoid receptors, comprising a step of administering an effective amount of the tetracyclic compound or a pharmaceutically acceptable salt thereof according to any one of (1) to (25).

(30) A method for treating and/or preventing a disease in which glucocorticoid receptors are involved, comprising a step of administering an effective amount of the tetracyclic compound or a pharmaceutically acceptable salt thereof according to any one of (1) to (25).

(31) Use of the tetracyclic compound or a pharmaceutically acceptable salt thereof according to any one of (1) to (25) for the manufacture of a glucocorticoid receptor modulator.

(32) Use of the tetracyclic compound or a pharmaceutically acceptable salt thereof according to any one of (1) to (25) for the manufacture of a therapeutic and/or preventive agent for a disease in which glucocorticoid receptors are involved.

(33) The tetracyclic compound or a pharmaceutically acceptable salt thereof according to any one of (1) to (25) for use in modulating glucocorticoid receptors.

(34) The tetracyclic compound or a pharmaceutically acceptable salt thereof according to any one of (1) to (25) for use in treating and/or preventing a disease in which glucocorticoid receptors are involved.

## EFFECTS OF INVENTION

The present invention provides, for example, a tetracyclic compound or a pharmaceutically acceptable salt thereof, having a modulating effect to glucocorticoid receptors and being useful for treatment of a disease in which glucocorticoid receptors are involved, such as inflammatory disease, chronic bronchial asthma, rheumatic disease, connective tissue disease, systemic lupus erythematosus, or systemic lupus erythematosus, or the like.

## MODES FOR CARRYING OUT INVENTION

Hereinafter, the compound represented by the formula (I) is referred to as Compound (I). The same applies to

the other compounds having different formula numbers.

The definitions of the respective groups in the formulae (I) are as follows.

(i) Examples of the lower alkyl and the lower alkyl moieties of the lower alkylsulfonyl, the lower alkanoyl, and the lower alkoxycarbonyl include linear or branched alkyl having 1 to 10 carbon atoms. Specific examples thereof include methyl, ethyl, propyl, isopropyl, butyl, isobutyl, sec-butyl, tert-butyl, pentyl, isopentyl, neopentyl, hexyl, heptyl, octyl, nonyl, decyl, and the like.

(ii) Examples of the lower alkenyl include linear or branched alkenyl having 2 to 10 carbon atoms. Specific examples thereof include vinyl, allyl, 1-propenyl, isopropenyl, methacryl, butenyl, crotyl, pentenyl, hexenyl, heptenyl, decenyl, and the like.

(iii) Examples of the lower alkynyl include linear or branched alkynyl having 2 to 10 carbon atoms. Specific examples thereof include ethynyl, propargyl, butynyl, pentynyl, hexynyl, heptynyl, octynyl, nonynyl, decynyl, and the like.

(iv) Examples of the cycloalkyl include cyclic alkyl having 3 to 10 carbon atoms. Specific examples thereof include cyclopropyl, cyclobutyl, cyclopentyl, cyclohexyl, cycloheptyl, cyclooctyl, cyclononyl, cyclodecyl, and the like.

(v) Examples of the aryl and the aryl moieties of the arylsulfonyl, and the aroyl include aryl having 6 to 14 carbon atoms. Specific examples thereof include phenyl, naphthyl, indenyl, anthryl, and the like.

(vi) Examples of the aliphatic heterocyclic group include a 5- or 6-membered monocyclic aliphatic heterocyclic group which contains at least one atom selected from a nitrogen atom, an oxygen atom, and a sulfur atom, a bicyclic or tricyclic condensed aliphatic heterocyclic group in which 3- to 8-membered rings are fused, which contains at least one atom selected from a nitrogen atom, an oxygen atom and a sulfur atom, and the like.

More specific examples thereof include aziridinyl, azetidinyl, pyrrolidinyl, piperidino, piperidinyl, azepanyl, 1,2,5,6-tetrahydropyridyl, imidazolidinyl, pyrazolidinyl, piperazinyl, homopiperazinyl, pyrazolinyl, oxiranyl, oxetanyl, tetrahydrofuranyl, tetrahydro-2H-pyranyl, 5, 6-dihydro-2H-pyranyl, tetrahydrothiopyranyl, oxazolidinyl, morpholino, morpholinyl, thioxazolidinyl, thiomorpholinyl, 2H-oxazolyl, 2H-thioxazolyl, dihydroindolyl, dihydroisoindolyl, dihydrobenzofuranyl, benzimidazolidinyl, dihydrobenzoxazolyl, dihydrobenzothioxazolyl, benzodioxolinyl, tetrahydroquinolyl, tetrahydroisoquinolyl, dihydro-2H-chromanyl, dihydro-1H-chromanyl, dihydro-2H-thiochromanyl, dihydro-1H-thiochromanyl, tetrahydroquinoxalinyl, tetrahydroquinazolinyl, dihydrobenzodioxanyl, dihydroquinolyl, dihydrodibenzoazepinyl, and the like.

(vii) Examples of the monocyclic aromatic heterocycle include a 5- or 6-membered monocyclic aromatic heterocycle which contains at least one atom selected from a nitrogen atom, an oxygen atom and a sulfur atom. Specific examples thereof include pyridine, pyrazine, pyrimidine, pyridazine, pyrrole, pyrazole, imidazole, triazine, triazole, thiophene, furan, thiazole, isothiazole, oxazole, isoxazole, oxadiazole, thiadiazole, and the like. More preferred examples include pyrazole, and the like.

(viii) Examples of the aromatic heterocyclic group include a 5- or 6-membered monocyclic aromatic heterocyclic group which contains at least one atom selected from a nitrogen atom, an oxygen atom and a sulfur atom, a bicyclic or tricyclic condensed aromatic heterocyclic group in which 3- to 8-membered rings are fused and which contains at least one atom selected from a nitrogen atom, an oxygen atom and a sulfur atom, and the like. Specific examples thereof include pyridyl, pyrazinyl, pyrimidinyl, pyridazinyl, oxopyridazinyl, quinolyl, isoquinolyl, phthalazinyl, quinazolinyl, quinoxalinyl, naphthyridinyl, cinnolinyl, pyrrolyl, pyrazolyl, imidazolyl, triazinyl, triazolyl, tetrazolyl, thienyl, furyl, thiazolyl, isothiazolyl, oxazolyl, isoxazolyl, oxadiazolyl, thiadiazolyl, indolyl, isoindolyl, indazolyl, benzofuryl, isobenzofuryl, benzothienyl, benzimidazolyl, benzotriazolyl, benzothiazolyl, benzoxazolyl, pyrazolopyridyl, pyrazolopyrimidinyl, purinyl, dibenzofuranyl, dibenzoazepinyl, and the like.

(ix) Examples of the nitrogen-containing heterocyclic group formed together with the adjacent nitrogen atom include a 5- or 6-membered monocyclic heterocyclic group which contains at least one nitrogen atom (the monocyclic heterocyclic group may further contain another nitrogen atom, an oxygen atom or a sulfur atom), a bicyclic or tricyclic condensed heterocyclic group in which 3- to 8-membered rings are fused, which contains at least one nitrogen atom (the condensed heterocyclic group may further contain another nitrogen atom, an oxygen atom or a sulfur atom), and the like. More specific examples thereof include aziridinyl, azetidinyl, pyrrolidinyl, piperidino, azepanyl, pyrrolyl, imidazolidinyl, imidazolyl, pyrazolidinyl, pyrazolinyl, pyrazolyl, piperazinyl, homopiperazinyl, oxazolidinyl, 2H-oxazolyl, thioxazolidinyl, 2H-thioxazolyl, morpholino, thiomorpholino, dihydroindolyl, dihydroisoindolyl, indolyl, isoindolyl, tetrahydroquinolyl, tetrahydroisoquinolyl, dihydrobenzoxazolyl, dihydrobenzothioxazolyl, benzimidazolidinyl, benzimidazolyl, dihydroindazolyl, indazolyl, benzotriazolyl, pyrrolopyridinyl, pyrrolopyrimidinyl, imidazopyridinyl, purinyl, and the like.

(x) The halogen means each atom of fluorine, chlorine, bromine, or iodine.

(xi) The substituents of the optionally substituted lower alkyl, optionally substituted lower alkenyl, optionally substituted lower alkynyl, optionally substituted lower alkoxycarbonyl, optionally substituted lower alkanoyl, and optionally substituted lower alkylsulfonyl may be the same or different and in number of, for example, 1 to 3, and examples thereof include halogen, hydroxy, sulfanyl, nitro, cyano, carboxy, carbamoyl, N-lower alkylcarbamoyl, N,N-di-lower

alkylcarbamoyl, cycloalkyl, optionally substituted aryl, an optionally substituted aliphatic heterocyclic group, an optionally substituted aromatic heterocyclic group, optionally substituted lower alkoxy, cycloalkoxy, aryloxy, lower alkanoyloxy, aroyloxy, lower alkylsulfanyl, lower alkylsulfonyloxy, arylsulfonyloxy, $-NR^{X1}R^{Y1}$ (wherein $R^{X1}$ and $R^{Y1}$ may be the same or different and each represent a hydrogen atom, optionally substituted lower alkyl, lower alkylsulfonyl, cycloalkyl, aryl, an aromatic heterocyclic group, lower alkanoyl, aroyl, or optionally substituted lower alkoxycarbonyl), lower alkanoyl, aroyl, lower alkoxycarbonyl, aryloxycarbonyl, and the like.

The substituents (xi-1) of the optionally substituted aryl, the optionally substituted aliphatic heterocyclic group, and the optionally substituted aromatic heterocyclic group described herein may be the same or different and in number of, for example, 1 to 3 and examples thereof include halogen; lower alkyl; lower alkoxy; lower alkoxycarbonyl; N-lower alkylcarbamoyl; N,N-di-lower alkylcarbamoyl; lower alkanoyl; carboxy; cyano; oxo; hydroxy; aryl, and the like.

The substituents (xi-2) of the optionally substituted lower alkyl, the optionally substituted lower alkoxycarbonyl, and the optionally substituted lower alkoxy described herein may be the same or different and in number of, for example, 1 to 3, and examples thereof include halogen; lower alkoxy; lower alkoxycarbonyl; N-lower alkylcarbamoyl; N,N-di-lower alkylcarbamoyl; lower alkanoyl; carboxy; cyano; hydroxy; aryl, and the like.

(xii) The substituents of the optionally substituted aryl, the optionally substituted aroyl, the optionally substituted arylsulfonyl, and the optionally substituted aromatic heterocyclic group may be the same or different and in number of, for example, 1 to 3, and examples thereof include halogen, hydroxy, sulfanyl, nitro, cyano, carboxy, carbamoyl, lower alkyl, trifluoromethyl, cycloalkyl, an aliphatic heterocyclic group, aryl, an aromatic heterocyclic group, optionally substituted lower alkoxy, cycloalkoxy, aryloxy, lower alkanoyloxy, aroyloxy, lower alkylsulfanyl, lower alkylsulfonyl, lower alkylsulfonyloxy, $-NR^{X2}R^{Y2}$ (wherein $R^{X2}$ and $R^{Y2}$ have the same meanings as those of $R^{X1}$ and $R^{Y1}$ as described above, respectively), lower alkanoyl, aroyl, lower alkoxycarbonyl, aryloxycarbonyl, N-lower alkylcarbamoyl, N,N-di-lower alkylcarbamoyl, and the like.

The substituents (xii-1) of the optionally substituted lower alkoxy described herein may be the same or different and in number of, for example, 1 to 3, and examples thereof include optionally substituted aryl and the like.

Examples of the substituent (xii-1-1) of the substituted aryl include
halogen; lower alkyl; lower alkoxy; lower alkoxycarbonyl; N-lower alkylcarbamoyl; N,N-di-lower alkylcarbamoyl; lower alkanoyl; carboxy; cyano; oxo; hydroxy; aryl, and the like.

(xiii) The substituents of the optionally substituted cycloalkyl, the optionally substituted aliphatic heterocyclic group, and the optionally substituted nitrogen-containing heterocyclic group formed together with the adjacent nitrogen atom may be the same or different and in number of, for example, 1 to 3, and examples thereof include oxo, halogen, hydroxy, sulfanyl, nitro, cyano, carboxy, carbamoyl, lower alkyl, cycloalkyl, aryl, an aliphatic heterocyclic group, an aromatic heterocyclic group, optionally substituted lower alkoxy, cycloalkoxy, aryloxy, lower alkanoyloxy, aroyloxy, lower alkylsulfanyl, lower alkylsulfonyl, $-NR^{X3}R^{Y3}$ (wherein $R^{X3}$ and $R^{Y3}$ have the same meanings as those of $R^{X1}$ and $R^{Y1}$ as described above, respectively), lower alkanoyl, aroyl, lower alkoxycarbonyl, aryloxycarbonyl, N-lower alkylcarbamoyl, N,N-di-lower alkylcarbamoyl, and the like.

The substituents (xiii-1) of the optionally substituted lower alkoxy described herein may be the same or different and in number of, for example, 1 to 3, and examples thereof include optionally substituted aryl.

Examples of the substituent (xiii-1-1) of the substituted aryl include the substituents illustrated in the above (xii-1-1).

Examples of the lower alkyl moieties of the lower alkyl, the lower alkoxy, the lower alkoxycarbonyl, the lower alkanoyl, the lower alkanoyloxy, the lower alkylsulfanyl, the lower alkylsulfonyl, the lower alkylsulfonyloxy, the N-lower alkylcarbamoyl, and the N,N-di-lower alkylcarbamoyl include the groups illustrated in the above (i) lower alkyl. The two lower alkyls in the N, N-di-lower alkylcarbamoyl may be the same or different.

Examples of the cycloalkyl and the cycloalkyl moiety of the cycloalkoxy include the groups illustrated in the above cycloalkyl.

Examples of the aryl moieties of the aryl, the aryloxy, aroyl, the arylsulfonyloxy, the aroyloxy, and the aryloxycarbonyl include the groups illustrated in the above aryl.

Examples of the aliphatic heterocyclic group, the aromatic heterocyclic group, and the halogen include the groups illustrated in the above aliphatic heterocyclic group, the above aromatic heterocyclic group, and the above halogen, respectively.

In addition to the above (1) to (25), as for the definitions of the groups in Compounds (I) and the like, in the definitions of the groups in Compounds (I), (Ia), (Ib), (Ic), and (Id), $R^1$, $R^{1a}$, and $R^{1b}$ each are preferably optionally substituted aryl, an optionally substituted aromatic heterocyclic group, optionally substituted lower alkyl, or the like;
$R^2$, $R^{2a}$, $R^{2b}$, and $R^3$ each are preferably a hydrogen atom;
$R^4$, $R^{4a}$, and $R^{4b}$ each are preferably a hydrogen atom, halogen, optionally substituted lower alkyl, optionally substituted lower alkenyl, $-CONR^{10}R^{11}$ (wherein $R^{10}$ and $R^{11}$ may be the same or different and each represent a hydrogen atom, optionally substituted lower alkyl, optionally substituted cycloalkyl, optionally substituted aryl, or an optionally substituted

aromatic heterocyclic group), or the like, and more preferably a hydrogen atom, methyl, ethyl, propyl, vinyl, 2-hydroxyethyl, or the like;

$R^5$, $R^{5a}$, and $R^{5b}$ each are preferably halogen, carboxy, optionally substituted aryl, an optionally substituted aromatic heterocyclic group, optionally substituted lower alkyl, optionally substituted lower alkoxycarbonyl, -$CONR^{10}R^{11}$ (wherein $R^{10}$ and $R^{11}$ have the same meanings as defined above, respectively), or the like, and more preferably substituted phenyl, a substituted aromatic heterocyclic group, substituted lower alkoxycarbonyl, -$CONHR^{10}$ (wherein $R^{10}$ has the same meaning as defined above), or the like.

Each of l, m, and n in Compound (I) is preferably 1.

Each of ma and mb in Compounds (Ia), (Ib), (Ic), and (Id) is preferably 1.

Each of X, $X^a$, and $X^b$ in Compounds (I), (Ia), (Ib), (Ic), and (Id) is preferably CH or a nitrogen atom.

Q in Compound (I) is preferably -$CH_2$-O- or -O-$CH_2$-.

$Q^a$ in Compounds (Ia) and (Ib) is preferably -$CH_2$-O- or -O-$CH_2$-.

Preferably,

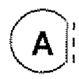

is pyrazole.

Examples of the pharmaceutically acceptable salt of Compound (I) include pharmaceutically acceptable acid addition salts, metal salts, ammonium salts, organic amine addition salts, amino acid addition salts, and the like.

Examples of the pharmaceutically acceptable acid addition salts of Compound (I) include inorganic acid salts such as hydrochlorides, sulfates, and phosphates; organic acid salts such as acetates, maleates, fumarates, tartrates, citrates, and methanesulfonates; and the like. Examples of the pharmaceutically acceptable metal salts include alkali metal salts such as sodium salts and potassium salts; alkaline earth metal salts such as magnesium salts and calcium salts; aluminum salts; zinc salts; and the like. Examples of the pharmaceutically acceptable ammonium salts include salts of ammonium, tetramethylammonium, or the like. Examples of the pharmaceutically acceptable organic amine addition salts include addition salts of morpholine, piperidine, or the like. Examples of the pharmaceutically acceptable amino acid addition salts include addition salts of lysine, glycine, phenylalanine, or the like.

Hereinafter, production methods of Compound (I) are described.

In the production methods described below, in the case where a defined group changes under the conditions of the production methods or is not suitable for carrying out the production methods, it is possible to produce a desired compound with use of a method commonly used in synthetic organic chemistry such as the methods for introducing and removing a protecting group (for example, the method described in T. W. Greene, Projective Groups in Organic Synthesis, fourth edition, John Wiley & Sons Inc. (1999), or the like). The order of reaction steps, such as introduction of a substituent, may be changed as necessary.

Production Method 1

Among Compounds (I), Compound (I-A) in which $R^5$ is a hydrogen atom, cyano, optionally substituted lower alkyl, optionally substituted lower alkenyl, optionally substituted lower alkynyl, optionally substituted cycloalkyl, optionally substituted aryl, or an optionally substituted aromatic heterocyclic group, Compound (I-A') in which $R^5$ is halogen, and Compound (I-A") in which $R^5$ is a hydrogen atom can be produced, for example, according to the following steps.

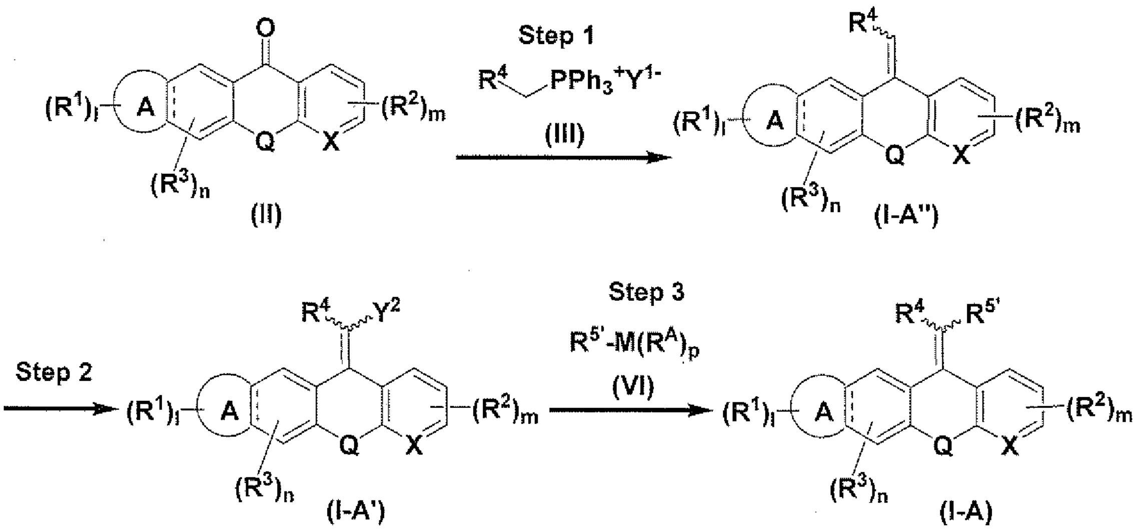


(In the formula, X, Q, $R^1$, $R^2$, $R^3$, $R^4$, l, m, and n have the same meanings as those defined in claim 1, respectively; $R^{5'}$ represents, among the meanings defined as the above $R^5$, a hydrogen atom, cyano, optionally substituted lower alkyl, optionally substituted lower alkenyl, optionally substituted lower alkynyl, optionally substituted cycloalkyl, optionally substituted aryl, or an optionally substituted aromatic heterocyclic group;
M represents, a tin atom, a boron atom, or a silicon atom; Ph represents phenyl;
$R^A$ represents a fluorine atom, a chlorine atom, a bromine atom, an iodine atom, hydroxy, $C_{1-6}$ alkyl, $C_{1-6}$ alkoxy, aryl, or aryloxy;
p represents an integer of 0 to 3;
$Y^1$ represents a chlorine atom, a bromine atom, or an iodine atom; $Y^2$ represents, among the meanings defined as the above $R^5$, halogen; and

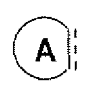

has the same meaning as defined above.)

Step 1

Compound (I-A") can be produced by reacting Compound (II) with 1 to 30 equivalents of a Wittig reagent represented by Compound (III), in a solvent, in the presence of 1 to 30 equivalents of a base, at a temperature between -90˚C and 200˚C for 5 minutes to 100 hours.

Examples of the solvent include methanol, ethanol, dichloromethane, acetonitrile, toluene, ethyl acetate, tetrahydrofuran (THF), 1,4-dioxane, N,N-dimethylformamide (DMF), N, N-dimethylacetamide (DMA), N-methylpyrrolidone (NMP), water, and the like. These solvents may be used alone or as a mixture thereof.

Examples of the base include n-butyllithium, sec-butyllithium, tert-butyllithium, lithium diisopropylamide, sodium hydride, potassium hydride, potassium carbonate, potassium tert-butoxide, sodium tert-butoxide, pyridine, triethylamine, dimethylaminopyridine, N-methylmorpholine, 1,8-diazabicyclo[5.4.0]undec-7-ene (DBU), N,N-diisopropylethylamine, and the like. These bases may be used alone or as a mixture thereof.

Compound (II) can be obtained according to a method described in Reference Examples, or known methods (for example, Chemical & Pharmaceutical Bulletin, vol. 39, p. 2429, (1991)).

Compound (III) can be obtained as a commercial product, or according to known methods (for example, Jikken Kagaku Kouza 4th edition, No. 24, p. 252, Maruzen (2000)).

Step 2

Compound (I-A') can be produced by reacting Compound (I-A") with 1 to 5 equivalents of a halogenating agent, in a solvent, at a temperature between -50˚C and 200˚C for 5 minutes to 100 hours. In this case, 0.01 to 30 equivalents

of an additive may be added to accelerate the reaction.

Examples of the solvent include methanol, ethanol, dichloromethane, chloroform, carbon tetrachloride, acetonitrile, toluene, ethyl acetate, THF, 1,4-dioxane, acetic acid, trifluoroacetic acid, and the like. These solvents may be used alone or as a mixture thereof.

Examples of the halogenating agent include chlorine, hydrogen chloride gas, concentrated hydrochloric acid, hydrobromic acid, tetra-n-butylammonium tribromide, bromine, iodine, N-chlorosuccinimide (NCS), N-bromosuccinimide (NBS), N-iodosuccinimide (NIS), iodine monochloride, pyridinium bromide perbromide, 4-dimethylaminopyridinium bromide perbromide, and the like. Examples of the additive include silver sulfate, copper acetate, calcium carbonate, zinc chloride, and the like.

Step 3

Compound (I-A) can be produced by reacting Compound (I-A') with 1 to 30 equivalents of Compound (VI), in a solvent, in the presence of 0.001 to 1 equivalent of a transition metal catalyst at a temperature between -50°C and 200°C for 5 minutes to 100 hours. In this case, 0.01 to 30 equivalents of a suitable additive may be added to accelerate the reaction.

Examples of the solvent include methanol, ethanol, dichloromethane, acetonitrile, toluene, ethyl acetate, THF, 1,4-dioxane, DMF, NMP, water, and the like. These solvents may be used alone or as a mixture thereof.

Examples of the transition metal catalyst include a palladium catalyst, such as palladium acetate, tetrakis (triphenylphosphine)palladium, palladium chloride, palladium bromide, bis(triphenylphosphine)palladium chloride, bis (acetonitrile)palladium dichloride, and [1,1'-bis(diphenylphosphino)ferrocene]palladium(II) dichloride dichloromethane complex (1:1), and the like; a nickel catalyst, such as nickel chloride, nickel acetylacetonate, bis(1,5-cyclooctadiene) nickel, and nickel bromide; and the like.

Examples of the additive include triphenylphosphine, tri(o-tolyl)phosphine, 1,1'-bis(diphenylphosphino)ferrocene, 1,2-bis(diphenylphosphino)propane, 2,2'-bis(diphenylphosphino)-1,1'-binaphthyl, 1,2-bis(diphenylphosphino) ethane, silver oxide, copper iodide, lithium chloride, cesium fluoride, triethylamine, diethylamine, sodium hydroxide, potassium hydroxide, sodium carbonate, tripotassium phosphate, and the like. These additives may be used alone or as a mixture thereof.

Compound (VI) can be obtained as a commercial product, or according to known methods (for example, Jikken Kagaku Kouza, 5th sedition, No. 18, Synthesis of organic compounds VI, Organic synthesis with the use of metal, p. 97, Maruzen (2005)).

Production Method 2

Among Compounds (I), Compound (I-C), Compound (I-B), and Compound (I-D), in which $R^5$ is carboxy, optionally substituted lower alkoxy carbonyl, or $-CONR^{10}R^{11}$ (wherein $R^{10}$ and $R^{11}$ have the same meanings as defined above, respectively) can be produced, for example, according to the following steps.

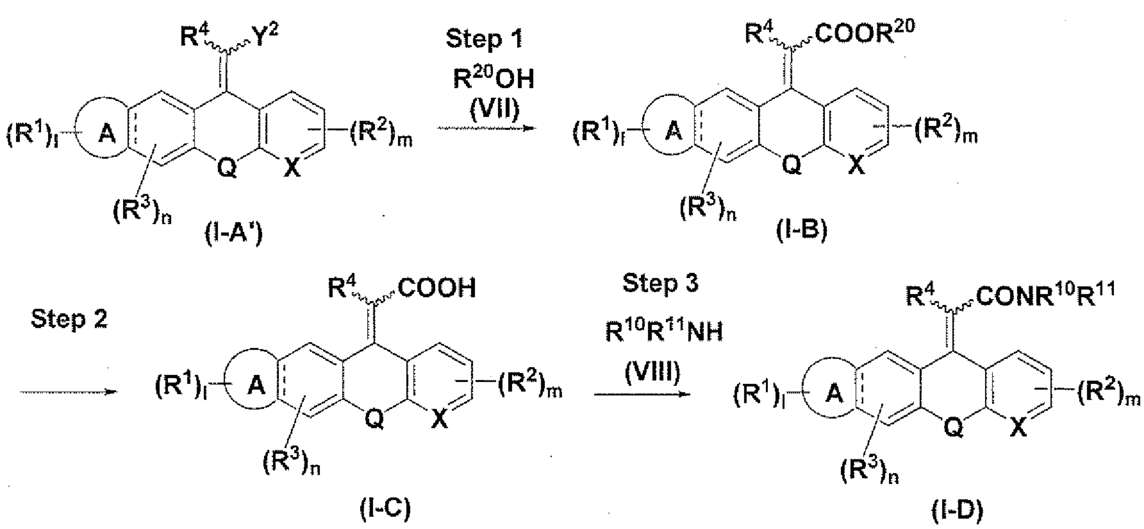

(I-A') (I-B) (I-C) (I-D)

(In the formula, X, $Y^2$, Q, $R^1$, $R^2$, $R^3$, $R^4$, $R^{10}$, $R^{11}$, l, m, and n have the same meanings as defined above, respectively;

$R^{20}$ represents, among the meanings defined as the above $R^5$, optionally substituted lower alkyl moieties of optionally substituted lower alkoxycarbonyl; and

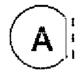

has the same meaning as defined above.)

Step 1

Compound (I-B) can be produced by reacting Compound (I-A') with 1 equivalent to a large excess amount of Compound (VII) , under a carbon monoxide atmosphere, in the absence of solvent or in a solvent, in the presence of 0.001 to 1 equivalent of a transition metal catalyst, at a temperature between -50˚C and 200˚C for 5 minutes to 100 hours. In this case, 0.01 to 30 equivalents of a suitable additive may be added to accelerate the reaction.
Examples of the solvent include dichloromethane, acetonitrile, toluene, ethyl acetate, THF, 1,4-dioxane, DMF, DMSO, NMP, water, and the like. These solvents may be used alone or as a mixture thereof.
Examples of the transition metal catalyst include a palladium catalyst, such as palladium acetate, tetrakis (triphenylphosphine)palladium, palladium chloride, palladium bromide, bis(triphenylphosphine)palladium chloride, bis (acetonitrile)palladium dichloride, and [1,1'-bis(diphenylphosphino)ferrocene]palladium(II) dichloride dichloromethane complex (1:1)); a nickel catalyst, such as nickel chloride, nickel acetylacetonate, bis(1,5-cyclooctadiene)nickel, and nickel bromide; and the like.
Examples of the additive include triphenylphosphine, tri (o-tolyl) phosphine, 1,1'-bis(diphenylphosphino)ferrocene, 1,2-bis(diphenylphosphino)propane, 2,2'-bis(diphenylphosphino)-1,1'-binaphthyl, 1,2-bis (diphenylphosphino) ethane, silver oxide, copper iodide, lithium chloride, cesium fluoride, triethylamine, diethylamine, sodium hydroxide, potassium hydroxide, sodium carbonate, tripotassium phosphate, and the like. These additives may be used alone or as a mixture thereof.
Compound (I-A') can be obtained according to Step 1 and Step 2 of Production Method 1.
Compound (VII) can be obtained, for example, as a commercial product.

Step 2

Compound (I-C) can be produced by treating Compound (I-B) with 1 equivalent to a large excess amount of a base, in a solvent at a temperature between 0˚C and the boiling point of the solvent used for 5 minutes to 72 hours.
Examples of the base include potassium carbonate, lithium hydroxide, potassium hydroxide, sodium hydroxide, sodium methoxide, and the like.
Examples of the solvent include a solvent which contains water. Examples of said solvent include methanol, ethanol, dichloromethane, chloroform, 1,2-dichloroethane, toluene, ethyl acetate, acetonitrile, diethyl ether, THF, 1,2-dimethoxyethane (DME), dioxane, DMF, DMA, NMP, pyridine, and the like. These solvents may be used as a mixture with water, or as a mixture thereof with water.

Step 3

Compound (I-D) can be produced by

(1) treating Compound (I-C) with preferably 1 equivalent to a large excess amount of a chlorinating agent or a brominating agent in the absence of solvent or in a solvent, preferably in the presence of 0.1 to 10 equivalnts of a suitable additive as necessary, at a temperature between -20˚C and 150˚C for 5 minutes to 72 hours, then
(2) reacting the compound obtained in (1) with preferably 1 to 10 equivalents of Compound (VIII) in the absence of solvent or in a solvent, preferably in the presence of 1 to 10 equivalents of a base as necessary, at a temperature between -20˚C and 150˚C for 5 minutes to 72 hours.

Examples of the chlorinating agent used in (1) include thionyl chloride, oxalyl chloride, phosphorus oxychloride, and the like and examples of the brominating agent include thionyl bromide, phosphorus oxybromide, and the like.
Examples of the additive used in (1) include DMF, pyridine, and the like.
Examples of the solvent used in (1) include dichloromethane, chloroform, 1,2-dichloroethane, toluene, ethyl acetate, acetonitrile, diethyl ether, THF, DME, 1,4-dioxane, DMF, DMA, NMP, pyridine, and the like. These solvents are

used alone or as a mixture thereof.

Examples of the base used in (2) include potassium carbonate, potassium hydroxide, sodium hydroxide, potassium tert-butoxide, triethylamine, diisopropylethylamine, N-methylmorpholine, pyridine, DBU, 4-dimethylaminopyridine (DMAP), and the like.

Examples of the solvent used in (2) include dichloromethane, chloroform, 1,2-dichloroethane, toluene, ethyl acetate, acetonitrile, diethyl ether, THF, DME, 1,4-dioxane, DMF, DMA, NMP, pyridine, water, and the like. These solvents are used alone or as a mixture thereof.

In an alternative method, Compound (I-D) can be produced by reacting Compound (I-C) with preferably 0.5 to 5 equivalents of Compound (VIII), in a solvent, preferably in the presence of 1 to 5 equivalents of a condensing agent, preferably in the presence of 1 to 5 equivalents of an additive as necessary, at a temperature between -20°C and the boiling point of the solvent used, for 5 minutes to 72 hours.

Examples of the condensing agent include 1,3-dicyclohexanecarbodiimide (DCC), 1-ethyl-3-(3-dimethylaminopropyl)carbodiimide hydrochloride (EDC), carbonyl diimidazole (CDI), 2-chloro-1-methylpyridinium iodide, and the like.

Examples of the additive include 1-hydroxybenzotriazole monohydrate ($HOBt \cdot H_2O$), and the like.

Examples of the solvent include dichloromethane, chloroform, 1,2-dichloroethane, toluene, ethyl acetate, acetonitrile, diethyl ether, THF, DME, 1,4-dioxane, DMF, DMA, NMP, pyridine, water, and the like. These solvents are used alone or as a mixture thereof.

Compound (VIII) can be obtained as a commercial product or according to known methods.

Production Method 3

Among Compounds (I), Compound (I-Aa) in which Q is $-CH_2-O-$ and $R^5$ is a hydrogen atom, cyano, optionally substituted lower alkyl, optionally substituted lower alkenyl, optionally substituted lower alkynyl, optionally substituted cycloalkyl, optionally substituted aryl, or an optionally substituted aromatic heterocyclic group can be produced, for example, according to the following steps.

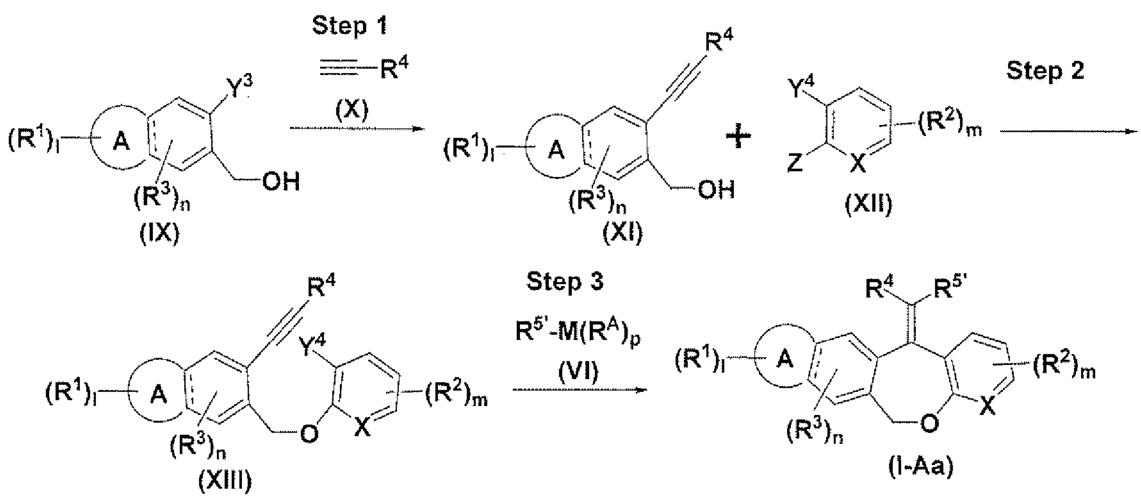


(In the formula, Z represents a fluorine atom, a chlorine atom, a bromine atom, an iodine atom, or a hydroxyl group; M, X, $R^A$, $R^1$, $R^2$, $R^3$, $R^4$, $R^5$, l, m, n, and p have the same meanings as defined above, respectively; $Y^3$ and $Y^4$ each represent a chlorine atom, a bromine atom, or an iodine atom; and

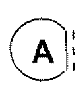

has the same meaning as defined above.)

Step 1

Compound (IX) can be obtained according to known methods [for example, Org. Synth., Coll. vol., 5, 450 (1973) or Synth. Comnun., 27, 1199 (1997)].

Compound (XI) can be obtained by subjecting Compound (IX) and Compound (X) to the Sonogashira reaction [for example, Jikken Kagaku Kouza, 5th edition, No. 13, Synthesis of organic compounds I, Hydrocarbons and halides, p. 298, Maruzen (2005)].

Compound (X) can be obtained as a commercial product, or according to known methods [for example, Jikken Kagaku Kouza, 5th edition, No. 13, Synthesis of organic compounds I, Hydrocarbons and halides, p. 283, Maruzen (2005)].

Step 2

Compound (XIII) can be obtained by subjecting Compound (XI) and Compound (XII) to, for example, the Williamson ether synthesis or the Mitsunobu reaction [for example, Jikken Kagaku Kouza, 5th edition, No. 14, Synthesis of organic compounds II, Alcohols and amines, p. 239, Maruzen (2005)].

Compound (XII) can be obtained as a commercial product, or according to known methods [for example, Jikken Kagaku Kouza, 5th edition, No. 13, Synthesis of organic compounds I, Hydrocarbons and halides, p. 341, Maruzen (2005)].

Step 3

Compound (I-Aa) can be produced by reacting Compound (XIII) with 1 to 30 equivalents of Compound (VI), in a solvent, in the presence of 0.001 to 1 equivalent of a transition metal catalyst at a temperature between -50˚C and 200˚C for 5 minutes to 100 hours. In this case, 0.01 to 30 equivalents of a suitable additive may be added to accelerate the reaction.

Examples of the solvent include methanol, ethanol, dichloromethane, acetonitrile, toluene, ethyl acetate, THF, 1,4-dioxane, DMF, NMP, water, and the like. These solvents may be used alone or as a mixture thereof.

Examples of the transition metal catalyst include a palladium catalyst, such as palladium acetate, tetrakis (triphenylphosphine)palladium, palladium chloride, palladium bromide, bis(triphenylphosphine)palladium chloride, bis (acetonitrile)palladium dichloride, and [1,1'-bis(diphenylphosphino)ferrocene]palladium(II) dichloride dichloromethane complex (1:1); a nickel catalyst, such as nickel chloride, nickel acetylacetonate, bis(1,5-cyclooctadiene)nickel, and nickel bromide; and the like.

Examples of the additive include triphenylphosphine, tri(o-tolyly phosphine, 1,1'-bis(diphenylphosphino)ferrocene, 1,2-bis(diphenylphosphino)propane, 2,2'-bis(diphenylphosphino)-1,1'-binaphthyl, 1,2-bis(diphenylphosphino) ethane, silver oxide, copper iodide, lithium chloride, cesium fluoride, triethylamine, diethylamine, sodium hydroxide, potassium hydroxide, sodium carbonate, tripotassium phosphate, and the like. These additives may be used alone or as a mixture thereof.

Compound (VI) can be obtained as a commercial product, or according to known methods [for example, Jikken Kagaku Kouza, 5th sedition, No. 18, Synthesis of organic compounds VI, Organic synthesis with the use of metal, p. 97, Maruzen (2005)].

Production Method 4

Among Compounds (I), Compound (I-Ab) in which Q is $-O-CH_2-$ and $R^5$ is a hydrogen atom, cyano, optionally substituted lower alkyl, optionally substituted lower alkenyl, optionally substituted lower alkynyl, optionally substituted cycloalkyl, optionally substituted aryl, or an optionally substituted aromatic heterocyclic group can be produced, for example, according to the following steps.

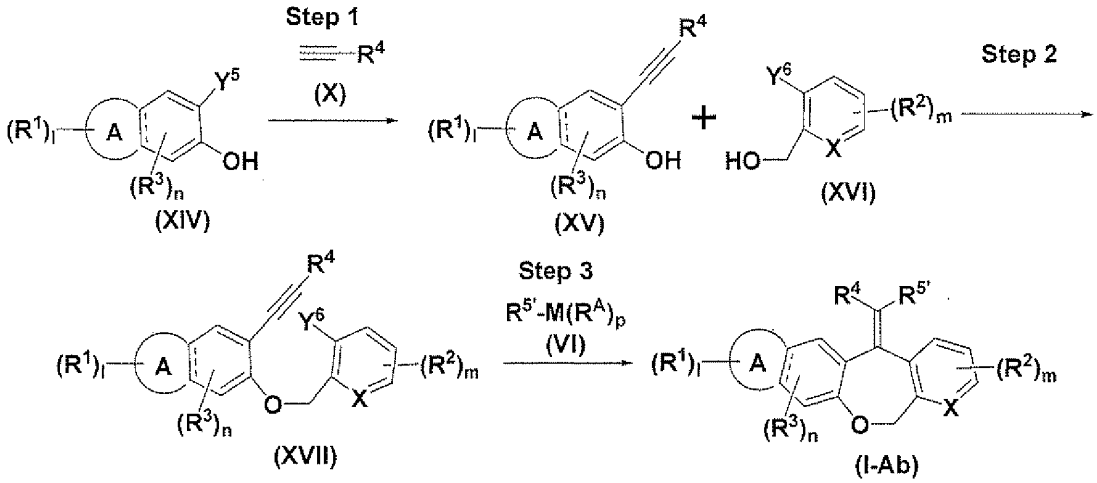


(In the formula, M, X, $R^A$, $R^1$ $R^2$, $R^3$, $R^4$, $R^{5'}$, l, m, n, and p have the same meanings as defined above, respectively; $Y^5$ and $Y^6$ each represent a chlorine atom, a bromine atom, or an iodine atom; and

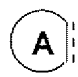

has the same meaning as defined above.)

Step 1

Compound (XIV) can be obtained according to known methods [for example, Org. Synth., Coll. vol., 5, 450 (1973) or Synth. Commun., 27, 1199 (1997)].

Compound (XV) can be produced from Compound (XIV) and Compound (X) in a similar manner as in the Step 1 of Production Method 3.

Compound (X) can be obtained as a commercial product, or according to known methods [for example, Jikken Kagaku Kouza, 5th edition, No. 13, Synthesis of organic compounds I, Hydrocarbons and halides, p. 283, Maruzen (2005)].

Step 2

Compound (XVII) can be produced from Compound (XV) and Compound (XVI) in a similar manner as in the Step 2 of Production Method 3.

Compound (XVI) can be obtained as a commercial product, or according to known methods [for example, Jikken Kagaku Kouza, 5th edition, No. 14, Synthesis of organic compounds II, Alcohols and amines, p. 1, Maruzen (2005)].

Step 3

Compound (VI) can be obtained as a commercial product, or according to known methods [for example, Jikken Kagaku Kouza, 5th edition, No. 18, Synthesis of organic compounds VI, Organic synthesis with the use of metal, p. 97, Maruzen (2005)].

Compound (I-Ab) can be produced from Compound (XVII) and Compound (VI) in a similar manner as in the Step 3 of Production Method 3.

Production Method 5

Among Compounds (I), Compound (I-Ac) in which $R^4$ is a hydrogen atom, cyano, optionally substituted lower alkyl, optionally substituted lower alkenyl, optionally substituted lower alkynyl, optionally substituted cycloalkyl, optionally

substituted aryl, or an optionally substituted aromatic heterocyclic group can be produced, for example, according to the following steps.

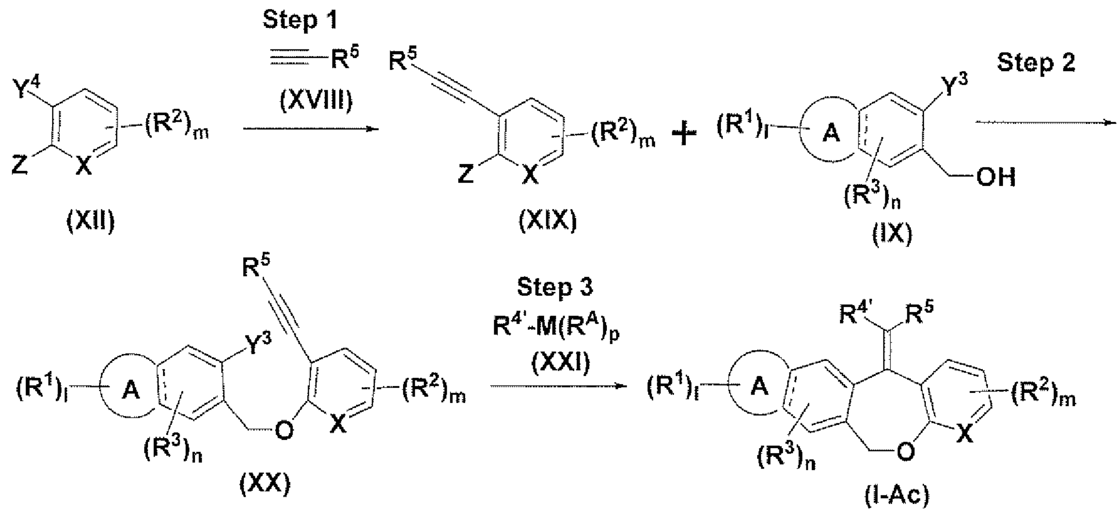


(XII) (XIX) (IX) (XX) (I-Ac)

(In the formula, M, X, $Y^3$, $Y^4$, Z, $R^A$, $R^1$, $R^2$, $R^3$, $R^5$, l, m, n, and p have the same meanings as defined above, respectively; $R^{4'}$ represents, among the meanings defined as the above $R^4$, a hydrogen atom, cyano, optionally substituted lower alkyl, optionally substituted lower alkenyl, optionally substituted lower alkynyl, optionally substituted cycloalkyl, optionally substituted aryl, or an optionally substituted aromatic heterocyclic group; and

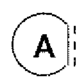

has the same meaning as defined above.)

Step 1

Compound (XIX) can be produced from Compound (XII) and Compound (XVIII) in a similar manner as in the Step 1 of Production Method 3.

Compound (XII) can be obtained as a commercial product, or according to known methods [for example, Jikken Kagaku Kouza, 5th edition, No. 13, Synthesis of organic compounds I, Hydrocarbons and halides, p. 341, Maruzen (2005)].

Compound (XVIII) can be obtained as a commercial product, or according to known methods [for example, Jikken Kagaku Kouza, 5th edition, No. 13, Synthesis of organic compounds I, Hydrocarbons and halides, p. 283, Maruzen (2005)].

Step 2

Compound (XX) can be produced from Compound (XIX) and Compound (IX) in a similar manner as in the Step 2 of Production Method 3.

Compound (IX) can be obtained according to known methods (for example, Org. Synth., Coll. vol., 5, 450 (1973) or Synth. Commun., 27, 1199 (1997)).

Step 3

Compound (I-Ac) can be produced from Compound (XX) and Compound (XXI) in a similar manner as in the Step 3 of Production Method 3.

Compound (XXI) can be obtained as a commercial product, or according to known methods (for example, Jikken Kagaku Kouza, 5th edition, No. 18, Synthesis of organic compounds VI, Organic synthesis with the use of metal,

p. 97, Maruzen (2005)).

Production Method 6

Among Compounds (I), Compound (I-Ad) in which $R^4$ is a hydrogen atom, cyano, optionally substituted lower alkyl, optionally substituted lower alkenyl, optionally substituted lower alkynyl, optionally substituted cycloalkyl, optionally substituted aryl, or an optionally substituted aromatic heterocyclic group can be produced, for example, according to the following steps.

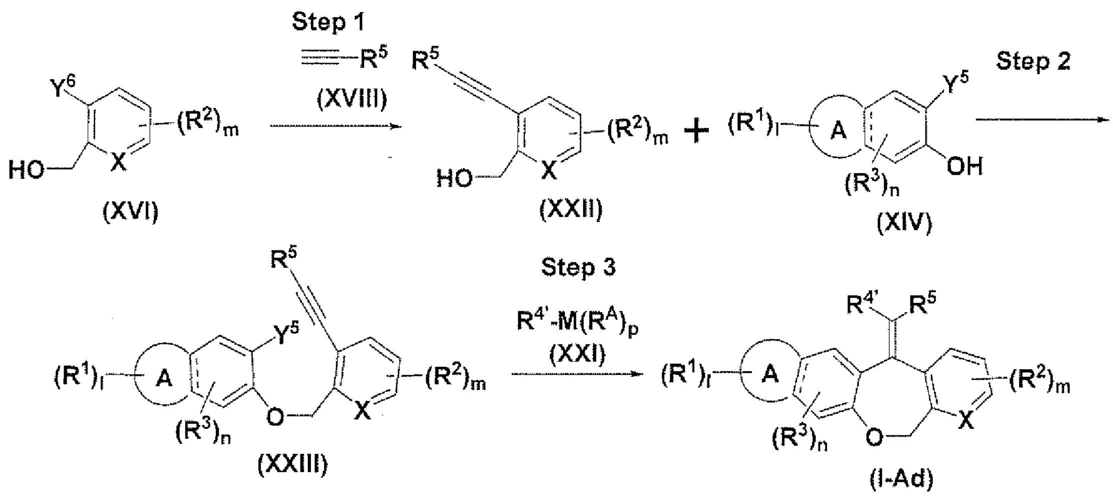

(XVI) (XXII) (XIV) (XXIII) (I-Ad)

(In the formula, M, X, $Y^5$, $Y^6$, $R^A$, $R^1$, $R^2$, $R^3$, $R^{4'}$, $R^5$, l, m, n, and p have the same meanings as defined above, respectively; and

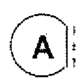

has the same meaning as defined above.)

Step 1

Compound (XXII) can be produced from Compound (XVI) and Compound (XVIII) in a similar manner as in the Step 1 of Production Method 3.

Step 2

Compound (XXIII) can be produced from Compound (XXII) and Compound (XIV) in a similar manner as in the Step 2 of Production Method 3.

Step 3

Compound (I-Ad) can be produced from Compound (XXIII) and Compound (XXI) in the same manner as in the Step 3 of Production Method 3.

Production Method 7

Among Compounds (I), Compound (I-Ae) in which $R^5$ is optionally substituted lower alkenyl, optionally substituted aryl, or an optionally substituted aromatic heterocyclic group can be produced, for example, according to the following steps.

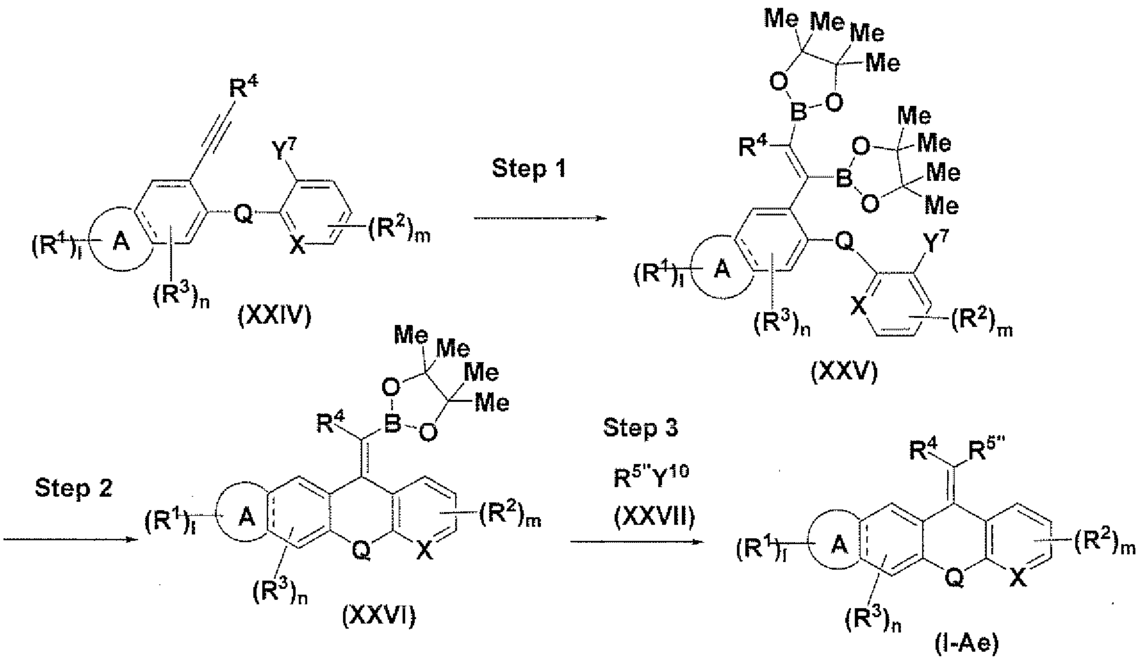


(XXIV) (XXV) (XXVI) (I-Ae)

(In the formula, Q, X, $R^1$, $R^2$, $R^3$, $R^4$, l, m, and n have the same meanings as defined above, respectively;
$Y^7$ and $Y^{10}$ each represent a chlorine atom, a bromine atom, or an iodine atom;
$R^{5''}$ represents, among the meanings defined as the above $R^5$, optionally substituted lower alkenyl, optionally substituted aryl, or an optionally substituted aromatic heterocyclic group; Me represents methyl; and

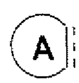

has the same meaning as defined above.)

Step 1

Compound (XXV) can be produced by reacting Compound (XXIV) with 1 to 30 equivalents of bis (pinacolato) diboron, in a solvent, in the presence off 0.001 to 1 equivalent of a transition metal catalyst, at a temperature between -50˚C and 200˚C for 5 minutes to 100 hours.

Examples of the solvent include methanol, ethanol, dichloromethane, acetonitrile, toluene, ethyl acetate, THF, 1,4-dioxane, DMF, NMP, water, and the like. These solvents may be used alone or as a mixture thereof.

Examples of the transition metal catalyst include a platinum catalyst, such as tetrakis(triphenylphosphine)platinum, bis(1,5-cyclooctadiene)platinum, and chloro(1,5-cyclooctadiene)platinum; and the like.

Compound (XXIV) can be produced in a similar manner as in the Step 2 of Production Method 3 or the Step 2 of Production Method 4.

Among Compounds (XXIV), a compound in which $R^1$ is bound to a nitrogen atom in

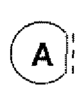

can be produced, for example, according to the following Step A or Step B.

Step A

Among Compounds (XXIV), Compound (XXIV-a) in which

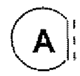

is

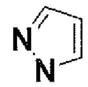

and $R^1$ is optionally substituted lower alkyl, optionally substituted cycloalkyl, or an optionally substituted aliphatic heterocyclic group can be produced, for example, according to the following steps.

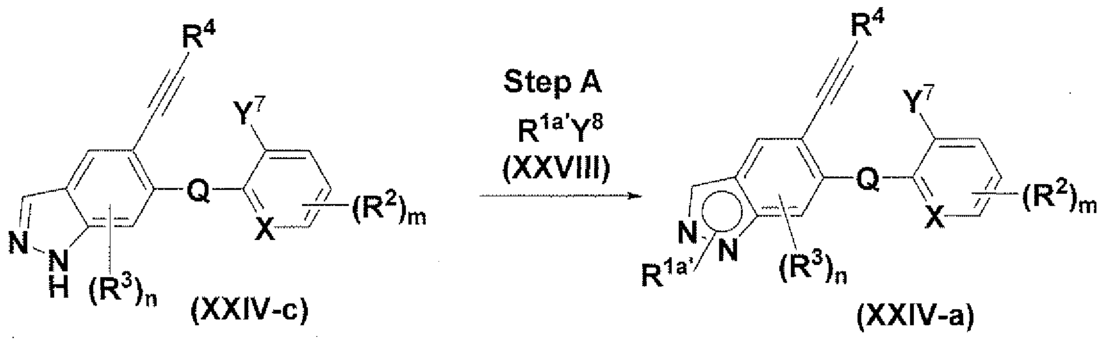


(XXIV-c) (XXIV-a)

(In the formula, $R^{1a'}$ represents, among the meanings defined as the above $R^1$, optionally substituted lower alkyl, optionally substituted cycloalkyl, or an optionally substituted aliphatic heterocyclic group;
Q, X, $R^2$, $R^3$, $R^4$, $Y^7$, m, and n have the same meanings as defined above, respectively; and
$Y^8$ represents a chlorine atom, a bromine atom, an iodine atom, methanesulfonyloxy, p-toluenesulfonyloxy, or trifluoromethanesulfonyloxy.)

Compound (XXIV-a) can be produced by reacting Compound (XXIV-c) with 1 to 30 equivalents of Compound (XXVIII), in the absence of solvent or in a solvent, in the presence of 1 to 30 equivalents of a base, at a temperature between -50°C and 200°C for 5 minutes to 100 hours.

Examples of the solvent include toluene, acetonitrile, THF, 1,4-dioxane, DMF, DMA, NMR, water, and the like. These solvents may be used alone or as a mixture thereof.

Examples of the base include sodium hydride, sodium hydroxide, potassium carbonate, potassium tert-butoxide, sodium tert-butoxide, and the like. These bases may be used alone or as a mixture thereof.

Compound (XXIV-c) can be obtained in a similar manner as in the Step 2 of Production Method 3 or the Step 2 of Production Method 4.

Compound (XXVIII) can be obtained, for example, as a commercial product.

Step B

Among Compounds (XXIV), Compound (XXIV-b) in which

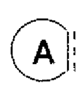

is

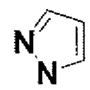

and $R^{1b'}$ is optionally substituted aryl, an optionally substituted aromatic heterocyclic group, or an optionally substituted lower alkenyl can be produced, for example, according to the following steps.

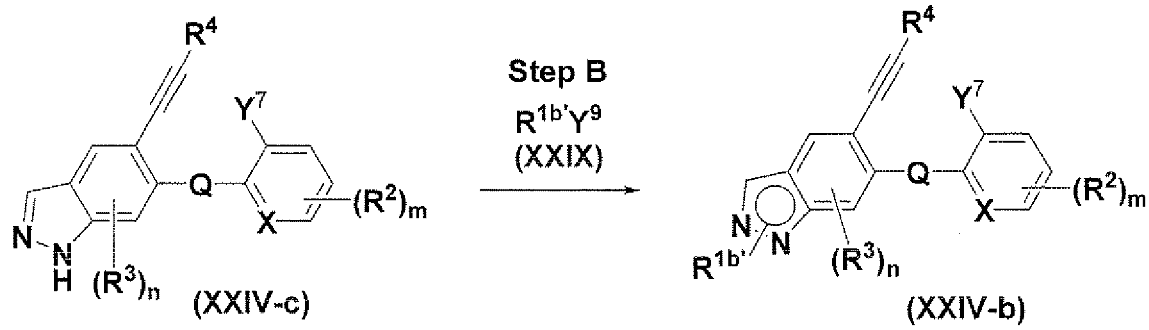

(XXIV-c) (XXIV-b)

(In the formula, $R^{1b'}$ represents, among the meanings defined as the above $R^1$, optionally substituted aryl, an optionally substituted aromatic heterocyclic group, or optionally substituted lower alkenyl;
Q, X, $Y^7$, $R^2$, $R^3$, $R^4$, m, and n have the same meanings as defined above, respectively; and
$Y^9$ represents a chlorine atom, a bromine atom, an iodine atom, or trifluoromethanesulfonyloxy.)

Compound (XXIV-b) can be produced by reacting Compound (XXIV-c) with 1 to 30 equivalents of Compound (XXIX), in a solvent, in the presence of 0.001 to 1 equivalent of a copper catalyst, at a temperature between -50˚C and 200˚C for 5 minutes to 100 hours. In this case, 0.01 to 30 equivalents of a suitable additive may be added to accelerate the reaction.

Examples of the solvent include methanol, ethanol, dichloromethane, acetonitrile, toluene, ethyl acetate, THF, 1, 4-dioxane, DMF, NMP, water, and the like. These solvents may be used alone or as a mixture thereof.

Examples of the copper catalyst include copper (0), copper(I) iodide, copper(II) iodide, copper(II) acetate, copper(II) oxide, copper(I) chloride, and the like.

Examples of the additive include ethylenediamine, trans-1,2-cyclohexanediamine, phenanthroline, potassium carbonate, cesium carbonate, lithium chloride, potassium chloride, potassium tert-butoxide, sodium tert-butoxide, triethylamine, potassium acetate, sodium ethoxide, sodium carbonate, sodium hydroxide, tripotassium phosphate, and the like. These additives may be used alone or as a mixture thereof.

Compound (XXIX) can be obtained, for example, as a commercial product.

Compound (XXIV-c) can be obtained in a similar manner as in the Step 2 of Production Method 3 or the Step 2 of Production Method 4.

Step 2

Compound (XXVI) can be produced by reacting Compound (XXV), in a solvent, in the presence of 0.001 to 1 equivalent of a transition metal catalyst, at a temperature between -50˚C and 200˚C for 5 minutes to 100 hours. In this case, 0.01 to 30 equivalents of a suitable additive may be added to accelerate the reaction.

Examples of the solvent include methanol, ethanol, dichloromethane, acetonitrile, toluene, ethyl acetate, THF, 1, 4-dioxane, DMF, NMP, water, and the like. These solvents may be used alone or as a mixture thereof.

Examples of the transition metal catalyst include a palladium catalyst, such as palladium acetate, tetrakis (triphenylphosphine)palladium, palladium chloride, palladium bromide, bis(triphenylphosphine)palladium chloride, bis (acetonitrile)palladium dichloride, and [1,1'-bis(diphenylphosphino)ferrocene]palladium(II) dichloride dichloromethane complex (1:1); a nickel catalyst, such as nickel chloride, nickel acetylacetonate, bis(1,5-cyclooctadiene)nickel, and nickel bromide; and the like.

Examples of the additive include triphenylphosphine, tri(o-tolyl) phosphine, 1,1'-bis(diphenylphosphino)ferrocene, 1,2-bis(diphenylphosphino)propane, 2,2'-bis(diphenylphosphino)-1,1'-binaphthyl, 1,2-bis(diphenylphosphino) ethane, silver oxide, copper iodide, lithium chloride, cesium fluoride, triethylamine, diethylamine, sodium hydroxide, potassium hydroxide, sodium carbonate, tripotassium phosphate, and the like. These additives may be used alone or as a mixture thereof.

Step 3

Compound (XXVII) can be obtained, for example, as a commercial product.

Compound (I-Ac) can be produced by reacting Compound (XXVI) with 1 to 30 equivalents of Compound (XXVII), in a solvent, in the presence of 0.001 to 1 equivalent of a transition metal catalyst, at a temperature between -50˚C and 200˚C for 5 minutes to 100 hours. In this case, 0.01 to 30 equivalents of a suitable additive may be added to accelerate the reaction.

Examples of the solvent include methanol, ethanol, dichloromethane, acetonitrile, toluene, ethyl acetate, THF, 1,4-dioxane, DMF, NMP, water, and the like. These solvents may be used alone or as a mixture thereof.

Examples of the transition metal catalyst include a palladium catalyst, such as palladium acetate, tetrakis (triphenylphosphine)palladium, palladium chloride, palladium bromide, bis(triphenylphosphine)palladium chloride, bis (acetonitrile)palladium dichloride, and [1,1'-bis(diphenylphosphino)ferrocene] palladium(II) dichloride dichloromethane complex (1:1); a nickel catalyst, such as nickel chloride, nickel acetylacetonate, bis(1,5-cyclooctadiene)nickel, and nickel bromide; and the like.

Examples of the additive include triphenylphosphine, tri (o-tolyl) phosphine, 1,1'-bis(diphenylphosphino) ferrocene, 1,2-bis(diphenylphosphino)propane, 2,2'-bis(diphenylphosphino)-1,1'-binaphthyl, 1,2-bis (diphenylphosphino) ethane, silver oxide, copper iodide, lithium chloride, cesium fluoride, triethylamine, diethylamine, sodium hydroxide, potassium hydroxide, sodium carbonate, tripotassium phosphate, and the like. These additives may be used alone or as a mixture thereof.

Production Method 8

Among Compounds (I), Compound (I-Ba) in which $R^5$ is optionally substituted lower alkoxycarbonyl can be produced, for example, according to the following steps.

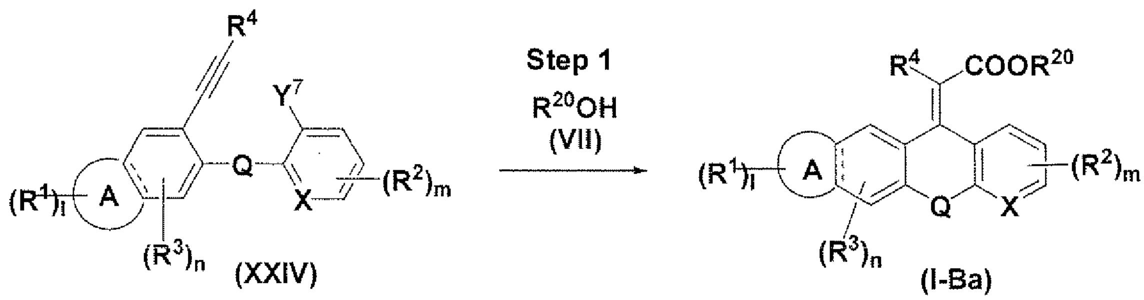


(XXIV) (I-Ba)

(In the formula, Q, X, $Y^7$, $R^1$, $R^2$, $R^3$, $R^4$, $R^{20}$, 1, m, and n have the same meanings as defined above, respectively; and

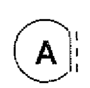

has the same meaning as defined above.)

Step 1

Compound (I-Ba) can be produced by reacting Compound (XXIV) with 1 equivalent to a large excess amount of Compound (VII) under a carbon monoxide atmosphere, in the absence of solvent or in a solvent, in the presence of 0. 001 to 1 equivalent of a transition metal catalyst, at a temperature between -50˚C and 200˚C for 5 minutes to 100 hours. In this case, 0.01 to 30 equivalents of a suitable additive may be added to accelerate the reaction.

Examples of the solvent include dichloromethane, acetonitrile, toluene, ethyl acetate, THF, 1, 4-dioxane, DMF, DMSO, NMP, water, and the like. These solvents may be used alone or as a mixture thereof.

Examples of the transition metal catalyst include a palladium catalyst, such as palladium acetate, tetrakis (triphenylphosphine)palladium, palladium chloride, palladium bromide, bis(triphenylphosphine)palladium chloride, bis (acetonitrile)palladium dichloride, and [1,1'-bis(diphenylphosphino)ferrocene] palladium(II) dichloride dichloromethane complex (1:1); a nickel catalyst, such as nickel chloride, nickel acetylacetonate, bis(1,5-cyclooctadiene)nickel, and nickel

bromide; and the like.

Examples of the additive include triphenylphosphine, tri (o-tolyl) phosphine, 1,1'-bis(diphenylphosphino)ferrocene, 1,2-bis(diphenylphosphino)propane, 2,2'-bis(diphenylphosphino)-1,1'-binaphthyl, 1,2-bis (diphenylphosphino) ethane, silver oxide, copper iodide, lithium chloride, cesium fluoride, triethylamine, diethylamine, sodium hydroxide, potassium hydroxide, sodium carbonate, tripotassium phosphate, and the like. These additives may be used alone or as a mixture thereof.

The intermediates and the desired compounds in the above-mentioned respective Production Methods can be isolated and purified through a separation and purification method generally employed in organic synthetic chemistry, for example, filtration, extraction, washing, drying, concentration, recrystallization, various types of chromatography, and the like. The intermediate may be subjected to the subsequent reaction without any particular purification.

Some of Compounds (I) exist as stereoisomers, such as geometric isomers, optical isomers, tautomers, and the like. Including these, all possible isomers and mixtures thereof are included in the present invention.

A salt of Compound (I) can be obtained as follows. When Compound (I) is obtained in the form of a salt, the salt may be simply purified as it is. When Compound (I) is obtained in a free form, the compound may be dissolved or suspended in a suitable solvent, followed by addition of an acid or a base, and then, the resulting salt may be isolated and purified.

Compounds (I) and pharmaceutically acceptable salts thereof may exist in the form of adducts with water or any of various solvents in some cases, and these adducts are also included in the present invention.

Specific examples of Compounds (I) obtained according to the present invention are shown in Tables 1 to 11. However, the compounds of the present invention are not limited thereto.

In the Tables, Me represents methyl, Et represents ethyl, iPr represents isopropyl, n-Pr represents n-propyl, and c-Pentyl represents cyclopentyl.

Table 1

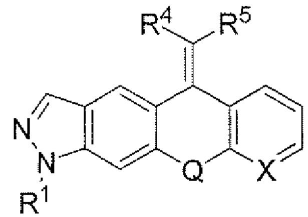

| Compound No. | Q | X | $R^1$ | $R^4$ | $R^5$ |
| --- | --- | --- | --- | --- | --- |
| 1 | $CH_2O$ | CH | Me | 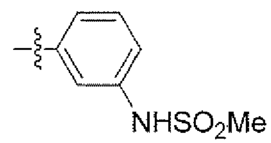 | Et |
| 2 | $CH_2O$ | CH | Me | Et | 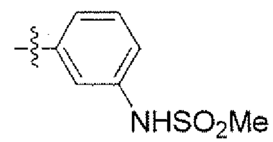 |
| 3 | $CH_2O$ | CH | 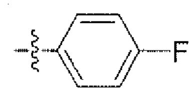 | 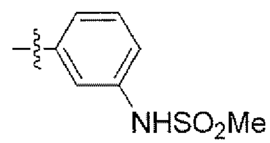 | Et |
| 4 | $CH_2O$ | CH | 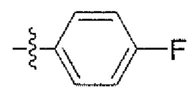 | Et | 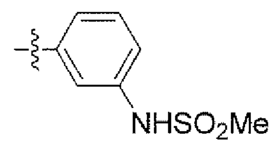 |
| 5 | $CH_2O$ | CH | 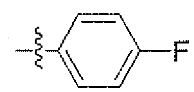 | 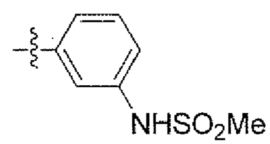 | Me |

(continued)

| Compound No. | Q | X | $R^1$ | $R^4$ | $R^5$ |
|---|---|---|---|---|---|
| 6 | $CH_2O$ | CH | 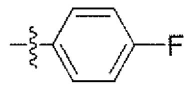 | Me | 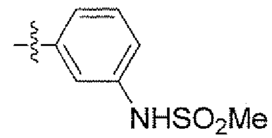 |
| 7 | $CH_2O$ | CH | iPr | 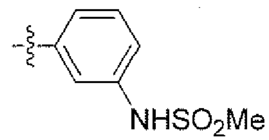 | Et |
| 8 | $CH_2O$ | CH | iPr | Et | 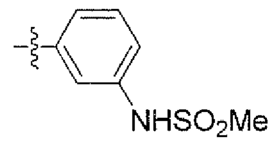 |
| 9 | $CH_2O$ | CH | c-Pentyl | 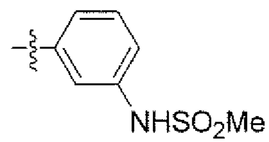 | Et |
| 10 | $CH_2O$ | CH | c-Pentyl | Et | 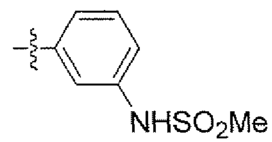 |

Table 2

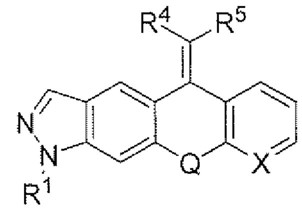

| Compound No. | Q | X | $R^1$ | $R^4$ | $R^5$ |
|---|---|---|---|---|---|
| 11 | $CH_2O$ | CH | 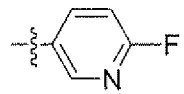 | 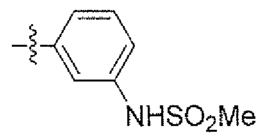 | Et |
| 12 | $CH_2O$ | CH | 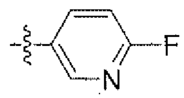 | Et | 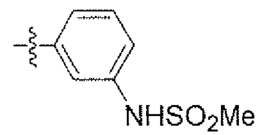 |
| 13 | $OCH_2$ | N | 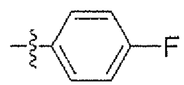 | 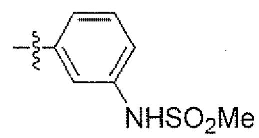 | Et |
| 14 | $OCH_2$ | N | 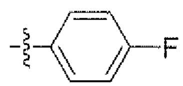 | Et | 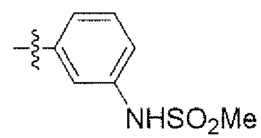 |
| 15 | $OCH_2$ | CH | 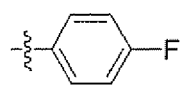 | 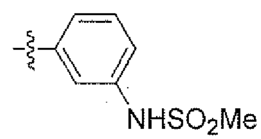 | Et |

(continued)

| Compound No. | Q | X | $R^1$ | $R^4$ | $R^5$ |
|---|---|---|---|---|---|
| 16 | $OCH_2$ | CH | 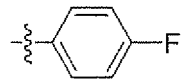 | Et | 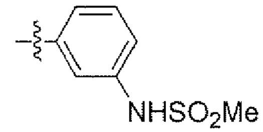 |
| 17 | $CH_2O$ | CH | 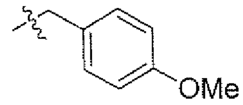 | Et | 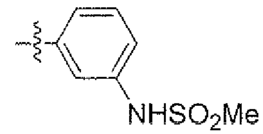 |
| 18 | $CH_2O$ | CH | H | Et | 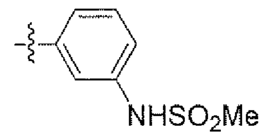 |
| 19 | $CH_2O$ | N | iPr | n-Pr | 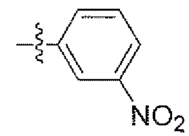 |
| 20 | $CH_2O$ | N | iPr | n-Pr | 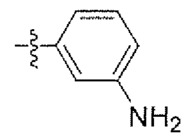 |

Table 3

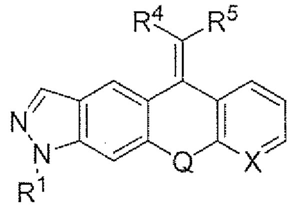

| Compound No. | Q | x | $R^1$ | $R^4$ | $R^5$ |
|---|---|---|---|---|---|
| 21 | $CH_2O$ | N | iPr | n-Pr | 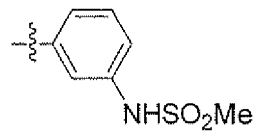 |
| 22 | $OCH_2$ | CH | iPr | Me | 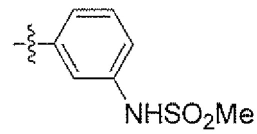 |
| 23 | $OCH_2$ | CH | iPr | Et | 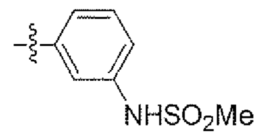 |
| 24 | $CH_2O$ | N | 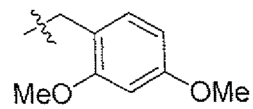 | Et | 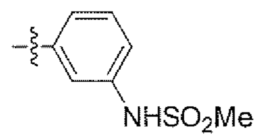 |
| 25 | $CH_2O$ | N | iPr | Et | 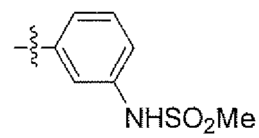 |
| 26 | $CH_2O$ | N | Et | Et | 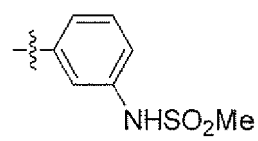 |
| 27 | $CH_2O$ | N | 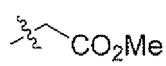 | Et | 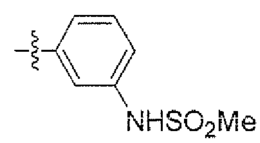 |
| 28 | $CH_2O$ | N | 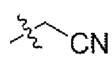 | Et | 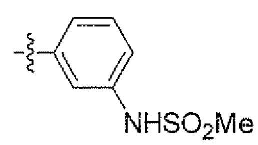 |
| 29 | $CH_2O$ | N | 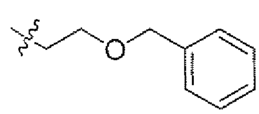 | Et | 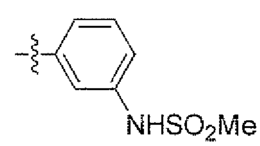 |

(continued)

| Compound No. | Q | x | $R^1$ | $R^4$ | $R^5$ |
|---|---|---|---|---|---|
| 30 | $CH_2O$ | N | 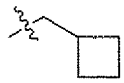 | Et | 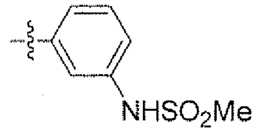 |

Table 4

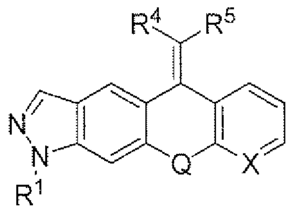

| Compound No. | Q | x | $R^1$ | $R^4$ | $R^5$ |
|---|---|---|---|---|---|
| 31 | $CH_2O$ | N | 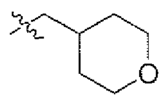 | Et | 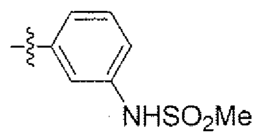 |
| 32 | $CH_2O$ | N | 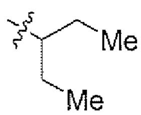 | Et | 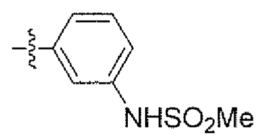 |
| 33 | $CH_2O$ | N | H | Et | 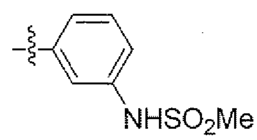 |
| 34 | $CH_2O$ | N | 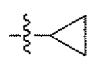 | Et | 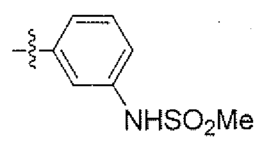 |
| 35 | $CH_2O$ | N | 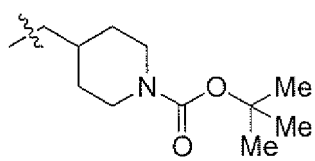 | Et | 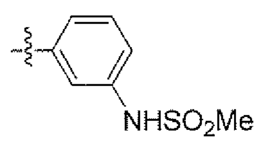 |
| 36 | $CH_2O$ | N | 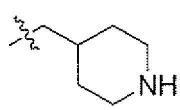 | Et | 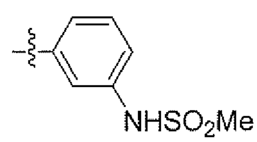 |
| 37 | $CH_2O$ | N | 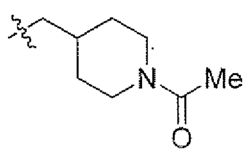 | Et | 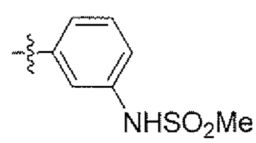 |

(continued)

| Compound No. | Q | x | $R^1$ | $R^4$ | $R^5$ |
|---|---|---|---|---|---|
| 38 | $CH_2O$ | N | 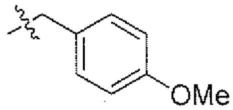 | Et | 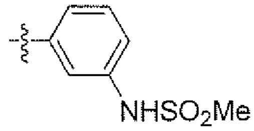 |
| 39 | $CH_2O$ | N | 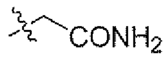 | Et | 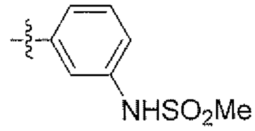 |
| 40 | $CH_2O$ | N | 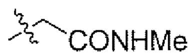 | Et | 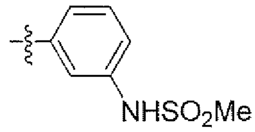 |

Table 5

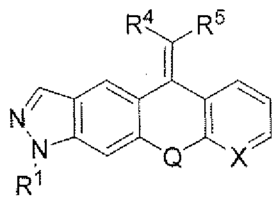

| Compound No. | Q | x | $R^1$ | $R^4$ | $R^5$ |
|---|---|---|---|---|---|
| 41 | $CH_2O$ | N | 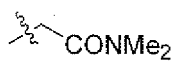 | Et | 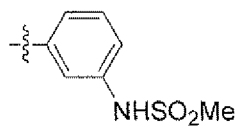 |
| 42 | $CH_2O$ | N | 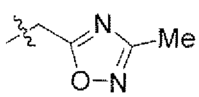 | Et | 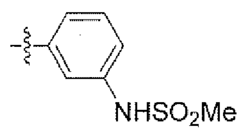 |
| 43 | $CH_2O$ | N | 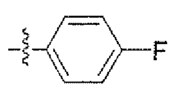 | Et | 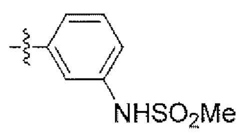 |
| 44 | $CH_2O$ | N | 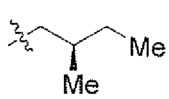 | Et | 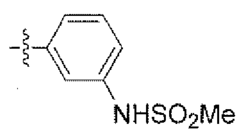 |
| 45 | $CH_2O$ | N | 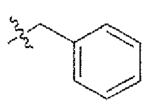 | Et | 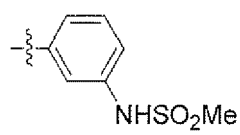 |
| 46 | $CH_2O$ | N | 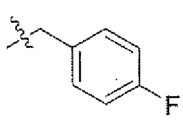 | Et | 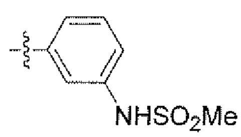 |

(continued)

| Compound No. | Q | x | $R^1$ | $R^4$ | $R^5$ |
|---|---|---|---|---|---|
| 47 | $CH_2O$ | N | 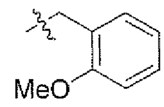 | Et | 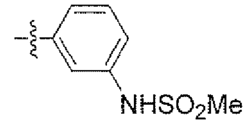 |
| 48 | $CH_2O$ | N | 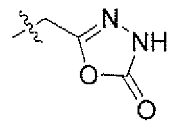 | Et | 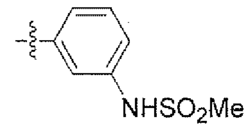 |
| 49 | $CH_2O$ | N | 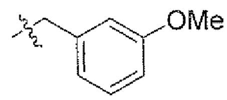 | Et | 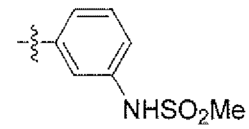 |
| 50 | $CH_2O$ | N | 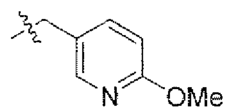 | Et | 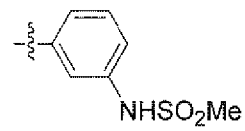 |
| 51 | $CH_2O$ | N | 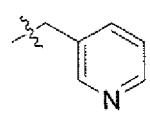 | Et | 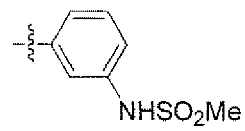 |

Table 6

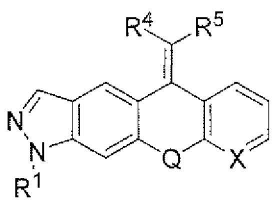

| Compound No. | Q | X | $R^1$ | $R^4$ | $R^5$ |
|---|---|---|---|---|---|
| 52 | $CH_2O$ | N | 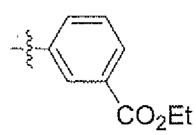 | Et | 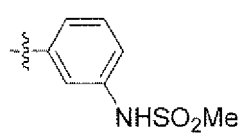 |
| 53 | $CH_2O$ | N | 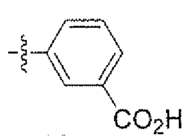 | Et | 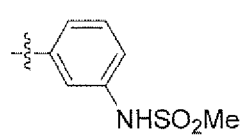 |
| 54 | $CH_2O$ | N | 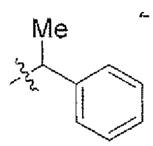 | Et | 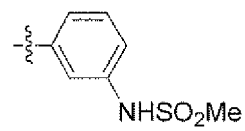 |
| 55 | $CH_2O$ | N | 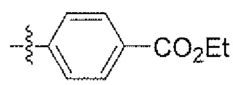 | Et | 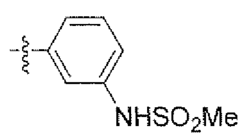 |

(continued)

| Compound No. | Q | X | $R^1$ | $R^4$ | $R^5$ |
|---|---|---|---|---|---|
| 56 | $CH_2O$ | N | 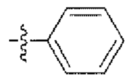 | Et | 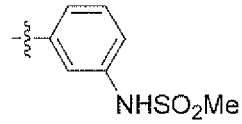 |
| 57 | $CH_2O$ | N | 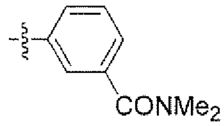 | Et | 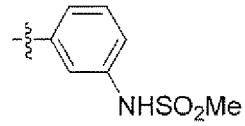 |
| 58 | $CH_2O$ | N | 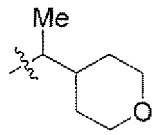 | Et | 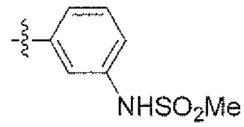 |
| 59 | $CH_2O$ | N | 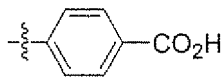 | Et | 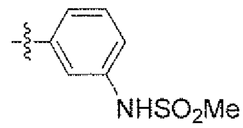 |
| 60 | $CH_2O$ | N | 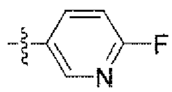 | Et | 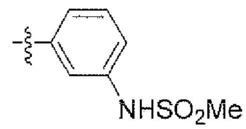 |
| 61 | $CH_2O$ | N | 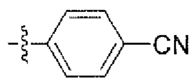 | Et | 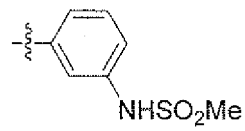 |
| 62 | $CH_2O$ | N | 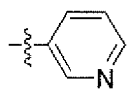 | Et | 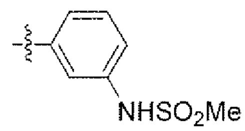 |

Table 7

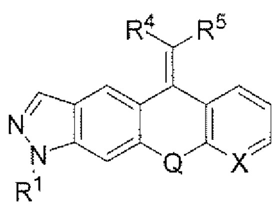

| Compound No. | Q | X | $R^1$ | $R^4$ | $R^5$ |
|---|---|---|---|---|---|
| 63 | $CH_2O$ | N | 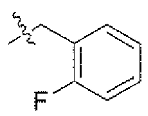 | Et | 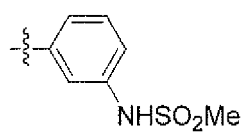 |
| 64 | $CH_2O$ | N | 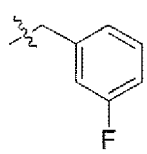 | Et | 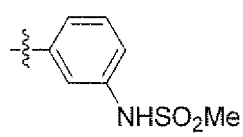 |

(continued)

| Compound No. | Q | X | $R^1$ | $R^4$ | $R^5$ |
|---|---|---|---|---|---|
| 65 | $CH_2O$ | N | 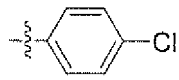 | Et | 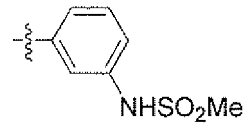 |
| 66 | $CH_2O$ | N | 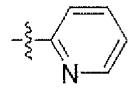 | Et | 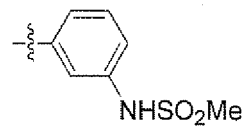 |
| 67 | $CH_2O$ | N | 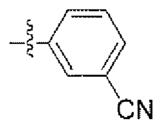 | Et | 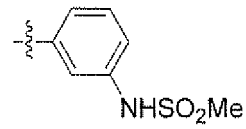 |
| 68 | $CH_2O$ | N | 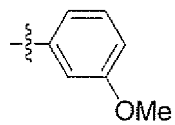 | Et | 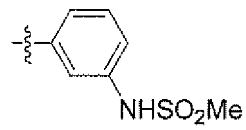 |
| 69 | $CH_2O$ | N | iPr | H | 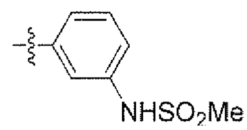 |
| 70 | $CH_2O$ | N | iPr | Me | 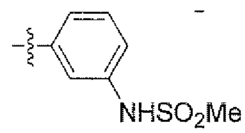 |
| 71 | $CH_2O$ | N | iPr | 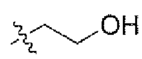 | 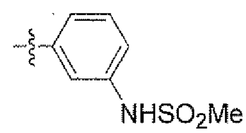 |
| 72 | $OCH_2$ | N | iPr | 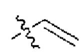 | 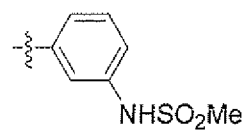 |
| 73 | $OCH_2$ | N | iPr | Et | 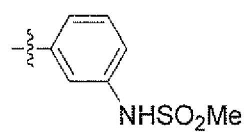 |

Table 8

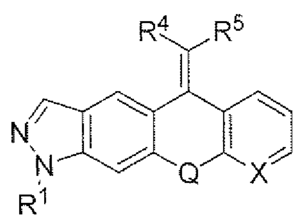

(continued)

| Compound No. | Q | x | $R^1$ | $R^4$ | $R^5$ |
|---|---|---|---|---|---|
| 74 | $CH_2O$ | N | iPr | Et | 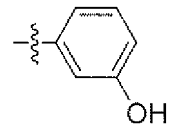 |
| 75 | $CH_2O$ | N | iPr | Et | 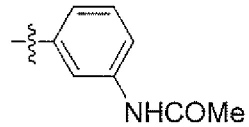 |
| 76 | $CH_2O$ | N | iPr | Et | 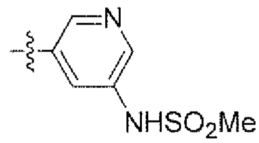 |
| 77 | $CH_2O$ | N | iPr | Et | 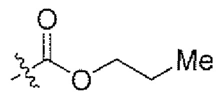 |
| 78 | $CH_2O$ | N | iPr | Et | 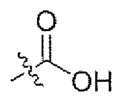 |
| 79 | $CH_2O$ | N | iPr | Et | 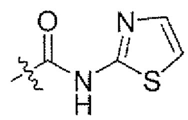 |
| 80 | $CH_2O$ | N | iPr | Et | 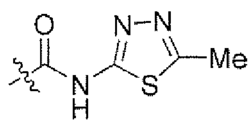 |
| 81 | $CH_2O$ | N | iPr | Et | 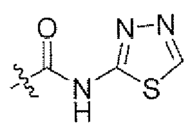 |
| 82 | $CH_2O$ | N | iPr | Et | 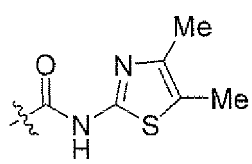 |
| 83 | $CH_2O$ | N | iPr | Et | 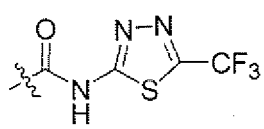 |

Table 9

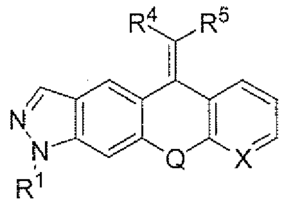

| Compound No. | Q | X | $R^1$ | $R^4$ | $R^5$ |
|---|---|---|---|---|---|
| 84 | $CH_2O$ | N | iPr | Et | 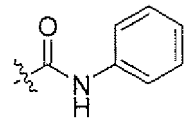 |
| 85 | $CH_2O$ | N | iPr | Et | 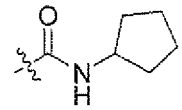 |
| 86 | $CH_2O$ | N | iPr | Et | 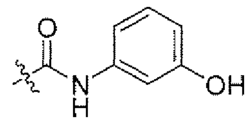 |
| 87 | $CH_2O$ | N | iPr | Et | 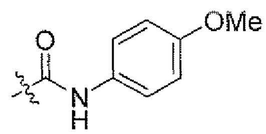 |
| 88 | $CH_2O$ | N | iPr | Et | 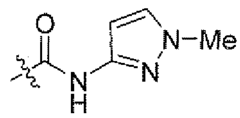 |
| 89 | $CH_2O$ | CH | 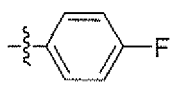 | Et | 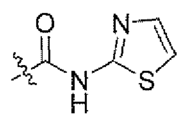 |
| 90 | $CH_2O$ | CH | 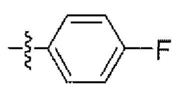 | 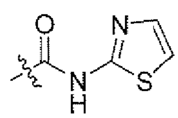 | Et |
| 91 | $CH_2O$ | CH | 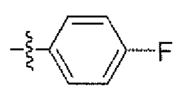 | Et | 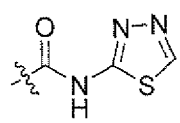 |
| 92 | $CH_2O$ | CH | 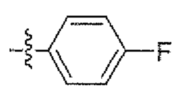 | Et | 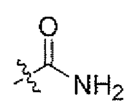 |
| 93 | $OCH_2$ | CH | 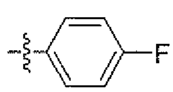 | 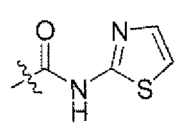 | Et |

Table 10

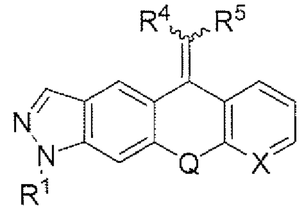

| Compound No. | Q | X | $R^1$ | $R^4$ | $R^5$ |
|---|---|---|---|---|---|
| 94 | $CH_2O$ | CH | 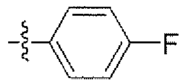 | H | 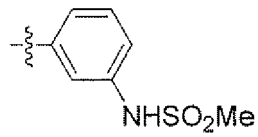 |
| 95 | $OCH_2$ | CH | 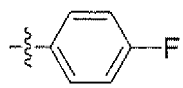 | Et | 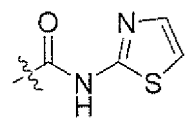 |
| 96 | $OCH_2$ | CH | 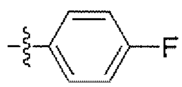 | Et | 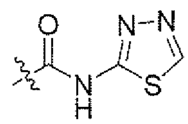 |
| 97 | $OCH_2$ | CH | 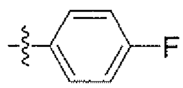 | Et | 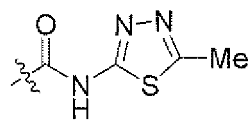 |
| 98 | $OCH_2$ | CH | 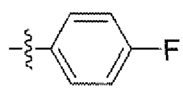 | Et | 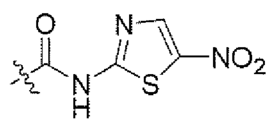 |

Table 11

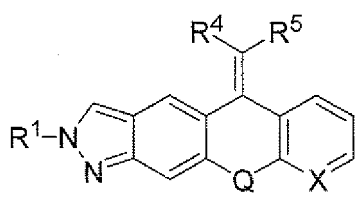

| Compound No. | Q | X | $R^1$ | $R^4$ | $R^5$ |
|---|---|---|---|---|---|
| 99 | $CH_2O$ | CH | iPr | Et | 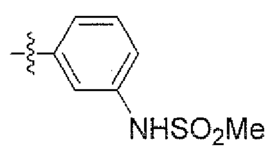 |

Next, pharmacological effects of some representative Compounds (I) will be specifically described with reference to Test Examples.

Test Example 1: Human glucocorticoid receptor assay

In this assay, a fluorescence polarization assay kit (Panvera/Invitrogen, product number: P2893) was used. The recombinant human glucocorticoid receptor (product number: P2812), the Fluoromore (trademark)-labeled dexamethasone (GS Green, product number: P2813), the stabilizing peptide solution (product number: P2815), and the basic buffer solution (product number: P2814) which were used in the assay kit had been stored at -80°C until used. A buffer solution for the assay was prepared by adding the basic buffer solution (10 fold dilution), the stabilizing peptide solution (10 fold dilution), and 1 mol/L DTT (1000 fold dilution to give a final concentration of 1 mmol/L) .

The assay was performed as follows. The test compound was diluted to 2-fold of the desired concentration with the buffer solution for the assay, and 16 μL of that was added to each well. Next, the Fluoromore (trademark)-labeled dexamethasone was diluted with the buffer solution for the assay so as to be 4 nmol/L, and 8 μL of that was added to each well. Further, the recombinant human glucocorticoid receptor was diluted with the buffer solution for the assay so as to be 16 nmol/L, and 8 μL of that was added to each well. After stirring of the mixture, the mixture was incubated under a light-shielded condition at room temperature for 2 hours or more. The fluorescence polarization value of each well was measured using a plate reader (PerkinElmer, Fusion or Envision). To a negative control group, the compound was not added. To a positive control group, dexamethasone was added so as to give a final concentration of 1 μmol/L. The glucocorticoid receptor binding rate (%) was calculated by the following formula.

Glucocorticoid receptor binding rate (%) = 100 × [1-(fluorescence polarization value of test compound solution - fluorescence polarization value of positive control solution)/(fluorescence polarization value of negative control solution - fluorescence polarization value of positive control solution)]

In this test, Compound 2 exhibited inhibitory activity of 80% or more at a concentration of 100 nmol/L or higher.

Test Example 2: Human progesterone receptor assay

In this assay, a fluorescence polarization assay kit (Panvera/Invitrogen, product number: P2895) was used. The recombinant human progesterone receptor (product number: P2899), the Fluoromore (trademark)-labeled progesterone (PL Green, product number: P2897), and the basic buffer solution (product number: P2901) which were used in the assay kit had been stored at -80°C until used. A buffer solution for the assay was prepared by adding 250 fold dilution of 1 mol/L DTT to the basic buffer solution to give a final concentration of 4 mmol/L.

The assay was performed as follows. The test compound was diluted to 2-fold of the desired concentration with the buffer solution for the assay, and 16 μL of that was added to each well. Next, the Fluoromore (trademark)-labeled progesterone was diluted with the buffer solution for the assay so as to be 8 nmol/L, and 8 μL of that was added to each well. Further, the recombinant human progesterone receptor was diluted with the buffer solution for the assay so as to be 160 nmol/L, and 8 μL of that was added to each well. After stirring of the mixture, the mixture was incubated under a light-shielded condition at room temperature for 1 hour or more. The fluorescence polarization value of each well was measured using a plate reader (PerkinElmer, Fusion or Envision). To a negative control group, the compound was not added. To a positive control group, mifepristone (RU-486) was added so as to give a final concentration of 1 μmol/L. The progesterone receptor binding rate (%) was calculated by the following formula.

Progesterone receptor binding rate (%) = 100 × [1 - (fluorescence polarization value of test compound solution - fluorescence polarization value of positive control solution)/(fluorescence polarization value of negative control solution - fluorescence polarization value of positive control solution)]

In this test, Compound 2 exhibited inhibitory activity of 10% or lower at a concentration of 1000 nmol/L.

The results of Test Examples 1 and 2 show that Compound 2 has a selective inhibitory activity to human glucocorticoid receptors.

Test Example 3: The inhibition assay for IL-1β induced IL-6 production

A549 cells cultured in F-12 medium supplemented with 10% bovine serum for 3 to 5 days were transferred to a 96-well plate (50000 cells/well) and then were cultured overnight. Next, the medium was exchanged for F-12 medium which did not contain bovine serum, and the cells were cultured for 8 to 12 hours. After the medium was removed by suction, 60 μL of F-12 medium was added to each well, and 20 μL of the test compound diluted to 5-fold of the desired concentration with F-12 medium or 20 μL of 4 μmol/L dexamethasone was added thereto. The test compound and the dexamethasone used here were prepared beforehand by dissolving in dimethyl sulfoxide (DMSO) and diluting with F-12 medium so that the final concentration of DMSO became 0.1% v/v or lower. After 30-minute incubation under 5% $CO_2$ atmosphere at 37°C, 20 μL of 4 nmol/L IL-1β diluted with F-12 medium was added to each well, and then incubation was performed under a 5% $CO_2$ atmosphere at 37°C for 12 to 18 hours. The 96-well plate was centrifuged at 300 × g at 4°C for 5 minutes, and then the culture supernatant was collected for use as a sample for IL-6 measurement. The IL-6 produced in the culture supernatant was quantified by the ELISA method (R&D Systems, product number: DY206, DouSet ELISA Development System Human IL-6), and the production inhibition rate was calculated by the following formula.

IL-6 production inhibition rate (%) = 100 × (amount of IL-6 production in the absence of the compound - amount of IL-6 production in the presence of the compound)/(amount of IL-6 production in the absence of the compound - amount of IL-6 production in the presence of dexamethasone)

In this test, Compound 2 exhibited IL-6 production inhibitory activity of 80% or more at a concentration of 100 nmol/L.

In addition, Compounds 6, 8, 14, 25, 43, 73, 89, and the like exhibited IL-6 production inhibitory activity of 80% or more at a concentration of 100 nmol/L.

It is reported that a glucocorticoid receptor agonist has an action of inhibiting IL-6 production. (Mol. Cell Endocrinol. vol. 275, No. 1-2, pp. 109-117 (2007))

Therefore, Compound (I) is considered to have an action of inhibiting IL-6 production, and from the results of Test Examples 1 and 5, is considered to have an agonistic activity to glucocorticoid receptors.

It is known that compounds having an agonistic activity to glucocorticoid receptors or IL-6 production inhibitory activity have, for example, anti-inflammatory effects. Therefore, it is considered that Compound (I) is effective for the treatment of, for example, an inflammatory disease, or the like.

Test Example 4: Mouse mammary tumor virus (MMTV) luciferase assay

A pGL4.14 plasmid (Promega, product number: AY864928) having a cloned MMTV-LTR fragment upstream of the luciferase gene (-200 to +100 relative to the transcription initiation site) was introduced into A549 cells, and the obtained strain which is constitutively resistant to a selective antibiotic hygromycin B (A549/MMTV-LTR) was used for evaluation. The A549/MMTV-LTR cells were transferred to a 96-well plate (25000 cells/well) and were cultured overnight. After that, the medium was exchanged for 80 μL of F-12 medium which did not contain bovine serum. Next, 20 μL of the test compound diluted to 5-fold of the desired concentration with F-12 medium or 20 μL of 4 μmol/L dexamethasone was added thereto, and the mixture was incubated under 5% $CO_2$ at 37°C for 6 hours. To each well, 50 μL of a luminescence reagent (Promega, product number: E2550 or E2650) was added, and the mixture was stirred to dissolve the cells, and the luciferase activity was measured by a plate reader (PerkinElmer, TopCount) . The MMTV activity, which shows the transcription-promoting activity via the glucocorticoid receptor, was calculated by applying the luminescence amount of luciferase to the following formula.

MMTV activity (%) = 100 × (luminescence amount in the presence of the compound - luminescence amount in the absence of the compound)/(luminescence amount in the presence of dexamethasone - luminescence amount in the absence of the compound)

In this test, Compound 2, dexamethasone, and prednisolone exhibited MMTV activities of 10% or less, 100% or more, and 80% or more, respectively at a concentration of 300 nmol/L.

In addition, Compounds 8 and 34 exhibited MMTV activities of 22% and 25%, respectively at the drug concentration of 300 nmol/L.

From the above results. Compounds 2, 8, and 34 have much weaker transcription-promoting activities via glucocorticoid receptors as compared to dexamethasone and prednisolone.

Therefore, these compounds are expected to reduce side effects, such as disorders of sugar metabolism.

Test Example 5: Human glucocorticoid receptor assay (2)

In this assay, a fluorescence polarization assay kit (Panvera/Invitrogen, product number: P2893) was used. The recombinant human glucocorticoid receptor (product number: P2812), the Fluoromore (trademark)-labeled dexamethasone (GS Green, product number: P2813), the stabilizing peptide solution (product number: P2815), and the basic buffer solution (product number: P2814) which were used in the assay kit had been stored at -80°C until used. A buffer solution for the assay was prepared by adding the basic buffer solution (10 fold dilation) the stabilizing peptide solution (10 fold dilution), and 1 mol/L DTT (dithiothreitol) (1000 fold dilution to give a final concentration of 1 mmol/L).

The assay was performed as follows. The test compound was diluted to 2-fold of the desired concentration with the buffer solution for the assay, and 16 μL of that was added to each well. Next, the Fluoromore (trademark)-labeled dexamethasone was diluted with the buffer solution for the assay so as to be 4 nmol/L, and 8 μL of that was added to each well. Further, the recombinant human glucocorticoid receptor was diluted with the buffer solution for the assay so as to be 16 nmol/L, and 8 μL of that was added to each well. After stirring the mixture, the mixture was incubated under a light-shielded condition at room temperature for 2 hours or more. The fluorescence polarization value of each well was measured using a plate reader (PerkinElmer, Fusion or Envision . To a negative control group, the compound was not added. To a positive control group, dexamethasone was added so as to give a final concentration of 1 μmol/L. The glucocorticoid receptor binding inhibitory rate (%) was calculated by the following formula.

Glucocorticoid receptor binding inhibitory rate (%) = 100 × [1 - (fluorescence polarization value of test compound solution - fluorescence polarization value of positive control solution)/(fluorescence polarization value of negative control solution - fluorescence polarization value of positive control solution)]

In this test, Compounds 6, 8, 14, 25, 34, 43, 73, 89, and the like exhibited binding inhibitory activities of 80% or more at a concentration of 100 nmol/L. Therefore, Compound (I) has been considered to have the binding activity to glucocorticoid receptors.

Test Example 6: Human progesterone receptor assay (2)

In this assay, a fluorescence polarization assay kit (Panvera/Invitrogen, product number: P2895) was used. The recombinant human progesterone receptor (product number: P2899), the Fluoromore (trademark)-labeled progesterone (PL Green, product number: P2897), and the basic buffer solution (product number: P2901) which were used in the assay kit had been stored at -80°C until used. A buffer solution for the assay was prepared by adding 250 fold dilution of 1 mol/L DTT (dithiothreitol) to the basic buffer solution to give a final concentration of 4 mmol/L.

The assay was performed as follows. The test compound was diluted to 2-fold of the desired concentration with the buffer solution for the assay, and 16 μL of that was added to each well. Next, the Fluoromore (trademark)-labeled progesterone was diluted with the buffer solution for the assay so as to be 8 nmol/L, and 8 μL of that was added to each well. Further, the recombinant human progesterone receptor was diluted with the buffer solution for the assay so as to be 160 nmol/L, and 8 μL of that was added to each well. After stirring the mixture, the mixture was incubated under a light-shielded condition at room temperature for 1 hour or more. The fluorescence polarization value of each well was measured using a plate reader (PerkinElmer, Fusion or Envision). To a negative control group, the compound was not added. To a positive control group, mifepristone (RU-486) was added so as to give a final concentration of 1 μmol/L. The progesterone receptor binding inhibitory rate (%) was calculated by the following formula.

MMTV activity (%) = 100 ×(luminescence amount in the presence of the compound - luminescence amount in the absence of the compound)/(luminescence amount in the presence of dexamethasone - luminescence amount in the absence of the compound)

In this test, Compounds 25, 34, 43, and the like exhibited binding inhibitory activities of 20% or less at a concentration of 1000 nmol/L.

From the results of Test Examples 5 and 6, Compound (I), such as 25, 34, 43, and the like, has been considered to show a selective binding activity to human glucocorticoid receptors.

Therefore, it is expected that these compounds are able to reduce the side effects originated from the progesterone receptor (for example, thrombosis, or the like).

Compound (I) or a pharmaceutically acceptable salt thereof can be administered alone. However, in general, Compound (I) or a pharmaceutically acceptable salt thereof is preferably provided as various pharmaceutical preparations. Such pharmaceutical preparations are used in animals and humans.

The pharmaceutical preparations according to the present invention may contain Compound (I) or a pharmaceutically acceptable salt thereof alone as an active ingredient or a mixture thereof with an optional active ingredient for any other therapy. The pharmaceutical preparations thereof are produced by mixing the active ingredient with one or

more pharmaceutically acceptable carriers and then subjecting the mixture to any production method well-known in the technical field of pharmaceutics.

The administration route is preferably the most effective route for the therapy. Examples of the administration route include oral administration and parenteral administration, such as intravenous administration.

Examples of the dosage form include tablets, injections, and the like.

For example, tablets and the like suitable for oral administration may be produced by use of excipients such as lactose, disintegrators such as starch, lubricants such as magnesium stearate, binders such as hydroxypropyl cellulose, and the like.

For example, injections and the like suitable for parenteral administration can be produced by use of a salt solution, a glucose solution, or a mixture of a salt solution and a glucose solution, and the like.

The doses and the frequencies of administration of Compound (I) or a pharmaceutically acceptable salt thereof may vary depending on the dosage form, the age and body weight of a patient, the nature or seriousness of the symptom to be treated, and the like. However, in the oral administration in general, a dose of 0.01 mg to 1 g, preferably, 0.05 to 100 mg is administered to an adult patient once or several times a day. In the parenteral administration, such as intravenous administration, a dose of 0.001 to 100 mg, preferably, 0.01 to 10 mg is administered to an adult patient once or several times a day. However, these doses and frequencies of administration vary depending on the various conditions described above.

Hereinafter, embodiments of the present invention will be described with reference to Examples and Reference Examples. Unless otherwise noted, starting materials and reagents used can be obtained as a commercial product, as a known substance, or according to a known method.

Reference Example 1

Step 1

N- (4-bromo-2, 5-dimethylphenyl) acetamide (Compound A1)

2,5-dimethylaniline (9.0 g, 74.3 mmol) was dissolved in toluene (90 mL). To this, acetic anhydride (7.71 mL, 81.7 mmol) was slowly added, and the mixture was stirred at 100˚C for 1 hour. The solvent was evaporated off under reduced pressure, and the residue was dissolved in acetic acid (54 mL) . To this, bromine (3.81 mL, 74.3 mmol) was slowly added, and the mixture was stirred at room temperature for 3 hours. To the reaction mixture, water (100 mL) was added. The precipitate was separated by filtration to give Compound A1 (16.0 g, yield: 89%) as a colorless crystal. ESI-MS *m/z*: 242 $[M+H]^+$; $^1$H-NMR (DMSO-$d_6$)δ(ppm) : 2.19 (s, 3H), 2.20 (s, 3H), 2.34 (s, 3H), 6.91 (br s, 1H), 7.34 (s, 1H), 7.67 (s, 1H).

Step 2

1-acetyl-5-bromo-6-methyl-1H-indazole (Compound A2)

Compound A1 (3.0 g, 12.4 mmol) was suspended in chloroform (27 mL) . To this, acetic anhydride (3.51 mL, 37.2 2 mmol), potassium acetate (2.43 g, 24. 8 mmol), 18-crown-6-ether (0.164 g, 6.20 mmol), and isoamyl nitrate (3.66 mL), 27.3 mmol) were added at room temperature, and the mixture was stirred at 65˚C for 3 days. After the reaction mixture was filtered, the solvent was evaporated off under reduced pressure. After an aqueous sodium hydrogen carbonate solution was added to the residue, extraction with ethyl acetate, washing with brine, and then drying over anhydrous sodium sulfate were performed. The solvent was evaporated off under reduced pressure, and the residue was purified by flash column chromatography (hexane/ethyl acetate = 9/1 to 4/1) to give Compound A2 (2.0 g, yield: 75%) as a colorless crystal.
ESI-MS *m/z*: 253 $(M+H)^+$; $^1$H-NMR ($CDCl_3$)δ(ppm): 2.57 (s, 3H), 2.77 (s, 3H), 7.92 (s, 1H), 8.02 (s, 1H), 8.36 (s, 1H).

Step 3

5-bromo-6-methyl-1H-indazole (Compound A3)

Compound A2 (3.90 g, 15.4 mmol) was suspended in methanol (12 mL) and 2. 0 mol/L hydrochloric acid (39 mL), and the mixture was stirred at 100˚C for 1 hour. Under ice-cooling, a 10 mol/L aqueous potassium hydroxide solution (10 mL) was added to the mixture. The precipitate was separated by filtration to give Compound A3 (3.20 g, yield: 97%) as a colorless crystal. ESI-MS *m/z*: 211 $[M+H]^+$; $^1$H-NMR ($CDCl_3$)δ(ppm): 2.53 (s, 3H), 7.38 (d, *J* = 1.0 Hz, 1H), 7.96 (s, 1H), 7.98 (d, *J* = 1.0 Hz, 1H).

Step 4

5-bromo-1,6-dimethyl-1H-indazole (Compound A4)

Compound A3 (2.16 g, 10.2 mmol) was dissolved in THF (45 mL). To this, 60% sodium hydride (0.61 g, 15.3 mmol) was added, and the mixture was stirred at room temperature for 15 minutes. Under ice-cooling, methyl iodide (1.26 mL, 20.5 mmol) was added, and the mixture was stirred for 1.5 hours. After water was added to the reaction mixture, extraction with ethyl acetate, washing with brine, and then drying over anhydrous sodium sulfate were performed. The solvent was evaporated off under reduced pressure, and the residue was purified by flash column chromatography (hexane/ethyl acetate = 85/15 to 30/70) to give Compound A4 (1.31 g, yield: 57%) as a colorless crystal. ESI-MS *m*/*z*: 225 $[M+H]^+$; $^1$H-NMR ($CDCl_3$)δ(ppm): 2.55 (s, 3H), 4.03 (s, 3H), 7.26 (s, 1H), 7.86 (d, *J* = 0.9 Hz, 1H), 7.96 (d, *J* = 0.9 Hz, 1H).

Step 5

Propyl-1,6-dimethyl-1H-indazole-5-carboxylate (Compound A5)

Compound A4 (1.30 g, 5.78 mmol) was dissolved in n-propanol (13 mL) and N,N-dimethylformamide (4 mL). To this, triethylamine (1.61 mL, 11.6 mmol) and [1,1'-bis(diphenylphosphino)ferrocene]palladiurm (II) dichloride (472 mg, 0.578 mmol) were added, and the mixture was stirred under carbon monoxide atmosphere at 90°C for 9 hours. After the reaction mixture was filtered through a Celite pad, extraction with water and ethyl acetate, washing with brine, and then drying over anhydrous sodium sulfate were performed. The solvent was evaporated off under reduced pressure, and the residue was purified by flash column chromatography (hexane/ethyl acetate = 85/15 to 30/70) to give Compound A5 (1.24 g, yield: 92%) as a colorless crystal.
ESI-MS *m*/*z*: 233 $[M+H]^+$; $^1$H-NMR ($CDCl_3$)δ(ppm): 1.06 (t, *J* = 7.2 Hz, 3H), 1.76-1.90 (m, 2H), 2.75 (s, 3H), 4.05 (s, 3H), 4.28 (t, *J* = 6.8 Hz, 2H), 7.20 (s, 1H), 8.01 (s, 1H), 8.42 (s, 1H).

Step 6

Propyl-6-bromomethyl-1-methyl-1H-indazole-5-carboxylate (Compound A6)

Compound A5 (1.51 g, 6.50 mmol) was dissolved in benzotrifluoride (30 mL). To this, N-bromosucciniimide (1.27 g, 7.15 mmol) and azobisisobutyronitrile (214 mg, 1.30 mmol) were added, and the mixture was stirred at 130°C for 3 minutes and then at room temperature for 30 minutes. The solvent was evaporated off under reduced pressure, and the residue was purified by flash column chromatography (hexane/ethyl acetate = 90/10 to 60/40) to give Compound A6 (1.51g, yield: 75%) as a colorless crystal.
ESI-MS *m*/*z*: 311 $[M+H]^+$; $^1$H-NMR ($CDCl_3$)δ(ppm) : 1.07 (t, *J* = 7.9 Hz, 3H), 1.78-1.92 (m, 2H), 4.10 (s, 3H), 4.34 (t, *J* = 6.9 Hz, 2H), 5.16 (s, 2H), 7.47 (s, 1H), 8.04 (s, 1H), 8.48 (s, 1H).

Step 7

n-propyl-1-rnethyl-6-phenoxymethyl-1H-indazole-5-carboylate (Compound A7)

Compound A6 (1.41 g, 4.53 mmol) was dissolved in N,N-dimethylformamide (28 mL). To this, phenol (0.515 g, 5.44 mmol) and potassium carbonate (0.945 g, 6.80 mmol) were added, and the mixture was stirred at room temperature for 5 hours. After the reaction mixture was diluted with water, extraction with ethyl acetate, washing with brine, and then drying over anhydrous sodium sulfate were performed. The solvent was evaporated off under reduced pressure, and the residue was purified by flash column chromatography (chloroform/methanol =99/1 to 90/10) to give roughly purified Compound A7 (2.03 g).
ESI-MS *m*/*z*: 325 $[M+H]^+$.

Step 8

1-methyl-6-phenoxymethyl-1H-indazole-5-carboxylic acid (Compound A8)

The above crude-purified Compound A7 (2.03g) was dissolved in ethanol (20 mL). To this, a 2 mol/L aqueous potassium hydroxide solution (10 mL) was added, and the mixture was stirred at 40°C for 5 hours. Under reduced pressure, the solvent was evaporated to about half the volume. To this, 2 mol/L hydrochloric acid was added, and the

precipitate was separated by filtration to give Compound A8 (1.17 g, 2-step yield: 91%) as a colorless crystal.
ESI-MS *m/z*: 283 $[M+H]^+$; $^1$H-NMR (DMSO-$d_6$)δ(ppm): 3.43 (br s, 1H), 4.05 (s, 3H), 5.55 (s, 2H), 6.96 (t, *J* = 7.3 Hz, 1H), 7.05 (d, *J* = 7.7 Hz, 2H), 7.32 (dd, *J* = 7.3, 7.7 Hz, 2H), 7.86 (s, 1H), 8.19 (s, 1H), 8.44 (s, 1H).

Step 9

1-methyl-5-on-5,11-dihydrobenzo[6,7]oxepino[4,3-f]indazole (Compound A9)

Polyphosphoric acid (15 mL) was added to Compound A8 (2.15 g, 7.62 mmol), and the mixture was stirred at 90˚C for 1 hour. To the reaction mixture, ice water was added. The precipitate was separated by filtration to give Compound A9 (1.48 g, yield: 74%) as a colorless crystal.
ESI-MS *m/z*: 265 $[M+H]^+$.

Step 10

1-methyl-5-propylidene-5,11-dihydrobenzo[6,7]oxepino[4,3-f] indazole (Compound A10)

n-propyl triphenyl phosphonium bromide (1.75g, 4.54 mmol)

was suspended in tetrahydrofuran (6 mL). To this, an n-butyllithium hexane solution (1.63 mol/L, 2.79 mL, 4.54 mmol) was added at -10˚C, and the mixture was stirred at room temperature for 2 hours. Subsequently, a suspension of Compound A9 (300 mg, 1.14 mmol) in tetrahydrofuran (1 mL) was slowly added thereto, and the mixture was stirred at room temperature for 12 hours. After the reaction mixture was diluted with water, extraction with ethyl acetate, washing with brine, and then drying over anhydrous sodium sulfate were performed. The solvent was evaporated off under reduced pressure, and the residue was purified by flash column chromatography (hexane/ethyl acetate = 4/1 to 1/1) to give Compound A10 (E/Z mixture) (266 mg, yield: 80%) as a colorless crystal.

ESI-MS *m/z*: 291 $[M+H]^+$; $^1$H-NMR (DMSO-$d_6$)δ(ppm): 1.00 (t, *J* = 7.6 Hz, 0.5H), 1.09 (t, *J* = 7.2 Hz, 2.5H), 2.33-2.43 (m, 2H), 4.05 (s, 2.5H), 4.07 (s, 0.5H), 5.30 (br s, 2H), 5.68 (t, *J* = 7.6 Hz, 0.83H), 6.05 (t, *J* = 7.2 Hz, 0.17H), 6.71 (d, *J* = 7.9 Hz, 0.17H), 6.79 (d, *J* = 8.6 Hz, 0.83H), 6.86-6.92 (m, 1H), 7.10-7.18 (m, 2H), 7.55 (s, 0.17H), 7.59 (s, 0.83H), 7.74 (s, 0.83H), 7.82 (s, 0.17H), 8.02 (s, 0.83H), 8.05 (s, 0.17H).

Step 11

(E)-5-(1-bromopropylidene)-1-methyl-5,11-dihydrobenzo-[6,7]oxepino[4,3-f]indazole (Compound A11)

(Z)-5-(1-bromopropylidene)-1-methyl-5,11-dihydrobenzo-[6,7]oxepino[4,3-f]indazole (Compound A12)

Compound A10 (265 mg, 0.913 mmol) was dissolved in acetonitrile (8 mL). To this, 4-(dimethylamino)pyridiniurm tribromide (994 mg, 2.74 mmol) was added, and the mixture was stirred at 80˚C for 4 hours. After water was added to the reaction mixture, extraction with ethyl acetate, washing with brine, and then drying over anhydrous sodium sulfate were performed. The solvent was evaporated off under reduced pressure, and the residue was purified by flash column chromatography (hexane/ethyl acetate = 97/3 to 80/20) to give Compound A11 (79 mg), yield: 23%) and Compound A12 (155 mg, yield: 46%) each as a colorless crystal.
Compound A11: ESI-MS *m/z*: 369 $[M+H]^+$; $^1$H-NMR ($CDCl_3$)δ(ppm): 1.31 (t, *J* = 7.0 Hz, 3H), 2.74-2.92 (m, 2H), 4.07 (s, 3H), 4.91 (d, *J* = 12.5 Hz, 1H), 5.75 (d, *J* = 12.5 Hz, 1H), 6.76-6.87 (m, 2H), 7.09-7.17 (m, 2H), 7.43 (s, 1H), 7.65 (s, 1H), 7.97 (s, 1H). Compound A12: ESI-MS *m/z*: 369 $[M+H]^+$; $^1$H-NMR ($CDCl_3$)δ(ppm): 1.16 (t, *J* = 7.3 Hz, 3H), 2.58 (q, *J* = 7.3 Hz, 2H), 4.06 (s, 3H), 4.92 (d, *J* = 12.5 Hz, 1H), 5.78 (d, *J* = 12.5 Hz, 1H), 6.75 (dd, *J* = 1.4, 8.4 Hz, 1H), 6.83-6.88 (m, 1H), 7.10-7.15 (m, 1H), 7.40 (dd, *J* = 1.8, 8.1 Hz, 1H), 7.44 (s, 1H), 7.53 (s, 1H), 7.95 (s, 1H).

Reference Example 2

Step 1

5-bromo-1-(4-fluorophenyl)-6-methyl-1H-indazole (Compound A13)

Compound A3 (500 mg, 2.37 mmol) obtained in the Step 3 of Reference Example 1 was dissolved in toluene (20 mL). To this, 4-fluoroiodobenzene (0.360 ml, 3.10 mmol), potassium phosphate (1.01 g, 4.74 mmol), copper iodide mmol), 0.237 mmol), and ethylenediamine (0.0640 mL, 0.948 mmol) were added, and the mixture was stirred at 120˚C for 10 hours. After water was added to the reaction mixture, extraction with ethyl acetate was performed. After washing

with brine, drying over anhydrous sodium sulfate was performed. The solvent was evaporated off under reduced pressure, and the residue was purified by flash silica gel column chromatography (hexane/ethyl acetate = 99/1 to 9/1) to give Compound A13 (0.74 g, yield: 44%).
ESI-MS *m/z*: 305 $[M+H]^+$; $^1$H-NMR ($CDCl_3$)δ(ppm): 2.54 (s, 3H), 7.22-7.26 (m, 2H), 7.53 (s, 1H), 7.60-7.68 (m, 2H), 7.99 (s, 1H), 8.08 (s, 1H).

Step 2

1-(4-fluorophenyl)-5,11-dihydro-1H-indazolo[5,6-e]benzo[b]-5-oxepinone (Compound A14)

With use of Compound A13, in the same manner as in the Steps 5, 6, 7, 8, and 9 of Reference Example 1, Compound A14 was obtained as a colorless crystal.
ESI-MS *m/z*: 345 $[M+H]^+$; $^1$H-NMR ($CDCl_3$) δ: 5.30 (d, *J* = 5.5 Hz, 2H), 7.07 (dd, *J* = 8.1, 1.1 Hz, 1H), 7.12-7.18 (m, 1H), 7.26-7.31 (m, 2H), 7.47-7.53 (m, 1H), 7.60 (s, 1H), 7.67-7.74 (m, 2H), 8.30-8.33 (m, 2H), 8.44 (s, 1H).

Step 3

(E)-1-(4-fluorophenyl)-5-(1-bromopropylidene)-5,11-dihydro-1H-indazolo[5,6-e] benzo[b]oxepin (Compound A15).

(Z)-1-(4-fluorophenyl)-5-(1-bromopropylidene)-5,11-dihydro-1H-indazolo[5,6-e]benzo[b]oxepin (Compound A16)

With use of Compound A14, in the same manner as in the Steps 10 and 11 of Reference Example 1, Compounds A15 and A16 were obtained each as a colorless crystal.
Compound A15: ESI-MS *m/z*: 449 $[M+H]^+$; $^1$H-NMR ($CDCl_3$)δ(ppm) : 1.32 (t, *J* = 7.3 Hz, 3H), 2.81-2.88 (m, 2H), 4.89 (d, *J* = 12.1 Hz, 1H), 5.75 (d, *J* = 12.1Hz, 1H), 6.78 (d, *J* = 8.1 Hz, 1H), 6.84-6.89 (m, 1H), 7.15-7.24 (m, 5H), 7.66-7.67 (m, 3H), 7.74 (s, 1H), 8.19 (s, 1H).
Compound A16: ESI-MS *m/z*: 449 $[M+H]^+$; $^1$H-NMR ($CDCl_3$)δ(ppm) : 1.18 (t, *J* = 7.3 Hz, 3H), 2.60-2.62 (m, 2H), 4.89 (d, *J* = 12.5 Hz, 1H), 5.77 (d, *J* = 12.5 Hz, 1H), 6.75 (dd, *J* = 1.8, 8.2 Hz, 1H), 6.85-6.90 (m, 1H), 7.11-7.17 (m, 1H), 7.23-7.27 (m, 2H), 7.42 (dd, *J* = 1.8, 7.7 Hz, 1H), 7.61-7.70 (m, 4H), 8.18 (d, *J* = 0.7 Hz, 1H).

Reference Example 3

(E)-1-(4-fluorophenyl)-5-(1-bromoethylidene)-5,11-dihydro-1H-indazolo[5,6-e]benzo[b]oxepin (Compound A17)

(Z)-1-(4-fluorophenyl)-5-(1-bromoethylidene)-5,11-dihydro-1H-indazolo[5,6-e]benzo[b]oxepin (Compound A18)

From Compound A14 obtained in the Step 2 of Reference Example 2 and ethyltriphenylphosphonium bromide, in the same manner as in the Steps 10 and 11 of Reference Example 1, Compounds A17 and A18 were obtained each as a colorless crystal. Compound A17: ESI-MS *m/z*: 435, 437 $[M+H]^+$; $^1$H-NMR ($CDCl_3$)δ(ppm): 2.66 (s, 3H), 4.89 (d, *J* = 12.2 Hz, 1H), 5.75 (d, *J* = 12.2 Hz, 1H), 6.76-6.90 (m, 2H), 7.11-7.27 (m, 5H), 7.64-7 .70 (m, 2H), 7.76 (s, 1H), 8.19 (d, *J* = 0.7 Hz, 1H).
Compound A18 : ESI-MS *m/z*: 435, 437 $[M+H]^+$; $^1$H-NMR ($CDCl_3$) δ(ppm) : 2.44 (s, 3H), 4.89 (d, *J* = 12.2 Hz, 1H), 5.76 (d, *J* = 12.2 Hz, 1H), 6.75 (dd, *J* = 8.3, 1.3 Hz, 1H), 6.85-6.91 (m, 1H), 7.11-7.18 (m, 1H), 7.22-7.28 (m, 1H), 7.45 (dd, *J* = 7.6, 1.7 Hz, 1H), 7.62-7.69 (m, 4H), 8.02 (br s, 1H), 8.17 (d, *J* = 1.0 Hz, 1H). Reference Example 4

Step 1

6-methyl-5-propyloxycarbonyl-1H-indazole (Compound A19)

With use of Compound A2 (37 .7 g, 0.149 mol), in the same manner as in the Step 5 of Reference Example 1, Compound A19 (35.5 g) was obtained as a roughly purified product.
ESI-MS m/z: 219 $[M+H]^+$.

Step 2

1-benzenesulphonyl-6-methyl-5-propyloxycarbonyl-1H-indazole (Compound A20)

With use of Compound A19 (35.5 g) and benzenesulphonyl chloride (31.6 g, 0.179 mol), in the same manner

as in the Step 4 in Reference Example 1, Compound A20 (32.6 g, 2-step yield: 72%) was obtained.
ESI-MS m/z: 359 $[M+H]^+$; $^1H$-NMR ($CDCl_3$) δ(pom) : 1.04 (t, J = 7.3 Hz, 3H), 1.80 (tq, J = 6.6, 7.3 Hz, 2H), 2.79 (s, 3H), 4.29 (t, J = 6.6 Hz, 2H), 7.44-7.51 (m, 2H), 7.56-7.63 (m, 1H), 7.96-8.02 (m, 2H), 8.08 (t, J = 1.0 Hz, 1H), 8.19 (d, J = 1.0 Hz, 1H), 8.31 (s, 1H).

Step 3

5,11-dihydro-1H-indazolo[5.6-e]benzo[b]oxepin-5-one (Compound A21)

With use of Compound A20, in the same manner as in the Steps 6, 7, 8, and 9 of Reference Example 1, Compound A21 was obtained as a colorless crystal.
ESI-MS m/z: 251 $[M+H]^+$; $^1H$-NMR (DMSO-$D_6$) δ(ppm) : 5.39 (s, 2H), 7.11 (d, J = 8.3 Hz, 1H), 7.18 (dd, J = 4.0, 11.0 Hz, 1H), 7.55-7.61 (m, 1H), 7.73 (s, 1H), 8.16 (dd, J = 1.7, 8.3 Hz, 1H), 8.29 (d, J = 4.0 Hz, 2H), 13.5 (s, 1H) .

Step 4

1-isopropyl-5,11-dihydro-1H-indazolo[5,6-e]benzo[b]oxepin (Compound A22)

2-isopropyl-5,11-dihydro-1H-indazolo[5,6-e]benzo[b]oxepin (Compound A23)

Compound A21 (2.00 g, 0.799 mmol) was dissolved in DMF (20 mL) . To this, 60% sodium hydride (0.64 g, 0.160 mmol) was added, and the mixture was stirred at room temperature for 30 minutes. Under ice-cooling, isopropyl iodide (1.99 mL, 20.0 mmol) was added thereto, and the mixture was stirred at room temperature for 2 hours. The mixture was neutralized with hydrochloric acid, and then extraction with ethyl acetate was performed. After washing with brine, drying over anhydrous sodium sulfate was performed. The solvent was evaporated off under reduced pressure, and the residue was purified by flash column chromatography (hexane/ethyl acetate = 99/1 to 70/30) to give Compound A22 (1.23 g, yield: 53%) and Compound A23 (0. 65 g, yield: 28%) each as a light yellow crystal.
CompoundA22: ESI-MS m/z: 293 $[M+H]^+$; $^1H$-NMR ($CDCl_3$) δ(ppm) : 1.63 (d, J = 6.6 Hz, 6H), 4.82-4.96 (m, 1H), 5.34 (d, J = 8.1 Hz, 2H), 7.06 (dd, J = 1.1, 8.4 Hz, 1H), 7.11-7.16 (m, 1H), 7.39 (s, 1H), 7.46-7.52 (m, 1H), 8.13 (s, 1H), 8.31 (dd, J = 1.8, 8.1 Hz, 1H), 8.38 (s, 1H).
CompoundA23: ESI-MS m/z: 293 $[M+H]^+$; $^1H$-NMR ($CDCl_3$) δ(ppm) : 1.68 (d, J = 6.6Hz, 6H), 4.78-4.87 (m, 1H), 5.29 (s, 2H), 7.06 (dd, J = 0.9, 8.2 Hz, 1H), 7.09-7.14 (m, 1H), 7.45-7.50 (m, 1H), 7.67 (s, 1H), 8.11 (t, J = 2.7 Hz, 1H), 8.29 (dd, J = 2.7, 8.2 Hz, 1H), 8.33 (s, 1H).

Step 5

(E)-5-(1-bromopropylidene)-1-isopropyl-5,11-dihydro-1H-indazolo[5,6-e]benzo[b]oxepin (Compound A24)

(Z)-5-(1-bromapropylidene)-1-isopropyl-5,11-dihydro-1H-indazolo[5,6-e]benzo[b]oxepin (Compound A25)

With use of Compound A22, in the same manner as in the Steps 10 and 11 of Reference Example 1, Compounds A24 and A25 were obtained each as a colorless crystal.
Compound A24: ESI-MS m/z: 397 $[M+H]^+$; $^1H$-NMR ($CDCl_3$)δ(ppm) : 1. 31 (t, J = 7.3 Hz, 3H), 1.56 (d, J = 7.0 Hz, 3H), 1.62 (d, J = 6.6 Hz, 3H), 2.74-2.92 (m, 1H), 4.83 (t, J = 6.6 Hz, 2H), 4.91 (d, J = 12.5 Hz, 1H), 5.76 (d, J = 12.5 Hz, 1H), 6.79-6.84 (m, 2H), 7.09-7.17 (m, 2H), 7.47 (s, 1H), 7.65 (s, 1H), 7.99 (s, 1H). Compound A25: ESI-MS m/z: 397 $[M+H]^+$; $^1H$-NMR ($CDCl_3$)δ(ppm) : 1.17 (t, J = 7.4 Hz, 3H), 1.57 (d, J = 6.9 Hz, 3H), 1.62 (d, J = 6.6 Hz, 3H), 2.56-2.64 (m, 2H), 4.82-4.88 (m, 1H), 4.91 (d, J = 12.2 Hz, 1H), 5.78 (d, J = 12.2 Hz, 1H), 6.75 (dd, J = 1.3, 8.3 Hz, 1H), 6.83-6.89 (m, 1H), 7.11-7.14 (m, 1H), 7.40 (dd, J = 1.7, 8.7 Hz, 1H), 7.49 (s, 1H), 7.53 (s, 1H), 7.99 (s, 1H).

Reference Example 5

Step 1

1-cyclopentyl-5,11-dihydro-1H-indazoio[5,6-e]benzo[b]oxepin (Compound A26)

With use of Compound A21 (500 mg, 2.00 mmol) obtained in the Step 3 of Reference Example 4 and cyclopentyl iodide (0.48 mL, 4.00 mmol), in the same manner as in the Step 4 of Reference Example 4, Compound A26 (0.25 g, yield: 39%) was obtained as a light yellow crystal.

ESI-MS *m/z*: 319 $[M+H]^+$; $^1H$-NMR ($CDCl_3$)δ(ppm) : 1.70-1.85 (m, 2H), 1.98-2.03 (m, 2H), 2.15-2.25 (m, 4H), 4.97-5.08 (m, 1H), 5.33 (s, 2H), 7.06 (dd, *J* = 1.0, 8.3 Hz, 1H), 7.10-7.16 (m, 1H), 7.40 (s, 1H), 7.47-7.50 (m, 1H), 8.11 (s, 1H), 8.31 (dd, *J* = 1.7, 8.3 Hz, 1H), 8.37 (s, 1H).

Step 2

(E)-5-(1-bromopropylidene)-1-cyclopentyl-5,11-dihydro-1H-indazolo[5,6-e]benzo[b]oxepin (Compound A27)

(Z)-5-(1-bromopropylidene)-1-cyclopentyl-5,11-dihydro-1H-indazolo[5,6-e]benzo[b]oxepin (Compound A28)

With use of Compound A26, in the same manner as in the Steps 10 and 11 of Reference Example 1, Compounds A27 and A28 were obtained each as a colorless crystal.
Compound A27: ESI-MS *m/z*: 423 $[M+H]^+$; $^1H$-NMR ($CDCl_3$) δ(ppm) : 1.31 (t, *J* = 7.1 Hz, 3H), 1.74 (dd, *J* = 7.0, 13.6 Hz, 2H), 1.99 (dd, *J* = 5.9, 11.7 Hz, 2H), 2.12-2.26 (m, 4H), 2.74-2.92 (m, 2H), 4.92 (d, *J* = 12.1 Hz, 1H), 4.97-4.99 (m, 1H), 5.76 (d, *J* = 12.1 Hz, 1H), 6.76-6.87 (m, 2H), 7.10-7.16 (m, 2H), 7.49 (s, 1H), 7.65 (s, 1H), 7.98 (s, 1H).
Compound A28: ESI-MS *m/z*: 423 $[M+H]^+$; $^1H$-NMR ($CDCl_3$) δ(ppm) : 1.16 (t, *J* = 7.3 Hz, 3H), 1.66-1.86 (m, 2H), 2.01-2.11 (m, 6H), 2.58-2.62 (m, 2H), 4.91 (d, *J* = 12.5 Hz, 1H), 4.99-5.01 (m, 1H), 5.78 (d, *J* = 12.5 Hz, 1H), 6.75 (dd, *J* = 1.1, 8.4 Hz, 1H), 6.83-6.88 (m, 1H), 7.08-7.16 (m, 1H), 7.40 (dd, *J* = 1.6, 7.9 Hz, 1H), 7.51 (d, *J* = 4.8 Hz, 2H), 7.97 (s, 1H).

Reference Example 6

Step 1

5,11-dihydro-1-(2-fluoropyridine-5-yl)-1H-indazolo[5,6-e] benzo[b]oxepin (Compound A29)

With use of Compound A21 (440 mg, 1.76 mmol) obtained in the Step 3 of Reference Example 4 and 2-fluoro-5-iodopyridine (783 mg, 3.52 mmol), in the same manner as in the Step 1 of Reference Example 2, Compound A29 was obtained as a colorless crystal.
ESI-MS *m/z:* 346 $[M+H]^+$; $^1H$-NMR ($CDCl_3$)δ(ppm): 5.29 (s, 2H), 7.01-7.20 (m, 2H), 7.60 (s, 1H), 7.70-7.73 (m, 2H), 8.29-8.32 (m, 2H), 8.35 (s, 1H), 8.45 (s, 1H), 8.53 (t, *J* = 2.7 Hz, 1H).

Step 2

(E)-5-(1-bromopropylidene)-1-(2-fluoropyridine-5-yl)-5,11-dihydrobenzo[b]oxepino[4,3-f]-1H-indazole (Compound A30)

(E)-5-(1-bromopropylidene)-1-(2-fluoropyridine-5-yl)-5,11-dihydrobenzo[b]oxepino [4,3-f]-1H-indazole (Compound A31)

With use of Compound A29, in the same manner as in the Steps 10 and 11 of Reference Example 1, Compounds A30 and A31 were obtained each as a colorless crystal.
Compound A30: ESI-MS *m/z*: 450 $[M+H]^+$; $^1H$-NMR ($CDCl_3$) δ(ppm): 1.33 (t, *J* = 7.1 Hz, 3H), 2.76-2.94 (m, 2H), 4.90 (d, *J* = 12.5 Hz, 1H), 5.75 (d, *J* = 12.5 Hz, 1H), 6.78 (dd, *J* = 1.3, 8.2 Hz, 1H), 6.84-6.90 (m, 1H), 7.11-7.19 (m, 3H), 7.70 (s, 1H), 7.77 (s, 1H), 8.13-8.19 (m, 1H), 8.24 (d, *J* = 0.7 Hz, 1H), 8.62-8.65 (m, 1H).
Compound A31: ESI-MS *m/z*: 450 $[M+H]^+$; $^1H$-NMR ($CDCl_3$) δ(ppm): 1.18 (t, *J* = 7.3 Hz, 3H), 2.57-2.65 (m, 2H), 4.90 (d, *J* = 12.1 Hz, 1H), 5.78 (d, *J* = 12.1 Hz, 1H), 6.75 (dd, *J* = 1.6, 8.2 Hz, 1H), 6.88 (m, 1H), 7.12-7.16 (m, 2H), 7.42 (dd, *J* = 1.6, 7.9 Hz, 1H), 7.64 (s, 1H), 7.71 (s, 1H), 8.13-8.16 (m, 1H), 8.23 (d, *J* = 1.6 Hz, 1H), 8.62 (dd, *J* = 1.6, 2.2 Hz, 1H).

Reference Example 7

Step 1

5-bromo-2-fluoro-4-methoxybenzaldehyde (Compound A32)

To methanol (175 mL), bromine (16.6 mL, 324 mmol) was added, and under ice-methanol-cooling, a solution of 2-fluoro-4-methoxybenzaldehyde (25 g, 162 mmol) inmethanol (25 mL) was added dropwise thereto. Stirring was continued at 0˚C until the starting materials disappeared, and then a saturated sodium hydrogen sulfite aqueous solution and water (500 mL) was added thereto. The produced solid substance was separated by filtration, washed with water, and then dried under reduced pressure to give Compound A32 (34.0 g, yield: 90%) as a white solid substance.

$^1$H-NMR (300 MHz, $CDCl_3$) δ(ppm): 3.98 (s, 3H), 6.68 (d, J = 11.7 Hz, 1H), 8.06 (d, J = 7.7 Hz, 1H), 10.17 (s, 1H).

Step 2

5-bromo-6-methoxy-1H-indazole (Compound A33)

Compound A32 (9 g, 38.6 mmol), o-hydroxylamine hydrochloride (3.23 g, 38.6 mmol), and potassium carbonate (5.87 g, 42.5 mmol) were dissolved in 1,2-dimethoxyethane (90 mL), and the mixture was stirred at 100˚C for 1 hour. After cooling to room temperature, the solid substance was filtered off, and the filtrate was evaporated off under reduced pressure. Dimethyl acetamide (90 mL) and hydrazine monohydrate (18 mL) were added thereto, and the mixture was stirred at 150˚C overnight. After cooling to room temperature, water was added to the reaction mixture, and the aqueous layer was extracted with ethyl acetate. The organic layer was washed with water and a saturated aqueous solution of sodium chloride, dried over anhydrous magnesium sulfate, and then the solvent was evaporated off under reduced pressure to give Compound A33 (8.26 g, yield: 94%) as a yellow paste.
ESI-MS m/z: 227, 229 $[M+H]^+$; $^1$H-NMR (300 MHz, $CDCl_3$) δ(ppm): 3.96 (s, 3H), 6.92 (s, 1H), 7.94 (d, J = 1.1 Hz, 2H).

Step 3

5-bromo-1-(4-fluorophenyl)-6-methoxy-1H-indazole (Compound A34)

With use of Compound A33 (3.09 g, 13.6 mmol), in the same manner as in the Step 1 of Reference Example 2, Compound A34 (2.15 g, yield: 49%) was obtained as a yellow solid substance. ESI-MS m/z: 321, 323 $[M+H]^+$; $^1$H-NMR (300 MHz, $CDCl_3$) δ(ppm): 3.94 (s, 3H), 7.00 (s, 1H), 7.22-7.28 (m, 2H), 7.62-7.67 (m, 2H), 7.97 (s, 1H), 8.04 (d, J = 0.7 Hz, 1H).

Step 4

5-bromo-1-(4-fluorophenyl)-6-hydroxy-1H-indazole (Compound A35)

Compound A34 (1.85 g, 5.76 mmol) was dissolved in dichloromethane (37 mL). To this, a solution of boron tribromide in dichloromethane (1.0 mol/L, 11.5 mL) was added under ice-cooling, and the mixture was stirred at room temperature overnight. To the reaction mixture, a solution of boron tribromide in dichloromethane (1.0 mol/L, 17.3 mL) was added, and stirring was further continued at room temperature overnight. Under ice-cooling, a saturated sodium hydrogen carbonate aqueous solution was added to stop the reaction, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 90/10 to 30/70) to give Compound A35 (1.64 g, yield: 93%) as a reddish brown solid substance. EST-MS m/z: 307, 309 $[M+H]^+$; $^1$H-NMR (270 MHz, DMSO) δ(ppm): 7.22 (s, 1H), 7.41-7.48 (m, 2H), 7.70-7.75 (m, 2H), 8.06 (s, 1H), 8.17 (d, J = 0.7 Hz, 1H), 10.70 (br s, 1H).

Step 5

Ethyl-2-(bromomethyl)nicotinate (Compound A36)

With use of ethyl-2-methyl nicotinate (4.66 mL, 30. 3 mmol), in the same manner as in the Step 6 of Reference Example 1, Compound A36 (3.09 g, yield: 42%) was obtained as a red oil. ESI-MS m/z: 244, 246 $[M+H]^+$; $^1$H-NMR (300 MHz, $CDCl_3$) δ(ppm): 1.44 (t, J = 7.1 Hz, 3H), 4.44 (q, J = 7.1 Hz, 2H), 5.04 (s, 2H), 7.34 (dd, J = 7.8, 4.8 Hz, 1H), 8.29 (dd, J = 7.8, 1.7 Hz, 1H), 8.71 (dd, J = 4.8, 1.7 Hz, 1H).

Step 6

Ethyl-2-[5-bromo-1-(4-fluorophenyl)-1H-indazole-6-yloxy] methyl nicotinate (Compound A37)

With use of Compound A35 (3.33 g, 10.8 mmol) obtained in the Step 4 of Reference Example 7 and Compound A36 (2.91g, 11.9 mmol), in the same manner as in the Step 7 of Reference Example 1, Compound A37 (3.84 g, yield: 75%) was obtained as a slightly yellow paste.
ESI-MS m/z: 470, 472 $[M+H]^+$; $^1$H-NMR (270MHz, $CDCl_3$) δ(ppm): 1.33 (t, J = 7.1 Hz, 3H), 4.39 (q, J = 7.1 Hz, 2H), 5.62

(s, 2H), 7.21-7.27 (m, 3H), 7.40 (dd, J = 7.9, 4.7 Hz, 1H), 7.59 (dd, J = 8.9, 4.7 Hz, 2H), 7.94 (s, 1H), 8.02 (d, J = 0.7 Hz, 1H), 8.23 (dd, J = 7. 9, 1.8 Hz, 1H), 8.72 (dd, J = 4.7, 1.8 Hz, 1H).

Step 7

2-[5-bromo-1-(4-fluorophenyl)-1H-indazole-6-yloxy]methylN-methoxy-N-methylnicotinamide (Compound A38)

Compound A37 (3.84 g, 8.16 mmol) and
N,O-dimethylhydroxylamine hydrochloride (2.39 g, 24.5 mmol) were dissolved in THF (77 mL), a solution of isopropylmagnesium chloride in THF (2.0 mol/L, 24.5 mL) was slowly added dropwise under ice-methanol-cooling. After the mixture was stirred under ice-methanol-cooling for 1 hour and 45 minutes, a saturated ammonium chloride aqueous solution was added thereto, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 90/10 to 60/40) to give Compound A38 (2.99 g, yield: 75%) as a light yellow amorphous.
ESI-MS m/z: 485, 487 $[M+H]^+$; $^1$H-NMR (300 MHz, $CDCl_3$) δ(ppm): 3.34 (br s, 3H), 3.39 (br s, 3H), 5.49 (s, 2H), 7.21-7.29 (m, 3H), 7.35 (dd, J = 7.7, 4.6 Hz, 1H), 7.57 (dd, J = 8.8, 4.6 Hz, 2H), 7.81 (d, J = 7.0 Hz, 1H), 7.92 (s, 1H), 8.00 (d, J = 0.7 Hz, 1H), 8.66 (dd, J = 4.6, 1.8 Hz, 1H).

Step 8

1-(4-fluorophenyl)-5,10-dihydro-1H-indazolo[6,5-b]pyrido[3, 2-e]-5-oxepinone (Compound A39)

Compound A38 (600 mg, 1.24 mmol) was dissolved in THF (12 mL), and the solution was cooled to -78˚C. To this, an n-butyl lithium hexane solution (1.65 mol/L, 0.974 mL) was added, and the mixture was stirred for 1 hour and 10 minutes with the temperature gradually raised from -78 to -20˚C. After a saturated ammonium chloride aqueous solution was added to the reaction mixture, the solvent was evaporated to some degree under reduced pressure. The produced solid substance was separated by filtration and reslurried with a mixed solvent of hexane/ethyl acetate/chloroform (20/1/1) to give Compound A39 (393 mg, yield: 92%) as a yellow solid substance.
ESI-MS m/z: 346 $[M+H]^+$; $^1$H-NMR (300 MHz, $CDCl_3$) δ(ppm) :5.40 (s, 2H), 7.23-7.29 (m, 2H), 7.35 (s, 1H), 7.48 (dd, J = 8.0, 4.8 Hz, 1H), 7.66-7.71 (m, 2H), 8.30 (s, 1H), 8.47 (dd, J = 8.0, 1.7 Hz, 1H), 8.74 (dd, J = 4.8, 1.7 Hz, 1H), 8.82 (s, 1H).

Step 9

(Z)-1-(4-fluorophenyl)-5-(1-bromopropylidene)-5,10-dihydro-1H-indazolo[6,5-b]pyrido[3,2-e]oxepin (Compound A40)

(E)-1-(4-fluorophenyl)-5-(1-bromopropylidene)-5,10-dihydro-1H-indazolo[6,5-b]pyrido[3,2-e]oxepin (Compound A41)

With use of Compound A39, in the same manner as in the Steps 10 and 11 of Reference Example 1, Compounds A40 and A41 were obtained each as an orange paste.
Compound A40: ESI-MS m/z:450, 452 $[M+H]^+$;
Compound A41: ESI-MS m/z:450, 452 $[M+H]^+$; $^1$H-NMR (300 MHz, $CDCl_3$) δ(ppm): 1.26 (t, J = 7.3 Hz, 3H), 2.67-2.80 (m, 2H), 5.03 (d, J = 14. 9 Hz, 1H), 5.67 (d, J = 14. 9 Hz, 1H), 7.19-7.25 (m, 3H), 7.34 (s, 1H), 7.55 (s, 1H), 7.61-7.65 (m, 2H), 7.84 (dd, J = 7.7, 1.5 Hz, 1H), 8.11 (d, J = 0.7 Hz, 1H), 8.47 (dd, J = 4.8, 1.5 Hz, 1H).

Reference Example 8

Step 1

Methyl-2-(bromomethyl)benzoate (Compound A42)

With use of methyl-2-methyl benzoate (9.23 mL, 65.9 mmol), in the same manner as in the Step 5 of Reference Example 5, Compound A42 (14.4 g, yield: 95%) was obtained as a colorless oil.
ESI-MS m/z: 229 $[M+H]^+$; $^1$H-NMR (270 MHz, $CDCl_3$) δ(ppm): 3.95 (s, 3H), 4.96 (s, 2H), 7.35-7.41 (m, 1H), 7.47-7.51 (m, 2H), 7.97 (d, J = 7.6 Hz, 1H).

Step 2

1-(4-fluorophenyl)-5-(1-bromopropylidene)-5,10-dihydro-1H-indazolo[6,5-b]benzo[e]oxepin (Compound A43)

With use of Compound A35 (2.62 g, 8.53 mmol) obtained in the Step 4 of Reference Example 6 and Compound 42 (2.15 g, 9.38 mmol), in the same manner as in the Steps 6 to 9 of Reference Example 5, Compound A43 (E/Z mixture) was obtained as a light yellow amorphous.
ESI-MS m/z: 449, 451 $[M+H]^+$; $^1$H-NMR (270 MHz, $CDCl_3$) δ(ppm): 1.20 (t, J = 7.4 Hz, 1.71H), 1.32 (t, J = 7.4 Hz, 1.29H), 2.56-2.66 (m, 1.14H), 2.79-2.89 (m, 0.86H), 4.87 (d, J = 12.6 Hz, 1.00H), 5.67 (d, J = 12.6 Hz, 0.43H), 5.69 (d, J = 12.6 Hz, 0.57H), 7.07-7.08 (m, 1.00H), 7.14-7.21 (m, 2.00H), 7.24-7.28 (m, 1.00H), 7.30-7.40 (m, 3.00H), 7.52 (s, 0.43H), 7.57-7.64 (m, 2.00H), 7.81 (s, 0.57H), 8.06 (d, J = 1.0 Hz, 0.43H), 8.08 (d, J = 1.0 Hz, 0.57H).

Reference Example 9

(Z)-5-(1-bromopropylidene)-1-(4-methoxybenzyl)-5,11-dihydro-1H-indazolo[5,6-e]benzo[b]oxepin (Compound A44)

With use of Compound A21 obtained in the Step 3 of Reference Example 4 and 4-methoxybenzyl chloride, in the same manner as in the Step 4 of Reference Example 4 and the Steps 10 and 11 of Reference Example 1, Compound A44 was obtained as a colorless crystal.
ESI-MS *m/z*: 567 $[M+H]^+$; $^1$H-NMR ($CDCl_3$)δ(ppm): 1.26 (t, *J* = 7.1 Hz, 3H), 2.39-2.44 (m, 1H), 2.60 (s, 3H), 2.82-2.87 (m, 1H), 3.77 (s, 3H), 4.96 (d, *J* = 12.2 Hz, 1H), 5.54 (s, 2H), 6.00 (d, *J* = 12.2 Hz, 1H), 6.30 (s, 1H), 6.38-6.44 (m, 1H), 6.57-6.73 (m, 2H), 6.81-6.85 (m, 3H), 7.00-7.02 (m, 1H), 7.12 (s, 1H), 7.17-7.33 (m, 4H), 7.44 (s, 1H), 7.66 (s, 1H), 8.03 (s, 1H).

Reference Example 10

Step 1

5-acetamide-2-iodo-4-methylbenzyl acetate (Compound A45)

(3-amino-4-methylphenyl)methanol (8.60 g, 62.7 mmol) and sodium hydrogen carbonate (10.3 g, 125 mmol) were dissolved in dichloromethane (84 mL) and methanol (84 mL). To this, benzyl trimethylammonium dichloro iodide (25.5 g, 75.2 mmol) was added, and the mixture was stirred at the room temperature for 40 minutes. The reaction mixture was concentrated under reduced pressure. To this, propionitrile (170 mL) and acetic anhydride (30 mL) were added, and the mixture was stirred at 80°C for 1.5 hours. To the reaction mixture, water and diisopropyl ether were added. The precipitate was separated by filtration to give Compound A45 (14.1 g, yield: 67%).
ESI-MS m/z: 348 $[M+H]^+$.

Step 2

6-acetoxy methyl-1-acetyl-5-iodo-1H-indazole (Compound A46)

With use of Compound A45 (14.1 g, 40.6 mmol), in the same manner as in the Step 2 of Reference Example 1, Compound A46 (13.4 g, yield: 92%) was obtained as a brownish-red crystal. ESI-MS m/z: 359 $[M+H]^+$.

Step 3

6-hydroxymethyl-5-iodo-1H-indazole (Compound A47)

Compound A46 (13.4 g, 37.4 mmol) was dissolved in methanol (250 mL). To this, potassium carbonate (15.5 g, 112 mmol) was added, and the mixture was stirred at room temperature for 1.5 hours. To the reaction mixture, water was added. The precipitate was separated by filtration to give Compound A47 (10.0 g, yield: 98%).
ESI-MS m/z: 275 $[M+H]^+$.

Step 4

6-(tert-butyldimethylsilyloxy)methyl-5-iodo-1H-indazole (Compound A48)

Compound A47 (650 mg, 2.37 mmol) was dissolved in DMF (6.5 mL). To this, triethylamine (0.668 mL, 4.74 mmol) and tert-butyldimethylsilyl chloride (536 mg, 3. 56 mmol) were added, and the mixture was stirred at room temperature for 2 hours. After water was added to the reaction mixture, extraction with ethyl acetate was performed. After washing with brine, drying over anhydrous sodium sulfate was performed.
The solvent was evaporated off under reduced pressure, and the residue was purified by flash silica gel column chromatography (hexane/ethyl acetate = 9/1 to 3/1) to give Compound A48 (795 mg, yield: 86%).
ESI-MS m/z: 389 $[M+H]^+$; $^1H$-NMR ($CDCl_3$)δ(ppm):0.18 (s, 6H), 1.01 (s, 9H), 4.72 (d, J = 1.3 Hz, 2H), 7.71 (d, J = 1.3 Hz, 1H), 7.98 (s, 1H), 8.22 (s, 1H).

Step 5

6-(tert-butyldimethylsilyloxy)methyl-5-iodo-1-isopropyl-1H-indazole (Compound A49)

With use of Compound A48 (2.40 g, 6.18 mmol) and isopropyliodide (1.54 mL, 15.5 mmol), in the same manner as in the Step 4 of Reference Example 4, Compound A49 (1.73 g, yield: 65%) was obtained.
ESI-MS m/z: 431 $[M+H]^+$; $^1H$-NMR ($CDCl_3$)δ(ppm): 0.19 (s, 6H), 1.01 (s, 9H), 1.57 (d, J = 7.6 Hz, 3H), 1.59 (d, J = 6.6 Hz, 3H), 4.74 (d, J = 1.3 Hz, 2H), 4.77-4.87 (m, 1H), 7.67 (d, J = 1.3 Hz, 1H), 7.89 (s, 1H), 8.17 (s, 1H).

Step 6

6-hydroxymethyl-5-iodo-1-isopropyl-1H-indazole (Compound A50)

Compound A49 (1.73 g, 4.02 mmol) was dissolved in THF (34 mL). To this, tetra(n-butyl)ammonium fluoride (1.0 mol/L, 4.80 mL), 4.80 mmol) was added, and the mixture was stirred at room temperature for 30 minutes. The solvent was evaporated off under reduced pressure, and the residue was purified by flash silica gel column chromatography (hexane/ethyl acetate = 3/2 to 1/4) to give Compound A50 (1.10 g, yield: 87%).
ESI-MS m/z: 317 $[M+H]^+$; $^1H$-NMR ($CDCl_3$)δ(ppm): 1.58 (d, J = 6.4 Hz, 6H), 2.37-2.42 (m, 1H), 4.84-4.88 (m, 3H), 7.62 (s, 1H), 7.91 (s, 1H), 8.20 (s, 1H).

Step 7

6-hydroxymethyl-1-isopropyl-5-(1-pentynyl)-1H-indazole (Compound A51)

Compound A50 (400 mg, 1.27 mmol) was dissolved in DMF (8.0 mL). To this, copper iodide (24.0 mg, 0.127 mmol) 1-pentyne (0.370 mL, 3.80 mmol), bis(triphenylphosphine)palladium(II) dichloride (89.0 mg, 0.127 mmol), and triethylamine (0.880 mL, 6.33 mmol) were added, and the mixture was stirred at room temperature for 12 hours. After water was added to the reaction mixture, extraction with ethyl acetate was performed. After washing with brine, drying over anhydrous sodium sulfate was performed. The solvent was evaporated off under reduced pressure, and the residue was purified by flash silica gel column chromatography (hexane/ethyl acetate = 9/1 to 7/3) to give Compound A51 (320 mg, yield: 99%).
ESI-MS m/z: 257 $[M+H]^+$; $^1H$-NMR ($CDCl_3$)δ(ppm): 1.08 (t, J = 7.3 Hz, 3H), 1.58 (d, J = 6.6 Hz, 6H), 1.63-1.73 (m, 2H), 2.39 (t, J = 6.3 Hz, 1H), 2.45 (t, J = 7.3 Hz, 2H), 4.81-4.85 (m, 1H), 4.93 (d, J = 6.3 Hz, 2H), 7.48 (s, 1H), 7.80 (s, 1H), 7.94 (s, 1H).

Step 8

6-(3-iodopyridine-2-yloxy)methyl-1-isopropyl-5-(1-pentynyl) -1H-indazole (Compound A52)

Compound A51 (320 mg, 1.25 mmol) was dissolved in DMF (6.5 mL). To this, 2-chloro-3-iodopyridine (450 mg, 1.87 mmol) and 60% sodium hydride (75.0 mg, 1.87 mmol) were added, and the mixture was stirred at room temperature for 2.5 hours. After water was added to the reaction mixture, extraction with ethyl acetate was performed. After washing with brine, drying over anhydrous sodium sulfate was performed. The solvent was evaporated off under reduced pressure, and the residue was purified by flash silica gel column chromatography (hexane/ethyl acetate = 9/1 to 85/15) to give Compound A52 (299 mg, yield: 52%).

ESI-MS m/z: 460 $[M+H]^+$; $^1$H-NMR ($CDCl_3$)(ppm): 1.06 (t, J = 7.3 Hz, 3H), 1.60 (d, J = 6.6 Hz, 6H), 1.64-1.67 (m, 2H), 2.43 (t, J = 6.9 Hz, 2H), 4.79-4.94 (m, 1H), 5.70 (s, 2H), 6.70 (dd, J = 5.0, 7.6 Hz, 1H), 7.79 (s, 1H), 7.82 (s, 1H), 7.94 (s, 1H), 8.09 (dd, J = 1.7, 7.6 Hz, 1H), 8.16 (dd, J = 1.7, 5.0 Hz, 1H).

Reference Example 11

Step 1

2-fluoro-5-iodo-4-methoxybenzaldehyde (Compound A53)

2-fluoro-4-methoxybenzaldehyde (9.9 g, 64.2 mmol) was dissolved in concentrated sulfuric acid (70 mL). Under ice-cooling, N-iodosuccinimide (13.7 g, 61.0 mmol) was added, and the mixture was stirred at room temperature overnight. The reaction mixture was poured into a cold sodium sulfite aqueous solution. The produced solid substance was separated by filtration to give Compound A53 (14.4 g, yield: 80%) as a brownish solid substance.
ESI-MS m/z: 281 $[M+H]^+$; $^1$H-NMR (300 MHz, $CDCl_3$) δ(ppm): 3.96 (s, 3H), 6.60 (d, J = 12.1 Hz, 1H), 8.29 (d, J = 7.7 Hz, 1H), 10.13 (s, 1H).

Step 2

5-iodo-6-methoxy-1H-indazole (Compound A54)

With use of Compound A53 (695 mg, 2.48 mmol), in the same manner as in the Step 2 of Reference Example 7, Compound A54 (429 mg, yield: 63%) was obtained as a yellow solid substance. ESI-MS m/z: 275 $[M+H]^+$; $^1$H-NMR (300 MHz, $CDCl_3$) δ(ppm): 3.94 (s, 3H), 6.87 (s, 1H), 7.91 (d, J = 1.1 Hz, 1H), 8.18 (s, 1H).

Step 3

5-iodo-1-isopropyl-6-methoxy-1H-indazole (Compound A55)

With use of Compound A54 (425 mg, 1.55 mmol), in the same manner as in the Step 4 of Reference Example 4, Compound A55 (287 mg, yield: 59%) was obtained as a yellow oil.
ESI-MS m/z: 317 $[M+H]^+$; $^1$H-NMR (300 MHz, $CDCl_3$) δ(ppm): 1.58 (d, J = 6.6 Hz, 6H), 3.96 (s, 3H), 4.68-4.81 (m, 1H), 6.74 (s, 1H), 7.84 (s, 1H), 8.13 (s, 1H).

Step 4

5-iodo-1-isopropyl-6-hydroxy-1H-indazole (Compound A56)

Compound A55 (285 mg, 0.902 mmol) was dissolved in DMF (2.9 mL). To this, dodecanethiol (0.643 mL, 2.70 mmol) and potassium tert-butoxide (198 mg, 2.70 mmol) were added, and the mixture was stirred at 100˚C for 1 hour and 45 minutes. To the reaction mixture, dodecanethiol (0.429 mL, 1.80 mmol) and potassium tert-butoxide (132 mg, 1.80 mmol) were added, and the mixture was further stirred at 100˚C for 50 minutes. After cooling to room temperature, a saturated ammonium chloride aqueous solution and a 1 mol/L hydrochloric acid aqueous solution were added, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 100/0 to 40/60) to give Compound A56 (287 mg, yield: 59%) as a white solid substance. ESI-MS m/z: 303 $[M+H]^+$; $^1$H-NMR (300 MHz, $CDCl_3$) δ(ppm): 1.56 (d, J = 6.6 Hz, 6H), 4.65-4.74 (m, 1H), 5.45 (br s, 1H), 7.03 (s, 1H), 7.84 (s, 1H), 8.05 (s, 1H).

Step 5

{2-[(3-nitrophenyl)ethynyl]phenyl}methanol (Compound A57)

1-ethynyl-3-nitrobenzene (100 mg, 0.680 mmol), 2-iodobenzyl alcohol (191 mg, 0.816 mmol), bis(triphenylphosphine)palladium(II) dichloride (47.7 mg, 0. 0680 mmol), and copper(I) iodide (25.9 mg, 0.136 mmol) were dissolved in acetonitrile (2 mL). To this, triethylamine (0.142 mL, 1.02 mmol) was added, and the mixture was stirred at room temperature for 50 minutes and then at 70˚C for 2.5 hours. After cooling to room temperature and filtration with Celite,

the filtrate was evaporated off under reduced pressure. The residue was purified by silica gel column chromatography (hexane/ethyl acetate = 100/0 to 55/45) to give Compound A57 (84.8 mg, yield: 49%) as an orange solid substance.
$^1$H-NMR (300 MHz, $CDCl_3$) δ(ppm): 1.97 (t, J = 6.0 Hz, 1H), 4.94 (d, J = 6.0 Hz, 2H), 7.33 (td, J = 7.6, 1.3 Hz, 1H), 7.43 (td, J = 7.6, 1.3 Hz, 1H), 7.56 (t, J = 8.1 Hz, 3H), 7.83 (dt, J = 7.7, 1.2 Hz, 1H), 8.20 (dq, J = 8.4, 1.2 Hz, 1H), 8.37 (t, J = 1.8 Hz, 1H).

Step 6

5-iodo-1-isopropyl-6-{2-[(3-nitrophenyl)ethynyl]-phenylmethyloxy}-1H-indazole (Compound A58)

Compound A56 (131 mg, 0.434 mmol) obtained in the Step 4 of Reference Example 11 and Compound A57 (165 mg, 0.652 mmol) were dissolved in toluene. To this, 1,1'-(azodicarbonyl) dipiperidine (219 mg, 0.869 mmol) and tributylphosphine (0.536 mL, 2.17 mmol) were added, and the mixture was stirred at room temperature for 3 hours and 20 minutes. The reaction mixture was filtered, and the filtrate was concentrated. The residue was purified by silica gel column chromatography (hexane/ethyl acetate = 95/5 to 65/35) to give Compound A58 (234 mg, quantitative yield) as a yellow solid substance.
ESI-MS m/z: 538 $[M+H]^+$; $^1$H-NMR (270 MHz, $CDCl_3$) δ(ppm): 1.51 (s, 3H), 1.53 (s, 3H), 4.64-4.73 (m, 1H), 5.46 (s, 2H), 6.88 (s, 1H), 7.38 (td, J = 7.6, 1.3 Hz, 1H), 7.45-7.54 (m, 2H), 7.63 (dd, J = 7.6, 1.3 Hz, 1H), 7.75 (dt, J = 7.9, 1.3 Hz, 1H), 7.79 (dd, J = 7.9, 1.3 Hz, 1H), 7.83 (s, 1H), 8.15 (s, 1H), 8.19 (dq, J = 8.3, 1.1 Hz, 1H), 8.33 (t, J = 1.8 Hz, 1H).

Reference Example 12

Step 1

1-(2,4-dimethoxybenzyl)-5-iodo-6-hydroxymethyl-1H-indazole (Compound A59)

With use of Compound A48 (10.0 g, 25.8 mmol) obtained in the Step 4 of Reference Example 10 and 2,4-dimethoxybenzyl chloride (9.61 g, 51.5 mmol), in the same manner as in the Steps 5 and 6 of Reference Example 10, Compound A59 (4.41 g, 2-step yield: 40%) was obtained.
ESI-MS m/z: 425 $[M+H]^+$.

Step 2

5-(1-butynyl)-1-(2,4-dimethoxybenzyl)-6-hydroxymethyl-1H-indazole (Compound A60)

With use of Compound A59 and 1-butyne, in the same manner as in the Steps 7 and 8 of Reference Example 10, Compound A60 was obtained.
ESI-MS: m/z 554 $[M+H]^+$; $^1$H NMR ($CDCl_3$)δ(ppm): 1.22 (t, J = 7.5 Hz, 3H), 2.44 (q, J = 7.5 Hz, 2H), 3.75 (s, 3H), 3.76 (s, 3H), 5.52 (s, 2H), 5.66 (s, 2H), 6.36 (dd, J = 2.2, 8.4 Hz, 1H), 6.41 (d, J = 2.2 Hz, 1H), 6.68 (dd, J = 4.9, 7.4 Hz, 1H), 6.94 (d, J = 8.4 Hz, 1H), 7.77 (s, 1H), 7.80 (s, 1H), 7.95 (s, 1H), 8.06 (dd, J = 1.5, 7.4 Hz, 1H), 8.13 (dd, J = 1.5, 4, 9 Hz, 1H).

Reference Example 13

Step 1

5-(1-butynyl)-6-hydroxymethyl-1H-indazole (Compound A61)

Compound A47 (10.0 g, 36.5 mmol) obtained in the Step3 of Reference Example 10 was dissolved in DMF (180 mL). To this, triethylamine (25.4 mL), 182 mmol) was added at room temperature, and a suitable amount of 1-butyne gas was blown thereinto. To this, bis(triphenylphasphine)palladium(II) dichloride (2.56 g, 3.65 mmol) and copper (I) iodide (695 mg, 3.65 mmol) were added, and the mixture was stirred at 40˚C for 7 hours. After the reaction mixture was cooled to room temperature, water was added and extraction with ethyl acetate was performed twice. The organic layer was washed with a saturated sodium chloride aqueous solution and dried over anhydrous magnesium sulfate. After filtration and concentration under reduced pressure, the residue was crystallized with use of a mixed solvent of hexane and chloroform to give Compound A61 (3.56 g, yield): 49%).
$^1$H NMR (DMSO)δ(ppm): 1.19 (t, J = 7.5 Hz, 3H), 2.45 (q, J = 7.5 Hz, 2H), 4.70 (d, J = 5.6 Hz, 2H), 5.32-5.39 (m, 1H), 7.61 (s, 1H), 7.76 (s, 1H), 8.00 (s, 1H).

Step 2

5-(1-butynyl)-6-(3-iodopyridine- 2-yloxy) methyl-1H-indazole (Compound A62)

Compound A61 (3.15 g, 15.7 mmol) was dissolved in DMF (70 mL). To this, 60% sodium hydride (1.57 g, 39.3 mmol) was added at room temperature and the mixture was stirred for 5 minutes. To this, 2-chloro-3-iodopyridine (4.52 g, 18.9 mmol) was added, and the mixture was further stirred at 40˚C for 90 minutes. After the reaction mixture was cooled to 0˚C, 1 mol/L hydrochloric acid and water were added to adjust the pH to 7, and the mixture was stirred for 30 minutes. The precipitate was separated by filtration, dried, and then purified by silica gel column chromatography to give Compound A62 (3.85 g, yield: 61%).
ESI-MS: m/z 404 $[M + H]^+$; $^1H$ NMR ($CDCl_3$)δ(ppm): 1.24 (t, J = 7.5 Hz, 3H), 2.45 (q, J = 7.5 Hz, 2H), 5.69 (s, 2H), 6.68 (d, J = 4.8, 7.5 Hz, 1H), 7.72 (s, 1H), 7.85 (s, 1H), 8.03 (s, 1H), 8.07 (dd, J = 1.8, 7.5 Hz, 1H), 8.13 (dd, J = 1.8, 4.8 Hz, 1H), 10.4-10.9 (br, 1H).

Step 3

5-(1-butynyl)-6-(3-iodopyridine-2-yloxy)methyl-1-isopropyl-1H-indazole (Compound A63)

Compound A62 (1.03 g, 2.55 mmol) was dissolved in DMF (4.0 mL). To this, 60% sodium hydride (204 mg, 5.10 mmol) was added at room temperature and the mixture was stirred for 5 minutes. To this, 2-iodopropane (382 μL, 3.83 mmol) was added, and the mixture was further stirred for 1 hour. After a saturated ammonium chloride aqueous solution was added to the reaction mixture, extraction with ethyl acetate was performed twice. The organic layer was washed with a saturated sodium chloride aqueous solution and dried over anhydrous magnesium sulfate. After filtration and concentration under reduced pressure, the residue was purified by silica gel column chromatography to give Compound A63 (450 mg, yield: 40%).
ESI-MS: m/z 446 $[M + H]^+$; $^1H$ NMR ($CDCl_3$)δ(ppm): 1.24 (t, J = 7.5 Hz, 3H), 2.45 (q, J = 7.5 Hz, 2H), 5.69 (s, 2H), 6.68 (d, J = 4.8, 7. 5 Hz, 1H), 7.72 (s, 1H), 7.85 (s, 1H), 8.03 (s, 1H), 8.07 (dd, J = 1.8, 7.5 Hz, 1H), 8.13 (dd, J = 1.8, 4.8 Hz, 1H).

Reference Example 14

1-(2-benzyloxy)ethyl-5-(1-butynyl)-6-(3-iodopyridine-2-yloxy)methyl-1H-indazole (Compound A64)

With use of Compound A62 (200 mg, 0.496 mmol) obtained in the Step 2 of Reference Example 13 and benzyl 2-bromoethyl ether (118 μL, 0.744 mmol), in the same manner as in the Step 3 of Reference Example 13, Compound A64 (150 mg, 56%) was obtained.
ESI-MS: m/z 538 $[M+H]^+$; $^1H$ NMR ($CDCl_3$)δ(ppm): 1.23 (t, J = 7.5 Hz, 3H), 2.45 (q, J = 7.5 Hz, 2H), 3.91 (t, J = 5.7 Hz, 2H), 4.43 (s, 2H), 4.58 (d, J = 5.7 Hz, 2H), 5.67 (s, 2H), 6.68 (dd, J = 4.8, 7.4 Hz, 1H), 7.12-7.26 (m, 5H), 7.79-7.82 (m, 2H), 7.95 (d, J = 0.7 Hz, 1H), 8.07 (dd, J = 1.8, 7.4 Hz, 1H), 8.15 (dd, J = 1.8, 4.8 Hz, 1H).

Reference Example 15

5-(1-butynyl)-1-cyclobutylmethyl-6-(3-iodopyridine-2-yloxy)methyl-1H-indazole (Compound A65)

With use of Compound A62 (50.0 mg, 0.124 mmol) obtained in the Step 2 of Reference Example 13 and bromomethyl cyclobutane (20.7 μL, 0.186 mmol), in the same manner as in the Step 3 of Reference Example 13, Compound A65 (37.0 mg, yield: 63%) was obtained.
ESI-MS: m/z 472 $[M + H]^+$: $^1H$ NMR ($CDCl_3$)δ(ppm): 1.24 (t, J = 7.5 Hz, 3H), 1.78-1.90 (m, 4H), 1.90-2.03 (m, 2H), 2.46 (q, J = 7.5 Hz, 2H), 2.86-2.98 (m, 1H), 4.39 (d, J = 7.2 Hz, 2H), 5.70 (s, 2H), 6.70 (dd, J = 4.8, 7.4 Hz, 1H), 7.75 (s, 1H), 7.80 (s, 1H), 7.91 (d, J = 1.0 Hz, 1H), 8.09 (dd, J = 1.8, 7.4 Hz, 1H), 8.15 (dd, J = 1.8, 4.8 Hz, 1H).

Reference Example 16

Step 1

4-methylsulfonyl oxymethyl tetrahydro-2H-pyran (Compound A66)

4-hydroxymethyl tetrahydro-2H-pyrane (1.16 g, 9.99 mmol) was dissolved in dichloromethane (30 mL). To this, triethylamine (2.09 mL, 15.0 mmol) and methanesulfonyl chloride (928 μL, 12.0 mmol) were added at room temperature,

and the mixture was stirred for 50 minutes. After a saturated aqueous solution of sodium hydrogen carbonate was added to the reaction mixture, extraction with chloroform was performed 3 times. The organic layer was dried over anhydrous magnesium sulfate, and was subjected to filtration and concentration under reduced pressure. The residue was crystallized with use of a mixed solvent of hexane and ethyl acetate to give Compound A66 (1.58 g, yield: 81%).
ESI-MS: m/z 195 $[M + H]^+$; $^1H$ NMR ($CDCl_3$)δ(ppm): 1.32-1.47 (m, 2H), 1.65-1.72 (m, 2H), 1.95-2.10 (m, 1H), 3.02 (s, 3H), 3.36-3.45 (m, 2H), 3.97-4.03 (m, 2H), 4.07 (d, J = 6.6 Hz, 2H).

Step 2

5-(1-butynyl)-6-(3-iodopyridine-2-yloxy)methyl-1-(tetrahydro-2H-pyrane-4-yl)methyl-1H-indazole (Compound A67)

With use of Compound A62 (50.0 mg, 0.124 mmol) obtained in the Step 2 of Reference Example 13 and Compound A66 (36.1 mg, 0.86 mmol), in the same manner as in the Step 3 of Reference Example 13, Compound A67 (32.9 mg, yield: 53%) was obtained. ESI-MS: m/z 502 $[M + H]^+$; $^1H$ NMR ($CDCl_3$)δ(ppm): 1.26 (t, J = 7.5 Hz, 3H), 1.39-1.50 (m, 4H), 2.20-2.32 (m, 1H), 2.47 (q, J = 7.5 Hz, 2H), 3.26-3.36 (m, 2H), 3.88-3.95 (m, 2H), 4.26 (d, J = 7.2 Hz, 2H), 5.70 (s, 2H), 6.71 (dd, J = 4.8,7.6 Hz, 1H), 7.73 (s, 1H), 7.81 (s, 1H), 7.94 (s, 1H), 8.10 (dd, J = 1.6, 7.6Hz, 1H), 8.16 (dd, J = 1.6, 4.8 Hz, 1H).

Reference Example 17

5-(1-butyryl)-6-(3-iodopyridine-2-yloxy)methyl-1-(3-pentyl)-1H-indazole (Compound A68)

With use of Compound A62 (50.0 mg, 0.124 mmol) obtained in the Step 2 of Reference Example 13 and 3-iodopentane (23.1 μL, 0.186 mmol), in the same manner as in the Step 3 of Reference Example 13, Compound A68 (30.7 mg, yield: 52%) was obtained. ESI-MS: m/z 474 $[M + H]^+$; $^1H$ NMR ($CDCl_3$)δ(ppm): 0.72 (t, J = 7.3 Hz, 6H), 1.25 (t, J = 7.3 Hz, 3H), 1.84-1.98 (m, 2H), 2.03-2.19 (m, 2H), 2.46 (q, J = 7.6 Hz, 2H), 4.24-4.34 (m, 1H), 5.68 (s, 2H), 6.70 (dd, J = 4.8,7.7 Hz, 1H), 7.79 (s, 1H), 7.81 (s, 1H), 7.98 (s, 1H), 8.09 (dd, J = 1.8,7.7 Hz, 1H), 8.15 (dd, J = 1.8, 4.8 Hz, 1H).

Reference Example 18

5-(1-butynyl)-1-cyclopropyl-6-(3-iodopyridine-2-yloxy)methyl-1H-indazole (Compound A69)

Compound A62 (100 mg, 0.248 mmol) obtained in the Step 2 of reference example 13, cyclopropyl boronic acid (42.6 mg, 0.496 mmol), and 2,2'-bipyridyl (38.7 mg, 0.248 mmol) were dissolved in 1,2-dichloroethane (1.0 mL). Copper(II) acetate (45.0mg, 0.248mmol) and sodium carbonate (52.6mg, 0.496 mmol) were added at room temperature, and the mixture was stirred at 70°C for 2 hours. After the reaction mixture was cooled to room temperature, a saturated ammonium chloride aqueous solution was added, and extraction with chloroform was performed twice. The organic layer was dried over anhydrous magnesium sulfate. After filtration and concentration under reduced pressure, the residue was purified by silica gel column chromatography to give Compound A69 (55.4 mg, yield: 50%).
ESI-MS: m/z 444$[M+H]^+$; $^1H$ NMR ($CDCl_3$)δ(ppm): 1.12-1.29 (m, 4H), 1.24 (t, J = 7.5 Hz, 3H), 2.46 (q, J = 7.5 Hz, 2H), 3.55-3.63 (m, 1H), 5.70 (d, J = 0.7 Hz, 2H), 6.70 (dd, J = 4.8, 7.5 Hz, 1H), 7.79 (s, 1H), 7.88 (d, J = 0.7 Hz, 1H), 7.95 (s, 1H), 8.10 (dd, J = 1.8, 7.5 Hz, 1H), 8.17 (dd, J = 1.8, 4.8 Hz, 1H).

Reference Example 19

Step 1

1-tert-butoxycarbonyl-4-(methylsulfonyloxy)ethyl piperidine (Compound A70)

1-tert-butoxycarbonyl-4-hydroxymethyl piperidine (2.15 g, 9.99 mmol) was dissolved in dichloromethane (30 mL). To this, triethylamine (2.09mL), 15.0 mmol) and methanesulfonyl chloride (928 μL, 12.0 mmol) were added at room temperature, and the mixture was stirred for 50 minutes. After a saturated aqueous solution of sodium hydrogen carbonate was added to the reaction mixture, extraction with chloroform was performed 3 times. The organic layer was dried over anhydrous magnesium sulfate, and was subjected to filtration and concentration under reduced pressure. The residue was crystallized with use of a mixed solvent of hexane and ethyl acetate to give Compound A70 (2.30 g, yield: 79%).
ESI-MS: m/z 294 $[M + H]^+$; $^1H$ NMR ($CDCl_3$)δ(ppm): 1.14-1.29 (m, 2H), 1.46 (s, 9H), 1.70-1.78 (m, 2H), 1.84-2.00 (m, 1H), 2.66-2.79 (m, 2H), 3.02 (s, 3H), 4.07 (d, J = 6.2 Hz, 2H), 4.10-4.22 (m, 2H).

Step 2

1-(1-tert-butoxycarbonylpiperidine-4-yl)methyl-5-(1-butynyl )-6-(3-iodopyridine-2-yloxy)methyl-1H-indazole (Compound A71)

With use of Compound A62 (500 mg, 1.24 mmol) obtained in the Step 2 of Reference Example 13 and Compound A70 (545 mg, 1.86 mmol), in the same manner as in the Step 3 of Reference Example 13, Compound A71 (415 mg, yield: 56%) was obtained. ESI-MS: m/z 601 $[M + H]^+$; $^1H$ NMR ($CDCl_3$)δ(ppm): 1.15-1.32 (m, 2H), 1.26 (t, J = 7.5 Hz, 3H), 1.45 (s, 9H), 1.46-1.58 (m, 2H), 2.09-2.24 (m, 1H), 2.47 (q, J = 7.5 Hz, 2H), 2.35-2.68 (m, 2H), 4.00-4.08 (m, 2H), 4.25 (d, J = 7.2 Hz, 2H), 5.70 (d, J = 1.0 Hz, 2H), 6.71 (dd, J = 4.8, 7.6 Hz, 1H), 7.71 (s, 1H), 7.81 (s, 1H), 7.93 (d, J = 0.7 Hz, 1H), 8.10 (dd, J = 1.8, 7.6 Hz, 1H), 8.16 (dd, J = 1.8, 4.8 Hz, 1H).

Reference Example 20

5-(1-butynyl)-6-(3-iodapyridine-2-yloxy)methyl-1-(4-methaxybenzyl)-1H-indazole (Compound A72)

With use of Compound A62 (65.2 mg, 0.162 mmol) obtained in the Step 2 of Reference Example 13 and 4-methoxybenzyl bromide (350 mg, 0.243 mmol), in the same manner as in the Step 3 of Reference Example 13, Compound A72 (46.8 mg, yield: 55%) was obtained.
ESI-MS: m/z 524 $[M + H]^+$; $^1H$ NMR ($CDCl_3$)δ(ppm): 2.24 (t, J = 7.3 Hz, 3H), 2.45 (q, J = 7.6Hz, 2H), 3.75 (s, 3H), 5.51 (s, 2H), 5.65 (s, 2H), 6.68 (dd, J = 4.8, 7.3 Hz, 1H), 6.79 (d, J = 8.4 Hz, 2H), 7.20 (d, J = 8.4 Hz, 2H), 7.70 (s, 1H), 7.81 (s, 1H), 7.96 (s, 1H), 8.06-8.13 (m, 2H).

Reference Example 21

1-tert-butoxycarbonylmethyl-5-(1-butynyl)-6-(3-iodopyridine -2-yloxy)methyl-1H-indazole (Compound A73)

With use of Compound A62 (100 mg, 0.248 mmol) obtained in the Step 2 of Reference Example 13 and tert-butyl bromoacetate (54.9 μL, 0.372 mmol), in the same manner as in the Step 3 of Reference Example 13, Compound A73 (61.3mg, yield: 48%) was obtained.
ESI-MS: m/z 518 $[M + H]^+$; $^1H$ NMR ($CDCl_3$)δ(ppm): 1.24 (t, J = 7.5 Hz, 3H), 1.41 (s, 9H), 2.46 (q, J = 7.5 Hz, 2H), 5.05 (s, 2H), 5.69 (d, J = 0.7 Hz, 2H), 6.69 (dd, J = 4.8, 7.7 Hz, 1H), 7.65 (s, 1H), 7.83 (s, 1H), 7.98 (d, J = 0.7 Hz, 1H), 8.07 (dd, J =1.8, 7.7 Hz, 1H), 8.15 (dd, J = 1.8, 4.8 Hz, 1H)

Reference Example 22

5-(1-butynyl)-1-(4-fluorophenyl)-6-(3-iodopyridine-2-yloxy) methyl-1H-indazole (Compound A74)

Compound A62 (350 mg, 0.868 mmol) obtained in the Step 2 of Reference Example 13 was dissolved in N, N-dimethylacetamide (7.0mL). To this, 4-fluoroiodobenzene (200 μL, 1.74 mmol), ethylenediamine (232 μL, 3.47 mmol), potassium phosphate (737 mg, 3.47 mmol), and copper(I) iodide (33.0 mag, 0.174 mmol) were added at room temperature, and the mixture was stirred at 100°C for 5 hours. After the reaction mixture was cooled to room temperature, water was added and extraction with ethyl acetate was performed twice. The organic layer was washed with a saturated sodium chloride aqueous
solution and dried over anhydrous magnesium sulfate. After filtration and concentration under reduced pressure, the residue was purified by silica gel column chromatography to give Compound A74 (192 mg, yield: 45%).
ESI-MS: m/z 498 $[M + H]^+$; $^1H$ NMR ($CDCl_3$)δ(ppm): 1.27 (t, J = 7.5 Hz, 2H), 2.49 (q, J = 7.5 Hz, 2H), 5.69 (d, J = 1.1 Hz, 2H), 6.70 (dd, J = 4.8, 7.7 Hz, 1H), 7.17-7.25 (m, 2H), 7.70-7.78 (m, 2H), 7.88 (s, 1H), 8.06-8.11 (m, 2H), 8.12-8.17 (m, 2H).

Reference Example 23

(S)-5-(1-butynyl)-6-(3-iodopyridine-2-yloxy)methyl-1-(2-methylbutyl)-1H-indazole (Compound A75)

With use of Compound A62 (200 mg, 0.496 mmol) obtained in the Step 2 of Reference Example 13 and (S)-1-iodo-2-methylbutane (96.6 μL, 0.74 mmol), in the same manner as in the Step 3 of Reference Example 13, Compound A75 (134 mg, yield: 57%) was obtained.
ESI-MS: m/z 474 $[M + H]^+$; $^1H$ NMR ($CDCl_3$)δ(ppm): 8.86 (d, J = 6.6 Hz, 3H), 0.91 (t, J = 7.5 Hz, 3H), 1.12-1.24 (m, 1H),

1.25 (t, J = 7.5Hz, 3H), 1.34-1.47 (m, 1H), 2.07-2.20 (m, 1H), 2.47 (q, J = 7.5 Hz, 2H), 4.15 (dd, J = 7.9, 14.1 Hz, 1H), 4.29 (dd, J = 6.9, 14.1 Hz, 1H), 5.69 (d, J = 1.1 Hz, 2H), 6.70 (dd, J = 4.8, 7.5 Hz, 1H), 7.73 (s, 1H), 7.81 (s, 1H), 7.93 (d, J = 0.7 Hz, 1H), 8.09 (dd, J = 1.5, 7.5 Hz, 1H), 8.15 (dd, J = 1.5, 4.8 Hz, 1H).

Reference Example 24

1-benzyl-5-(1-butynyl)-6-(3-iodopyridine-2-yloxy)methyl-1H-indazole (Compound A76)

With use of Compound A62 (50.8 mg, 0.126 mmol) obtained in the Step 2 of Reference Example 13 and benzyl bromide (22.5 μL, 0.189 mmol), in the same manner as in the Step 3 of Reference Example 13, Compound A76 (25.1 mg, yield: 40%) was obtained. ESI-MS: m/z 494 $[M + H]^+$; $^1H$ NMR ($CDCl_3$)δ(ppm): 1.24 (t, J = 7.6 Hz, 3H), 2.45 (q, J = 7.5 Hz, 2H), 5.58 (s, 2H), 5.64 (d, J = 0.7 Hz, 2H), 6.67 (dd, J = 4.9, 7.5 Hz, 1H), 7.20-7.35 (m, 5H), 7.69 (s, 1H), 7.82 (s, 1H), 7.97 (d, J = 0.7 Hz, 1H), 8.06 (dd, J = 1.6, 7.5 Hz, 1H), 8.11 (dd, J = 1.6, 4.9 Hz, 1H).

Reference Example 25

5-(1-butynyl)-1-(4-fluorobenzyl)-6-(3-iodopyridine-2-yloxy)methyl-1H-indazole (Compound A77)

With use of Compound A62 (50.8 mg, 0.126 mmol) obtained in the Step 2 of Reference Example 13 and 4-fluorobenzyl bromide (23.5 μL, 0.189 mmol), in the same manner as in the Step 3 of Reference Example 13, Compound A77 (34.4 mg, yield: 53%) was obtained.
ESI-MS: m/z 512 $[M + H]^+$; $^1H$ NMR ($CDCl_3$)δ(ppm): 1.25 (t, J = 7.4 Hz, 3H), 2.46 (q, J = 7.4 Hz, 2H), 5.54 (s, 2H), 5.66 (d, J = 1.0 Hz, 2H), 6.69 (dd, J = 4.8, 7.4 Hz, 1H), 6.90-5.99 (m, 2H), 7.18-7.24 (m, 2H), 7.63 (s, 1H), 7.82 (s, 1H), 7.97 (d, J = 0.7 Hz, 1H), 8.07 (dd, J = 1.8, 7.4 Hz, 1H), 8.11 (dd, J = 1.8, 4.8 Hz, 1H).

Reference Example 26

5-(1-butynyl)-6-(3-iodopyridine-2-yloxy)methyl-1-(2-methoxybenzyl)-1H-indazol (Compound A78)

With use of Compound A62 (60.0 mg, 0.149 mmol) obtained in the Step 2 of Reference Example 13 and 2-methoxybenzyl chloride (31.1 μL, 0.223 mmol), in the same manner as in the Step 3 of Reference Example 13, Compound A78 (34.4 mg, yield: 44%) was obtained.
ESI-MS: m/z 524 $[M + H]^+$; $^1H$ NMR ($CDCl_3$)δ(ppm): 1.22 (t, J = 7.5 Hz, 3H), 2.44 (q, J = 7.5 Hz, 2H), 3.81 (s, 3H), 5.60 (s, 2H), 5.65 (d, J = 0.7 Hz, 2H), 6.67 (dd, J = 4.8, 7.4 Hz, 1H) 6.78-6.93 (m, 3H), 7.18-7.25 (m, 1H), 7.75 (s, 1H), 7.82 (s, 1H), 7.97 (d, J = 0.7 Hz, 1H), 8.05 (dd, J = 1.8, 7.4 Hz, 1H), 8.12 (dd, J = 1.8, 4.8 Hz, 1H).

Reference Example 27

5-(1-butynyl)-6-(3-iodopyridine-2-yloxy)methyl-1-(3-methoxybenzyl)-1H-indazole (Compound A79)

With use of Compound A62 (65.0 mg, 0.161 mmol) obtained in the Step 2 of Reference Example 13 and 3-methoxybenzyl chloride (32.6 μL, 0.242 mmol), in the same manner as in the Step 3 of Reference Example 13, Compound A79 (22.2 mg, yield: 26%) was obtained.
ESI-MS: m/z 524 $[M + H]^+$; $^1H$ NMR ($CDCl_3$)δ(ppm): 1.24 (t, J = 7.5 Hz, 3H), 2.46 (q, J = 7.5 Hz, 2H), 3.72 (s, 3H), 5.56 (s, 2H), 5.64 (d, J = 1.1Hz, 2H), 6.68 (dd, J = 4.8, 7.3 Hz, 1H), 6.74-6.83 (m, 3H), 7.15-7.21 (m, 1H), 7.70 (s, 1H), 7.82 (s, 1H), 7.98 (d, J = 1.1 Hz, 1H), 8.06 (dd, J = 1.8, 7.3 Hz, 1H), 8.12 (dd, J = 1.8, 4.8 Hz, 1H).

Reference Example 28

Step 1

5-chloromethyl-2-methoxypyridine (Compound A80)

2-methoxy-5-pyridinecarbaldehyde (137 mg, 0.999 mmol) was dissolved in methanol (5.0mL). To this, sodium borohydride (37.8 mg, 0.999 mmol) was added at 0˚C, and the mixture was stirred for 1 hour. To the reaction mixture, a saturated ammonium chloride aqueous solution was added, and extraction with ethyl acetate was performed twice. The organic layer was washed with a saturated sodium chloride aqueous solution and dried over anhydrous magnesium sulfate. After filtration and concentration under reduced pressure, the residue was dissolved in dichloromethane (5.0

mL). To this, triethylamine (278 μL, 2.00 mmol) and methanesulfonyl chloride (116 μL, 1.50 mmol) were added, and the mixture was stirred overnight. After a saturated aqueous solution of sodium hydrogen carbonate was added to the reaction mixture, extraction with chloroform was performed twice. The organic layer was dried over anhydrous magnesium sulfate. After filtration and concentration under reduced pressure, the residue was purified by silica gel column chromatography to give Compound A80 (116 mg, yield: 74%).
ESI-MS: m/z 158 [M + H]$^+$; $^1$H NMR ($CDCl_3$)δ(ppm): 3.94 (s, 3H), 4.55 (s, 2H), 6.76 (d, J = 8.4 Hz, 1H), 7.62 (dd, J = 2.4, 8.4 Hz, 1H), 8.15 (d, J = 2.4 Hz, 1H).

Step 2

5-(1-butynyl)-6-(3-iodopyridine-2-yloxy)methyl-1-(2-methoxypyridine-5-yl)methyl-1H-indazole (Compound A81)

With use of Compound A62 (61.0 mg, 0.151 mmol) obtained in the Step 2 of Reference Example 13 and Compound A80 (35.8 mg, 0.227 mmol), in the same manner as in the Step 3 of Reference Example 13, Compound A81 (49.9 mg, yield: 63%) was obtained. ESI-MS: m/z 525 [M + H]$^+$; $^1$H NMR ($CDCl_3$)δ(ppm): 1.25 (t, J = 7.5 Hz, 3H), 2.46 (q, J = 7.5 Hz, 2H), 3.89 (s, 3H), 5.50 (s, 2H), 5.65 (d, J = 1.1 Hz, 2H), 6.63 (d, J = 8.4 Hz, 1H), 6.69 (dd, J = 4.8, 7.3 Hz, 1H), 7.45 (dd, J = 2.4, 8.4 Hz, 1H), 7.72 (s, 1H), 7.81 (s, 1H), 7.96 (d, J = 0.7 Hz, 1H), 8.08 (dd, J = 1.8, 7.3 Hz, 1H), 8.13 (dd, J = 1.8, 4.8 Hz, 1H), 8.18 (d, J = 2.4 Hz, 1H).

Reference Example 29

5-(1-butynyl)-6-(3-iodopyridine-2-yloxy)methyl-1-(pyridine-3-yl)tnethyl-1H-indazole (Compound A82)

With use of Compound A62 (94.1 mg, 0.233 mmol) obtained in the Step 2 of Reference Example 13 and 3-bromomethylpyridine hydrochloride (88.5 mg, 0.350 mmol), in the same manner as in the Step 3 of Reference Example 13, Compound A82 (28.3 mg, yield: 25%) was obtained.
ESI-MS: m/z 495 [M + H]$^+$; $^1$H NMR ($CDCl_3$)δ(ppm): 1.25 (t, J = 7.5 Hz, 3H), 2.47 (q, J = 7.5 Hz, 2H), 5.60 (s, 2H), 5.65 (d, J = 1.0 Hz, 2H), 6.69 (dd, J = 5.0, 7.6 Hz, 1H), 7.19 (dd, J = 4.8, 7.4 Hz, 1H), 7.50 (d, J = 7.9 Hz, 1H), 7.70 (s, 1H), 7.83 (s, 1H), 7.99 (d, J = 1.0 Hz, 1H), 8.08 (dd, J = 1.8, 7.6 Hz, 1H), 8.13 (dd, J = 1.8, 5.0 Hz, 1H), 8.52 (s, 1H), 8.63 (s, 1H).

Reference Example 30

5-(1-butynyl)-1-(3-ethoxycarbonylphenyl)-6-(3-iodopyridine-2-yloxy)methyl-1H-indazole (Compound A83)

With use of Compound A62 (50.0 mg, 0.124 mmol) obtained in the Step 2 of Reference Example 13 and 3-iodoethyl benzoate (140 mg, 0.248 mmol), in the same manner as in Reference Example 22, Compound A83 (95.0 mg, yield: 46%) was obtained. ESI-MS: m/z 552 [M + H]$^+$; $^1$H NMR ($CDCl_3$)δ(ppm): 1.27 (t, J = 7.5 Hz, 3H), 1.41 (t, J = 7.1 Hz, 3H), 2.49 (q, J = 7.5 Hz, 2H), 4.42 (q, J = 7.1 Hz, 2H), 5.59 (s, 2H), 6.69 (dd, J = 4.8, 7.5 Hz, 1H), 7.60 (dd, J = 7.9, 7.9Hz, 1H), 7.90 (s, 1H), 7.95-8.00 (m, 1H), 8.02-8.08 (m, 2H), 8.14-8.18 (m, 3H), 8.44-8.46 (m, 1H).

Reference Example 31

5-(1-butynyl)-6-(3-iodopyridine-2-yloxy)methyl-1-(1-phenylethyl)-1H-indazole (Compound A84)

With use of Compound A62 (80.2 mg, 0.199 mmol) obtained in the Step 2 of Reference Example 13 and 1-chloroethylbenzene (39.2 μL, 0.298 mmol), in the same manner as in the Step 3 of Reference Example 13, Compound A84 (51.6 mg, yield: 51%) was obtained.
ESI-MS: m/z 508 [M + H]$^+$; $^1$H NMR ($CDCl_3$)δ(ppm): 1.23 (t, J = 7.5 Hz, 3H), 2.04 (d, J = 7.2Hz, 3H), 2.44 (q, J = 7.5 Hz, 2H), 5.53-5.68 (m, 2H), 5.82 (q, J = 7.2 Hz, 1H), 6.68 (dd, J = 4.8, 7.4 Hz, 1H), 7.15-7.28 (m, 5H), 7.63 (s, 1H), 7.80 (s, 1H), 8.00 (d, J = 0.7 Hz, 1H), 8.07 (dd, J = 1.8, 7.4 Hz, 1H), 8.10 (dd, J = 1.8, 4.8 Hz, 1H).

Reference Example 32

5-(1-butynyl)-1-(4-ethoxycarbonylphenyl)-6-(3-iodopyridine-2-yloxy)methyl-1H-indazole (Compound A85)

With use of Compound A62 (220 mg, 0.546 mmol) obtained in the Step 2 of Reference Example 13 and 4-iodoethyl benzoate (181 μL, 1.09 mmol), in the same manner as in Reference Example 22, Compound A85 (80.0 mg, yield: 27%) was obtained. ESI-MS: m/z 552 [M + H]$^+$; $^1$H NMR ($CDCl_3$)δ(ppm): 1.28 (t, J = 7.5Hz, 3H), 1.44 (t, J = 7.2

Hz, 3H), 2.50 (q, J = 7.5 Hz, 2H), 4.42 (q, J = 7.2 Hz, 2H), 5.70 (s, 2H), 6.71 (dd, J = 4.8, 7.6 Hz, 1H), 7.89-7.95 (m, 3H), 8.10 (dd, J = 1.8, 7.6 Hz, 1H), 8.14-8.26 (m, 5H).

Reference Example 33

5-(1-butynyl)-6-(3-iodopyridine-2-yloxy)methyl-1-phenyl-1H-indazole (Compound A86)

With use of Compound A62 (50.0 mg, 0.124 mmol) obtained in the Step 2 of Reference Example 13 and iodobenzene (27.8 μL, 0.248 mmol), in the same manner as in Reference Example 22, Compound A86 (17.0 mg, yield: 29%) was obtained.
ESI-MS: m/z 480 $[M + H]^+$

Reference Example 34

Step 1

4-[1-(methanesulfonyloxy)ethyl]tetrahydro-2H-pyrane (Compound A87)

Tetrahydro-2H-pyrane-4-carbaldehyde (571 mg, 5.00 mmol) was dissolved in THE (25 mL). To this, a 1.0 mol/L methylmagnesium bromide/THF solution (6.50 mL, 6.50 mmol) was added at 0˚C, and the mixture was stirred for 30 minutes. To the reaction mixture, 1 mol/L hydrochloric acid was added, and extraction with ethyl acetate was performed twice. The organic layer was washed with a saturated sodium chloride aqueous solution and dried over anhydrous magnesium sulfate. After filtration and concentration under reduced pressure, a part (193 mg) of the residue (825 mg) was dissolved in dichloromethane (5.0mL). To this, triethylamine (326 μL, 2.34 mmol) and methanesulfonyl chloride (118 μL, 1.52 mmol) were added at room temperature, and the mixture was stirred for 10 minutes. After a saturated aqueous solution of sodium hydrogen carbonate was added to the reaction mixture, extraction with ethyl acetate was performed twice. The organic layer was washed with a saturated sodium chloride aqueous solution and dried over anhydrous magnesium sulfate. After filtration and concentration under reduced pressure, the residue was purified by silica gel column chromatography to give Compound A87 (167 mg, yield: 69%).
$^1$H NMR ($CDCl_3$)δ(ppm): 1.42 (d, J = 6.3 Hz, 3H), 1.42-1.90 (m, 5H), 3.01 (s, 3H), 3.31-3.44 (m, 2H), 3.99-4.17 (m, 2H). 4.57-4.67 (m, 1H).

Step 2

5-(1-butynyl)-6-(3-iodopyridine-2-yloxy)methyl-1-[1-(tetrahydro-2H-pyrane-4-yl)ethyl]-1H-indazole (Compound A88)

With use of Compound A62 (100 mg, 0.248 mmol) obtained in the Step 2 of Reference Example 13 and Compound A87 (103 mg, 0.496 mmol), in the same manner as in the Step 3 of Reference Example 13, Compound A88 (47.6 mg, yield: 37%) was obtained. ESI-MS: m/z 516$[M + H]^+$; $^1$H NMR ($CDCl_3$)δ(ppm): 0.89-0.97 (m, 1H), 1.18-1.32 (m, 1H), 1.25 (t, J = 7.5 Hz, 3H), 1.36-1.50 (m, 1H), 1.61 (d, J = 6.6 Hz, 3H), 1.75-1.83 (m, 1H), 2.15-2.29 (m, 1H), 2.46 (q, J = 7.5 Hz, 2H), 3.22 (ddd, J = 2.2, 11,7, 11,7 Hz, 1H), 3.38 (ddd, J = 2.2, 11,7, 11,7 Hz, 1H), 3.79 (dd, J = 3.0, 11,7 Hz, 1H), 4.01 (dd, J = 3.0,11,7 Hz, 1H), 4.29-4.42 (m, 1H), 5.70 (d, J = 1.0 Hz, 2H), 6.71 (dd, J = 4.8, 7.3 Hz, 1H), 7.75 (s, 1H), 7.81 (s, 1H), 7.95 (s, 1H), 8.10 (dd, J = 1.6, 7.3 Hz, 1H), 8.16 (dd, J = 1.6, 4.8 Hz, 1H).

Reference Example 35

5-(1-butynyl)-1-(2-fluoropyridine-5-yl)-6-(3-iodopyridine-2-yloxy)methyl-1H-indazole (Compound A89)

With use of Compound A62 (150 mg, 0.372 mmol) obtained in the Step 2 of Reference Example 13 and 2-fluoro-5-iodopyridine (166 mg, 0.744 mmol), in the same manner as in Reference Example 22, Compound A89 (86.0mg, yield: 46%) was obtained.
ESI-MS: m/z 499 $[M + H]^+$; $^1$H NMR ($CDCl_3$)δ(ppm): 1.28 (t, J = 7.5 Hz, 3H), 2.50 (q, J = 7.5 Hz, 2H), 5.70 (s, 2H), 6.71 (dd, J = 4.8, 7.7 Hz, 1H), 7.11 (dd, J = 3.3, 8.4 Hz, 1H), 7.90 (s, 1H), 8.07-8.25 (m, 5H), 8.72 (s, 1H).

Reference Example 36

5-(1-butynyl)-1-(4-cyanophenyl)-6-(3-iodopyridine-2-yloxy)methyl-1H-indazole (Compound A90)

With use of Compound A62 obtained in the Step 2 of Reference Example 13 and 4-iodobenzonitrile, in the same manner as in Reference Example 22, Compound A90 was obtained.
ESI-MS: m/z 505 $[M + H]^+$; $^1H$ NMR ($CDCl_3$)δ(ppm): 1.29 (t, J = 7.5 Hz, 3H), 2.51 (q, J = 7.5 Hz, 2H), 5.72 2 (s, 2H), 6.73 (dd, J = 5.0, 7.6 Hz, 1H), 7.81 (d, J = 8.9 Hz, 2H), 7.89 (s, 1H), 7.99 (d, J = 8.9 Hz, 2H), 8.11 (dd, J = 1.7, 7.6Hz, 1H), 8.16 (dd, J = 1.7,5.0 Hz, 1H), 8.20 (d, J = 1.0 Hz, 1H), 8.23 (s, 1H).

Reference Example 37

5-(1-butynyl)-6-(3-iodopyridine-2-yloxy)methyl-1-(pyridine-3-yl)-1H-indazole (Compound A91)

With use of Compound A62 (100 mg, 0.248 mmol) obtained in the Step 2 of Reference Example 13 and 3-iodopyridine (102 mg, 0.496 mmol), in the same manner as in Reference Example 22, Compound A91 (86.0 mg, yield: 46%) was obtained.
ESI-MS: m/z 481 $[M + H]^+$; $^1H$ NMR ($CDCl_3$)δ(ppm): 1.28 (t, J = 7.6 Hz, 3H), 2.50 (q, J = 7.6 Hz, 2H), 5.70 (s, 2H), 6.71 (dd, J = 4.8, 7.7 Hz, 1H), 7.47 (dd, J = 4.8, 8.4 Hz, 1H), 7.90 (s, 1H), 8.07-8.21 (m, 5H), 8.61 (dd, J = 1.5, 4.8 Hz, 1H), 9.16 (d, J = 2.2 Hz, 1H).

Reference Example 38

5-(1-butynyl)-1-(2-fluorobenzyl)-6-(3-iodopyridine-2-yloxy)methyl-1H-indazole (Compound A92)

With use of Compound A62 (53.0 mg, 0.131 mmol) obtained in the Step 2 of Reference Example 13 and 2-fluorobenzyl bromide (23.8 μL, 0.197 mmol), in the same manner as in the Step 3 of Reference Example 13, Compound A92 (39.6 mg, yield: 59%) was obtained.
ESI-MS: m/z 512 $[M + H]^+$; $^1H$ NMR ($CDCl_3$)δ(ppm): 1.24 (t, J = 7.5 Hz, 3H), 2.45 (q, J = 7.5 Hz, 2H), 5.60 (s, 2H), 5.65 (d, J = 3.3 Hz, 2H), 6.68 (dd, J = 5.0, 7.6Hz, 1H), 6.95-7.08 (m, 3H), 7.18-7.30 (m, 1H), 7.74 (s, 1H), 7.82 (s, 1H), 7.99 (s, 1H), 8.07 (dd, J = 1.7, 7.6 Hz, 1H), 8.12 (dd, J = 1.7, 5.0 Hz, 1H).

Reference Example 39

5-(1-butynyl)-1-(3-fluorobenzyl)-6-(3-iodopyridine-2-yloxy)methyl-1H-indazole (Compound A93)

With use of Compound A62 (56.0 mg, 0.139 mmol) obtained in the Step 2 of Reference Example 13 and 3-fluorobenzyl bromide (25.6 μL, 0.208 mmol), in the same manner as in the Step 3 of Reference Example 13, Compound A93 (42.3 mg, yield: 60%) was obtained.
ESI-MS: m/z 512 $[M+ H]^+$; $^1H$ NMR ($CDCl_3$)δ(ppm): 1.25 (t, J = 7.5 Hz, 3H), 2.46 (q, J = 7.5 Hz, 2H), 5.57 (s, 2H), 5.65 (d, J = 1.0 Hz, 2H), 6.68 (dd, J = 5.0, 7.6 Hz, 1H), 6.87-7.03 (m, 3H), 7.18-7.28 (m, 1H), 7.65 (d, J = 1.0 Hz, 1H), 7.83 (s, 1H), 7.99 (d, J = 1.0 Hz, 1H), 8.06 (dd, J = 1.8, 7.6 Hz, 1H), 8.16 (dd, J = 1.7, 5.0 Hz, 1H).

Reference Example 40

5-(1-butynyl)-1-(4-chlorophenyl)-6-(3-iodopyridine-2-yloxy)methyl-1H-indazole (Compound A94)

With use of Compound A62 (100 mg, 0.248 mmol) obtained in the Step 2 of Reference Example 13 and 4-chloro iodopyridine (118 mg, 0.496 mmol), in the same manner as in Reference Example 22, Compound A94 (95.7 mg, yield: 75%) was obtained. ESI-MS: m/z 514 $[M + H]^+$; $^1H$ NMR ($CDCl_3$)δ(ppm): 1.27 (t, J = 7.5 Hz, 3H), 2.49 (q, J = 7.5 Hz, 2H), 5.70 (d, J = 1.0 Hz, 2H), 6.71 (dd, J = 4.8, 7.4 Hz, 1H), 7.45-7.53 (m, 2H), 7.72-7.79 (m, 2H), 7.88 (s, 1H), 8.07-8.17 (m, 4H).

Reference Example 41

5-(1-butynyl)-6-(3-iodopyridine-2-yloxy)methyl-1-(pyridine-2-yl)-1H-indazole (Compound A95)

With use of Compound A62 (100 mg, 0.248 mmol) obtained in the Step 2 of Reference Example 13 and 2-

iodopyridine (53.0 μL, 0.496 mmol), in the same manner as in Reference Example 22, Compound A95 (83.0 mg, yield: 70%) was obtained.
ESI-MS: m/z 481 [M + H]$^+$; $^1$H NMR ($CDCl_3$)δ(ppm): 1.24 (t, J = 7.6 Hz, 3H), 2.46 (q, J = 7.6 Hz, 2H), 5.75 (s, 2H), 6.69 (dd, J = 4.8, 7.4 Hz, 1H), 7.13-7.18 (m, 1H), 7.78-7.86 (m, 2H), 8.00-8.17 (m, 4H), 8.50-8.54 (m, 1H), 9.17 (s, 1H).

Reference Example 42

5-(1-butynyl)-1-(3-cyanophenyl)-6-(3-iodopyridine-2-yloxy)methyl-1H-indazole (Compound A96)

With use of Compound A62 (100 mg, 0.248 mmol) obtained in the Step 2 of Reference Example 13 and 3-iodobenzonitrile (113 mg, 0.496 mmol), in the same manner as in Reference Example 22, Compound A96 (111 mg, yield: 89%) was obtained.
ESI-MS: m/z 505 [M + H]$^+$; $^1$H NMR ($CDCl_3$)δ(ppm): 1.30 (t, J = 7.5 Hz, 3H), 2.52 (q, J = 7.5 Hz, 2H), 5.71 (d, J = 0.7 Hz, 2H), 6.72 (dd, J = 5.0, 7.6 Hz, 1H), 7.60-7.67 (m, 2H), 7.90 (s, 1H), 8.09-8.19 (m, 5H), 8.23 (s, 1H).

Reference Example 43

5-(1-butynyl)-6-(3-iodopyridine-2-yloxy)methyl-1-(3-methoxypheny)-1H-indazole (Compound A97)

With use of Compound A62 (100 mg, 0.248 mmol) obtained in the Step 2 of Reference Example 13 and 3-iodoanisole (59.0 μL, 0.496 mmol), in the same manner as in Reference Example 22, Compound A97 (76.0 mg, yield: 60%) was obtained.
ESI-MS: m/z 510 [M + H]$^+$; $^1$H NMR ($CDCl_3$)δ(ppm): 1.27 (t, J = 7.5 Hz, 3H), 2.49 (q, J = 7.5 Hz, 2H), 3.88 (s, 3H), 5.68 (d, J = 1.0 Hz, 2H), 6.70 (dd, J = 4.8, 7.6 Hz, 1H), 6.87-6.92 (m, 1H), 7.35-7.46 (m, 3H), 7.88 (s, 1H), 8.07 (dd, J = 1.8, 7.6 Hz, 1H), 8.14-8.17 (m, 2H), 8.23 (s, 1H).

Reference Example 44

Step 1

2-chloro-3-[(3-nitrophenyl)ethynyl]pyridine (Compound A98)

With use of 2-chloro-3-iodopyridine (300 mg, 1.25 mmol) and 1-ethynyl-3-nitrobenzene (221 mg, 1.50 mmol), in the same manner as in the Step 5 of Reference Example 11, Compound A98 (312 mg, yield: 96%) was obtained as a yellow solid substance. ESI-MSm/z:259 [M+H]$^+$; $^1$H-NMR (270MHz, $CDCl_3$) δ(ppm): 7.29 (dd, J = 7.9, 5.0 Hz, 1H), 7.59 (t, J = 7.9 Hz, 1H), 7.87-7.91 (m, 2H), 8.25 (ddd, J = 8.3, 2.3, 1.3 Hz, 1H), 8.39-8.43 (m, 2H).

Step 2

5-iodo-1-isopropyl-6-{3-[(3-nitrophenyl)ethynyl]pyridine-2-ylooy}methyl-1H-indazole (Compound A99)

With use of Compound A98 (344 mg, 1.09 mmol) and Compound A50 (172 mg, 0.545 mmol) obtained in the Step 6 of Reference Example 10, in the same manner as in the Step 8 of Reference Example 10, Compound A99 (255 mg, yield: 40%) was obtained as a light yellow solid substance.
ESI-MS m/z:539 [M+H]$^+$; $^1$H-NMR (300 MHz, $CDCl_3$) δ(ppm): 1.44 (d, J = 6.6 Hz, 6H), 4.61-4.70 (m, 1H), 5.62 (s, 2H), 6.99 (dd, J = 7.3, 5.1 Hz, 1H), 7.54 (t, J = 8.1 Hz, 1H), 7.77 (s, 1H), 7.84 (dd, J = 7.3, 2.0 Hz, 2H), 7.91 (s, 1H), 8.19-8.25 (m, 2H), 8.28 (s, 1H), 8.41 (t, J = 2.0 Hz, 1H).

Reference Example 45

Step 1

6-(tert-butyldimethylsilyloxy)methyl-1-isopropyl-5-(1-propynyl)-1H-indazole (Compound A100)

Under ice-cooling, a zinc (II) chloride THF solution (0.5 mol/L, 3.99 mL) and a 1-propynyl magnesium bromide THF solution (0.5 mol/L, 3.74 mL) were mixed, and the mixture was stirred at room temperature for 1.5 hours. After the precipitate was filtered off, tetrakis(triphenylphosphine)palladium (0) (144 mg, 0.125 mmol) and Compound A49 (536 mg, 1.25 mmol) obtained in the Step 5 of Reference Example 10 was added, and the mixture was stirred at 80˚C

overnight. After the mixture was cooled to room temperature, saturated sodium hydrogen carbonate aqueous solution was added thereto, and filtration with Celite was performed. The aqueous layer was extracted with ethyl acetate, and the organic layer was washed with a saturated sodium chloride aqueous solution and then dried over anhydrous magnesium sulfate. After the solvent was evaporated off under reduced pressure, the residue was purified by silica gel column chromatography (hexane/ethyl acetate = 100/0 to 85/15) to give Compound A100 (209 mg, yield: 49%).
ESI-MS m/z:343 $[M+H]^+$; $^1$H-NMR (270 MHz, $CDCl_3$) δ(ppm) : 0.17 (s, 6H), 1.01 (s, 9H), 1.59 (d, J = 6.9 Hz, 6H), 2.10 (s, 3H), 4.78-4.88 (m, 1H), 4.96 (d, J = 1.3 Hz, 2H), 7.59 (s, 1H), 7.74 (s, 1H), 7.92 (s, 1H).

Step 2

6-(3-iodopyridine-2-yloxy)methyl-1-isopropyl-5-(propynyl)-1H-indazole (Compound A101)

With use of Compound A100 (207 mg, 0.604 mmol), in the same manner as in the Steps 6 and 8 of Reference Example 10, Compound A101 (135 mg, 2-step yield: 54%) was obtained. ESI-MS m/z:432 $[M+H]^+$; $^1$H-NMR (300 MHz, $CDCl_3$) δ(ppm) : 1.61 (d, J = 6.6 Hz, 6H), 2.09 (s, 3H), 4.82-4.91 (m, 1H), 5.70 (s, 2H), 6.71 (dd, J = 7.4, 4.8 Hz, 1H), 7.81 (s, 2H), 7.94 (s, 1H), 8.10 (dd, J = 7.4, 1.7 Hz, 1H), 8.17 (dd, J = 4.8, 1.7 Hz, 1H).

Reference Example 46

6-(3-iodopyridine-2-yloxy)methyl-1-isopropyl-5-[4-(tetrahydro-2H-pyrane-2-yloxy)-1-butynyl]-1H-indazole (Compound A102)

With use of Compound A50 (200 mg, 0.633 mmol) obtained in the Step 6 of Reference Example 10 and 2-(3-butynyloxy)tetrahydro-2H-pyrane (0.198 mL, 1.27 mmol), in the same manner as in the Steps 7 and 8 of Reference Example 10, Compound A102 (88.9 mg, 2-step yield: 27%) was obtained as a yellow oil.
ESI-MS m/z:546 $[M+H]^+$; $^1$H-NMR (300MHz, $CDCl_3$) δ(ppm) : 1.50-1.88 (m, 6H), 1.60 (d, J = 6.6 Hz, 6H), 2.76 (t, J = 7.1 Hz, 2H), 3.50-3.57 (m, 1H), 3.61-3.69 (m, 1H), 3.88-3.97 (m, 2H), 4.71 (t, J = 3.5 Hz, 1H), 4.82-4.91 (m, 1H), 5.69 (s, 2H), 6.70 (dd, J = 7.5, 4.9 Hz, 1H), 7.79 (s, 1H), 7.82 (s, 1H), 7.95 (s, 1H), 8.09 (dd, J = 7.5, 1.6 Hz, 1H), 8.16 (dd, J = 4.9, 1.6 Hz, 1H). Reference Example 47

Step 1

(3-iodopyridine-2-yl)methanol (Compound A103)

Methyl-3-iodopicolinate produced by a method known from literature (Synth. Commun., 27, 1075-1086 (1997)) (230 mg, 0.874 mmol) and calcium chloride (243 mg, 2.19 mmol) were dissolved in methanol, and under ice-cooling, sodium borohydride (165 mg, 4.37 mmol) was slowly added. After 1.5-hour stirring at 0˚C, water was added to the reaction mixture, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 90/10 to 30/70) to give Compound A103 (160 mg, yield: 78%) as a white solid substance.
ESI-MS m/z:236 $[M+H]^+$; $^1$H-NMR (270 MHz, $CDCl_3$) δ(ppm) : 4.49 (t, J = 4.6 Hz, 1H), 4.66 (d, J = 4.6 Hz, 2H), 6.99-7.04 (m, 1H), 8.10 (dd, J = 7.8, 1.5 Hz, 1H), 8.54 (dd, J = 4.6, 1.3 Hz, 1H).

Step 2

5-iodo-1-isopropyl-6-{3-[(3-nitrophenyl)ethynyl]pyridine-2-yl}methoxy-1H-indazole (Compound A104)

With use of Compound A103 (600 mg, 2.55 mmol), in the same manner as in the Steps 5 and 6 of Reference Example 11, Compound A104 (849 mg, 2-step yield: 65%) was obtained as a yellow substance.
ESI-MSm/z:539 $[M+H]^+$; $^1$H-NNR (300 MHz, $CDCl_3$) δ(ppm) : 1.53 (s, 3H), 1.56 (s, 3H), 4.68-4.77 (m, 1H), 5.53 (s, 2H), 7.16 (s, 1H), 7.35 (dd, J = 7.8, 5.0 Hz, 1H), 7.46 (t, J = 7.8 Hz, 1H), 7.72 (d, J = 7.8 Hz, 1H), 7.80 (s, 1H), 7.92 (dd, J = 7.8, 1.6 Hz, 1H), 8.09 (s, 1H), 8.16-8.19 (m, 1H), 8.33-8.35 (m, 1H), 8.63 (dd, J = 5.0, 1.5 Hz, 1H).

Reference Example 48

Step 1

5-iodo-1-isopropyl-6-(tetrahydro-2H-pyrane-2-yloxy)-1H-indazole (Compound A105)

Compound A56 (1.74 g, 5.77 mmol) obtained in the Step 4 of Reference Example 11 was dissolved in dichloromethane (35 mL). To this, pyridinium p-toluene sulfonate (0.290 g, 1.16 mmol) was added, and 3, 4-dihydro-2H-pyrane (1.58 mL, 17.3 mmol) was added under ice-cooling. After the mixture was stirred at room temperature overnight, a saturated sodium hydrogen carbonate aqueous solution was added to the reaction mixture, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 100/0 to 75/25) to give Compound A105 (2.08 mg, yield: 93%) as a yellow paste.
ESI-MS m/z:387 $[M+H]^+$; $^1$H-NMR (300 MHz, $CDCl_3$) δ(ppm) : 1.53 (d, J = 6.6 Hz, 3H), 1.57 (d, J = 6.6 Hz, 3H), 1.64-1.96 (m, 4H), 2.02-2.08 (m, 1H), 2.14-2.27 (m, 1H), 3.62-3.68 (m, 1H), 3.90 (td, J = 11.2, 2.9 Hz, 1H), 4.68-4.77 (m, 1H), 5.62 (s, 1H), 7.10 (s, 1H), 7.83 (s, 1H), 8.13 (s, 1H).

Step 2

5-(1-butynyl)-1-isopropyl-6-(tetrahydro-2H-pyrane-2-yloxy)-1H-indazole (Compound A106)

With use of Compound A105 (2.08 g, 5.39 mmol), in the same manner as in the Step 1 of Reference Example 13, Compound A106 (1.30 g, yield: 77%) was obtained as a dark yellow oil. ESI-MS m/z:313 $[M+H]^+$; $^1$H-NMR (270 MHz, $CDCl_3$) δ(ppm) : 1.28 (d, J = 7.5 Hz, 3H), 1.54 (d, J = 7.5 Hz, 3H), 1.56 (d, J = 7.3 Hz, 3H), 1.61-2.17 (m, 6H), 2.48 (q, J = 7.5 Hz, 2H), 3.61-3.67 (m, 1H), 4.00 (td, J = 10.9, 3.1 Hz, 1H), 4.66-4.76 (m, 1H), 5.60 (t, J = 2.8 Hz, 1H), 7.06 (s, 1H), 7.72 (s, 1H), 7.86 (s, 1H).

Step 3

5-(1-butynyl)-1-isopropyl-1H-indazole-6-ol (Compound A107)

Compound A106 (1.30g, 4.17 mmol) was dissolved in methanol (26 mL). To this, concentrated hydrochloric acid (0.423 mL, 13.9 mmol) was added, and the mixture was stirred at room temperature for 2 hours. To the reaction mixture, a saturated sodium hydrogen carbonate aqueous solution was added, and the aqueous layer was extracted with ethyl acetate. The organic layer was washed with a saturated sodium chloride aqueous solution , dried over anhydrous magnesium sulfate, and then the solvent was evaporated off under reduced pressure to give Compound A107 (1.01 g, quantitative yield) as an orange solid substance.
ESI-MS m/z:229 $[M+H]^+$; $^1$H-NMR (300 MHz, $CDCl_3$) δ(ppm) : 1.29 (t, J = 7.5 Hz, 3H), 1.56 (d, J = 7.0 Hz, 6H), 2.52 (q, J = 7.5 Hz, 2H), 4.64-4.73 (m, 1H), 6.03 (s, 1H), 6.89 (s, 1H), 7.68 (s, 1H), 7.87 (s, 1H).

Step 4

5-(1-butynyl)-6-(3-iodopyridine-2-yl)methoxy-1-isopropyl-1H-indazole (Compound A108)

Compound A103 (770 mg, 3.28 mmol) obtained in the Step 1 of Reference Example 47 was dissolved in THF (7.7 mL). To this, triethylamine (0.594 mL, 4.26 mmol) was added, and then methanesulfonyl chloride (0.281 mL, 3.60 mmol) was added under ice-cooling. The mixture was stirred at 0˚C for 30 minutes, and filtered. To the filtrate, Compound A107 (1.00 g, 4.38 mmol) and THF (11 mL) were added. Sodium tert-butoxide (315 mg, 3.28 mmol) was added under ice-cooling, and the mixture was stirred at room temperature overnight. After water was added to the reaction mixture, the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 90/10 to 40/60) to give Compound A108 (1.35 g, yield: 92%) as a white solid substance. ESI-MS m/z:446 $[M+H]^+$; $^1$H-NMR (270 MHz, $CDCl_3$) δ(ppm) : 1.22 (t, J = 7.4 Hz, 3H), 1.55 (d, J = 6.6 Hz, 6H), 2.45 (q, J = 7.5 Hz, 2H), 4.67-4.77 (m, 1H), 5.42 (s, 2H), 6.97-7.02 (m, 2H), 7.72 (s, 1H), 7.84 (s, 1H), 8.18 (dd, J=7.9, 1.3 Hz, 1H), 8.59 (dd, J = 4.6, 1.7 Hz, 1H).

Step 5

(Z)-5-[1,2-bis(4,4,5,5-tetramethyl-1,3,2-dioxaborolane-2-yl)-1-butenyl]-6-(3-iodopyridine-2-yl)methoxy-1-isopropyl-1H-indazole (Compound A109)

Compound A108 (1.24 g, 2.78 mmol) was dissolved in DMF (27 . 8 mL) . To this, bis (pinacolato) diboron (2.83 g, 11.1 mmol) and tetrakis(triphenylphosphine)platinum(0) (277 mg, 0.223 mmol) were added, and the mixture was stirred at 80˚C overnight. Then, after cooling to room temperature, water was added thereto, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 90/10 to 40/60) to give Compound A109 (1.61 g, yield: 83%) as a slightly yellow amorphous.
ESI-MS m/z:700 $[M+H]^+$; $^1$H-NMR (300 MHz, $CDCl_3$) δ(ppm) : 0.96 (t, J = 7.7 Hz, 3H), 1.14 (br s, 6H), 1.18 (br s, 6H), 1.35 (s, 12H), 1.54 (d, J = 6.6 Hz, 6H), 2.13 (q, J = 7.5 Hz, 2H), 4.65-4.76 (m, 1H), 5.27 (s, 2H), 6.92-6.96 (m, 2H), 7.28 (s, 1H), 7.82 (s, 1H), 8.13 (dd, J = 8.1, 1.1 Hz, 1H), 8.52 (dd, J = 4.4, 1.1 Hz, 1H).

Step 6

(Z)-1-isopropyl-5-[1-(4,4,5,5-tetramethyl-1,3,2-dioxaborolane-2-yl)propylidene]-5,10-dihydro-1H-indazolo[6,5-b]pyrido [3,2-e]oxepin (Compound A110)

Compound A109 (50 mg, 0.072 mmol) was dissolved in 1,4-dioxane (7.2 mL). To this, tripotassium phosphate (45.5 mg, 0.215 mmol) and [1,1'-bis(diphenylphosphino)ferrocene]palladium(II) dichloride dichloromethane complex (1: 1) (11.7 mg, 0.014 mmol) was added, and the mixture was stirred at 100˚C for 6 hours. After cooling to room temperature, water was added thereto, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 85/15 to 40/60) to give Compound A110 (25.8 mg, yield: 81%) as a yellow paste.
ESI-MS m/z:446 $[M+H]^+$; $^1$H-NMR (300 MHz, $CDCl_3$) δ(ppm) : 1.05 (t, J = 7.5 Hz, 3H), 1.11 (s, 6H), 1.19 (s, 6H), 1.53-1.59 (m, 6H), 2.32-2.50 (m, 2H), 4.68-4.77 (m, 1H), 5.05 (d, J = 15.0 Hz, 1H), 5.73 (d, J = 15.0 Hz, 1H), 7.07-7.12 (m, 2H), 7.41 (s, 1H), 7.63 (dd, J = 7.5, 1.6 Hz, 1H), 7.91 (s, 1H), 8.40 (dd, J = 4.9, 1.6 Hz, 1H).

Reference Example 49

(E)-1-isopropyl-5-[1-(4,4,5,5-tetramethyl-1,3,2-dioxaborane-2-yl)propylidene]-5,11-dihydro-1H-indazolo[5,6-e]pyrido [2,3-b]oxepin (Compound A111)

With use of Compound A53 obtained in the Step 3 of Reference Example 13, in the same manner as in the Steps 5 and 6 of Reference Example 48, Compound A111 was obtained.
ESI-MS m/z: 446 $[M+H]^+$; $^1$H-NMR (270 MHz, $CDCl_3$) δ(ppm): 0.98 (t, *J* = 7.6 Hz, 3H), 1.14 (s, 6H), 1.24 (s, 6H), 1.57 (d, *J* = 6.6 Hz, 3H), 1.62 (d, *J* = 6.6 Hz, 3H), 2.22-2.42 (m, 2H), 4.81-4.91 (m, 1H), 5.02 (d, *J* = 12.2 Hz, 1H), 5.91 (d, *J* = 12.2 Hz, 1H), 6.77 (dd, *J* = 7.4, 4.8 Hz, 1H), 7.47 (s, 1H), 7.52 (s, 1H), 7.56 (dd, *J* = 7.4, 2.0 Hz, 1H), 7.98 (s, 1H), 8.07 (dd, *J* = 4.8, 2.0 Hz, 1H).

Reference Example 50

3-bromo-5-methanesulfonamidepyridine (Compound A112)

With use of 3-amino-5-bromopyridine (300 mg, 1.73 mmol), in the same manner as in Example 20, Compound A112 (131 mg, yield: 30%) was obtained.

ESI-MS: m/z 251 $[M + H]^+$; $^1$H NMR (DMSO)δ(ppm): 3.14 (s, 3H), 7.80 (dd, J = 2.2, 2.2 Hz, 1H), 8.41 (d, J = 2.2 Hz, 1H), 8.44 (d, J = 2.2 Hz, 1H), 10.28 (s, 1H).

Reference Example 51

(Z)-5-[1-bromo(propylidene)]-2-isopropyl-5,11-dihydro-2H-indazolo[5,6-e]benzo[b]oxepin (Compound A113)

With use of Compound A23 obtained in the Step 4 of Reference Example 4, in the same manner as in the Steps 10 and 11 of Reference Example 1, Compound A113 was obtained as a colorless crystal.
ESI-MS m/z: 397 $[M+H]^+$; $^1$H-NMR ($CDCl_3$)δ(ppm): 1.26-1.30 (m, 6H), 1.63 (d, J = 7.0 Hz, 3H), 2.78-2.87 (m, 1H), 4.09-4.14 (m, 1H), 4.70-4.84 (m, 1H), 4.91 (q, J = 12.1 Hz, 1H), 5.69 (d, J = 12.1 Hz, 1H), 6.74-6.89 (m, 2H), 7.09-7.14 (m, 2H), 7.55 (s, 1H), 7.75 (s, 1H), 7.94 (s, 1H).

Reference Example 52

1-(4-fluorophenyl)-5-bromomethylidene-5,11-dihydro-1H-indazolo[5,6-e]benzo[b]oxepin (Compound A114)

With use of Compound A14 obtained in the Step 2 of Reference Example 4 and methyltriphenylphosphonium bromide, in the same manner as in the Steps 10 and 11 of Reference Example 1, Compound A114 (159 mg, yield: 92%, E/Z = 89/11 or 11/89) was obtained as a light yellow amorphous.
ESI-MS m/z: 421, 423 $[M+H]^+$; $^1$H-NMR (270 MHz, $CDCl_3$) δ(ppm) : 4.93 (br s, 1.00H), 5.66 (br s, 1.00H), 6.57 (s, 0.11H), 6.79-6.83 (m, 1.00H), 6.86 (s, 0.89H), 6.87-6.98 (m, 1.00H), 7.17-7.31 (m, 3.11H), 7.62-7.72 (m, 3.89H), 7.85 (s, 1.00H), 8.18 (d, *J* = 1.0 Hz, 0.11H), 8.23 (d, *J* = 1.0 Hz, 0.89H).

Example 1

(E)-5-{1-[3-(methanesulfonamide)phenyl]propylidene}-1-methyl-5,11-dihydrobenzo[6,7]oxepino[4,3-f]indazole (Compound 1)

Compound A11 (79 mg, 0.213 mmol) obtained in the Step 11 of Reference Example 1, 3-(methanesulfonamide) phenylboronic acid (138 mg, 0.642 mmol), sodium carbonate (45 mg, 0. 428 mmol), triphenylphosphine (34 mg, 0.128 mmol), and palladium acetate (9.6 mg, 0.0428 mmol) were dissolved in 1,4-dioxane (2 mL) and water (1 mL), and the mixture was stirred at 70˚C for 7 hours. After water was added to the reaction mixture, extraction with ethyl acetate, washing with brine, and then drying over anhydrous sodium sulfate were performed. The solvent was evaporated off under reduced pressure, and the residue was purified by flash column chromatography (chloroform/methanol = 1/0 to 4/1) to give Compound 1 (32.0 mg, yield: 33%) as a colorless crystal.
ESI-MS m/z: 460 $[M+H]^+$; $^1$H-NMR ($CDCl_3$)δ(ppm) : 0.92 (t, *J* = 7.6 Hz, 3H), 2.29 (s, 3H), 2.57-2.71 (m, 1H), 2.91-3.05 (s, 1H), 4.00 (s, 3H), 5.04 (d, *J* = 12.2 Hz, 1H), 5.99 (d, *J* = 12.2 Hz, 1H), 6.16 (s, 1H), 6.76-6.84 (m, 3H), 6.86-6.92 (m, 1H), 6.96-6.99 (m, 1H), 7.03 (s, 1H), 7.12-7.20 (m, 2H), 7.25-7.28 (m, 2H), 7.38 (s, 1H).

Example 2

(Z)-5-{1-[3-(methanesulfonamide)phenyl]propylidene}-1-methyl-5,11-dihydrobenzo[6,7]oxepino[4,3-f]indazole

(Compound 2)

With use of Compound A12 (155 mg, 0.420 mmol) obtained in the Step 11 of Reference Example 1, in the same manner as in Example 1, Compound 2 (60.0 mg, yield: 31%) was obtained as a colorless crystal.
ESI-MS m/z: 460 $[M+H]^+$; $^1$H-NMP, ($CDCl_3$)δ(ppm): 0.71 (t, *J* = 7.6 Hz, 3H), 2.35-2.49 (m, 1H), 2.60 (s, 3H), 2.79-2.93 (m, 1H), 4.11 (s, 3H), 5.03 (d, *J* = 12.0 Hz, 1H), 6.05 (d, *J* = 12.0 Hz, 1H), 6.25 (s, 1H), 6.39-6.45 (m, 1H), 6.58-6.61 (m, 1H), 6.65-6.69 (m, 1H), 6.83-6.89 (m, 1H), 6.99-7.03 (m, 1H), 7.13-7.15 (m, 1H), 7.21-7.25 (m, 1H), 7.31 (t, *J* = 7.9 Hz, 1H), 7.50 (s, 1H), 7.67 (s, 1H), 7.99 (s, 1H).

Example 3

(E)-1-(4-fluorophenyl)-5-{1-[3-(methanesulfonamide)phenyl] propylidene}-5,11-dihydrobenzo[6,7]oxepino[4,3-f]indazole (Compound 3)

With use of Compound A15 (90.0 mg, 0.200 mmol) obtained in the Step 3 of Reference Example 12, in the same manner as in Example 1, Compound 3 (55.0 mg, yield: 51%) was obtained as a colorless crystal.
ESI-MS m/z: 540 $[M+H]^+$; $^1$H-NMR ($CDCl_3$)δ(ppm) : 0. 93 (t, *J* = 7.0 Hz, 3H), 2.32 (s, 3H), 2.62-2.71 (m, 1H), 2.93-3.04

(m, 1H), 5.00 (d, *J* = 12.5 Hz, 1H), 5.98 (d, *J* = 12.5 Hz, 1H), 6.08 (s, 1H), 6.81-6.83 (m, 3H), 6.89-7.02 (m, 2H), 7.11 (s, 1H), 7.13-7.30 (m, 4H), 7.58-7.63 (m, 2H), 7.65 (s, 1H), 7.88 (s, 1H).

Example 4

(Z)-1-(4-fluorophenyl)-5-{1-[3-(methanesulfonamide)phenyl] propylidene}-5,11-dihydrobenzo[6,7]oxepino[4,3-f]indazole (Compound 4)

With use of Compound A16 (105.0 mg, 0.234 mmol) obtained in the Step 3 of Reference Example 2, in the same manner as in Example 1, Compound 4 (82.5 mg, yield: 65%) was obtained as a colorless crystal.
ESI-MS m/z: 540 $[M+H]^+$; $^1$H-NMR ($CDCl_3$)δ(ppm) : 0. 73 (t, *J* = 7.3 Hz, 3H), 2.42-2.49 (m, 1H), 2.61 (s, 3H), 2.85-2.92 (m, 1H), 5.00 (d, *J* = 12.5 Hz, 1H), 6.05 (d, *J* = 12.5 Hz, 1H), 6.20 (s, 1H), 6.41-6.46 (m, 3H), 6.60-6.69 (m, 2H), 6.84-6.91 (m, 1H), 6.99-7.02 (m, 1H), 7.14-7.19 (m, 1H), 7.23-7.35 (m, 1H), 7.67-7.75 (m, 4H), 8.20 (s, 1H)

Example 5

(E)-1-(4-fluorophenyl)-5-{1-[3-(methanesulfonamide)phenyl] ethylidene}-5,11-dihydrobenzo[6,7]oxepino[4,3-f]indazole (Compound 5)

With use of Compound A17 (100 mg, 0.230 mmol) obtained in Reference Example 3, in the same manner as in Example 1, Compound 5 (12.3 mg, yield: 10%) was obtained as a slightly yellow crystal.
ESI-MS *m*/*z*: 526 $[M+H]^+$; $^1$H-NMR (DMSO-$D_6$)δ(ppm): 2.26 (s, 3H), 2.34 (s, 3H), 5.19 (d, *J* = 11.9 Hz, 1H), 5.85 (d, *J* = 11.9 Hz, 1H), 6.77-6.80 (m, 1H), 6.84-6.99 (m, 4H), 7.14-7.20 (m, 3H), 7.33-7.37 (m, 1H), 7.40-7.46 (m, 2H), 7.74-7.79 (m, 2H), 7.96 (s, 1H), 8.11-8.12 (m, 1H), 9.43 (br s, 1H).

Example 6

(Z)-1-(4-fluorophenyl)-5-{1-[3-(methanesulfonamide)phenyl] ethylidene}-5,11-dihydrobenzo[6,7]oxepino[4,3-f]indazole (Compound 6)

With use of Compound A18 (100 mg, 0.230 mmol) obtained in Reference Example 3, in the same manner as in Example 1, Compound 6 (8.8 mg, yield: 7%) was obtained as a slightly yellow crystal.
ESI-MS m/z: 526 $[M+H]^+$; $^1$H-NMR (DMSO-$D_6$)δ(ppm): 2.09 (s, 3H), 2.64 (s, 3H), 5.19 (d, *J* = 12.5 Hz, 1H), 5.89 (d, *J* = 12.5 Hz, 1H), 6.43-6.48 (m, 1H), 6.64-6.67 (m, 1H), 6.89-6.92 (m, 1H), 6.98-7.06 (m, 2H), 7.12-7.37 (m, 4H), 7.44-7.48 (m, 2H), 7.82-7.90 (m, 2H), 8.05 (s, 1H), 8.40 (s, 1H), 9.61 (s, 1H).

Example 7

(E)-5-{1-[3-(methanesulfonamide)phenyl]propylidene}-1-isopropyl-5,11-dihydro-1H-indazolo[5,6-e]benzo[b]oxepin (Compound 7)

With use of Compound A24 (80 mg, 0.201 mmol) obtained in the Step 5 of Reference Example 4, in the same manner as in Example 1, Compound 7 (41.0 mg, yield: 41%) was obtained as a colorless crystal.
ESI-MS m/z: 488 $[M+H]^+$; $^1$H-NMR ($CDCl_3$)δ(ppm): 0.91 (t, J = 7.5 Hz, 3H), 1.50 (d, J = 6.6 Hz, 3H), 1.56 (d, J = 6.6 Hz, 3H), 2.20 (s, 3H), 2.61-2.66 (m, 1H), 2.97-3.01 (m, 1H), 4.73-4.82 (m, 1H), 5.03 (d, J = 12.1 Hz, 1H), 5.99 (d, J = 12.1 Hz, 1H), 6.07 (s, 1H), 6.75 (t, J = 1.8 Hz, 1H), 6.82 (d, J = 8.1 Hz, 2H), 6.87-6.90 (m, 1H), 7.02-7.04 (m, 2H), 7.14-7.19 (m, 2H), 7.25-7.26 (m, 1H), 7.43 (s, 1H), 7.68 (s, 1H).

Example 8

(Z)-5-{1-[3-(methanesulfonamide)phenyl]propylidene}-1-isopropyl-5,11-dihydro-1H-indazolo[5,6-e]benzo[b]oxepin (Compound 8)

With use of Compound A25 (97.0 mg, 0.244 mmol) obtained in the Step 5 of Reference Example 4, in the same manner as in Example 1, Compound 8 (58.0 mg, yield: 49%) was obtained as a colorless crystal.
ESI-MS m/z: 488 $[M+H]^+$; $^1$H-NMR ($CDCl_3$)δ(ppm): 0.72 (t, J = 7.4 Hz, 3H), 1.59 (d, J = 6.6 Hz, 3H), 1.64 (d, J = 6.6 Hz, 3H), 2.43 (dd, J = 13.9, 7.6 Hz, 1H), 2.60 (s, 3H), 2.88 (dd, J = 7.3, 13.9 Hz, 1H), 4.83-4.92 (m, 1H), 5.02 (d, J = 12.2 Hz, 1H), 6.05 (d, J = 12.2 Hz, 1H), 6.16 (s, 1H), 6.39-6.43 (m, 1H), 6.60 (dd, J = 1.8, 7.8 Hz, 1H), 6.67 (dd, J = 1.3, 8.3

Hz, 1H), 6.99-7.02 (m, 1H), 7.13 (s, 1H), 7.25-7.32 (m, 3H), 7.54 (s, 1H), 7.65 (d, J = 3.6 Hz, 1H), 8.01 (s, 1H).

Example 9

(E)-1-cyclopentyl-5-{1-[3-(methanesulfonamide)phenyl] propylidene}-5,11-dihydro-1H-indazolo[5,6-e]benzo[b]oxepin (Compound 9)

With use of Compound A27 (60.0 mg, 0.141 mmol) obtained in the Step 2 of Reference Example 5, in the same manner as in Example 1, Compound 9 (64.0 mg, yield: 88%) was obtained as a colorless crystal.
ESI-MS m/z: 514 $[M+H]^+$; $^1$H-NMR ($CDCl_3$)δ(ppm): 0.91 (t, J = 7.4 Hz, 3H), 1. 72 (t, J = 6.9 Hz, 2H), 1.90-2.15 (m, 6H), 2.18 (s, 3H), 2.60-2.66 (m, 1H), 2.96-3.02 (m, 1H), 4.89-4.92 (m, 1H), 5.03 (d, J = 12.2 Hz, 1H), 5.99 (d, J = 12.2 Hz, 1H), 6.19 (s, 1H), 6.74 (t, J = 1.8 Hz, 1H), 6.80-6.91 (m, 3H), 7.01-7.04 (m, 2H), 7.11-7.28 (m, 3H), 7.44 (s, 1H), 7.66 (s, 1H).

Example 10

(Z)-1-cyclopentyl-5-{1-[3-(methanesulfonamide)phenyl] propylidene}-5,11-dihydro-1H-indazolo[5,6-e]benzo[b]oxepin (Compound 10)

With use of Compound A28 (100 mg, 0.236 mmol) obtained in the Step 2 of Reference Example 5, in the same manner as in Example 1, Compound 10 (57.0 mg, yield: 47%) was obtained as a colorless crystal.
ESI-MS m/z: 514 $[M+H]^+$; $^1$H-NMR ($CDCl_3$)δ(ppm): 1.26 (t, J = 7.1 Hz, 3H), 1.76-1.78 (m, 2H), 1.98-2.00 (m, 2H), 2.14-2.26 (m, 4H), 2.43 (t, J = 6.9 Hz, 1H), 2.60 (s, 3H), 2.85-2.87 (m, 1H), 5.00-5.03 (m, 2H), 6.05 (d, J = 11.9 Hz, 1H), 6.29 (s, 1H), 6.38-6.44 (m, 1H), 6.60 (dd, J = 7.8, 1.8 Hz, 1H), 6.67 (dd, J = 8.3, 1.0 Hz, 1H), 6.82-6.88 (m, 1H), 7.00-7.03 (m, 1H), 7.14 (t, J = 1.8 Hz, 1H), 7.25-7.31 (m, 2H), 7.55 (s, 1H), 7.65 (s, 1H), 7.99 (s, 1H).

Example 11

(E)-1-(2-fluoropyridine-5-yl)-5-{1-[3-(methanesulfonamide) phenyl]propylidene}-5,11-dihydro-1H-indazolo[5,6-e]benzo [b] oxepin (Compound 11)

With use of Compound A30 (50.0 mg, 0.111 mmol) obtained in the Step 2 of Reference Example 6, in the same manner as in Example 1, Compound 11 (32.5 mg, yield: 53%) was obtained as a colorless crystal.
ESI-MS m/z: 541 $[M+H]^+$; $^1$H-NMR ($CDCl_3$)δ(ppm): 0.73 (t, J = 7.4 Hz, 3H), 2.43-2.48 (m, 1H), 2.62 (s, 3H), 2.84-2.89 (m, 1H), 5.01 (d, J = 12.2 Hz, 1H), 6.06 (d, J = 12.2 Hz, 1H), 6.25 (s, 1H), 6.44 (t, J = 7.6 Hz, 1H), 6.61 (d, J = 8.3 Hz, 1H), 6.67 (d, J = 8.3 Hz, 1H), 6.88 (t, J = 7.8 Hz, 1H), 7.01 (d, J = 7.8 Hz, 1H), 7.18-7.31 (m, 4H), 7.78 (d, J = 3.0 Hz, 2H), 8.19 (t, J = 7.6 Hz, 1H), 8.26 (s, 1H), 8.66 (s, 1H).

Example 12

(Z)-1-(6-fluoropyridine-3-yl)-5-{1-[3-(methanesulfonamide) phenyl]propylidene}-5,11-dihydro-1H-indazolo[5,6-e]benzo [b] oxepin (Compound 12)

With use of Compound A31 (39.0 mg, 0.0866 mmol) obtained in the Step 2 of Reference Example 6, in the same manner as in Example 1, Compound 12 (31.9 mg, yield: 68%) was obtained as a colorless crystal.
ESI-MS m/z: 541 $[M+H]^+$; $^1$H-NMR ($CDCl_3$)δ(ppm): 0.94 (t, J = 7.4 Hz, 3H), 2.47 (s, 3H), 2.64-2.69 (m, 1H), 2.95-3.00 (m, 1H), 5.01 (d, J = 12.2 Hz, 1H), 5.98 (d, J = 12.2 Hz, 1H), 6.15 (s, 1H), 6.84-6.93 (m, 5H), 7.10-7.21 (m, 4H), 7.27-7.29 (m, 1H), 7.65 (s, 1H), 7.94 (d, J = 1.0 Hz, 1H), 8.09-8.12 (m, 1H), 8.58 (s, 1H).

Example 13

(Z)-1-(4-flulorophenyl)-5-{1-[3-(methanesulfonamide) phenyl]propylidene}-5,10-dihydro-1H-indazolo[6,5-b]pyrido [3,2-e]oxepin (Compound 13)

With use of Compound A40 (46 mg, 0.102 mmol) obtained in the Step 9 of Reference Example 7, in the same manner as in Example 1, Compound 13 (20.0 mg, yield: 36%) was obtained as a light yellow amorphous.
ESI-MS m/z:541 $[M+H]^+$; $^1$H-NMR (300 MHz, DMSO) δ(ppm) : 0.77 (t, J = 7.5 Hz, 3H), 2.46-2.67 (m, 2H), 2.48 (s, 3H), 5.07 (d, J = 14.3 Hz, 1H), 5.85 (d, J = 14.3 Hz, 1H), 6.94 (dd, J = 7.7, 2.0 Hz, 1H), 7.05 (d, J = 7.7 Hz, 1H), 7.11 (s, 1H), 7.14 (s, 1H), 7.22 (t, J = 7.7 Hz, 1H), 7.27 (s, 1H), 7.35-7.45 (m, 3H), 7.68-7.72 (m, 2H), 7.90 (dd, J = 7.7, 1.5 Hz, 1H),

8.00 (s, 1H), 8.51 (dd, J = 4.6, 1.3 Hz, 1H), 9.54 (br s, 1H).

Example 14

(E)-1-(4-fluorophenyl)-5-{1-[3-(methanesulfonamide)phenyl] propylidene}-5,10-dihydro-1H-indazolo[6,5-b]pyrido [3,2-e] oxepin (Compound 14)

With use of Compound A41 (43 mg, 0.095 mmol) obtained in the Step 9 of Reference Example 7, in the same manner as in Example 1, Compound 14 (20.3 mg, yield: 39%) was obtained as a white solid substance.
ESI-MS m/z:541 $[M+H]^+$; $^1$H-NMR (270 MHz, DMSO) δ(ppm) : 0.77 (t, J = 7.4 Hz, 3H), 2.46-2.59 (m, 1H), 2.68 (s, 3H), 2.71-2.85 (m, 1H), 5.06 (d, J = 15.1 Hz, 1H), 5.73 (d, J = 15.1 Hz, 1H), 6.92-7.10 (m, 5H), 7.24 (t, J = 7.7 Hz, 1H), 7.39-7.46 (m, 2H), 7.51 (s, 1H), 7.79-7.86 (m, 3H), 8.23 (dd, J = 4.8, 1.7 Hz, 1H), 8.34 (d, J = 0.8 Hz, 1H), 9.60 (br s, 1H).

Example 15

(Z)-1-(4-fluorophenyl)-5-{1-[3-(methanesulfonamide)phenyl] propylidene}-5,10-dihydro-1H-indazolo[6,5-b]benzo[e]oxepin (Compound 15)

(E)-1-(4-fluorophenyl)-5-{1-[3-(methanesulfonamide)phenyl] propylidene}-5,10-dihydro-1H-indazolo[6,5-b]benzo[e]oxepin (Compound 16)

After Compound A43 (445 mg, 0.990 mmol) obtained in the Step 2 of Reference Example 8 was subjected to the same reaction as in Example 1, purification by reversed phase preparative HPLC (ammonium bicarbonate buffer solution (pH 8.6) /acetonitrile = 35/65)] to give Compound 15 (25.8 mg, yield: 5%) as a white solid substance and Compound 16 (19.1 mg, yield: 4%) as a light yellow amorphous.
Compound 15; ESI-MS m/z:540 $[M+H]^+$; $^1$H-NMR (300 MHz, DMSO) δ(ppm) : 0.70 (t, J = 7.3 Hz, 3H), 2.39-2.50 (m, 1H), 2.48 (s, 3H), 2.62-2.73 (m, 1H), 5.10 (d, J = 12.5 Hz, 1H), 5.86 (d, J = 12.5 Hz, 1H), 6.95-6.98 (m, 1H), 7.00 (s, 1H), 7.10 (s, 1H), 7.12-7.14 (m, 1H), 7.20-7.27 (m, 2H), 7.32-7.41 (m, 3H), 7.44-7.45 (m, 2H), 7.54 (d, J = 7.0 Hz, 1H), 7.63-7.67 (m, 2H), 7.93 (s, 1H), 9.54 (br s, 1H).
Compound 16; ESI-MS m/z:540 $[M+H]^+$; $^1$H-NMR (300 MHz, DMSO) δ(ppm) : 0.85 (t, J = 7.3 Hz, 3H), 2.55-2.67 (m, 1H), 2.62 (s, 3H), 2.78-2.90 (m, 1H), 5.07 (d, J = 12.7 Hz, 1H), 5.80 (d, J = 12.7 Hz, 1H), 6.74 (d, J = 7.3 Hz, 1H), 6.92-7.02 (m, 4H), 7.09 (t, J = 7.3 Hz, 1H), 7.16-7.22 (m, 2H), 7.33-7.42 (m, 3H), 7.73-7.78 (m, 2H), 7.81 (s, 1H), 8.29 (s, 1H), 9.53 (br s, 1H).

Example 16

(Z)-1-(4-methoxybenzyl)-5-{1-[3-(methanesulfonamide)phenyl] propylidene}-5,11-dihydro-1H-indazolo[5,6-e]benzo[b] oxepin (Compound 17)

With use of Compound A44 (160 mg, 0.337 mmol) obtained in Reference Example 9, in the same manner as in Example 1, Compound 17 (165 mg, yield: 87%) was obtained as a colorless crystal.
ESI-MSm/z:566 $[M+H]^+$; $^1$H-NMR ($CDCl_3$)δ(ppm) :1.26 (t, J = 7.1 Hz, 3H), 2.39-2.44 (m, 1H), 2.60 (s, 3H), 2.82-2.87 (m, 1H), 3.77 (s, 3H), 4.96 (d, J = 12.2 Hz, 1H), 5.54 (s, 2H), 6.00 (d, J = 12.2 Hz, 1H), 6.30 (s, 1H), 6.38-6.44 (m, 1H), 6.57-6.73 (m, 2H), 6.81-6.85 (m, 3H), 7.00-7.02 (m, 1H), 7.12 (s, 1H), 7.17-7.33 (m, 4H), 7.44 (s, 1H), 7.66 (s, 1H), 8.03 (s, 1H).

Example 17

(Z)-5-{1-[3-(methanesulfonamide)phenyl]propylidene}-5,11-dihydro-1H-indazolo[5,6-e]benzo[b]oxepin (Compound 18)

Compound 17 (85.0 mg, 0.150 mmol) was dissolved in trifluoroacetic acid (0.5 mL). To this, anisole (0.0527 mL, 0.467 mmol) and trifluoromethanesulfonic acid (0.0413 mL, 0.467 mmol) were added, and the mixture was stirred at 90˚C for 3 hours. After water was added to the reaction mixture, extraction with ethyl acetate, washing with brine, and then drying over anhydrous sodium sulfate were performed. The solvent was evaporated off under reduced pressure, and the residue was purified by flash column chromatography (chloroform/methanol = 1/0 to 90/10) to give Compound 18 (33.0 mg, yield: 47%) as a colorless crystal.
ESI-MS m/z: 446 $[M+H]^+$; $^1$H-NMP, ($CDCl_3$)δ(ppm): 1.17 (t, J = 7.1 Hz, 3H), 2.39-2.44 (m, 1H), 2.84 (s, 3H), 2.82-2.87 (m, 1H), 4.98 (br s, 2H), 6.66 (d, J = 8.1 Hz, 1H), 6.77 (d, J = 7.3 Hz, 1H), 6.87-6.90 (m, 5H), 7.33 (d, J = 8.1 Hz, 1H),

7.52 (s, 1H), 7.72 (s, 1H), 7.97 (s, 1H), 9.40 (s, 1H), 9.50 (br s, 1H).

Example 18

(Z)-1-isopropyl-5-[1-(3-nitrophenyl)butylidene]-5,11-dihydro-1H-indazolo[6,5-b]pyrido[3,2-e]oxepin (Compound 19)

Compound A52 (100 mg, 0.218 mmol) obtained in the Step 8 of Reference Example 10, 3-nitrophenyl boronic acid (44.0 mg, 0.261 mmol), palladium acetate (10.0 mg, 0.0435 mmol), and sodium carbonate (69.0 mg, 0.653 mmol) were dissolved in dioxane (3.0 mL) and water (0.75 mL), and the mixture was stirred at 100˚C for 2 hours. After the reaction mixture was filtered through a Celite pad, water was added and extraction with ethyl acetate, washing with brine, and then drying over anhydrous sodium sulfate were performed. The solvent was evaporated off under reduced pressure, and the residue was purified by flash silica gel column chromatography (hexane/ethyl acetate = 9/1 to 1/1) to give Compound 19 (41.5 mg, yield: 42%). ESI-MS m/z: 455 $[M+H]^+$; $^1$H-NMR ($CDCl_3$)δ(ppm): 0.65 (t, J = 7.3 Hz, 3H), 1.21-1.31 (m, 2H), 1.59 (d, J = 6.6 Hz, 3H), 1.66 (d, J = 6.6Hz, 3H), 2.50-2.59 (m, 1H), 2.87-2.92 (m, 1H), 4.85-4.94 (m, 1H), 5.19 (d, J = 12.1 Hz, 1H), 6.09 (d, J = 12.1 Hz, 1H), 6.44 (dd, J = 4.8, 7.7 Hz, 1H), 6.87 (dd, J = 2.0, 7.5 Hz, 1H), 7.38 (t, J = 7.9 Hz, 1H), 7.52-7.55 (m, 1H), 7.59 (s, 1H), 7.66 (s, 1H), 7.93 (dd, J = 2.0, 4.6 Hz, 1H), 8.04-8.08 (m, 2H), 8.22 (t, J = 2.0 Hz, 1H).

Example 19

(Z)-5-[1-(3-aminophenyl)butylidene]-1-isopropyl-5,11-dihydro-1H-indazolo[6,5-b]pyrido[3,2-e]oxepin (Compound 20)

Compound 19 (200 mg, 0.440 mmol) was dissolved in ethanol (4.0 mL) and water (2.0 mL). To this, iron (0.299 g, 2.20 mmol) and ammonium chloride (12.0 mg, 0.0881 mmol) were added, and the mixture was stirred at 75˚C for 1 hour. After
Celite-filtration of the reaction mixture and concentration of the filtrate under reduced pressure, the residue was purified by flash silica gel column chromatography (hexane/ethyl acetate = 6/4 to 1/9) to give Compound 20 (101 mg, yield: 54%). ESI-MS m/z: 425 $[M+H]^+$; $^1$H-NMR ($CDCl_3$)δ(ppm): 0.63 (t, J = 7.3 Hz, 3H), 1.06-1.13 (m, 2H), 1.58 (d, J = 6.6 Hz, 3H), 1.65 (d, J = 7.0 Hz, 3H), 2.39-2.48 (m, 1H), 2.75-2.80 (m, 1H), 3.58 (br s, 2H), 4.84-4.92 (m, 1H), 5.13 (d, J = 12.1 Hz, 1H), 6.10 (d, J = 12.1 Hz, 1H), 6.45-6.52 (m, 2H), 6.58-6.59 (m, 1H), 6.65-6.68 (m, 1H), 7.00-7.07 (m, 2H), 7.56 (s, 1H), 7.64 (s, 1H), 7.90 (dd, J = 1.8, 4.8 Hz, 1H), 8.01 (s, 1H).

Example 20

(Z)-1-isopropyl-5-{1-[3-(methanesulfonamide)phenyl] butylidene}-3,11-dihydro-1H-indazolo[6,5-b]pyrido[3,2-e] oxepin (Compound 21)

Compound 20 (95 mg, 0.224 mmol) was dissolved in dichloromethane (2.0 mL). To this, pyridine (0.0260 mL, 0.336 mmol) and methanesulfonyl chloride (0.0260 mL, 0.336 mmol) were added, and the mixture was stirred at room temperature for 1 hour. After water was added to the reaction mixture, extraction with ethyl acetate was performed. After washing with brine, drying over anhydrous sodium sulfate was performed. The solvent was evaporated off under reduced pressure, and the residue was subjected to slurry purification with use of ethyl acetate to give Compound 21 (92.4 mg, yield: 82%).
ESI-MS m/z: 503 $[M+H]^+$; $^1$H-NMR (DMSO-$d_6$)δ(ppm): 0.56 (t, J = 7.3 Hz, 3H), 0.96-1.24 (m, 2H), 1.49 (d, J = 6.6 Hz, 3H), 1.55 (d, J = 6.6 Hz, 3H), 2.45-2.54 (m, 2H), 2.66-2.70 (m, 1H), 2.71 (s, 3H), 4.98-5.06 (m, 1H), 5.24 (d, J = 12.1 Hz, 1H), 5.98 (d, J = 12.1 Hz, 1H), 6.56 (dd, J = 4.6, 7.5 Hz, 1H), 6.98 (dd, J = 2.0, 7.5 Hz, 1H), 7.02-7.05 (m, 1H), 7.14 (d, J = 7.7 Hz, 1H), 7.19-7.22 (m, 1H), 7.28 (t, J = 7.7 Hz, 1H), 7.84 (dd, J = 2.0, 4.8 Hz, 1H), 8.01 (s, 1H), 8.11 (s, 1H), 9.60 (s, 1H).

Example 21

Step 1

(E)-1-isopropyl-5-[1-(3-nitrophenyl)ethylidene]-5,10-dihydro-1H-indazolo[6,5-b]benzo[e]oxepin (Compound C1)

Compound A58 (20 mg, 0.037 mmol) obtained in the Step 6 of Reference Example 11,
[1,1'-bis(diphenylphosphino)ferrocene]palladium(II) dichloride dichloromethane complex (1:1) (6.1 mg, 0.00744 mmol), and cesium carbonate (36.4 mg, 0.112 mmol) were dissolved in 1,4-dioxane (1.19 mL) and water (0.30 mL). To this,

methyl boronic acid (3.3 mg, 0.056 mmol) was added, and the mixture was stirred at 100°C for 5 hours. After the reaction mixture was cooled to room temperature, the solvent was evaporated off, and purification by silica gel column chromatography (hexane/ethyl acetate = 90/10 to 50/50) was performed to give Compound C1 (10.7 mg, yield: 68%) as a light yellow paste.
ESI-MS m/z:426 $[M+H]^+$; $^1$H-NMR (300 MHz, $CDCl_3$) δ(ppm) : 1.52 (d, J = 7.0 Hz, 3H), 1.58 (d, J = 7.0 Hz, 3H), 2.36 (s, 3H), 4.65-4.74 (m, 1H), 5.04 (d, J = 13.0 Hz, 1H), 5.87 (d, J = 13.0 Hz, 1H), 6.64-6.67 (m, 1H), 6.90-6.96 (m, 2H), 7.11 (td, J = 7.5, 1.3 Hz, 1H), 7.25-7.34 (m, 3H), 7.62 (s, 1H), 7.92 (s, 1H), 7.95-7.99 (m, 2H).

Step 2

(E)-5-[1-(3-aminophenyl)ethylidene]-1-isopropyl-5,10-dihydro-1H-indazolo[6,5-b]benzo[e]oxepin (Compound C2)

Compound C1 (95 mg, 0.223 mmol) was dissolved in ethanol (2 mL). To this, 10% palladium/carbon (19.0 mg) was added, and the mixture was stirred under hydrogen atmosphere at room temperature for 6 hours. After Celite-filtration of the reaction mixture and concentration of the filtrate under reduced pressure, purification by silica gel column chromatography (hexane/ethyl acetate = 90/10 to 40/60) was performed to give Compound C2 (47.3 mg, yield: 54%) as a light yellow amorphous.
ESI-MS m/z:396 $[M+H]^+$; $^1$H-NMR (300 MHz, $CDCl_3$) δ (ppm) : 1.51 (d, J = 6.6 Hz, 3H), 1.57 (d, J = 7.0 Hz, 3H), 2.28 (s, 3H), 3.49 (br s, 2H), 4.63-4.72 (m, 1H), 4.97 (d, J = 12.5 Hz, 1H), 5.87 (d, J = 12.5 Hz, 1H), 6.39-6.45 (m, 3H), 6.79-6.82 (m, 1H), 6.89-7.00 (m, 3H), 7.08 (td, J = 7.4, 1.3 Hz, 1H), 7.21-7.24 (m, 1H), 7.59 (s, 1H), 7.89 (s, 1H).

Step 3

(E)-1-isopropyl-5-{1-[3-(methanesulfonamide)phenyl] ethylidene}-5,10-dihydro-1H-indazolo[6,5-b]benzo[e]oxepin (Compound 22)

With use of Compound C2 (45 mg, 0.114 mmol), in the same way as in Example 20, Compound 22 (51.8 mg, yield: 87%) was obtained as a light pink paste.
ESI-MS m/z:474 $[M+H]^+$; $^1$H-NMR (300 MHz, DMSO) δ(ppm) : 1.38 (d, J = 6.6 Hz, 3H), 1.46 (d, J = 6.6 Hz, 3H), 2.23 (s, 3H), 2.60 (s, 3H), 4.77-4.85 (m, 1H), 5.05 (d, J = 12.8 Hz, 1H), 5.76 (d, J = 12.8 Hz, 1H), 6.70 (d, J = 7.7 Hz, 1H), 6.92-7.00 (m, 4H), 7.06-7.20 (m, 3H), 7.34 (dd, J = 7.3, 1.1 Hz, 1H), 7.67 (s, 1H), 7.96 (s, 1H), 9.52 (br s, 1H).

Example 22

Step 1

(E)-1-isopropyl-5-[1-(3-nitrophenyl)-2-propenylidene]-5,10-dihydro-1H-indazolo[6,5-b]benzo[e]oxepin (Compound C3)

Compound A58 (130 mg, 0.242 mmol) obtained in the Step 6 of Reference Example 11, and [1,1'-bis(diphenylphosphino)ferrocene]palladium(II) dichloride dichloromethane complex (1:1) (39.5 mg, 0.048 mmol) were dissolved in 1,4-dioxane (9.7 mL). To this, tributyl(vinyl)tin (0.213 mL, 0.726 mmol) was added, and the mixture was stirred at 100°C for 3 hours. After the reaction mixture was cooled to room temperature, the solvent was evaporated off under reduced pressure and the residue was purified by silica gel column chromatography (hexane/ethyl acetate = 90/10 to 50/50) to give Compound C3 (83.4 mg, yield: 79%) as a light yellow paste.
ESI-MS m/z:438 $[M+H]^+$; $^1$H-NMR (300 MHz, $CDCl_3$) δ(ppm) : 1.52 (d, J = 7.0 Hz, 3H), 1.58 (d, J = 6.6 Hz, 3H), 4.64-4.73 (m, 1H), 4.84 (dd, J = 17.2, 0.7 Hz, 1H), 4.97 (d, J = 13.0 Hz, 1H), 5.27 (dd, J = 10.6, 0.7 Hz, 1H), 5.80 (d, J = 13.0 Hz, 1H), 6.67 (d, J = 7.3 Hz, 1H), 6.90-6.95 (m, 2H), 7.07 (t, J = 7.0 Hz, 1H), 7.22 (br s, 1H), 7.25 (br s, 1H), 7.38-7.40 (m, 2H), 7.68 (s, 1H), 7.94 (s, 1H), 7.97 (br s, 1H), 8.03-8.07 (m, 1H).

Step 2

(E)-1-isopropyl-5-{1-[3-(methanesulfonamide)phenyl] propylidene}-5,10-dihydro-1H-indazolo[6,5-b]benzo[e]oxepin (Compound 23)

Compound C3 (95 mg, 0.217 mmol) was dissolved in a 2 mol/L hydrochloric acid/ethanol solution (2 mL). To this, palladium/carbon (9.5 mg) was added, and the mixture was stirred under hydrogen atmosphere overnight. After Celite-filtration of the reaction mixture, the organic layer was washed with a saturated sodium hydrogen carbonate aqueous solution and a saturated sodium chloride aqueous solution, dried over anhydrous magnesium sulfate, and then

the solvent was evaporated off under reduced pressure. The residue was purified by silica gel column chromatography (hexane/ethyl acetate = 100/0 to 60/40). The roughly purified product was dissolved in dichloromethane (1 mL). To this, pyridine (0.015 mL, 0.183mmol) was added, and then methanesulfonyl chloride (0.011 mL, 0.147 mmol) was added under ice-cooling. After the mixture was stirred at room temperature for 2.5 hours, water and a saturated sodium hydrogen carbonate aqueous solution were added thereto, and the aqueous layer was extracted with ethyl acetate. The organic layer was washed with a saturated sodium chloride aqueous solution and dried over anhydrous magnesium sulfate. Then, the solvent was evaporated off under reduced pressure. The residue was purified by semipreparative high-performance liquid chromatography to give Compound 23 (16.8 mg, yield: 16%) as a light yellow solid substance. ESI-MS m/z:488 $[M+H]^+$; $^1$H-NMR (270 MHz, DMSO) δ(ppm) : 0.82 (t, J = 7.4 Hz, 3H), 1.38 (d, J = 6.6 Hz, 3H), 1.45 (d, J = 6.6 Hz, 3H), 2.55-2.64 (m, 1H), 2.60 (s, 3H), 2.75-2.86 (m, 1H), 4.76-4.86 (m, 1H), 5.04 (d, J = 12.7 Hz, 1H), 5.76 (d, J = 12.7 Hz, 1H), 6.70 (d, J = 7.6 Hz, 1H), 6.90-6.99 (m, 4H), 7.03-7.10 (m, 2H), 7.15-7.21 (m, 1H), 7.27-7.33 (m, 1H), 7.64 (s, 1H), 7.96 (s, 1H), 9.51 (br s, 1H).

Example 23

Step 1

(Z)-1-(2,4-dimethoxybenzyl)-5-[1-(3-nitrophenyl) propylidene]-5,11-dihydro-1H-indazolo[5,6-e]pyrido[2,3-b]-oxepin (Compound C4)

With use of Compound A60 (2.86 g, 5.17 mmol) obtained in the Step 2 of Reference Example 12, in the same manner as in Example 18, Compound C4 (1.15 g, yield: 41%) was obtained. ESI-MS: m/z 549 $[M + H]^+$; $^1$H NMR ($CDCl_3$) δ(ppm): 0.73 (t, J = 7.5 Hz, 3H), 2.42-2.56 (m, 1H), 2.86-3.00 (m, 1H), 3.77 (s, 3H), 3.86 (s, 3H), 5.14 (d, J = 12.2 Hz, 1H), 5.57 (d, J = 2.2 Hz, 2H), 6.04 (d, J = 12.2 Hz, 1H), 6.35-6.47 (m, 3H), 6.88 (dd, J = 2.0, 7.5 Hz, 1H), 7.00 (d, J = 8.4 Hz, 1H), 7.38 (dd, J = 7.9, 7.9 Hz, 1H), 7.52 (d, J = 7.7 Hz, 1H), 7.62 (s, 1H), 7.66 (s, 1H), 7.92 (dd, J = 2.0, 4.6 Hz, 1H), 8.04-8.08 (m, 2H), 8.20-8.23 (m, 1H).

Step 2

(Z)-1-(2,4-dimethoxybenzyl)-5-{1-[3-(methanesulfonamide) phenyl]propylidene}-5,11-dihydro-1H-indazolo[5,6-e]pyrido-[2,3-b]oxepin (Compound 24)

With use of Compound C4 (100 mg, 0.182 mmol), in the same manner as in the Examples 19 and 20, Compound 24 (80.4 mg, 2-step yield: 75%) was obtained.
ESI-MS: m/z 597 $[M + H]^+$; $^1$H NMR ($CDCl_3$)δ(ppm): 0.73 (t, J = 7.5 Hz, 3H), 2.37-2.50 (m, 1H), 2.77 (s, 3H), 2.80-2.92 (m, 1H), 3.76 (s, 3H), 3.85 (s, 3H), 5.10 (d, J = 12.1 Hz, 1H), 5.55 (s, 2H), 6.05 (d, J = 12.1 Hz, 1H), 6. 39 (dd, J = 2.2, 8.4 Hz, 1H), 6.36-6.47 (m, 3H), 6.84 (s, 1H),6.95-7.00 (m, 2H), 7.09 (d, J = 8.1 Hz, 1H), 7.12-7.18 (m, 1H), 7.26-7.31 (m, 1H), 7.60 (s, 1H), 7.66 (m, 1H), 7.89 (dd, J = 2.0, 4.8 Hz, 1H), 8.03 (d, J = 0.7 Hz, 1H).

Example 24

(Z)-1-isopropyl-5-{1-[3-(methanesulfonamide)phenyl] propylidene}-5,11-dihydro-1H-indazolo[5,6-e]pyrido-[2,3-b]oxepin (Compound 25)

Compound A63 (450 mg, 1.01 mmol) obtained in the Step 3 of Reference Example 13 and 3-(methanesulfonamide)phenylboronic acid (261 mg, 1.21 mmol) were dissolved in a mixed solvent of 1,4-dioxane (8.0 mL) and water (2.0 mL). To this, sodium carbonate (321 mg, 3.03 mmol) and palladium acetate (45.4 mg, 0.202 mmol) were added at room temperature, and the mixture was stirred at 80˚C for 40 minutes. To the reaction mixture, a saturated ammonium chloride aqueous solution was added, and extraction with ethyl acetate was performed twice. The organic layer was dried over anhydrous magnesium sulfate. After filtration and concentration under reduced pressure, the residue was purified by silica gel column chromatography to give Compound 25 (220 mg, yield: 45%).
ESI-MS: m/z 489 $[M + H]^+$; $^1$H NMR (DMSO)δ(ppm): 0.66 (t, J = 7.6 Hz, 3H), 1.49 (d, J = 6.3 Hz, 3H), 1.54 (d, J = 6.3 Hz, 3H), 2.35-2.46 (m, 1H), 2.70-2.80 (m, 1H), 2.73 (s, 3H), 4.96-5.08 (m, 1H), 5.22 (d, J = 12.2 Hz, 1H), 5. 95 (d, J = 12.2 Hz, 1H), 6.56 (dd, J = 4.6, 7.6 Hz, 1H), 6.96-7.06 (m, 2H), 7.12 (d, J = 7.3 Hz, 1H), 7.20 (s, 1H), 7.24-7.32 (m, 1H), 7.76 (s, 1H), 7.81-7.85 (m, 1H), 8.02 (s, 1H), 8.11 (s, 1H), 9.64 (s, 1H).

Example 25

Step 1

(Z)-5-[1-(3-nitrophenyl)propylidene]-5,11-dihydro-1H-indazolo[5,6-e]pyrido[2,3-b]oxepin (Compound C5)

With use of Compound C4 (793 mg, 1.45 mmol) obtained in the Step 1 of Example 23, in the same manner as in Example 17, Compound C5 (410 mg, yield: 71%) was obtained.
ESI-MS: m/z 399 $[M + H]^+$; $^1H$ NMR (DMSO)δ(ppm): 0.66 (t, J = 7.4 Hz, 3H), 2.45-2.58 (m, 1H), 2.70-2.83 (m, 1H), 5.27 (d, J = 12.2 Hz, 1H), 6.04 (d, J = 12.2 Hz, 1H), 6.54 (dd, J = 4.8, 7.4 Hz, 1H), 6.98 (dd, J = 2.0, 7.4 Hz, 1H), 7.58 (dd, J = 7.9, 7.9 Hz, 1H), 7.76-7.86 (m, 4H), 8.08 (d, J = 8.2 Hz, 1H), 8.15 (s, 1H), 8.21 (s, 1H), 13.29 (s, 1H).

Step 2

(Z)-1-ethyl-5-[1-(3-nitrophenyl)propylidenel-5,11-dihydro-1H-indazolo[5,6-e]pyrido[2,3-b]oxepin (Compound C6)

Compound C5 (51.0 mg, 0.128 mmol) was dissolved in DMF (0.5 mL). To this, 60% sodium hydride (7.7 mg, 0.192 mmol) was added at room temperature and the mixture was stirred for 5 minutes. Iodoethane (20.5 μL, 0.256 mmol) was added thereto, and the mixture was further stirred for 7 hours. To the reaction mixture, a saturated ammonium chloride aqueous solution was added, and extraction with ethyl acetate was performed twice. The organic layer was washed with a saturated sodium chloride aqueous solution and dried over anhydrous magnesium sulfate. After filtration and concentration under reduced pressure, the residue was purified by silica gel column chromatography to give Compound C6 (39.5 mg, yield: 72%).
ESI-MS: m/z 427 $[M + H]^+$; $^1H$ NMR ($CDCl_3$)δ(ppm): 0.74 (t, J = 7.4 Hz, 3H), 1.55 (t, J = 7.2 Hz, 3H), 2.50 (dt, J = 7.2, 14.4 Hz, 1H), 2.95 (dt, J = 7.2, 14.4 4 Hz, 1H), 4.48 (q, J = 7.2 Hz, 2H), 5.20 (d, J = 12.2 Hz, 1H), 6.08 (d, J = 12.2 Hz, 1H), 6.46 (dd, J =4.8, 7.6 Hz, 1H), 6.90 (dd, J = 1.8, 7.6 Hz, 1H), 7.40 (dd, J = 7.9, 7.9 Hz, 1H), 7.54 (d, J = 7.4 Hz, 1H), 7.58 (s, 1H), 7.69 (s, 1H), 7.93 (dd, J = 1.8, 4.8 Hz, 1H), 8.03 (s, 1H), 8.05-8.09 (m, 1H), 8.21-8.24 (m, 1H).

Step 3

(Z)-1-ethyl-5-{1-[3-(methanesulfonamide)phenyl]propylidene} -5,11-dihydro-1H-indazolo[5,6-e]pyrido[2,3-b]oxepin (Compound 26)

With use of Compound C6, in the same manner as in Examples 19 and 20, Compound 26 was obtained.
ESI-MS: m/z 475 $[M + H]^+$; $^1H$ NMR (DMSO)δ(ppm): 0.66 (t, J = 7.3 Hz, 3H), 1.43 (t, J = 7.2 Hz, 3H), 2.35-2.45 (m, 1H), 2.68-2.78 (m, 1H), 2.72 (s, 3H), 4.48 (q, J = 7.2 Hz, 2H), 5.22 (d, J = 12.2 Hz, 1H), 5.96 (d, J = 12.2 Hz, 1H), 6.56 (dd, J = 4.6, 7.6 Hz, 1H), 6.99 (dd, J = 2.0, 7.6 Hz, 1H), 7.01-7.07 (m, 1H), 7.12 (d, J = 7.6 Hz, 1H), 7.20 (s, 1H), 7.29 (dd, J = 7.8, 7.8 Hz, 1H), 7.76 (s, 1H), 7.83 (dd, J = 2.0, 4. 6 Hz, 1H), 7.98 (s, 1H), 8.01 (s, 1H), 9.63 (s, 1H).

Example 26

(Z)-1-methoxycarbonylmethyl-5-[1-(3-nitrophenyl) propylidene]-5,11-dihydro-1H-indazolo[5,6-e]pyrido[2,3-b] oxepin (Compound 27)

With use of Compound C5 obtained in the Step 1 of Example 25 and methyl bromoacetate, in the same manner as in the Step 4 of Reference Example 1 and Examples 19 and 20, Compound 27 was obtained.
ESI-MS: m/z 519 $[M + H]^+$; $^1H$ NMR ($CDCl_3$)δ(ppm): 0.74 (t, J = 7.5 Hz, 3H), 2.45 (dq, J = 7.2, 14.4 Hz, 1H), 2.78 (s, 3H), 2.87 (dq, J = 7.2, 14.4 Hz, 1H), 3.78 (s, 3H), 5.11 (d, J = 12.2 Hz, 1H), 5.19-5.22 (m, 2H), 6.06 (d, J = 12.2 Hz, 1H), 6.48 (dd, J = 4.8, 7. 6 Hz, 1H), 7.00 (dd, J = 1.8, 7.6 Hz, 1H), 7.08-7.18 (m, 4H), 7.28 (d, J = 8.4 Hz, 1H), 7.49 (s, 1H), 7.72 (s, 1H), 7.89 (dd, J = 1.8, 4.8 Hz, 1H), 8.09 (d, J = 1.2 Hz, 1H).

Example 27

(Z)-1-cyanomethyl-5-{1-[3-(methanesulfonamide)phenyl] propylidene}-5,11-dihydro-1H-indazolo[5,6-e]pyrido[2,3-b] oxepin (Compound 28)

With use of Compound C5 obtained in the Step 1 of Example 25 and bromoacetonitrile, in the same manner as in the Step 4 of Reference Example 1 and Examples 19 and 20, Compound 28 was obtained.

ESI-MS: m/z 486 [M + H]$^+$; $^1$H NMR (DMSO)δ(ppm): 0.66 (t, J = 7.3 Hz, 3H), 2.36-2.44 (m, 1H), 2.68-2.76 (m, 1H), 2.73 (s, 3H), 5.22 (d, J = 12.2 Hz, 1H), 5.82-5.86 (m, 2H), 5.98 (d, J = 12.2 Hz, 1H), 6.57 (dd, J = 4.8, 7.7 Hz, 1H), 6.97-7.07 (m, 2H), 7. 13 (d, J = 8.1 Hz, 1H), 7.21 (s, 1H), 7.29 (dd, J = 7.9, 7.9 Hz, 1H), 7.84 (dd, J = 1.8, 4.8 Hz, 1H), 7.86 (s, 1H), 8.08 (s, 1H), 8.28 (d, J = 0.7 Hz, 1H), 9.64 (s, 1H).

Example 28

(Z)-1-(2-benzyloxy)ethyl-5-{1-[3-(methanesulfonamide)-phenyl]propylidene}-5,11-dihydro-1H-indazolo[5,6-e]pyrido[2 , 3-b] oxepin (Compound 29)

With use of Compound A64 (49.5 mg, 0.0921 mmol) obtained in Reference Example 14, in the same manner as in Example 24, Compound 29 (22.0 mg, yield: 41%) was obtained.
ESI-MS: m/z 581 [M + H]$^+$; $^1$H NMR ($CDCl_3$)δ(ppm): 0.74 (t, J = 7.2 Hz, 3H), 2.45 (dq, J = 7.2, 14.4 Hz, 1H), 2.78 (s, 3H), 2.88 (dq, J = 7.2, 14. 4 Hz, 1H), 3.92 (d, J = 5.4 Hz, 2H), 4.42-4.44 (m, 2H), 4.60 (d, J = 5.4 Hz, 2H), 5.07 (d, J = 12.2 Hz, 1H), 6.05 (d, J = 12.2 Hz, 1H), 6.48 (dd, J = 4.8, 7. 7 Hz, 1H), 7.01 (dd, J = 1.8, 7.7 Hz, 1H), 7.10-7.33 (m, 9H), 7.46 (s, 1H), 7.62 (s, 1H), 7. 68 (s, 1H), 7.89 (dd, J = 1.8, 4.8 Hz, 1H), 8.03 (s, 1H).

Example 29

(Z)-1-cyclobutylmethyl-5-{1-[3-(methanesulfonamide)phenyl]-propylidene}-5,11-dihydro-1H-indazolo[5,6-e]pyrido [2,3-b] oxepin (Compound 30)

With use of Compound A65 (37.0 mg, 0.0785 mmol) obtained in Reference Example 15, in the same manner as in Example 24, Compound 30 (19.2 mg, yield: 48%) was obtained.
ESI-MS: m/z 515 [M + H]$^+$; $^1$H NMR ($CDCl_3$)δ(ppm): 0.74 (t, J = 7.5 Hz, 3H), 1.82-1.98 (m, 4H), 2.00-2.10 (m, 2H), 2.38-2.50 (m, 1H), 2.78 (s, 3H), 2.82-2.99 (m, 2H), 4.41 (d, J = 7.2 Hz, 2H), 5.16 (d, J = 12.2 Hz, 1H), 6.07 (d, J = 12.2 Hz, 1H), 6.46 (dd, J = 4.8, 7.7 Hz, 1H), 6.50 (s, 1H), 6.98 (dd, J = 1.8, 7.7 Hz, 1H), 7.04-7.09 (m, 1H), 7.12-7.18 (m, 2H), 7.26-7.32 (m, 1H), 7.55 (s, 1H), 7.67 (s, 1H), 7.89 (dd, J = 1.8, 4.8 Hz, 1H), 8.00 (s, 1H).

Example 30

(Z)-5-{1-[3-(methanesulfonamide)phenyl]propylidene}-1-(tetrahydro-2H-pyrane-4-yl)methyl-5,11-dihydro-1H-indazolo [5,6-e]pyrido[2,3-b]oxepin (Compound 31)

With use of Compound A67 (32.4 mg, 0.0646 mmol) obtained in the Step 2 of Reference Example 16, in the same manner as in Example 24, Compound 31 (15.1 mg, yield: 43%) was obtained. ESI-MS: m/z 545 [M + H]$^+$; $^1$H NMR ($CDCl_3$)δ(ppm): 0.75 (t, J = 7.4 Hz, 3H), 1.42-1.54 (m, 4H), 2.20-2.30 (m, 1H), 2.39-2.52 (m, 1H), 2.79 (s, 3H), 2.79-2.94 (m, 1H), 3.30-3.41 (m, 2H), 3.92-4.00 (m, 2H), 4.28 (d, J = 6.9 Hz, 2H), 5.14 (d, J = 12.2 Hz, 1H), 6.08 (d, J = 12.2 Hz, 1H), 6.48 (dd, J = 4.8, 7.4 Hz, 1H), 7.00 (dd, J = 2.0, 7.4 Hz, 1H), 7.05-7.18 (m, 4H), 7.26-7.33 (m, 1H), 7.53 (s, 1H), 7.69 (s, 1H), 7.89 (dd, J = 2.0, 4.8 Hz, 1H), 8.03 (d, J = 0.7 Hz, 1H).

Example 31

(Z)-5-{1-[3-(methanesulfonamide)phenyl]propylidene}-1-(3-pentyl)-5,11-dihydro-1H-indazolo[5,6-e]pyrido[2,3-b]oxepin (Compound 32)

With use of Compound A68 (30.4 mg, 0.0646 mmol) obtained in Reference Example 17, in the same manner as in Example 24, Compound 32 (14.0 mg, yield: 42%) was obtained.
ESI-MS: m/z 517 [M + H]$^+$; $^1$H NMR ($CDCl_3$)δ(ppm): 0.70 (t, J = 7.3 Hz, 3H), 0.76 (t, J = 7.3 Hz, 3H), 0.78 (t, J = 7.3 Hz, 3H), 1.86-2.00 (m, 2H), 2.01-2.15 (m, 2H), 2.40-2.52 (m, 1H), 2.78 (s, 3H), 2.80-2.94 (m, 1H), 4.23-4.34 (m, 1H), 5.13 (d, J = 12.2 Hz, 1H), 6.08 (d, J = 12.2 Hz, 1H), 6.47 (dd, J = 4.6, 7.6 Hz, 1H), 7.00 (dd, J = 2.0, 7.6 Hz, 1H), 7.04-7.19 (m, 4H), 7.26-7.33 (m, 1H), 7.55 (s, 1H), 7.67 (s, 1H), 7.89 (dd, J = 2.0, 4.6 Hz, 1H), 8.06 (s, 1H).

Example 32

(Z)-5-{1-[3-(methanesulfonamide)phenyl]propylidene}5,11-dihydro-1H-indazolo[5,6-e]pyrido[2,3-b]oxepin (Compound 33)

With use of Compound C5 (50.8 mg, 0.128 mmol) obtained in the Step 1 of Reference Example 25, in the same manner as in Examples 19 and 20, Compound 33 (17.2 mg, 2-step yield: 30%) was obtained.
ESI-MS: m/z 447 $[M + H]^+$; $^1H$ NMR (DMSO)δ(ppm): 0.65 (t, J = 7.4 Hz, 3H), 2.35-2.48 (m, 1H), 2.68-2.80 (m, 1H), 2.73 (s, 3H), 5.27 (d, J = 12.5 Hz, 1H), 5.92 (d, J = 12.5 Hz, 1H), 6.55 (dd, J = 4.8, 7.3 Hz, 1H), 6.99 (dd, J = 1.8, 7.3 Hz, 1H), 7.04 (d, J = 9.5 Hz, 1H), 7.12 (d, J = 7.7 Hz, 1H), 7.18-7.21 (m, 1H), 7.26-7.32 (m, 1H), 7.77 (s, 1H), 7.80-7.83 (m, 2H), 8.11 (s, 1H), 9.63 (s, 1H), 13.29 (s, 1H).

Example 33

(Z)-1-cyclopropyl-5-{1-[3-(methanesulfonamide)phenyl] propylidene}-5,11-dihydro-1H-indazolo[5,6-e]pyrido[2,3-b] oxepin (Compound 34)

With use of Compound A69 (53.0 mg, 0.120 mmol) obtained in Reference Example 42, in the same manner as in Example 24, Compound 34 (24.3 mg, yield: 42%) was obtained.
ESI-MS: m/z 487 $[M + H]^+$; $^1H$ NMR ($CDCl_3$)δ(ppm): 0.74 (t, J = 7.3 Hz, 3H), 1.18-1.34 (m, 4H), 2.38-2.50 (m, 1H), 2.78 (s, 3H), 2.79-2.92 (m, 1H), 3.58-3.66 (m, 1H), 5.18 (d, J = 12.1 Hz, 1H), 6.08 (d, J = 12.1 Hz, 1H), 6. 47 (dd, J = 4.8, 7.3 Hz, 1H), 6.61 (s, 1H), 6.98 (dd, J = 1.8, 7.3 Hz, 1H), 7.08 (dd, J = 1.5, 7.0 Hz, 1H), 7.13-7.18 (m, 2H), 7.25-7.32 (m, 1H), 7.66 (s, 1H), 7.74 (s, 1H), 7.89 (dd, J = 1.8, 4.8 Hz, 1H), 7.97 (s, 1H).

Example 34

(Z)-1-(1-tert-butoxycarbonylpiperidine-4-yl)methyl-5-{1-[3-(methanesulfonamide)phenyl]propylidene}-5,11-dihydro-1H-indazolo[5,6-e]pyrido[2,3-b]oxepin (Compound 35)

With use of Compound A71 (413 mg, 0.688 mmol) obtained in the Step 2 of Reference Example 19, in the same manner as in Example 24, Compound 35 (186 mg, yield: 42%) was obtained. ESI-MS: m/z 644 $[M + H]^+$; $^1H$ NMR ($CDCl_3$)δ(ppm): 0.75 (t, J = 7.5 Hz, 3H), 1.18-1.33 (m, 2H), 1.45 (s, 9H), 1.48-1.63 (m, 2H), 2.12-2.28 (m, 1H), 2.39-2.52 (m, 1H), 2.58-2.72 (m, 2H), 2.78 (s, 3H), 2.78-2.94 (m, 1H), 4.02-4.18 (m, 2H), 4.27 (d, J = 7.0 Hz, 2H), 5.14 (d, J = 12.2 Hz, 1H), 6.08 (d, J = 12.2 Hz, 1H), 6.48 (dd, J = 4.8, 7.7 Hz, 1H), 7.00 (dd, J = 1.8, 7.7 Hz, 1H), 7.05-7.18 (m, 4H), 7.29 (dd, J = 7.7, 7.7 Hz, 1H), 7.51 (s, 1H), 7.68 (s, 1H), 7.89 (dd, J = 1.8, 4.8 Hz, 1H), 8.02 (s, 1H).

Example 35

(Z)-5-{1-[3-(methanesulfonamide)phenyl]propylidene}-1-(piperidine-4-yl)methyl-5,11-dihydro-1H-indazolo[5,6-e] pyrido[2,3-b]oxepin dihydrochloride (Compound 36)

Compound 35 (176 mg, 0.273 mmol) was suspended in ethyl acetate (2.0 mL). To this, a 4 mol/L hydrochloric acid/ethyl acetate solution (4.0 mL) was added at room temperature, and the mixture was stirred at 60°C for 2 hours. After the reaction mixture was cooled to room temperature, the precipitate was separated by filtration and dried to give Compound 36 (154 mg, yield: 91%).
ESI-MS : m/z 544 $[M + H]^+$; $^1H$ NMR (DMSO)δ(ppm): 0.67 (t, J = 7.5 Hz, 3H), 1.45-1.53 (m, 2H), 1.62-1.72 (m, 2H), 2.12-2.30 (m, 1H), 2.37-2.50 (m, 1H), 2.70-2.88 (m, 3H), 2.75 (s, 3H), 3.19-3.28 (m, 2H), 4.33-4.42 (m, 2H), 5.32 (d, J = 12.5 Hz, 1H), 6.07 (d, J = 12.5 Hz, 1H), 6.73 (dd, J = 5.1, 7.7 Hz, 1H), 6.95-7.02 (m, 1H), 7.08-7.23 (m, 3H), 7.31 (dd, J = 7.7, 7.7 Hz, 1H), 7.82 (s, 1H), 7.93 (dd, J = 1.8, 5.1 Hz, 1H), 8.09 (s, 1H), 8.17 (s, 1H), 8.55-8.75 (br, 1H), 8.92-9.10 (br, 1H), 9.70 (s, 1H).

Example 36

(Z)-1-(1-acetylpiperidine-4-yl)methyl-5-{1-[3-(methanesulfonamide)phenyl]propylidene}-5,11-dihydro-1H-indazolo [5,6-e]pyrido[2,3-b]oxepin (Compound 37)

Compound 36 (12.0 mg, 0.0194 mmol) was dissolved in pyridine (0.25 mL). To this, acetic anhydride (0.5 mL) was added at room temperature, and the mixture was stirred for 5 hours. After the reaction mixture was concentrated

under reduced pressure, the residue was purified by preparative thin-layer chromatography to give Compound 37 (7.8 mg, yield: 68%).
ESI-MS: m/z 586 $[M + H]^+$; $^1H$ NMR ($CDCl_3$)δ(ppm): 0.75 (t, J = 7.4 Hz, 3H), 1.20-1.36 (m, 2H), 1.58-1.77 (m, 2H), 2.07 (s, 3/2H), 2.09 (s, 3/2H), 2.20-2.36 (m, 1H), 2.39-2.57 (m, 2H), 2.79 (s, 3H), 2.79-3.02 (m, 2H), 3.75-3.85 (m, 1H), 4.20-4.37 (m, 2H), 4.60-4.70 (m, 1H), 5.15 (d, J = 12.2 Hz, 1H), 6.08 (d, J = 12.2 Hz, 1H), 6.48 (dd, J = 4.8, 7.7 Hz, 1H), 6.86 (s, 1H), 7.00 (dd, J = 1.8, 7.7 Hz, 1H), 7.09 (d, J = 7.0 Hz, 1H), 7 .14-7 .19 (m, 2H), 7.30 (dd, J = 7.9, 7.9 Hz, 1H), 7.51 (d, J = 2.2 Hz, 1H), 7.69 (s, 1H), 7.90 (dd, J = 1.8, 4.8 Hz, 1H), 8.01-8.05 (m, 1H).

Example 37

(Z)-1-(4-methoxybenzyl)-5-{1-[3-(methanesulfonamide)phenyl] propylidene}-5,11-dihydro-1H-indazolo[5,6-e]pyrido [2,3-b] oxepin (Compound 38)

With use of Compound A72 (46.5 mg, 0.0888 mmol) obtained in Reference Example 20, in the same manner as in Example 24, Compound 38 (20.1 mg, yield: 40%) was obtained.
ESI-MS: m/z 567 $[M + H]^+$; $^1H$ NMR ($CDCl_3$)δ(ppm): 0.73 (t, J = 7.5 Hz, 3H), 2.37-2.50 (m, 1H), 2.77 (s, 3H), 2.77-2.92 (m, 1H), 3.77 (s, 3H), 5.08 (d, J = 12.2 Hz, 1H), 5.55 (s, 2H), 6.03 (d, J = 12.2 Hz, 1H), 6.46 (dd, J = 4.8, 7.3 Hz, 1H), 6.84 (d, J = 8.8 Hz, 2H), 6.90-6.94 (br, 1H), 6.98 (dd, J = 2.0, 7.3 Hz, 1H), 7.07-7.16 (m, 3H), 7.20 (d, J = 8.8 Hz, 2H), 7.26-7.32 (m, 1H), 7.47 (s, 1H), 7.68 (s, 1H), 7.87 (dd, J = 2.0, 4.8 Hz, 1H), 8.05 (d, J = 0.7 Hz, 1H).

Example 38

Step 1

(Z)-1-tert-butoxycaxbonylmethyl-5-{1-[3-(methanesulfonamide)phenyl]propylidene}-5,11-dihydro-1H-indazolo[5,6-e] pyrido[2,3-b]oxepin (Compound C7)

With use of Compound A73 (61.0 mg, 0.118 mmol) obtained in Reference Example 21, in the same manner as in Example 24, Compound C7 (35.0 mg, yield: 53%) was obtained.
ESI-MS: m/z 561 $[M + H]^+$; $^1H$ NMR ($CDCl_3$)δ(ppm): 0.73 (t, J = 7.3 Hz, 3H), 1.47 (s, 9H), 2.38-2.50 (m, 1H), 2.78 (s, 3H), 2.79-2.92 (m, 1H), 5.02-5.15 (m, 3H), 6.06 (d, J = 12.2 Hz, 1H), 6.47 (dd, J = 4.8, 7.3 Hz, 1H), 6.84 (s, 1H), 6.99 (dd, J = 1.8, 7.3 Hz, 1H), 7.07-7.17 (m, 3H), 7.29 (dd, J = 8.4, 8.4 Hz, 1H), 7.47 (s, 1H), 7.70 (s, 1H), 7.88 (dd, J = 1.8, 4.8 Hz, 1H), 8.07 (d,

J = 0.7 Hz, 1H).

Step 2

(Z)-1-carboxymethyl-5-{1-[3-(methanesulfonamide)phenyl]-propylidene}]-5,11-dihydro-1H-indazolo[5,6-e]pyrido[2,3-b] oxepin (Compound C8)

Compound C7 (35.0 mg, 0.0624 mmol) was dissolved in trifluoroacetic acid (1.0 mL), and the mixture was stirred at room temperature for 3 hours. After diethylether (4.0 mL) was added to the reaction mixture, the precipitate was separated by filtration and dried to give Compound C8 (26.4 mg, yield: 84%).
ESI-MS: m/z 505 $[M + H]^+$; $^1H$ NMR (DMSO)δ(ppm): 0.67 (t, J = 7.5 Hz, 3H), 2.36-2.50 (m, 1H), 2.69-2.79 (m, 1H), 2.73 (s, 3H), 5.21 (d, J = 12.2 Hz, 1H), 5.31 (s, 2H), 5.97 (d, J = 12.2 Hz, 1H), 6.59 (dd, J = 4.8, 7.7 Hz, 1H), 7.01-7.07 (m, 2H), 7.13 (d, J = 8.1 Hz, 1H), 7.20 (s, 1H), 7.29 (dd, J = 7.9, 7.9 Hz, 1H), 7.79 (s, 1H), 7.85 (dd, J = 1.8, 4.8 Hz, 1H), 7.94 (s, 1H), 8.15 (d, J = 0.7 Hz, 1H), 9.63 (s, 1H).

Step 3

(Z)-1-carbamoylmethyl-5-{1-[3-(methanesulfonamide)phenyl] propylidene}-5,11-dihydro-1H-indazolo[5,6-e]pyrido [2,3-b] oxepin (Compound 39)

Compound C8 (15.0 mg, 0.0297 mmol) was dissolved in DMF (0.3 mL). To this, 30% aqueous ammonia (0.1 mL) and bromotripyrrolidinophosphonium hexafluorophosphate (23.2 mg, 0.0446 mmol) was added at room temperature, and the mixture was stirred for 4 hours. After a saturated aqueous solution of sodium hydrogen carbonate was added to the reaction mixture, extraction with chloroform was performed twice. The organic layer was washed with saturated

sodium chloride aqueous solution and dried over anhydrous magnesium sulfate. After filtration and concentration under reduced pressure, the residue was purified by preparative thin-layer chromatography to give Compound 39 (8.1 mg, yield: 54%).
ESI-MS: m/z 504 $[M + H]^+$; $^1H$ NMR ($CDCl_3$)δ(ppm): 0.73 (t, J = 7.4 Hz, 3H), 2.37-2.52 (m, 1H), 2.77 (s, 3H), 2.77-2.90 (m, 1H), 5.14 (s, 2H), 5.16 (d, J = 12.2 Hz, 1H), 5.76-5.84 (br, 1H), 5.91-5.99 (br, 1H), 6.07 (d, J = 12.2 Hz, 1H), 6.48 (dd, J = 4.8, 7.5 Hz, 1H), 7.02 (dd, J = 1.8, 7.5 Hz, 1H), 7.09-7.19 (m, 4H), 7.26-7.32 (m, 1H), 7.68 (s, 1H), 7.73 (s, 1H), 7.87 (dd, J = 1.8, 4.8 Hz, 1H), 8.13 (s, 1H).

Example 39

(Z)-5-{1-[3-(methanesulfonamide)phenyl]propylidene}-1-methylcarbamoylmethyl-5,11-dihydro-1H-indaz.olo[5,6-e]pyrido [2,3-b]oxepin (Compound 40)

With use of Compound C8 (15.0 mg, 0.0297 mmol) obtained in the Step 2 of Example 38 and a 2.0 mol/L methylamine/THF solution (44.6 μL, 0.0892 mmol), in the same manner as in the Step 3 of Example 38, Compound 40 (8.2 mg, yield: 53%) was obtained.
ESI-MS: m/z 518 $[M + H]^+$: $^1H$ NMR ($CDCl_3$)δ(ppm): 0.75 (t, J = 7.5 Hz, 3H), 2.40-2.52 (m, 1H), 2.77 (d, J = 4.8 Hz, 3H), 2.77-2.90 (m, 1H), 2.79 (s, 3H), 5.11 (s, 2H), 5.14 (d, J = 12.2 Hz, 1H), 5.84-5.92 (m, 1H), 6.07 (d, J = 12.2 Hz, 1H), 6.49 (dd, J = 4.8, 7.7 Hz, 1H), 7.01 (dd, J = 1.8, 7 .7 Hz, 1H), 7.11-7.20 (m, 4H), 7.26-7.33 (m, 1H), 7.63 (s, 1H), 7.73 (s, 1H), 7.89 (dd, J = 1.8, 4.8 Hz, 1H), 8.14 (s, 1H).

Example 40

(Z)-1-dimethylcarbamoylmethyl-5-{1-[3-(methanesulfonamide)-phenyl]propylidene}-5,11-dihydro-1H-indazolo[5,6-e] pyrido-[2,3-b]oxepin (Compound 41)

With use of Compound C8 (15.0 mg, 0.0297 mmol) obtained in the Step 2 of Example 38 and a 2.0 mol/L dimethylamine/THF solution (44.6 μL, 0.0892 mmol), in the same manner as in the Step 3 of Example 38, the title compound (8.6 mg, yield: 54%) was obtained.
ESI-MS: m/z 532 $[M + H]^+$; $^1H$ NMR ($CDCl_3$)δ(ppm): 0.73 (t, J = 7.4 Hz, 3H), 2.38-2.52 (m, 1H), 2.78 (s, 3H), 2.80-2.94 (m, 1H), 2.99 (s, 3H), 3.16 (s, 3H), 5.10 (d, J = 12.2 Hz, 1H), 5.20 (d, J = 16.2 Hz, 1H), 5.34 (d, J = 16.2 Hz, 1H), 6.03 (d, J = 12.2 Hz, 1H), 6.47 (dd, J = 4.8, 7.6 Hz, 1H), 6.99 (dd, J = 1.8, 7.6 Hz, 1H), 7.08-7.18 (m, 4H), 7.28 (dd, J = 7.9, 7.9 Hz, 1H), 7.56 (s, 1H), 7.68 (s, 1H), 7.88 (dd, J = 1.8, 4.8 Hz, 1H), 8.05 (s, 1H).

Example 41

(Z)-5-{1-[3-(methanesulfonamide)phenyl]propylidene}-1-(3-methyl-1,2,4-oxadiazolo-5-yl)methyl-5,11-dihydro-1H-indazolo[5,6-e]pyrido[2,3-b]oxepin (Compound 42)

With use of Compound C8 (15.0 mg, 0.0297 mmol) obtained in the Step 2 of Example 38 and N'-hydroxy acetamidine (3.3 mg, 0.0446 mmol), in the same manner as in the Step 3 of Example 38, Compound 42 (11.4 mg, yield: 71%) was obtained.
ESI-MS: m/z 543 $[M + H]^+$; $^1H$ NMR ($CDCl_3$)δ(ppm): 0.74 (t, J = 7.5 Hz, 3H), 2.38 (s, 3H), 2.38-2.52 (m, 1H), 2.79 (s, 3H), 2.80-2.93 (m, 1H), 5.12 (d, J = 12.2 Hz, 1H), 5.77-5.90 (m, 2H), 6.04 (d, J = 12.2 Hz, 1H), 6.49 (dd, J = 4.8, 7.7 Hz, 1H), 7.00 (dd, J = 2.0, 7.7 Hz, 1H), 7.10-7.18 (m, 4H), 7.26-7.33 (m, 1H), 7.63 (s, 1H), 7.73 (s, 1H), 7.89 (dd, J = 2.0, 4.8 Hz, 1H), 8.12 (d, J = 0.7 Hz, 1H).

Example 42

(Z)-1-(4-fluorophenyl)-5-{1-[3-(methanesulfonamide)phenyl]-propylidene}-5,11-dihydro-1H-indazolo[5,6-e]pyrido [2,3-b] oxepin (Compound 43)

With use of Compound A74 (290 mg, 0.583 mmol) obtained in Reference Example 22, in the same manner as in Example 24, Compound 43 (145 mg, yield: 46%) was obtained.
ESI-MS: m/z 541 $[M + H]^+$; $^1H$ NMR ($CDCl_3$)δ(ppm): 0.75 (t, J = 7.4 Hz, 3H), 2.40-2.54 (m, 1H), 2.78 (s, 3H), 2.82-2.96 (m, 1H), 5.13 (d, J = 12.2 Hz, 1H), 6.07 (d, J = 12.2 Hz, 1H), 6.49 (dd, J = 4.8, 7.4 Hz, 1H), 6.55 (s, 1H), 7.00 (dd, J = 2.2, 7.4 Hz, 1H), 7.02-7.09 (m, 1H), 7.13-7.18 (m, 2H), 7.24-7.32 (m, 3H), 7.64-7.72 (m, 2H), 7.77 (s, 1H), 7.79 (s, 1H), 7.91 (dd, J = 2.2, 4.8 Hz, 1H), 8.22 (d, J = 0.7 Hz, 1H).

Example 43

(S,Z)-5-{1-[3-methanesulfonamide)phenyl]propylidene}-1-(2-methyl)butyl-5,11-dihydro-1H-indazolo[5,6-e]pyrido [2,3-b] oxepin (Compound 44)

With use of Compound A75 (133 mg, 0.281 mmol) obtained in Reference Example 23, in the same manner as in Example 24, Compound 44 (58.2 mg, yield: 40%) was obtained.
ESI-MS: m/z 517 $[M + H]^+$; $^1H$ NMR ($CDCl_3$)δ(ppm): 0.75 (t, J = 7.4 Hz, 3H), 0.89 (dd, J = 1.0, 6.6 Hz, 3H), 0.94 (t, J = 7.4 Hz, 3H), 1.17-1.28 (m, 1H), 1.35-1.47 (m, 1H), 2.08-2.21 (m, 1H), 2.38-2.50 (m, 1H), 2.79 (s, 3H), 2.79-2.93 (m, 1H), 4.11-4.21 (m, 1H), 4.25-4.35 (m, 1H), 5.13 (d, J = 12.2 Hz, 1H), 6.08 (d, J = 12.2 Hz, 1H), 6.48 (dd, J = 5.0, 7.6 Hz, 1H), 7.00 (dd, J = 2.0, 7.6 Hz, 1H), 7.11-7.19 (m, 3H), 7.26-7.33 (m, 2H), 7.51 (s, 1H), 7.68 (s, 1H), 7.89 (dd, J = 2.0, 5.0 Hz, 1H), 8.02 (s, 1H).

Example 44

(Z)-1-benzyl-5-{1-[3-(methanesulfonamide)phenyl]-propylidene}-5,11-dihydro-1H-indazolo[5,6-e]pyrido-[2,3-b]oxepin (Compound 45)

With use of Compound A76 (24.0 mg, 0.0486 mmol) obtained in Reference Example 24, in the same manner as in Example 24, Compound 45 (5.6 mg, yield: 21%) was obtained.
ESI-MS: m/z 537 $[M + H]^+$; $^1H$ NMR ($CDCl_3$)δ(ppm): 0.73 (t, J = 7.4 Hz, 3H), 2.40-2.52 (m, 1H), 2.77 (s, 3H), 2.77-2.90 (m, 1H), 5.08 (d, J = 12.2 Hz, 1H), 5.62 (s, 2H), 6.03 (d, J = 12.2 Hz, 1H), 6.46 (dd, J = 4.8, 7.4 Hz, 1H), 6.74 (s, 1H), 6.98 (dd, J = 1.7, 7.4 Hz, 1H), 7.05-7.18 (m, 4H), 7.20-7.35 (m, 5H), 7.47 (s, 1H), 7.69 (s, 1H), 7.88 (dd, J = 1.7, 4.6 Hz, 1H), 8.07 (s, 1H).

Example 45

(Z)-1-(4-fluorobenzyl)-5-{1-[3-(methanesulfonamide)phenyl] propylidene}-5,11-dihydro-1H-indazolo[5,6-e]pyrido[2,3-b] oxepin (Compound 46)

With use of Compound A77 (34.0 mg, 0.0665 mmol) obtained in Reference Example 25, in the same manner as in Example 24, Compound 46 (9.1 mg, yield: 25%) was obtained.
ESI-MS: m/z 555 $[M + H]^+$; $^1H$ NMR ($CDCl_3$)δ(ppm): 0.73 (t, J = 7.3 Hz, 3H), 2.38-2.50 (m, 1H), 2.78 (s, 3H), 2.78-2.92 (m, 1H), 5.08 (d, J = 12.2 Hz, 1H), 5.58 (s, 2H), 6.04 (d, J = 12.2 Hz, 1H), 6.47 (dd, J = 4.8, 7.3 Hz, 1H), 6.91 (s, 1H), 6.96-7.04 (m, 3H), 7.07-7.17 (m, 3H), 7.19-7.32 (m, 3H), 7.47 (s, 1H), 7.70 (s, 1H), 7.88 (dd, J = 1.8, 4.8 Hz, 1H), 8.07 (d, J = 0.7 Hz, 1H).

Example 46

(Z)-5-{1-[3-(methanesulfonamide)phenyl]propylidene}-1-(2-methoxybenzyl)-5,11-dihydro-1H-indazolo[5,6-e]pyrido [2,3-b] oxepin (Compound 47)

With use of Compound A78 (34.0 mg, 0.0665 mmol) obtained in Reference Example 26, in the same manner as in Example 24, Compound 47 (7.0 mg, yield: 19%) was obtained.
ESI-MS: m/z 567 $[M + H]^+$; $^1H$ NMR ($CDCl_3$)δppm): 0.73 (t, J = 7.5 Hz, 3H), 2.44 (dt, J = 7.5,14.4 Hz, 1H), 2.77 (s, 3H), 2.81-2.94 (m, 1H), 3.89 (s, 3H), 5.09 (d, J = 12.2 Hz, 1H), 5.64 (s, 2H), 6.05 (d, J = 12.2 Hz, 1H), 6.46 (dd, J = 4.8, 7.7 Hz, 1H), 6.79-6.95 (m, 4H), 6.98 (dd, J = 2.0, 7.7 Hz, 1H), 7.06-7.11 (m, 1H), 7.13-7.18 (m, 2H), 7.22-7.32 (m, 2H), 7.58 (s, 1H), 7.67 (s, 1H), 7.87 (dd, J = 2.0, 4.8 Hz, 1H), 8.06 (d, J = 0.7 Hz, 1H).

Example 47

Step 1

(Z)-2-(5-{1-[3-(methanesulfonamide)phenyl]propylidene}-5,11-dihydro-1H-indazolo[5,6-e]pyrido[2,3-b]oxepin-1-yl) acetohydrazide (Compound C9)

With use of Compound C8 (48.0 mg, 0.0951 mmol) obtained in the Step 2 of Example 38 and hydrazine monohydrate (46.3 μL, 0.951 mmol), in the same manner as in the Step 3 of Example 38, Compound C9 (15.4 mg,

yield: 31%) was obtained.
ESI-MS: m/z 519 [M + H]$^+$; $^1$H NMR (DMSO)δ(ppm): 0.66 (t, J = 7.5 Hz, 3H), 2.36-2.48 (m, 1H), 2.70-2.80 (m, 1H), 2.72 (s, 3H), 4.24-4.40 (br, 2H), 5.08 (s, 2H), 5.19 (d, J = 12.5 Hz, 1H), 5.95 (d, J = 12.5 Hz, 1H), 6.56 (dd, J = 4.8, 7.7 Hz, 1H), 6.99 (dd, J = 1.8, 7.7 Hz, 1H), 7.04 (d, J = 8.1 Hz, 1H), 7.12 (d, J = 7.7 Hz, 1H), 7.20 (s, 1H), 7.29 (dd, J = 7.9, 7.9 Hz, 1H), 7 .77 (s, 1H), 7.83 (dd, J = 1.8, 4.8 Hz, 1H), 7.89 (s, 1H), 8.11 (s, 1H), 9.45 (s, 1H), 9.64 (s, 1H).

Step 2

(Z)-5-{1-[3-(methanesulfonamide)phenyl]propylidene}-1-[1,3, 4-oxadiazole-2(3H)-one-5-yl]methyl-5,11-dihydro-1H-indazolo [5,6-e]pyrido[2,3-b]oxepin (Compound 48)

Compound C9 (15.4 mg, 0.0297 mmol) was dissolved in THF (2.0 mL). To this, carbonyldiimidazole (7.2 mg, 0.0445 mmol) was added at room temperature, and the mixture was stirred overnight. After the reaction mixture was concentrated under reduced pressure, the residue was purified by silica gel column chromatography to give Compound 48 (13.8 mg, yield: 85%). ESI-MS: m/z 545 [M + H]$^+$; $^1$H NMR (DMSO)δ(ppm): 0.66 (t, J = 7.4 Hz, 3H), 2.36-2.48 (m, 1H), 2.68-2.80 (m, 1H), 2.73 (s, 3H), 5.22 (d, J = 12.2 Hz, 1H), 5.72 (s, 2H), 5.96 (d, J = 12.2 Hz, 1H), 6.57 (dd, J = 4.8, 7.6 Hz, 1H), 7.00 (dd, J = 2.0, 7.6 Hz, 1H), 7.05 (d, J = 8.2 Hz, 1H), 7.13 (d, J = 7.6 Hz, 1H), 7.20 (s, 1H), 7.26-7.32 (m, 1H), 7.81-7.86 (m, 2H), 8.02 (s, 1H), 8.21 (s, 1H), 9.64 (s, 1H), 12.3-12.5 (br, 1H).

Example 48

(Z)-5-{1-[3-(methanesulfonamide)phenyl]propylidene}-1-(3-methoxybenzyl)-5,11-dihydro-1H-indazolo[5,6-e]pyrido [2,3-b]oxepin (Compound 49)

With use of Compound A79 (21.9 mg, 0.0418 mmol) obtained in Reference Example 27, in the same manner as in Example 24, Compound 49 (13.0 mg, yield: 55%) was obtained.
ESI-MS: m/z 567 [M + H]$^+$; $^1$H NMR ($CDCl_3$)δ(ppm): 0.73 (t, J = 7.4 Hz, 3H), 2.38-2.51 (m, 1H), 2.76 (s, 3H), 2.79-2.92 (m, 1H), 3.74 (s, 3H), 5.05 (d, J = 12.2 Hz, 1H), 5.58 (s, 2H), 6.03 (d, J = 12.2 Hz, 1H), 6.47 (dd, J = 4.6, 7.6 Hz, 1H), 6.74-6.84 (m, 3H), 6.99 (dd, J = 2.0, 7.6 Hz, 1H), 7.10-7.28 (m, 6H), 7.46 (s, 1H), 7.69 (s, 1H), 7.87 (dd, J = 2.0, 4.8 Hz, 1H), 8.07 (d, J = 0.7 Hz, 1H).

Example 49

(Z)-5-{1-[3-(methanesulfonamide)phenyl]propylidene}-1-(2-methoxypyridine-5-yl)methyl-5,11-dihydro-1H-indazolo-[5,6-e]pyrido[2,3-b]oxepin (Compound 50)

With use of Compound A81 (49.0 mg, 0.0934 mmol) obtained in the Step 2 of Reference Example 28, in the same manner as in Example 24, Compound 50 (22.2 mg, yield: 42%) was obtained. ESI-MS: m/z 568 [M + H]$^+$; $^1$H NMR ($CDCl_3$)δ(ppm): 0.73 (t, J = 7.4 Hz, 3H), 2.38-2.51 (m, 1H), 2.78 (s, 3H), 2.79-2.92 (m, 1H), 3.90 (s, 3H), 5.07 (d, J = 12.2 Hz, 1H), 5.54 (s, 2H), 6.03 (d, J = 12.2 Hz, 1H), 6.47 (dd, J = 4.8, 7.4 Hz, 1H), 6.67 (dd, J = 0.7, 8.6 Hz, 1H), 6.99 (dd, J = 1.8, 7.4 Hz, 1H), 7.10-7.18 (m, 3H), 7.26-7.34 (m, 2H), 7.47 (dd, J = 2.6, 8.6 Hz, 1H), 7.51 (s, 1H), 7.69 (s, 1H), 7.88 (dd, J = 1.8, 4.8 Hz, 1H), 8.05 (d, J = 1.0 Hz, 1H), 8.14 (d, J = 2.6 Hz, 1H).

Example 50

(Z)-5-{1-[3-(methanesulfonamide)phenyl]propylidene}-1-(3-pyridylmethyl)-5,11-dihydro-1H-indazolo[5,6-e]pyrido [2,3-b] oxepin (Compound 51)

With use of Compound A82 (28.0 mg, 0.0566 mmol) obtained in Reference Example 29, in the same manner as in Example 24, Compound 51 (7.2 mg, yield: 24%) was obtained.
ESI-MS: m/z 538 [M + H]$^+$; $^1$H NMR ($CDCl_3$)δ(ppm): 0.72 (t, J = 7.5 Hz, 3H), 2.38-2.50 (m, 1H), 2.78-2.90 (m, 1H), 2.79 (s, 3H), 5.07 (d, J = 12.2 Hz, 1H), 5.69 (dd, J = 16.1, 27.5 Hz, 2H), 5.97 (d, J = 12.2 Hz, 1H), 6.48 (dd, J = 4.8, 7.4 Hz, 1H), 6.97 (s, 1H), 6.99 (dd, J = 2.0, 7.4 Hz, 1H), 7.09-7.17 (m, 3H), 7.22-7.28 (m, 2H), 7.47 (d, J = 8.1 Hz, 1H), 7.58 (s, 1H), 7.72 (s, 1H), 7.90 (dd, J = 2.0, 4.8 Hz, 1H), 8.10 (d, J = 1.1 Hz, 1H), 8.51-8.58 (m, 2H).

Example 51

(Z)-1-(3-ethoxycarbonylphenyl)-5-{1-[3-(methanesulfonamide) phenyl]propylidene}-5,11-dihydro-1H-indazolo[5,6-e] pyrido-[2,3-b]oxepin (Compound 52)

With use of Compound A83 (120 mg, 0.218 mmol) obtained in Reference Example 30, in the same manner as in Example 24, Compound 52 (41 mg, yield: 31%) was obtained.
ESI-MS: m/z 595 $[M + H]^+$; $^1H$ NMR ($CDCl_3$)δ(ppm): 0.75 (t, J = 7.5 Hz, 3H), 1.44 (t, J = 7.3 Hz, 3H), 2.40-2.54 (m, 1H), 2.79 (s, 3H), 2.80-2.94 (m, 1H), 4.45 (q, J = 7.3 Hz, 2H), 5.17 (d, J = 12.2 Hz, 1H), 6.08 (d, J = 12.2 Hz, 1H), 6.43 (s, 1H), 6.49 (dd, J = 4.8, 7.6 Hz, 1H), 7.01 (dd, J = 2.0, 7.6 Hz, 1H), 7.02-7.09 (m, 1H), 7.14-7.19 (m, 2H), 7.25-7.31 (m, 1H), 7.66 (dd, J = 7.9, 7.9 Hz, 1H), 7.78 (s, 1H), 7.88 (s, 1H), 7.89-7.96 (m, 2H), 8.07-8.11 (m, 1H), 8.26 (d, J = 0.7 Hz, 1H), 8.40-8.42 (m, 1H).

Example 52

(Z)-1-(3-carboxyphenyl)-5-{1-[3-(methanesulfonamide)phenyl] propylidene}-5,11-dihydro-1H-indazolo[5,6-e]pyrido [2,3-b] oxepin (Compound 53)

Compound 52 (32.0 mg, 0.0538 mmol) was dissolved in ethanol (0.5 mL). To this, a 2 mol/L sodium hydroxide aqueous solution (0.5 mL) was added at room temperature, and the mixture was stirred for 1 hour. To the reaction mixture, 2 mol/L hydrochloric acid and water were added to adjust the pH to 7. The precipitate was separated by filtration, dried, and then subjected to slurry purification with use of a mixed solvent of ethyl acetate and diethylether to give Compound 53 (26.5 g, yield: 87%).
ESI-MS: m/z 567 $[M + H]^+$; $^1H$ NMR (DMSO)δ(ppm): 0.67 (t, J = 7.5 Hz, 3H), 2.38-2.52 (m, 1H), 2.68-2.80 (m, 1H), 2.73 (s, 3H), 5.35 (d, J = 12.5 Hz, 1H), 5.95 (d, J = 12.5 Hz, 1H), 6.58 (dd, J = 4.8, 7.3 Hz, 1H), 6.99-7.07 (m, 2H), 7.15 (d, J = 7.3 Hz, 1H), 7.21 (s, 1H), 7.30 (dd, J = 7.9, 7.9 Hz, 1H), 7.76 (dd, J = 7.9, 7.9 Hz, 1H), 7.85 (dd, J = 1.8, 4.8 Hz, 1H), 7.94 (s, 1H), 8.00 (d, J = 7.7 Hz, 1H), 8.13 (d, J = 7.3 Hz, 1H), 8.20 (s, 1H), 8.29-8.32 (m, 1H), 8.48 (s, 1H), 9.64 (s, 1H), 13.23-13.41 (br, 1H).

Example 53

(Z)-5-{1-[3-(methanesulfonamide)phenyl]propylidene}-1-(1-phenylethyl)-5,11-dihydro-1H-indazolo[5,6-e]pyrido[2,3-b] oxepin (Compound 54)

With use of Compound A84 (51.6 mg, 0.102 mmol) obtained in Reference Example 31, in the same manner as in Example 24, Compound 54 (23.8 mg, yield: 42%) was obtained.
ESI-MS: m/z 551 $[M + H]^+$; $^1H$ NMR ($CDCl_3$)δ(ppm): 0.72 (dt, J = 5.2, 7.4 Hz, 3H), 2.05 (dd, J = 7.4, 7.4 Hz, 3H), 2.38-2.50 (m, 1H), 2.76 (s, 3H), 2.78-2.90 (m, 1H), 5.04 (d, J = 12.2 Hz, 1H), 5.78-5.88 (m, 2H), 6.01 (dd, J = 6.9, 12.2 Hz, 1H), 6.42-6.48 (m, 1H), 6.89 (d, J = 10.9 Hz, 1H), 6.97 (dt, J = 2.5, 5.0 Hz, 1H), 7.06-7.17 (m, 3H), 7.21-7.29 (m, 5H), 7.42 (d, J = 5.6 Hz, 1H), 7.67 (d, J = 1.8 Hz, 1H), 7.83-7.89 (m, 1H), 8.09 (s, 1H).

Example 54

(Z)-1-(4-ethoxyoarbonyl)phenyl-5-{1-[3-(methanesulfonamide) phenyl]propylidene}-5,11-dihydro-1H-indazolo[5,6-e] pyrido[2 ,3-b]oxepin (Compound 55)

With use of Compound A85 (80.0 mg, 0.145 mmol) obtained in Reference Example 32, in the same manner as in Example 24, Compound 55 (34.7 mg, yield: 40%) was obtained.
ESI-MS: m/z 595 $[M + H]^+$; $^1H$ NMR ($CDCl_3$)δ(ppm): 0.75 (t, J = 7.5 Hz, 3H), 1.45 (t, J = 7.2 Hz, 3H), 2.40-2.54 (m, 1H), 2.79 (s, 3H), 2.80-2.94 (m, 1H), 4.44 (q, J = 7.2 Hz, 2H), 5.17 (d, J = 12.2 Hz, 1H), 6.09 (d, J = 12.2 Hz, 1H), 6.42-6.53 (m, 2H), 7.01 (dd, J = 2.0, 7.6 Hz, 1H), 7.07 (d, J = 9.3 Hz, 2H), 7.14-7.19 (m, 2H), 7.30 (dd, J = 7.9, 7.9 Hz, 1H), 7.79 (s, 1H), 7.86 (d, J = 8.6 Hz, 1H), 7.91 (dd, J = 1.8, 4.8 Hz, 1H), 7.95 (s, 1H), 8.24-8.28 (m, 3H).

Example 55

(Z)-5-{1-[3-(methanesulfonamide)phenyl]propylidene}-1-phenyl-5,11-dihydro-1H-indazolo[5,6-e]pyrido[2,3-b]oxepin (Compound 56)

With use of Compound A86 (17.0 mg, 0.0354 mmol) obtained in Reference Example 33, in the same manner as in Example 24, Compound 56 (4.5 mg, yield: 24%) was obtained.
ESI-MS: m/z 523 $[M + H]^+$; $^1H$ NMR ($CDCl_3$)δ(ppm): 0.75 (t, J = 7.5 Hz, 3H), 2.40-2.53 (m, 1H), 2.78 (s, 3H), 2.81-2.94 (m, 1H), 5.15 (d, J = 12.1 Hz, 1H), 6.07 (d, J = 12.1 Hz, 1H), 6.31 (s, 1H), 6.48 (dd, J = 4.8, 7.7 Hz, 1H), 7.00 (dd, J = 2.0, 7.7 Hz, 1H), 7.05 (d, J = 8.4 Hz, 1H), 7.13-7.19 (m, 2H), 7.26-7.32 (m, 1H), 7.42 (dd, J = 7.5, 7.5 Hz, 1H), 7.55-7.61 (m, 2H), 7.72-7.78 (m, 3H), 7 .88 (s, 1H), 7.91 (dd, J = 2.0, 4.8 Hz, 1H), 8.24 (d, J = 0.7 Hz, 1H).

Example 56

(Z)-1-[3-(dimethylcarbamoyl)phenyl]-5-{1-[3-(methanesulfonamide)phenyl]propylidene}-5,11-dihydro-1H-indazolo [5,6-e]pyrido[2,3-b]oxepin (Compound 57)

With use of Compound 53 (17.0 mg, 0.0300 mmol) obtained in Reference Example 52, in the same manner as in Example 40, Compound 57 (13.0 mg, yield: 73%) was obtained.
ESI-MS: m/z 594 $[M + H]^+$; $^1H$ NMR ($CDCl_3$)δ(ppm): 0.75 (t, J = 7.3 Hz, 3H), 2.40-2.54 (m, 1H), 2.79 (s, 3H), 2.81-2.94 (m, 1H), 3.06 (s, 3H), 3.17 (s, 3H), 5.14 (d, J = 12.1 Hz, 1H), 6.05 (d, J = 12.1 Hz, 1H), 6.49 (dd, J = 4.8, 7.7 Hz, 1H), 6.53 (s, 1H), 7.01 (dd, J = 1.8, 7.7 Hz, 1H), 7.08 (d, J = 7.0 Hz, 1H), 7.13-7.19 (m, 2H), 7.30 (dd, J = 7.9, 7.9 Hz, 1H), 7.44-7.48 (m, 1H), 7.59-7.65 (m, 1H), 7.77 (s, 1H), 7.79-7.84 (m, 2H), 7.88 (s, 1H), 7.91 (dd, J = 1.8, 4.8 Hz, 1H), 8.24 (d, J = 0.7 Hz, 1H).

Example 57

(Z)-5-{1-[3-(methanesulfonamide)phenyl]propylidene}-1-[1-(tetrahydro-2H-pyrane-4-yl)ethyl]-5,11--dihydro-1H-indazolo [5,6-e]pyrido[2,3-b]oxepin (Compound 58)

With use of Compound A88 (63.5 mg, 0.123 mmol) obtained in the Step 2 of Reference Example 34, in the same manner as in Example 24, Compound 58 (28.4 mg, yield: 41%) was obtained. ESI-MS: m/z 559 $[M + H]^+$; $^1H$ NMR ($CDCl_3$)δ(ppm): 0.75 (dt, J = 7.5, 7.5 Hz, 3H), 0.91-1.10 (m, 1H), 1.18-1.34 (m, 1H), 1.60 (dd, J = 6.0, 14.5 Hz, 3H), 1.78-1.91 (m, 1H), 2.20-2.52 (m, 2H), 2.78 (s, 3H), 2.78-2.90 (m, 1H), 3.18-3.52 (m, 2H), 3.78-3.90 (m, 1H), 4.00-4.11 (m, 1H), 4.32-4.42 (m, 1H), 5.16 (d, J = 12.2 Hz, 1H), 6.08 (d, J = 12.2 Hz, 1H), 6.40-6.50 (m, 3H), 6.93-7.02 (m, 1H), 7.03-7.08 (m, 1H), 7.13-7.18 (m, 2H), 7.25-7.32 (m, 1H), 7.55 (s, 1H), 7.67 (s, 1H), 7.87-7.92 (m, 1H), 8.05 (d, J = 3.3 Hz, 1H).

Example 58

(Z)-1-(4-carboxyphenyl)-5-{1-[3-(methanesulfonamide)phenyl] propylidene}-5,11-dihydro-1H-indazolo[5,6-e]pyrido [2,3-b] oxepin (Compound 59)

With use of Compound 55 (32.0 mg, 0.0538 mmol) obtained in Example 54, in the same manner as in Example 52, Compound 59 (8.7 mg, yield: 35%) was obtained.
ESI-MS: m/z 567 $[M + H]^+$; $^1H$ NMR (DMSO)δ(ppm): 0.67 (t, J = 7.5 Hz, 3H), 2.38-2.52 (m, 1H), 2.67-2.80 (m, 1H), 2.73 (s, 3H), 5.35 (d, J = 12.1 Hz, 1H), 5.96 (d, J = 12.1 Hz, 1H), 6.58 (dd, J = 4.8, 7.3 Hz, 1H), 7.00-7.07 (m, 2H), 7.15 (d, J = 7.3 Hz, 1H), 7.22 (s, 1H), 7.30 (dd, J = 7.9, 7.9 Hz, 1H), 7.82-7.87 (m, 1H), 7.95 (s, 1H), 8.00 (d, J = 8.8 Hz, 2H), 8.17 (d, J = 8.8 Hz, 2H), 8.33 (s, 1H), 8.51 (s, 1H), 9.64 (s, 1H).

Example 59

(Z)-1-(2-fluoropyridine-5-yl)-5-{1-[3-(methanesulfonamide) phenyl]propylidene}-5,11-dihydro-1H-indazolo[5,6-e]pyrido [2 ,3-b]oxepin (Compound 60)

With use of Compound A89 (126 mg, 0.253 mmol) obtained in Reference Example 35, in the same manner as in Example 24, Compound 60 (52.0 mg, yield: 38%) was obtained.
ESI-MS: m/z 542 $[M + H]^+$; $^1H$ NMR ($CDCl_3$)δ(ppm): 0.75 (t, J = 7.5 Hz, 3H), 2.40-2.53 (m, 1H), 2.80 (s, 3H), 2.80-2.94

(m, 1H), 5.15 (d, J = 12.2 Hz, 1H), 6.08 (d, J = 12.2 Hz, 1H), 6.47-6.52 (m, 2H), 7.01 (dd, J = 2.0, 7.6 Hz, 1H), 7.07 (d, J = 8.4 Hz, 1H), 7.12-7.20 (m, 3H), 7.26-7.32 (m, 1H), 7.80 (s, 1H), 7.81 (s, 1H), 7.91 (dd, J = 2. 0, 4.6 Hz, 1H), 8.14-8.22 (m, 1H), 8.28 (d, J = 0.7 Hz, 1H), 8.63-8.67 (m, 1H).

Example 60

(Z)-1-(4-cyanophenyl)-5-{1-[3-(methanesulfonamide)phenyl] propylidene}-5,11-dihydro-1H-indazolo[5,6-e]pyrido [2,3-b] oxepin (Compound 61)

With use of Compound A90 (40.0 mg, 0.0793 mmol) obtained in Reference Example 36, in the same manner as in Example 24, Compound 61 (18.0 mg, yield: 41%) was obtained.
ESI-MS: m/z 548 $[M + H]^+$; $^1H$ NMR ($CDCl_3$)δ(ppm): 0.75 (t, J = 7.5 Hz, 3H), 2.40-2.52 (m, 1H), 2.80 (s, 3H), 2.80-2.92 (m, 1H), 5.18 (d, J = 12.5 Hz, 1H), 6.09 (d, J = 12.5 Hz, 1H), 6.43 (s, 1H), 6.49 (dd, J = 4.8, 7.7 Hz, 1H), 7.01 (dd, J = 1.8, 7.7 Hz, 1H), 7.06 (d, J= 6.6 Hz, 1H), 7.14-7.18 (m, 2H), 7.26-7.30 (m, 1H), 7.80 (s, 1H), 7.85-7.96 (m, 6H), 8.29 (d, J = 0.7 Hz, 1H).

Example 61

(Z)-5-{1-[3-(methanesulfonamide)phenyl]propylidene}-1-(pyridine-3-yl)-5,11-dihydro-1H-indazolo[5,6-e]pyrido[2,3-b ] oxepin (Compound 62)

With use of Compound A91 (40.0 mg, 0.0833 mmol) obtained in Reference Example 37, in the same manner as in Example 24, Compound 62 (7.4 mg, yield: 17%) was obtained.
ESI-MS: m/z 524 $[M + H]^+$; $^1H$ NMR ($CDCl_3$)δ(ppm): 0.76 (t, J = 7.4 Hz, 3H), 2.40-2.54 (m, 1H), 2.80 (s, 3H), 2.80-2.94 (m, 1H), 5.16 (d, J = 12.6 Hz, 1H), 6.09 (d, J = 12.6 Hz, 1H), 6.49 (dd, J = 4.8, 7.6 Hz, 1H), 6.52 (s, 1H), 7.01 (dd, J = 2.0, 7.6 Hz, 1H), 7.07 (d, J = 7.9 Hz, 1H), 7.14-7.19 (m, 2H), 7.26-7.33 (m, 1H), 7.54 (dd, J = 5.0, 8.3Hz, 1H), 7.80 (s, 1H), 7.89 (s, 1H), 7.91 (dd, J = 2.0, 4.8 Hz, 1H), 8.06-8.11 (m, 1H), 8.29 (s, 1H), 8.67 (dd, J = 1.5, 4.8 Hz, 1H), 9.10 (d, J = 2.6 Hz, 1H).

Example 62

(Z)-1-(2-fluorobenzyl)-5-{1-[3-(methanesulfonamide)phenyl] propylidene}-5,11-dihydro-1H-indazolo[5,6-e]pyrido[2,3-b] oxepin (Compound 63)

With use of Compound A92 (39.6 mg, 0.0774 mmol) obtained in Reference Example 38, in the same manner as in Example 24, Compound 63 (22.7 mg, yield: 53%) was obtained.
ESI-MS: m/z 555 $[M + H]^+$; $^1H$ NMR ($CDCl_3$)δ(ppm): 0.74 (t, J = 7.5 Hz, 3H), 2.38-2.50 (m, 1H), 2.77 (s, 3H), 2.77-2.90 (m, 1H), 5.10 (d, J= 12.5 Hz, 1H), 5.66 (s, 2H), 6.05 (d, J = 12.5 Hz, 1H), 6.47 (dd, J = 4.8, 7.7 Hz, 1H). 6.99 (dd, J = 1.8, 7.7 Hz, 1H), 7.02-7.33 (m, 9H), 7.55 (s, 1H), 7.69 (s, 1H), 7.87 (dd, J = 1.8, 4.8 Hz, 1H), 8.07 (d, J = 0.7 Hz, 1H).

Example 63

(Z)-1-(3-fluorobenzyl)-5-{1-[3-(methanesulfonamide)phenyl] propylidene}-5,11-dihydro-1H-indazolo[5,6-e]pyrido[2,3-b] oxepin (Compound 64)

With use of Compound A93 (42.3 mg, 0.0827 mmol) obtained in Reference Example 39, in the same manner as in Example 24, Compound 64 (23.0 mg, yield: 50%) was obtained.
ESI-MS: m/z 555 $[M + H]^+$; $^1H$ NMR ($CDCl_3$)δ(ppm): 0.74 (t, J = 7.2 Hz, 3H), 2.45 (dq, J = 7.2, 14. 4 Hz, 1H), 2.77 (s, 3H), 2.77-2.90 (m, 1H), 5.06 (d, J = 12.1 Hz, 1H), 5.60 (s, 2H), 6.04 (d, J = 12.1 Hz, 1H), 6.47 (dd, J = 4.8, 7.7 Hz, 1H), 6.89 (dd, J = 1.8, 9.5 Hz, 1H), 6.95 (dd, J = 2.2, 8.8 Hz, 1H), 6.97-7.03 (m, 2H), 7.10-7.18 (m, 3H), 7.24-7.32 (m, 3H), 7.45 (s, 1H), 7.71 (s, 1H), 7.87 (dd, J = 1.8, 4.8 Hz, 1H), 8.09 (d, J = 0.7 Hz, 1H).

Example 64

(Z)-1-(4-chlorophenyl)-5-{1-[3-(methanesulfonamide)phenyl] propylidene}-5,11-dihydro-1H-indazolo[5,6-e]pyrido [2,3-b] oxepin (Compound 65)

With use of Compound A94 (94.0 mg, 0.183 mmol) obtained in Reference Example 40, in the same manner

as in Example 24, Compound 65 (33.0 mg, yield: 32%) was obtained.
ESI-MS: m/z 557 [M + H]$^+$; $^1$H NMR ($CDCl_3$)δ(ppm): 0.75 (t, J = 7.4 Hz, 3H), 2.40-2.54 (m, 1H), 2.79 (s, 3H), 2.81-2.96 (m, 1H), 5.14 (d, J = 12.2 Hz, 1H), 6.07 (d, J = 12.2 Hz, 1H), 6.46-6.51 (m, 2H), 7.00 (dd, J = 2.0, 7.6 Hz, 1H), 7.03-7.09 (m, 1H), 7.13-7.19 (m, 2H), 7.25-7.33 (m, 1H), 7.52-7.57 (m, 2H), 7.65-7.70 (m, 2H), 7.77 (s, 1H), 7.83 (s, 1H), 7.91 (dd, J = 2.0, 4.6 Hz, 1H), 8.23 (d, J = 1.0 Hz, 1H).

Example 65

(Z)-5-{1-[3-(methanesulfonamide)phenyl]propylidene}-1-(pyridine-2-yl)-5,11-dihydro-1H-indazolo[5,6-e]pyrido[2,3-b ] oxepin (Compound 66)

With use of Compound A95 (80.0 mg, 0.0833 mmol) obtained in Reference Example 41, in the same manner as in Example 24, Compound 66 (9.3 mg, yield: 11%) was obtained. ESI-MS: m/z 524 [M + H]$^+$; $^1$H NMR ($CDCl_3$)δ(ppm): 0. 74 (t, J = 7.4 Hz, 3H), 2.38-2.52 (m, 1H), 2.80 (s, 3H), 2.80-2.94 (m, 1H), 5.29 (d, J = 12. 2 Hz, 1H), 6.10 (d, J = 12.2 Hz, 1H), 6.45-6.51 (m, 2H) 7.01 (dd, J = 2.0, 7.4 Hz, 1H), 7.08 (d, J = 8.3 Hz, 1H), 7.15-7.23 (m, 3H), 7.26-7.33 (m, 1H), 7.73 (s, 1H), 7.82-7.89 (m, 1H), 7.91 (dd, J = 2.0, 4.6 Hz, 1H), 8.05 (d, J = 8.6 Hz, 1H), 8.23 (s, 1H), 8.55-8.58 (m, 1H), 9.02 (s, 1H).

Example 66

(Z)-1-(3-cyanophenyl)-5-{1-[3-(methanesulfonamide)phenyl] propylidene}-5,11-dihydro-1H-indazolo[5,6-e]pyrido [2,3-b] oxepin (Compound 67)

With use of Compound A96 (110 mg, 0.218 mmol) obtained in Reference Example 42, in the same manner as in Example 24, Compound 67 (43.0 mg, yield: 36%) was obtained.
ESI-MS: m/z 548 [M + H]$^+$; $^1$H NMR ($CDCl_3$)δ(ppm): 0.75 (t, J = 7.5 Hz, 3H), 2.40-2.54 (m, 1H), 2.80 (s, 3H), 2.79-2.93 (m, 1H), 5.18 (d, J = 12. 6 Hz, 1H), 6.09 (d, J = 12.6 Hz, 1H), 6.47-6.52 (m, 2H), 7.00 (dd, J = 1.8, 7.5 Hz, 1H), 7.05-7.09 (m, 1H), 7.15-7.19 (m, 2H), 7.25-7.34 (m, 1H), 7.65-7.72 (m, 2H), 7.80 (s, 1H), 7.89 (s, 1H), 7.92 (dd, J = 1.8, 4.8 Hz, 1H), 8.01-8.06 (m, 1H), 8.07-8.10 (m, 1H), 8.27 (d, J = 0.7 Hz, 1H).

Example 67

(Z)-5-{1-[3-(methanesulfonamide)phenyl]propylidene}-1-(3-methoxypheny)-5,11-dihydro-1H-indazolo[5,6-e]pyrido [2,3-b] oxepin (Compound 68)

With use of Compound A97 (76.0 mg, 0.149 mmol) obtained in Reference Example 43, in the same manner as in Example 24, Compound 68 (18.2 mg, yield: 22%) was obtained.
ESI-MS: m/z 553 [M + H]$^+$; $^1$H NMR ($CDCl_3$)δ(ppm): 0.75 (t, J = 7.5 Hz, 3H), 2.40-2.54 (m, 1H), 2.79 (s, 3H), 2.80-2.94 (m, 1H), 3.90 (s, 3H), 5.15 (d, J = 12.2 Hz, 1H), 6.07 (d, J = 12.2 Hz, 1H), 6.44 (s, 1H), 6.48 (dd, J = 4.8, 7.5 Hz, 1H), 6.92-6.98 (m, 1H), 7.00 (dd, J = 2.0, 7.5 Hz, 1H), 7.04-7.09 (m, 1H), 7.14-7.19 (m, 3H), 7.24-7.33 (m, 2H), 7.45-7.52 (m, 1H), 7.76 (s, 1H), 7.88-7.92 (m, 2H), 8.23 (s, 1H).

Example 68

Step 1

(Z)-1-isopropyl-5-[1-(3-nitrophenyl)methylidene]-5,11-dihydro-1H-indazolo[5,6-e]pyrido[2,3-b]oxepin (Compound C10)

Compound A99 (190 mg, 0.353 mmol) obtained in the Step 2 of Reference Example 44 was dissolved in propionitrile (7.1mL). To this, palladium acetate (15.9 mg, 0.071 mmol) and tri-o-tolylphosphine (43.0 mg, 0.141 mmol) were added, and then formic acid (0.081 mL, 2.12 mmol) and piperidine (0.280 mL, 2.82 mmol) were added. The mixture was stirred at 70˚C for 5 hours and 15 minutes, and then cooled to room temperature. Water was added thereto, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 100/0 to 40/60) to give Compound C10 (42.1 mg, yield: 29%) as a yellow paste. ESI-MS m/z:413 [M+H]$^+$; $^1$H-NMR (270 MHz, $CDCl_3$) δ(ppm) : 1.62 (d, J = 6.6 Hz, 6H), 4.84-4.94 (m, 1H), 5.58 (br s, 2H), 6.68 (dd, J = 7.7, 4.8 Hz, 1H), 6.82 (s, 1H), 7.35 (dd, J = 7.7, 2.0 Hz, 1H), 7.42 (t, J = 8.1 Hz, 1H), 7.53 (s, 1H), 7.66 (d, J = 7.7 Hz, 1H), 7.81 (s, 1H), 8.05-8.09 (m, 2H), 8.16

(dd, J = 4.8, 1.8 Hz, 1H), 8.23 (t, J = 2.0 Hz, 1H).

Step 2

(Z)-1-isopropyl-5-{1-[3-(methanesulfonamide)phenyl] methylidene}-5,11-dihydro-1H-indazolo[5,6-e]pyrido[2,3-b]oxepin (Compound 69)

With use of Compound C10 (40 mg, 0.097 mmol), in the same manner as in Examples 19 and 20, Compound 69 (6.1 mg, 2-step yield: 14%) was obtained as a white solid substance.
ESI-MS m/z:461 $[M+H]^+$; $^1$H-NMR (270 MHz, DMSO) δ(ppm) : 1.50 (d, J= 6.6 Hz, 6H), 2.87 (s, 3H), 4.97-5.07 (m, 1H), 5.53 (br s, 2H), 6.78 (dd, J = 7.6, 4.8 Hz, 1H), 6.81 (s, 1H), 7.06 (d, J = 7.6 Hz, 1H), 7.13 (d, J = 7.6 Hz, 1H), 7.24-7.30 (m, 2H), 7.37 (dd, J = 7.6, 2.0 Hz, 1H), 7.86 (s, 1H), 7.94 (s, 1H), 8.06 (dd, J = 4.8, 2.0 Hz, 1H), 8.12 (s, 1H), 9.69 (s, 1H).

Example 69

(Z)-1-isopropyl-5-{1-[3-(methanesulfonamide)phenyl] ethylidene}-5,11-dihydro-1H-indazolo[5,6-e]pyrido[2,3-b] oxepin (Compound 70)

With use of Compound A101 (133 mg, 0.308 mmol) obtained in the Step 2 of Reference Example 45, in the same manner as in Example 24, Compound 70 (5.5 mg, yield: 4%) was obtained as a light yellow solid substance.
ESI-MS m/z:475 $[M+H]^+$; $^1$H-NMR (270 MHz, DMSO) δ(ppm): 1.49 (d, J = 6.6 Hz, 3H), 1.53 (d, J = 6. 6 Hz, 3H), 2.09 (s, 3H), 2.71 (s, 3H), 4.97-5.07 (m, 1H), 5.20 (d, J = 12.6 Hz, 1H), 5.94 (d, J = 12.6 Hz, 1H), 6.57 (dd, J = 7.6, 4.8 Hz, 1H), 6.98-7.03 (m, 2H), 7.12 (d, J = 7.6 Hz, 1H), 7.19 (br s, 1H), 7.26 (t, J = 7. 6 Hz, 1H), 7.75 (s, 1H), 7.85 (dd, J = 4.8, 2. 0 Hz, 1H), 8.00 (s, 1H), 8.11 (s, 1H), 9.63 (br s, 1H).

Example 70

(Z)-1-isopropyl-5-(3-hydroxy-1-[3-(methanesulfonamide) phenyl]propylidene)-5,11-dihydro-1H-indazolo[5,6-e]pyrido [2 ,3-b]oxepin (Compound 71)

With use of Compound A102 (86 mg, 0.158 mmol) obtained in Reference Example 46, in the same manner as in Example 24, Compound 71 (38.1 mg, yield: 44%) was obtained as a white solid substance.
ESI-MS m/z:505 $[M+H]^+$; $^1$H-NMR (300 MHz, DMSO) δ(ppm) : 1.50 (d, J = 6.6 Hz, 3H), 1.54 (d, J = 6.6 Hz, 3H), 2.50-2.58 (m, 1H), 2.72 (s, 3H), 2.88-2.97 (m, 1H), 3.02-3.12 (m, 1H), 3.18-3.25 (m, 1H), 4.33 (t, J = 4.9 Hz, 1H), 4.97-5.06 (m, 1H), 5.18 (d, J = 12.1 Hz, 1H), 6.05 (d, J = 12.1 Hz, 1H), 6.55 (dd, J = 7.3, 4.8 Hz, 1H), 6.97 (dd, J = 7.3, 1.8 Hz, 1H), 7.03 (d, J = 7.9 Hz, 1H), 7.12 (d, J = 7.3 Hz, 1H), 7.20 (s, 1H), 7.27 (t, J = 7.9 Hz, 1H), 7.76 (s, 1H), 7.82 (dd, J = 4.8, 1.8 Hz, 1H), 7.99 (s, 1H), 8.10 (s, 1H), 9.63 (br s, 1H).

Example 71

Step 1

(E)-1-isopropyl-5-[1-(3-nitrophenyl)-2-propenylidene]-5,10-dihydro-1H-indazolo[6,5-b]pyrido[2,2-e]oxepin (Compound C11)

With use of Compound A104 (847 mg, 1.57 mmol) obtained in the Step 2 of Reference Example 47, in the same manner as in the Step 1 of Example 22, Compound C11 (391 mg, yield: 57%) was obtained as a yellow amorphous.
ESI-MS m/z:439 $[M+H]^+$; $^1$H-NMR (270MHz, $CDCl_3$) δ(ppm) : 1.57-1.62 (m, 6H), 4.72-4.82 (m, 1H), 4.86 (d, J = 18.1 Hz, 1H), 5.12 (d, J = 16.0 Hz, 1H), 5.24 (d, J = 11.5 Hz, 1H), 5.75 (d, J = 16.0 Hz, 1H), 6.77 (dd, J = 7.9, 4.8 Hz, 1H), 6.97-7.10 (m, 2H), 7.22 (s, 1H), 7.43-7.45 (m, 2H), 7.68 (s, 1H), 7.99 (s, 1H), 8.08-8.14 (m, 2H), 8.25 (dd, J = 4.8, 1.6 Hz, 1H).

Step 2

(E)-5-[1-(3-aminophenyl)-2-prcapenylidene]-1-isopropyl-5,10-dihydro-1H-indazolo[6,5-b]pyrido[3,2-e]oxepin (Compound C12)

With use of Compound C11 (290 mg, 0.661 mmol) and 10% platinum/carbon (58.0 mg), in the same manner

as in the Step 2 of Example 21, Compound C12 (165 mg, yield: 61%) was obtained as a slightly orange amorphous. ESI-MS m/z:409 $[M+H]^+$; $^1$H-NMR (300MHz, $CDCl_3$) δ(ppm): 1.55-1.61 (m, 6H), 3.58 (br s, 2H), 4.71-4.80 (m, 1H) 5.03-5.21 (m, 3H), 5.74 (d, J = 14.7 Hz, 1H), 6.48-6.58 (m, 3H), 6.81 (dd, J = 7.7, 5.1 Hz, 1H), 6.94-7.06 (m, 2H), 7.16-7.20 (m, 2H), 7.65 (s, 1H), 7.95 (s, 1H), 8.24 (dd, J = 4.8, 1.5 Hz, 1H).

Step 3

(E)-1-isopropyl-5-{1-[3-(methanesulfonamide)phenyl]-2-propenylidene}-5,10-dihydro-1H-indazolo[6,5-b]pyrido[3,2-e] oxepin (Compound 72)

With use of Compound C12 (165 mg, 0.404 mmol), in the same manner as in Example 20, Compound 7.2 (170 mg, yield: 83%) was obtained as a white solid substance.
ESI-MS m/z:487 $[M+H]^+$; $^1$H-NMR (270 MHz, DMSO) δ(ppm) : 1.43 (d, J = 6.6 Hz, 3H), 1.48 (d, J = 6.6 Hz, 3H), 2.70 (s, 3H), 4.88-4.97 (m, 2H), 5.01 (d, J = 14.9 Hz, 1H), 5.26 (dd, J= 10.6, 1.7 Hz, 1H), 5.69 (d, J = 14.9 Hz, 1H), 6.90-7.00 (m, 3H), 7.04-7.08 (m, 2H), 7.12 (dd, J = 7.8, 1.5 Hz, 1H), 7.28 (t, J = 8.1 Hz, 1H), 7.46 (s, 1H), 7.72 (s, 1H), 8.05 (s, 1H), 8.23 (dd, J = 4.8, 1.5 Hz, 1H), 9.62 (br s, 1H).

Example 72

Step 1

(E)-5-[1-(3-aminophenyl)propylidene]-1-isopropyl-5,10-dihydro-1H-indazolo[6,5-b]pyrido[3,2-e]oxepin (Compound C13)

Compound A110 (682 mg, 1.53 mmol) obtained in the Step 6 of Reference Example 48 and 3, 5-dimethoxyphenol (1.18g, 7.66 mmol) were dissolved in 1,4-dioxane (20 mL). To this, 3-iodoaniline (0.276 mL, 2.30 mmol), a potassium hydroxide aqueous solution (2 mol/L, 7.66 mL), and tetrakis(triphenylphosphine)palladium(0) (354 mg, 0.306 mmol) were added, and the mixture was stirred at 80˚C for 2 hours. After the mixture was cooled to room temperature, a saturated ammonium chloride aqueous solution was added, and the aqueous layer was extracted with ethyl acetate. The organic layer was washed with a saturated sodium chloride aqueous solution and dried over anhydrous magnesium sulfate, and then the solvent was evaporated off under reduced pressure. The residue was purified by silica gel column chromatography (hexane/ethyl acetate = 50/50 to 0/100) to give Compound C13 (537 mg, yield: 85%) as a light yellow amorphous.
ESI-MS m/z:411 $[M+H]^+$; $^1$H-NMR (300 MHz, $CDCl_3$) δ(ppm) : 0.81 (t, J = 7.4 Hz, 3H), 1.57 (d, J = 6.6 Hz, 3H), 1.61 (d, J = 6.6 Hz, 3H), 2.37-2.50 (m, 1H), 2.75-2.88 (m, 1H), 3.56 (br s, 2H), 4.72-4.82 (m, 1H), 5.10 (d, J = 15.3 Hz, 1H), 5.75 (d, J = 15.3 Hz, 1H), 6.47-6.55 (m, 3H), 6.78 (dd, J = 7.9, 4.6 Hz, 1H), 6.96-7.02 (m, 1H), 7.13 (dd, J = 7.9, 1.7 Hz, 1H), 7.20 (s, 1H), 7.60 (s, 1H), 7.94 (s, 1H), 8.22 (dd, J = 5.0, 1.7 Hz, 1H).

Step 2

(E)-1-isopropyl-5-{1-[3-(methanesulfonamide)phenyl] propylidene}-5,10-dihydro-1H-indazolo[6,5-b]pyrido[3,2-e] oxepin (Compound 73)

With use of Compound C13 (535 mg, 1.30 mmol), in the same manner as in Example 20, Compound 73 (450 mg, yield: 71%) was obtained as a white solid substance.
ESI-MS m/z:489 $[M+H]^+$; $^1$H-NMR (270 MHz, DMSO) δ(ppm): 0.75 (t, J = 7.4 Hz, 3H), 1.43 (d, J = 6.6 Hz, 3H), 1.49 (d, J = 6.6 Hz, 3H), 2.41-2.54 (m, 1H), 2.67 (s, 3H), 2.70-2.82 (m, 1H), 4.86-4.96 (m, 1H), 5.01 (d, J = 15.2 Hz, 1H), 5.71 (d, J = 15.2 Hz, 1H), 6.91 (dd, J = 7.8, 4.8 Hz, 1H), 6.99-7.06 (m, 4H), 7.24 (t, J= 7.9 Hz, 1H), 7.46 (s, 1H), 7.69 (s, 1H), 8.02 (s, 1H), 8.21 (dd, J = 4.6, 1.7 Hz, 1H), 9.59 (br s, 1H).

Example 73

Step 1

(Z)-1-isopropyl-5-[1-(3-methoxypheny)propylidene]-5,11-dihydro-1H-indazolo[5,6-e]pyrido[2,3-b]oxepin (Compound C14)

With use of Compound A63 (44.3 mg, 0.0995 mmol) obtained in the Step 3 of Reference Example 13 and 3-methoxy phenylboronic acid (18.1 mg, 0.119 mmol), in the same manner as in Example 24, Compound C14 (17.0 mg, yield: 40%) was obtained.
ESI-MS: m/z 426 [M + H]$^+$; $^1$H NMR ($CDCl_3$)δ(ppm): 0.73 (t, J = 7.5 Hz, 3H), 1.59 (d, J = 6.6 Hz, 3H), 1.65 (d, J = 6.6 Hz, 3H), 2.38-2.50 (m, 1H), 2.80-2.92 (m, 1H), 3.74 (s, 3H), 4.84-4.94 (m, 1H), 5.15 (d, J = 12.1 Hz, 1H), 6.09 (d, J = 12.1 Hz, 1H), 6.46 (dd, J = 4.6, 7.5 Hz, 1H), 6.73 (dd, J = 2.4, 7.9 Hz, 1H), 6.80-6.87 (m, 2H), 7.02 (dd, J = 2.0, 7.5 Hz, 1H), 7.16 (dd, J = 7.9, 7.9 Hz, 1H), 7.58 (s, 1H), 7.68 (s, 1H), 7.87-7.92 (m, 1H), 8.02 (s, 1H).

Step 2

(Z)-5-[1-(3-hydroxyphenyl)propylidene]-1-isopropyl-5,11-dihydro-1H-indazolo[5,6-e]pyrido[2,3-b]oxepin (Compound 74)

With use of Compound C14 (17.0 mg, 0.0400 mmol), in the same manner as in the Step 4 of Reference Example 7, Compound 74 (6.0 mg, yield: 36%) was obtained.
ESI-MS: m/z 412 [M + H]$^+$; $^1$H NMR ($CDCl_3$)δ(ppm): 0.79 (t, J = 7.3 Hz, 3H), 1.57 (d, J = 6.6 Hz, 3H), 1.63 (d, J = 6.6 Hz, 3H), 2.43-2.55 (m, 1H), 2.86-2.98 (m, 1H), 4.81-4.91 (m, 1H), 5.13 (d, J = 11.7 Hz, 1H), 6.18 (d, J = 11.7 Hz, 1H), 6.51 (dd, J = 4.8, 7.5 Hz, 1H), 6.72 (dd, J = 2.8, 7.7 Hz, 1H), 6.92 (d, J = 7.7 Hz, 1H), 6.96-7.00 (m, 1H), 7.08 (dd, J = 1.8, 7.5 Hz, 1H), 7.21 (dd, J = 7.9, 7.9 Hz, 1H), 7.55 (s, 1H), 7.70 (s, 1H), 7.75 (dd, J = 1.8, 4.8 Hz, 1H), 8.02 (s, 1H).

Example 74

(Z)-5-[1-(3-acetamidephenyl)propylidene]-1-isopropyl-5,11-d ihydro-1H-indazolo[5,6-e]pyrido[2,3-b]oxepin (Compound 75)

With use of Compound A63 (32.7 mg, 0.0734 mmol) obtained in the Step 3 of Reference Example 13 and 3-acetamidephenylboronic acid (15.8 mg, 0.0881 mmol), in the same manner as in Example 24, Compound 75 (70 mg, yield: 21%) was obtained.
ESI-MS: m/z 453 [M + H]$^+$; $^1$H NMR ($CDCl_3$)δ(ppm): 0.72 (t, J = 7.5 Hz, 3H), 1.59 (d, J = 6.6 Hz, 3H), 1.64 (d, J = 6.6 Hz, 3H), 2.13 (s, 3H), 2.37-2.50 (m, 1H), 2.80-2.92 (m, 1H), 4.84-4.95 (m, 1H), 5.12 (d, J = 12.1 Hz, 1H), 6.07 (d, J = 12.1 Hz, 1H), 6.47 (dd, J = 4.8, 7.3 Hz, 1H), 6.99-7.05 (m, 2H), 7.21 (dd, J = 7.9, 7.9 Hz, 1H), 7.38-7. 48 (m, 3H), 7.57 (s, 1H), 7.67 (s, 1H), 7.89 (dd, J = 1.8, 4.8 Hz, 1H), 8.03 (s, 1H).

Example 75

(Z)-1-isopropyl-5-{1-[3-(methanesulfonamide)pyridine-5-yl] propylidene}-5,11-dihydro-1H-indazolo[5,6-e]pyrido[2,3-b] oxepin (Compound 76)

With use of Compound A111 (27.9 mg, 0.0626 mmol) obtained in Reference Example 49 and Compound A112 obtained in Reference Example 50, in the same manner as in the Step 1 of Example 72, Compound 76 (4.0 mg, yield: 13%) was obtained.
ESI-MS: m/z 490 [M + H]$^+$; $^1$H NMR ($CDCl_3$)δ(ppm): 0.79 (t, J = 7.4 Hz, 3H), 1.59 (d, J = 6.6 Hz, 3H), 1.65 (d, J = 6.6 Hz, 3H), 2.42-2.56 (m, 1H), 2.86 (m, 3H), 2.86-2.99 (m, 1H), 4.84-4.95 (m, 1H), 5.13 (d, J = 12.2 Hz, 1H), 6.04 (d, J = 12.2 Hz, 1H), 6.53 (dd, J = 5.0, 7.6 Hz, 1H), 6.98 (dd, J=2.0, 7.6 Hz, 1H), 7.58-7.62 (m, 2H), 7.69 (s, 1H), 7.93 (dd, J = 2.0, 5.0 Hz, 1H), 8.05 (s, 1H), 8.34 (d, J = 2.6 Hz, 1H), 8.44 (d, J = 2.0 Hz, 1H).

Example 76

(Z)-1-isopropyl-5-[1-(3-propyloxycarbonyl)propylidene]-5,11 -dihydro-1H-indazolo[5,6-e]pyrido[2,3-b]oxepin (Compound 77)

Compound A63 (550 mg, 1.24 mmol) obtained in the Step 3 of Reference Example 13 was dissolved in DMF (27.5 mL) and n-propanol (27.5 mL). To this, N,N-diisopropylethylamine (0.863 mL, 4.94 mmol) and [1,1'-bis(diphenylphosphino)ferrocene]palladium(II) dichloride dichloromethane complex (1:1) (101 mg, 0.124 mmol) were added, and the mixture was stirred under carbon monoxide atmosphere at 100˚C for 6 hours. After the reaction mixture was cooled to room temperature, water was added and the aqueous layer was extracted with ethyl acetate. The organic layer was washed with water and a saturated sodium chloride aqueous solution and then dried over anhydrous magnesium sulfate. After that, the solvent was evaporated off under reduced pressure. The residue was purified by silica gel column chromatography (hexane/ethyl acetate = 95/5 to 30/70) to give Compound 77 (293 mg, yield: 58%) as

a light yellow paste.
ESI-MS m/z:406 $[M+H]^+$; $^1$H-NMR (300 MHz, $CDCl_3$) δ(ppm) : 0.75 (t, J = 7.3 Hz, 3H), 0.97 (t, J = 7.5 Hz, 3H), 1.35-1.53 (m, 2H), 1.58 (d, J = 6.6 Hz, 3H), 1.63 (d, J = 6.6 Hz, 3H), 2.40-2.60 (m, 2H), 3.86-3.94 (m, 1H), 3.98-4.06 (m, 1H), 4.83-4.91 (m, 1H), 5.08 (d, J = 12.5 Hz, 1H), 5.90 (d, J = 12.5 Hz, 1H), 6.80 (dd, J = 7.7, 4.8 Hz, 1H), 7.48 (dd, J = 7.7, 2.0 Hz, 1H), 7.55 (s, 1H), 7.56 (s, 1H), 8.01 (s, 1H), 8.10 (dd, J = 4. 8, 2. 0 Hz, 1H).

Example 77

(Z)-1-isopropyl-5-[1-(3-hydroxycarbonyl)propylidene]-5,11-dihydro-1H-indazolo[5,6-e]pyrido[2,3-b]oxepin hydrochloride (Compound 78)

Compound 77 (370 mg, 0.912 mmol) was dissolved in ethanol (3.7 mL), dimethyl sulfoxide (1.85mL), and a potassium hydroxide aqueous solution (2 mol/L, 3.7 mL), and the mixture was stirred at 100˚C for 2 hours. After the reaction mixture was cooled to room temperature, the solvent was evaporated to some degree under reduced pressure. 1 mol/L hydrochloric acid aqueous solution was added thereto, and the produced solid substance was separated by filtration to give Compound 78 (308 mg, yield: 84%) as a brown solid substance.
ESI-MS m/z:364 $[M+H]^+$; $^1$H-NMR (300 MHz, DMSO) δ(ppm) : 0.91 (t, J = 7.5 Hz, 3H), 1.48 (d, J = 6.6 Hz, 3H), 1.51 (d, J = 6. 6 Hz, 3H), 2.27-2.47 (m, 2H), 4.97-5.05 (m, 1H), 5.14 (d, J = 12.5 Hz, 1H), 5.72 (d, J = 12.5 Hz, 1H), 6.93 (dd, J = 7.6, 4.8 Hz, 1H), 7.53 (dd, J = 7.6, 2.0 Hz, 1H), 7.63 (s, 1H), 8.00 (s, 1H), 8.06 (dd, J = 4.8, 2.0 Hz, 1H), 8.09 (s, 1H), 12.85 (s, 1H).

Example 78

(Z)-1-isoprapyl-5-{1-[(thiazole-2-yl)aminocarbonyl] propylidene}-5,11-dihydro-1H-indazolo[5,6-e]pyrido[2,3-b] oxepin (Compound 79)

Compound 78 (50 mg, 0.125 mmol) was dissolved in 1,2-dichloroethane (1 mL). To this, dimethylformamide (catalyst amount) was added, and then oxalylchloride (0.016 mL, 0.188 mmol) was added under ice-cooling. After the reaction mixture was stirred at room temperature for 1 hour, N-methyl pyrrolidone (1 mL), triethylamine (0.174 mL, 1.25 mmol), and 2-aminothiazole (37.6 mg, 0.375 mmol) were added under ice-cooling, and stirring was continued at room temperature overnight. After water was added to the reaction mixture, the aqueous layer was extracted with ethyl acetate and methanol. The organic layer was dried over anhydrous magnesium sulfate and then the solvent was evaporated off under reduced pressure. The residue was purified by preparative thin-layer chromatography (chloroform/methanol = 30/1) to give Compound 79 (16.5 mg, yield: 29%) as a white solid substance.
ESI-MS m/z:446 $[M+H]^+$; $^1$H-NMR (270 MHz, DMSO) δ(ppm): 0.88 (t, J = 7.4 Hz, 3H), 1.49 (d, J = 6.6 Hz, 3H), 1.53 (d, J = 6.6 Hz, 3H), 2.46 (q, J = 7.4 Hz, 2H), 4.97-5.07 (m, 1H), 5.09 (d, J = 12.4 Hz, 1H), 6.35 (d, J = 12.4 Hz, 1H), 6.77 (dd, J = 7.4, 4.8 Hz, 1H), 7.23 (d, J= 3.6 Hz, 1H), 7.45-7.49 (m, 2H), 7.66 (s, 1H), 7.98-8.00 (m, 2H), 8.10 (s, 1H), 12.61 (br s, 1H).

Example 79

(Z)-1-isopropyl-5-{1-[(5-methyl-1,3,4-thiadiazole-2-y1) aminocarbonyl]propylidene}-5,11-dihydro-1H-indazolo[5,6-e] pyrido[2,3-b]oxepin (Compound 80)

With use of 2-amino-5-methyl-1,3,4-thiadiazole (43. 2 mg, 0.375 mmol), in the same manner as in Example 78, Compound 80 (7.9 mg, yield: 13%) was obtained as a colorless solid substance.
ESI-MS m/z:461 $[M+H]^+$; $^1$H-NMR (270 MHz, DMSO) δ(ppm): 0.88 (t, J = 7.6 Hz, 3H), 1.49 (d, J = 6.6 Hz, 3H), 1.52 (d, J = 6.6 Hz, 3H), 2.46 (q, J = 7.3 Hz, 2H), 2.61 (s, 3H), 4.97-5.07 (m, 1H), 5.10 (d, J = 12.2 Hz, 1H), 6.32 (d, J = 12.2 Hz, 1H), 6.78 (dd, J = 7.5, 5.0 Hz, 1H), 7.43 (dd, J = 7.5, 1.8 Hz, 1H), 7.66 (s, 1H), 7.99-8.02 (m, 2H), 8.11 (s, 1H), 12.89 (br s, 1H).

Example 80

(Z)-1-isopropyl-5-{1-[(1,3,4-thiadiazole-2-yl)aminocarbonyl ]propylidene}-5,11-dihydro-1H-indazolo[5,6-e]pyrido[2,3-b] oxepin (Compound 81)

With use of 2-amino-1,3,4-thiadiazole (73.6 mg, 0.728 mmol), in the same manner as in Example 78, Compound 81 (156 mg, yield: 71%) was obtained as a cream-colored solid substance. ESI-MS m/z:447 $[M+H]^+$; $^1$H-NMR (300 MHz,

DMSO) δ(ppm): 0.88 (t, J = 7.5 Hz, 3H), 1.49 (d, J = 6.6 Hz, 3H), 1.53 (d, J = 6.6 Hz, 3H), 2.47 (q, J = 7.5 Hz, 2H), 4.98-5.07 (m, 1H), 5.11 (d, J = 12.1 Hz, 1H), 6.33 (d, J = 12.1 Hz, 1H), 6.76 (dd, J = 7.3, 4.8 Hz, 1H), 7.43 (d, J = 7.3 Hz, 1H), 7.67 (s, 1H), 7.99 (br s, 2H), 8.11 (s, 1H), 9.18 (s, 1H), 13.08 (br s, 1H).

Example 81

(Z)-1-isopropyl-5-{1-[(4,5-dimethylthiazole-2-yl) aminocarbonyl]propylidene}-5,11-dihydro-1H-indazolo[5,6-e] pyrido [2,3-b]oxepin (Compound 82)

With use of 2-amino-4,5-dimethylthiazole hydrochloride (30.9 mg, 0.188 mmol), in the same manner as in Example 78, Compound 82 (51.5 mg, yield: 84%) was obtained as a brown solid substance.
ESI-MS m/z:474 $[M+H]^+$; $^1H$-NMR (300 MHz, DMSO) δ(ppm): 0.86 (t, J = 7.6 Hz, 3H), 1.49 (d, J = 6.6 Hz, 3H), 1.52 (d, J = 6.6 Hz, 3H), 2.11 (s, 3H), 2.23 (s, 3H), 2.43 (q, J = 7.6 Hz, 2H), 4.97-5.06 (m, 1H), 5.07 (d, J = 12.5 Hz, 1H), 6.33 (d, J = 12.5 Hz, 1H), 6.78 (dd, J = 7.5, 4.6 Hz, 1H), 7.46 (dd, J = 7. 5, 2.0 Hz, 1H), 7.64 (s, 1H), 7.97 (s, 1H), 7.99 (dd, J = 4.6, 2.0 Hz, 1H), 8.09 (s, 1H), 12.35 (br s, 1H).

Example 82

(Z)-1-isopropyl-5-{1-[(5-trifluoromethyl-1,3,4-thiadiazole-2-yl)aminocarbonyl]propylidene}-5,11-dihydro-1H-indazolo[5, 6-e]pyrido[2,3-b]oxepin (Compound 83)

With use of 2-amino-5-trifluoromethyl-1,3,4-thiadiazole (31.7 mg, 0.188 mmol), in the same manner as in Example 78, Compound 83 (23.3 mg, yield: 36%) was obtained as a white solid substance.
ESI-MS m/z:515 5 $[M+H]^+$; $^1H$-NMR (300 MHz, DMSO) δ(ppm) 0. 89 (t, J = 7.5 Hz, 3H), 1. 50 (d, J = 7. 0 Hz, 3H), 1.53 (d, J = 6.6 Hz, 3H), 2.50 (q, J = 7.5 Hz, 2H), 4.98-5.07 (m, 1H), 5.13 (d, J = 12.5 Hz, 1H), 6.30 (d, J = 12.5 Hz, 1H), 6.79 (dd, J = 7.5, 4.8 Hz, 1H), 7.39 (dd, J = 7.5, 1.8 Hz, 1H), 7.69 (s, 1H), 8.01 (s, 1H), 8.02 (dd, J = 4.8, 1.8 Hz, 1H), 8.12 (s, 1H), 13.80 (br s, 1H).

Example 83

(Z)-1-isopropyl-5-[1-(phenylaminocarbonyl)propylidene]-5,11 -dihydro-1H-indazolo[5,6-e]pyrido[2,3-b]oxepin (Compound 84)

With use of aniline (0.057 mL, 0.625 mmol), in the same manner as in Example 78, Compound 84 (45.9 mg, yield: 80%) was obtained as a brown solid substance.
EST-MS m/z:439 $[M+H]^+$; $^1H$-NMR (270 MHz, DMSO) δ(ppm): 0.95 (t, J = 7.4 Hz, 3H), 1.50 (d, J = 6.6 Hz, 3H), 1.53 (d, J = 6.6 Hz, 3H), 2.43 (q, J = 7.4 Hz, 2H), 4.97-5.07 (m, 1H), 5.12 (d, J = 12.2 Hz, 1H), 6.21 (d, J = 12.2 Hz, 1H), 6.84 (dd, J = 7.6, 4.6 Hz, 1H), 7.07 (t, J = 7.6 Hz, 1H), 7.29 (t, J = 7.6 Hz, 2H), 7.40 (d, J = 7.6 Hz, 2H), 7.64 (s, 1H), 7.68 (dd, J = 7.6, 1.7 Hz, 1H), 7.97-8.00 (m, 2H), 8.10 (s, 1H), 9.97 (br s, 1H).

Example 84

(Z)-1-isopropyl-5-[1-(cyclopentylaminocarbonyl)propylidene] -5,11-dihydro-2H-indazolo[5,6-e]pyrido[2,3-b]oxepin (Compound 85)

With use of cyclopentylamine (0.019 mL, 0.188 mmol), in the same manner as in Example 78, Compound 85 (50.9 mg, yield: 92%) was obtained as a brown solid substance.
ESI-MS m/z:431 $[M+H]^+$; $^1H$-NMR (270 MHz, DMSO) δ(ppm) 0.86 (t, J = 7.6 Hz, 3H), 0.90-1.00 (m, 1H), 1.33-1.56 (m, 6H), 1.48 (d, J = 6.6 Hz, 3H), 1.52 (d, J = 6.6 Hz, 5H), 1.67-1.76 (m, 1H), 2.31 (q, J = 7.6 Hz, 2H), 4.00 (dd, J = 13.7, 6.8 Hz, 1H), 4.95-5.05 (m, 1H), 5.05 (d, J = 12.2 Hz, 1H), 6.14 (d, J = 12.2 Hz, 1H), 6.85 (dd, J = 7.6, 4.8 Hz, 1H), 7.57-7.61 (m, 2H), 7.90 (d, J = 7.6 Hz, 1H), 7.94 (s, 1H), 8.01 (dd, J = 4.8, 1.8 Hz, 1H), 8.07 (s, 1H).

Example 85

(Z)-1-isopropyl-5-{1-[(3-hydroxyphenyl)aminocarbonyl] propyllidene}-5,11-dihydro-1H-indazolo[5,6-e]pyrido[2,3-b] oxepin (Compound 86)

With use of 3-aminophenol (20.5 mg, 0.188 mmol), in the same manner as in Example 78, Compound 86 (3.3

mg, yield: 6%) was obtained as a brown solid substance.
ESI-MS m/z:455 $[M+H]^+$; $^1$H-NMR (270 MHz, DMSO) δ(ppm) 0.94 (t, J = 7.4 Hz, 3H), 1.49 (d, J = 6.6 Hz, 3H), 1.53 (d, J = 6.6 Hz, 3H), 2.42 (q, J = 7.4 Hz, 2H), 4.97-5.07 (m, 1H), 5.10 (d, J = 12.2 Hz, 1H), 6.21 (d, J = 12.2 Hz, 1H), 6.46 (dd, J = 8.1, 1.6 Hz, 1H), 6.78-6.85 (m, 2H), 6.98-7.00 (m, 1H), 7.05 (t, J = 8.1 Hz, 1H), 7.63 (s, 1H), 7.67 (dd, J = 7.6, 2.0 Hz, 1H), 7.97-8.00 (m, 2H), 8.10 (s, 1H), 9.37 (s, 1H), 9.86 (br s, 1H).

Example 86

(Z)-1-isopropyl-5-{1-[(4-methoxypheny)aminocarbonyl] propylidene}-5,11-dihydro-1H-indazolo[5,6-e]pyrido[2,3-b] oxepin (Compound 87)

With use of p-anisidine (23.1 mg, 0.188 mmol), in the same manner as in Example 78, Compound 87 (64.1 mg, quantitative yield) was obtained as a brownish solid substance.
ESI-MS m/z:469 $[M+H]^+$; $^1$H-NMR (300 MHz, DMSO) δ(ppm): 0.94 (t, J = 7. 4 Hz, 3H), 1.49 (d, J = 6.6 Hz, 3H), 1.53 (d, J = 6.6 Hz, 3H), 2.42 (q, J = 7.4 Hz, 2H), 3.71 (s, 3H), 4.97-5.06 (m, 1H), 5.10 (d, J = 12.5 Hz, 1H), 6.20 (d, J = 12.5 Hz, 1H), 6.82-6.87 (m, 3H), 7.26 (d, J = 8.8 Hz, 2H), 7.63 (s, 1H), 7.67 (dd, J = 7.5,1.6 Hz, 1H), 7.98-8.00 (m, 2H), 8.10 (s, 1H), 9.80 (s, 1H).

Example 87

(Z)-1-isopropyl-5-{1-[(1-methyl-1H-pyrazole-3-yl) amninocarbonyl]propylidene}-5,11-dihydro-1H-indazolo[5,6-e] pyrido [2,3-b]oxepin (Compound 88)

With use of 1-methyl-1H-pyrazole-3-amine (18.2 mg, 0.188 mmol), in the same manner as in Example 78, Compound 88 (37.0 mg, yield: 66%) was obtained as a brown solid substance.
ESI-MS m/z:443 $[M+H]^+$; $^1$H-NMR (270 MHz, DMSO) δ(ppm): 0.88 (t, J = 7.4 Hz, 3H), 1.49 (d, J = 6. 6 Hz, 3H), 1.52 (d, J = 6.6 Hz, 3H), 2.39 (q, J = 7.4 Hz, 2H), 3.70 (s, 3H), 4.96-5.06 (m, 1H), 5.04 (d, J = 12.2 Hz, 1H), 6.33 (d, J = 12.2 Hz, 1H), 6.46 (d, J = 2.0 Hz, 1H), 6.80 (dd, J = 7.4, 4.8 Hz, 1H), 7.52 (d, J = 2.0 Hz, 1H), 7.60-7.63 (m, 2H), 7.95 (s, 1H), 7.97 (dd, J = 4.8, 2.0 Hz, 1H), 8.09 (s, 1H), 10.79 (br s, 1H).

Example 88

Step 1

(Z)-1-(4-fluorophenyl)-5-[1-(propyioxycarbonyl)propylidene] -5,11-dihydro-1H-indazolo[5,6-e]benzo[b]oxepain (Compound C15)

Compound A16 (380 mg, 0.846 mmol) obtained in the Step 3 of Reference Example 2 was dissolved in 1-propanol (4 mL) and dimethylformamide (1 mL). To this, tributylamine (0.806 mL, 3.38 mmol) and bis(triphenylphosphine) palladium(II) dichloride (59 mg, 0.0846 mmol) were added, and the mixture was refluxed under CO atmosphere for 7 hours. After the reaction mixture was allowed to cool to room temperature, water was added thereto, and the aqueous layer was extracted with ethyl acetate. The organic layer was washed with a 0.05 mol/L hydrochloric acid aqueous solution and a saturated sodium chloride aqueous solution and then dried over anhydrous magnesium sulfate. The solvent was evaporated off under reduced pressure. The residue was purified by silica gel column chromatography (hexane/ethyl acetate) to give Compound C15 (283 mg, yield: 73%). ESI-MS *m/z*: 457 $[M+H]^+$; $^1$H-NMR (270 MHz, $CDCl_3$) δ(ppm): 0.72 (t, *J* = 7.4 Hz, 3H), 0.97 (t, *J* = 7.4 Hz, 3H), 1.22-1.47 (m, 2H), 2.45-2.54 (m, 2H), 3.85-4.04 (m, 2H), 4.91 (d, *J* = 12.2 Hz, 1H), 5.86 (d, *J* = 12.2 Hz, 1H), 6.72-6.82 (m, 2H), 7.07-7.14 (m, 2H), 7.22-7.28 (m, 2H), 7.63-7.72 (m, 4H), 8.18 (d, *J* = 1.0 Hz, 1H).

Step 2

(Z)-1-(4-fluorophenyl)-5-[1-(hydroxycarbonyl)propylidene]-5 ,11-dihydro-1H-indazolo[5,6-e]benzo[b]oxepin (Compound C16)

With use of Compound C15, in the same manner as in Example 77, Compound C16 was obtained.
ESI-MS m/z: 415 $[M+H]^+$; $^1$H-NMR (300 MHz, DMSO) δ(ppm) : 0.91 (t, J = 7.3 Hz, 3H), 2.29-2.43 (m, 2H), 5.11 (d, J = 12.1 Hz, 1H), 5.70 (d, J =12.1 Hz, 1H), 6.70 (dd, J =8.1, 1.1 Hz, 1H), 6.80 (dt, J = 1.1, 7.3 Hz, 1H). 7.09-7.19 (m, 2H), 7.42-7.48 (m, 2H), 7.73 (s, 1H), 7.81-7.85 (m, 2H), 8.03 (s, 1H), 8.38 (s, 1H).

Step 3

(Z)-1-(4-fluorophenyl)-5-{1-[(thiazole-2-yl)aminocarbonyl] propylidene}-5,11-dihydro-1H-indazolo[5,6-e]benzo[b]axepin (Compound 89)

With use of Compound C16 (150 mg, 0.362 mmol), in the same manner as in Example 78, Compound 89 (43 mg, yield: 24%) was obtained.
ESI-MS m/z:497 $[M+H]^+$; $^1$H-NMR (300 MHz, $CDCl_3$) δ(ppm): 1.00 (t, J = 7.5 Hz, 3H), 2.65 (q, J = 7.5 Hz, 2H), 5.00 (d, J = 12.5 Hz, 1H), 5.81 (d, J = 12.5 Hz, 1H), 6.69-6.79 (m, 2H), 6.96 (d, J = 3.7 Hz, 1H), 7.07-7.16 (m, 2H), 7.24-7.29 (m, 2H), 7.36 (d, J = 3.7 Hz, 1H), 7.66-7.71 (m, 3H), 7.74 (s, 1H), 8.21 (d, J = 0.7 Hz, 1H), 8.89 (br s, 1H).

Example 89

(E)-1-(4-fluorophenyl)-5-{1-[(thiazole-2-yl)aminocarbonyl] propylidene}-5,11-dihydro-1H-indazolo [5,6-e]benzo[b]oxepin (Compound 90)

With use of Compound A15 obtained in the Step 3 of Reference Example 2, in the same manner as in Example 88, Compound 90 was obtained as a yellow solid substance.
ESI-MS m/z: 497 $[M+H]^+$; $^1$H-NMR (300 MHz, DMSO) δ(ppm): 1.07 (t, J = 7.3 Hz, 3H), 2.61-2.69 (m, 2H), 5.14 (d, J = 12.1 Hz, 1H), 5.97 (d, J = 12.1Hz, 1H), 6.79 (d, J = 8.4 Hz, 1H), 6.92 (t, J = 7.3 Hz, 1H), 7.13 (d, J = 3.3 Hz, 1H), 7.18-7.23 (m, 2H), 7.37-7.45 (m, 3H), 7.64 (s, 1H), 7.76-7.80 (m, 2H), 7.97 (s, 1H), 8.29 (s, 1H), 12.27 (br s, 1H).

Example 90

(Z)-1-(4-fluorophenyl)-5-{1-[(1,3,4-thiadiazole-2-yl) amninocarbonyl]propylidene}-5,11-dihydro-1H-indazolo[5,6-e]b enzo[b]oxepin (Compound 91)

With use of 2-amino-1,3, 4-thiadiazole (124 mg, 1.23mmol), in the same manner as in the Step 3 of Example 88, Compound 91 (11 mg, yield: 6%) was obtained.
ESI-MS m/z:498 $[M+H]^+$; $^1$H-NMR (300 MHz, $CDCl_3$) δ(ppm) : 1.01 (t, J = 7.4 Hz, 3H), 2.68 (q, J = 7.4 Hz, 2H), 5.00 (d, J = 12.5 Hz, 1H), 5.86 (d, J = 12.5 Hz, 1H), 6.67-6.72 (m, 1H), 6.77-6.81 (m, 1H), 7.08-7.13 (m, 2H), 7.24-7.30 (m, 2H), 7.66-7.75 (m, 4H), 8.22 (s, 1H), 8.81 (s, 1H), 9.67 (br s, 1H).

Example 91

(Z)-1-(4-fluorophenyl)-5-[1-(aminocarbonyl)propylidene]-5,1 1-dihydro-1H-indazolo[5,6-e]benzo[b]oxepin (Compound 92)

With use of an ammonia/methanol solution (7 mol/L, 0.424 mL), in the same manner as in the Step 3 of Example 88, Compound 92 (105 mg, yield: 78%) was obtained as a pink solid substance. ESI-MS m/z:414 $[M+H]^+$; $^1$H-NMR (270 MHz, $CDCl_3$) δ(pom) : 0.98 (t, J = 7.5 Hz, 3H), 2.56 (q, J = 7.5 Hz, 2H), 4.97 (d, J = 12.2 Hz, 1H), 5.40 (br s, 2H), 5.76 (d, J = 12.2 Hz, 1H), 6.76 (dd, J = 8.3, 1.2 Hz, 1H), 6.85 (td, J = 7.5, 1.2 Hz, 1H), 7.12-7.18 (m, 1H), 7.22-7.33 (m, 3H), 7.64-7.70 (m, 3H), 7.72 (s, 1H), 8.20 (d, J = 1.0 Hz, 1H).

Example 92

(Z)-1-(4-fluorophenyl)-5-{1-[(thiazole-2-yl)aminocarbonyl] propylidene}-5,10-dihydro-1H-indazolo[6,5-b]benzo[e]oxepin (Compound 93)

With use of Compound A40 obtained in the Step 9 of Reference Example 7, in the same manner as in Example 88, Compound 93 was obtained as an orange solid substance.
ESI-MS m/z:498 $[M+H]^+$; $^1$H-NMR, (300 MHz, DMSO) δ(ppm) : 0. 96 (t, J = 7.3 Hz, 3H), 2.34-2.53 (m, 2H), 4.98 (d, J = 13.4 Hz, 1H), 6.27 (d, J = 13.4 Hz, 1H), 7.18 (d, J = 3.7 Hz, 1H), 7.21 (s, 1H), 7.33-7.41 (m, 3H), 7.45 (dd, J = 7.7, 4.8 Hz, 1H), 7.67-7.73 (m, 3H), 7.81 (dd, J = 7.7,1.5 Hz, 1H), 8.17 (d, J = 1.1 Hz, 1H), 8.55 (dd, J = 4.8, 1.5 Hz, 1H), 12.57 (br s, 1H).

Example 93

1-(4-fluorophenyl)-5-{1-[3-(methanesulfonamide)phenyl] methylidene}-5,11-dihydro-1H-indazolo[5,6-e]benzo[b]oxepin (Compound 94)

With use of Compound A114 (E/Z mixture) (155 mg, 0.368 mmol) obtained in Reference Example 52, in the same manner as in Example 1, Compound 95 (166 mg, yield: 88%, E/Z = 84/16 or 16/84) was obtained as a slightly yellow amorphous.

ESI-MS m/z: 512 $[M+H]^+$; $^1$H-NMR (300 MHz, DMSO) δ(ppm): 2.53 (s, 3.00H), 5.23 (d, J = 11.2 Hz, 1.00H), 5.71 (d, J=11.2Hz, 1.00H), 6.65-6.70 (m, 0.14H), 6.77-7.29 (m, 8.00H), 7.43-7.50 (m, 2.86H), 7.60 (dd, J = 7.9, 1. 3 Hz, 0.86H), 7.82-7.87 (m, 2.00H), 7.98 (s, 0.28H), 8.11 (s, 0.86H), 8.24 (s, 0.86H), 8.41 (s, 0.14H), 9.55 (br s, 1.00H).

Example 94

Step 1

1-(4-fluorophenyl)-5-[1-(hydroxycarbonyl)propylidene]-5,10-dihydro-1H-indazolo[6,5-b]benzo[e]oxepin (Compound C17)

With use of Compound A43 (E/Z mixture) obtained in the Step 2 of Reference Example 8, in the same manner as in the Steps 1 and 2 of Example 88, Compound C17 (E/Z = 1/1) was obtained. ESI-MS m/z:415 $[M+H]^+$; $^1$H-NMR (270 MHz, DMSO) δ(ppm): 0.94 (t, J = 7.5 Hz, 1.5H), 1.05 (t, J = 7.5 Hz, 1.5H), 2.34 (q, J = 7.5 Hz, 1.0H), 2.53-2.66 (m, 1.0H), 5.01 (d, J = 13.0 Hz, 0.5H), 5.02 (d, J = 13.0 Hz, 0.5H), 5.61 (d, J = 13.0 Hz, 0.5H), 5.66 (d, J = 13.0 Hz, 0.5H), 7.07 (s, 0.5H), 7.16 (s, 0.5H), 7.21-7.41 (m, 5.5H), 7.50-7.53 (m, 0.5H), 7.63 (s, 0.5H), 7.65 (s, 0.5H), 7.70-7.77 (m, 2.0H), 8.24 (s, 0.5H), 8.27 (s, 0.5H).

Step 2

1-(4-fluorophenyl)-5-{1-[(thiazole-2-yl)aminocarbonyl] propylidene}-5,10-dinydro-1H-andazolo[6,5-b]benzo[e]oxepin (Compound 95)

With use of Compound C17 (200 mg, 0.483 mmol), in the same manner as in the Step 3 of Example 88, Compound 95 (E/Z = 1/1, 140 mg, yield: 56%) was obtained as a brownish solid substance. ESI-MS m/z:497 $[M+H]^+$; $^1$H-NMR (300 MHz, DMSO) δ(ppm): 0.93 (t, J = 7.5 Hz, 1.5H), 1.06 (t, J = 7.5 Hz, 1.5H), 2.37-2.46 (m, 1 .0H), 2.69 (q, J = 7.5 Hz, 1.0H), 4.96 (d, J = 12.5 Hz, 0.5H), 5.02 (d, J = 12.5 Hz, 0.5H), 5.89 (d, J = 12.5 Hz, 0.5H), 6.25 (d, J = 12.5 Hz, 0.5H), 7.05 (s, 0.5H), 7.13 (s, 0.5H), 7.16-7.25 (m, 2.5H), 7.31-7.46 (m, 5.0H), 7.52-7.55 (m, 0.5H), 7.62 (s, 0.5H), 7.65-7.77 (m, 2.5H), 8.12 (d, J = 0.7 Hz, 0.5H), 8.31 (d, J = 1.1 Hz, 0.5H), 12.22 (br s, 0.5H), 12.55 (br s, 0.5H).

Example 95

1-(4-fluorophenyl)-5-{1-[(1,3,4-thiadiazole-2-yl) aminocarbonyl]propylidene}-5,10-dihydro-1H-indazolo[6,5-b] benzo[e] oxepin (Compound 96)

With use of Compound C17 (200 mg, 0.483 mmol) obtained in the Step 1 of Example 94 and 2-amino-1, 3, 4-thiadiazole (146 mg, 1.45 mmol), in the same manner as in the Step 3 of Example 88, Compound 96 (E/Z = 1/1, 50.0 mg, yield: 19%) was obtained as a brownish solid substance.

ESI-MS m/z:498 $[M+H]^+$; $^1$H-NMR (300 MHz, DMSO) δ(ppm): 0.94 (d, J = 7.3 Hz, 1.5H), 1.06 (t, J = 7.5 Hz, 1.5H), 2.40-2.47 (m, 1.0H), 2.71 (q, J = 7.5 Hz, 1.0H), 4.98 (d, J = 12.8 Hz, 0.5H), 5.03 (d, J = 12.5 Hz, 0.5H), 5.89 (d, J = 12.8 Hz, 0.5H), 6.23 (d, J = 12.5 Hz, 0.5H), 7.06 (s, 0.5H), 7.15 (s, 0.5H), 7.17-7.26 (m, 1.5H), 7.31-7.45 (m, 3.5H), 7.53-7.56 (m, 0.5H), 7.57 (s, 0.5H), 7.65-7.77 (m, 3.0H), 8.12 (d, J = 1.1 Hz, 0.5H), 8.32 (d, J = 1.1 Hz, 0.5H), 9.12 (d, J = 1.5 Hz, 1.0H), 12.71 (br s, 0.5H), 13.02 (br s, 0.5H).

Example 96

1-(4-fluorophenyl)-5-{1-[(5-methyl-1,3,4-thiadiazole-2-yl) aminocarbonyl]propylidene}-5,1p-dihydro-1H-indazolo[6,5-b] benzo[e]oxepin (Compound 97)

With use of Compound C17 (70 mg, 0.169 mmol) obtained in the Step 1 of Example 94 and 2-amino-5-methyl-1, 3, 4-thiadiazole (58.4 mg, 0.507 mmol), in the same manner as in the Step 3 of Example 88, Compound 97 (E/Z = 1/1, 12.4 mg, yield 14%) was obtained as a brownish solid substance.
ESI-MS m/z:512 $[M+H]^+$; $^1$H-NMR (300 MHz, DMSO) δ(ppm): 0.92 (t, J = 7.5 Hz, 1.5H), 1.05 (t, J = 7.5 Hz, 1.5H), 2.39-2.46 (m, 1.0H), 2.57 (s, 1.5H), 2.57 (s, 1.5H), 2.70 (q, J = 7.5 Hz, 1.0H), 4.97 (d, J = 12.5 Hz, 0.5H), 5.03 (d, J = 12.5 Hz, 0.5H), 5.88 (d, J = 12.5 Hz, 0.5H), 6.23 (d, J = 12.5 Hz, 0.5H), 7.06 (s, 0.5H), 7.14 (s, 0.5H), 7.17-7.27 (m, 1.5H), 7.31-7.46 (m, 4.5H), 7.53-7.56 (m, 0.5H), 7.57 (s, 0.5H), 7.66-7.77 (m, 2.0H), 8.13 (d, J = 0.7 Hz, 0.5H), 8.32 (d, J = 0.7 Hz, 0.5H), 12.51 (br s, 0.5H), 12.83 (br s, 0.5H).

Example 97

1-(4-flucrophenyl)-5-{1-[(5-nitrothiazole-2-yl) aminocarbonyl]propylidene}-5,10-dihydro-1H-indazolo[6,5-b] benzo[e]oxepin (Compound 98)

With use of Compound C17 (95 mg, 0.229 mmol) obtained in the Step 1 of Example 94 and 2-amino-5-nitrothiazole (100 mg, 0.688 mmol), in the same manner as in the Step 3 of Example 88, Compound 98 (E/Z = 1/1, 25.1 mg, yield 18%) was obtained as a brownish solid substance.
ESI-MS m/z:542 $[M+H]^+$; $^1$H-NMR (270 MHz, DMSO) δ(ppm): 0.91 (t, J = 7.4 Hz, 1.5H), 1.03 (t, J = 7.4 Hz, 1.5H), 2.41-2.47 (m, 1.0H), 2.71 (q, J = 7.4 Hz, 1.0H), 4.99 (d, J = 12.2 Hz, 0.5H), 5.03 (d, J = 12.2 Hz, 0.5H), 5.82 (d, J = 12.6 Hz, 0.5H), 6.11 (d, J = 12.2Hz, 0.5H), 7.06 (s, 0.5H), 7.15 (s, 1.0H), 7.19-7.26 (m, 1.0H), 7.31-7.44 (m, 3.5H), 7.53-7.56 (m, 1.0H), 7.66-7.77 (m, 3.0H), 8.14 (s, 0.5H), 8.31 (s, 0.5H), 8.50 (s, 0.5H), 8.53 (s, 0.5H), 13.44 (br s, 1.0H).

Example 98

(Z)-2-isopropyl-5-{1-[3-(methanesulfonamide)phenyl] propylidene}-5,11-dihydro-1H-indazolo[5,6-e]benzo[b]oxepin (Compound 99)

With use of Compound A113 (184 mg, 0.463 mmol) obtained in Reference Example 51, in the same manner as in Example 1, Compound 99 (118 mg, yield: 52%) was obtained as a colorless crystal.
ESI-MS m/z: 488 $[M+H]^+$; $^1$H-NMR (CDCl3)δ(ppm): 0.72 (t, J = 7.5 Hz, 3H), 1.66 (d, J= 6.6 Hz, 6H), 2.39-2.43 (m, 1H), 2.61 (s, 3H), 2.88-2.93 (m, 1H), 4.76-4.85 (m, 1H), 5.00 (d, J = 12.1 Hz, 1H), 5.99 (d, J = 12.1 Hz, 1H), 6.20 (s, 1H), 6.40 (t, J = 7.3 Hz, 1H), 6.58 (dd, J = 1.5,7.7 Hz, 1H), 6.67 (d, J = 8.4 Hz, 1H), 6.81-6.87 (m, 1H), 7.02-7.03 (m, 1H), 7.11 (s, 1H), 7.23-7.30 (m, 2H), 7.56 (s, 1H), 7.81 (s, 1H), 7.96 (s, 1H).

Example 99

In a usual manner, a tablet having the following
composition is prepared. Compound 2 (40g), lactose 286.8g, and potato starch 60g are mixed, and to this mixture, 120 g of a 10% hydroxypropylcellulose aqueous solution is added. This mixture is kneaded in a usual manner, granulate, dried, and fine-granulated to prepare granules for tableting. Magnesium stearate 1.2g is added thereto and mixed with this, and tableting is performed with use of a tableting machine (Type RT-15, made by KIKUSUI) having a pestle 8 rom in diameter to give tablets (containing 20 mg of the active ingredient per tablet).

Table 12

| | | |
|---|---|---|
| Formula | Compound 2 | 20 mg |
| | Lactose | 143.4 mg |
| | Potato starch | 30 mg |
| | Hydroxypropylcellulose | 6 mg |
| | Magnesium stearate | 0.6 mg |
| | | 200 mg |

INDUSTRIAL APPLICABILITY

The present invention provides, for example, a tetracyclic compound or a pharmaceutically acceptable salt thereof having a modulating effect to glucocorticoid receptors and being useful for treatment of a disease in which glucocorticoid receptors are involved, such as inflammatory disease, chronic bronchial asthma, rheumatic disease, connective tissue disease, systemic lupus erythematosus, or systemic lupus erythematosus, or the like.

**Claims**

1. A tetracyclic compound represented by the formula (I):

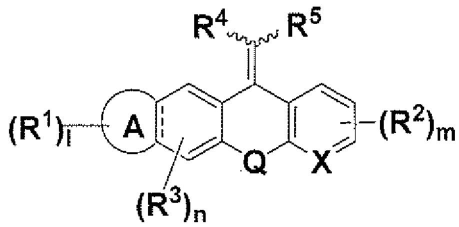

(I)

(in the formula(I),

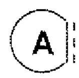

represents a benzene ring or a monocyclic aromatic heterocycle; X represents CH or a nitrogen atom; Q represents -O-, -S-, $-NR^6-$ (wherein $R^6$ represents optionally substituted lower alkyl, optionally substituted cycloalkyl, optionally substituted aryl, an optionally substituted aliphatic heterocyclic group, or an optionally substituted aromatic heterocyclic group), $-CH_2-CH_2-$, -CH=CH-, $-CH_2-O-$, $-O-CH_2-$, $-CH_2-S-$, $-S-CH_2-$, $-NR^6-CO-$ (wherein $R^6$ has the same meaning as defined above), $-CO-NR^6-$ (wherein $R^6$ has the same meaning as defined above), $-NR^6-CH_2-$ (wherein $R^6$ has the same meaning as defined above), $-CH_2-NR^6-$ (wherein $R^6$ has the same meaning as defined above), -CH=N-, or -N=CH-;
$R^1$, $R^2$, and $R^3$ may be the same or different and each represent a hydrogen atom, hydroxy, cyano, carboxy, halogen, optionally substituted lower alkyl, optionally substituted lower alkenyl, optionally substituted lower alkynyl, optionally substituted cycloalkyl, optionally substituted lower alkanoyl, optionally substituted lower alkoxycarbonyl, optionally substituted aryl, optionally substituted aroyl, optionally substituted arylsulfonyl, optionally substituted lower alkylsulfonyl, an optionally substituted aliphatic heterocyclic group, an optionally substituted aromatic heterocyclic group, $-NR^7R^8$ (wherein $R^7$ and $R^8$ may be the same or different and each represent a hydrogen atom, optionally substituted lower alkylsulfonyl, optionally substituted lower alkyl, optionally substituted lower alkanoyl, optionally substituted aryl, an optionally substituted aliphatic heterocyclic group, or an optionally substituted aromatic heterocyclic group, or $R^7$ and $R^8$ are combined together with the adjacent nitrogen atom to form an optionally substituted nitrogen-containing heterocyclic group), or $-OR^9$ (wherein $R^9$ represents optionally substituted lower alkyl, optionally substituted lower cycloalkyl, optionally substituted lower alkanoyl, optionally substituted aryl, an optionally substituted aliphatic heterocyclic group, or an optionally substituted aromatic heterocyclic group); 1, m, and n may be the same or different and each represent an integer from one to the maximum substitutable number; and $R^4$ and $R^5$ may be the same or different and each represent a hydrogen atom, cyano, carboxy, halogen, optionallysubstituted lower alkyl, optionally substituted lower alkenyl, optionally substituted lower alkynyl, optionally substituted cycloalkyl, optionally substituted lower alkanoyl, optionally substituted lower alkoxycarbonyl, optionally substituted aroyl, optionally substituted arylsulfonyl, optionally substituted lower alkylsulfonyl, optionally substituted aryl, an optionally substituted aromatic heterocyclic group, or $-CONR^{10}R^{11}$ (wherein $R^{10}$ and $R^{11}$ may be the same or different and each represent a hydrogen atom, optionally substituted lower alkyl, optionally substituted cycloalkyl, optionally substituted aryl, or an optionally substituted aromatic heterocyclic group)],
or a pharmaceutically acceptable salt thereof.

2. The tetracyclic compound or a pharmaceutically acceptable salt thereof according to claim 1, wherein $R^4$ is a hydrogen atom, cyano, carboxy, halogen, optionally substituted lower alkyl, optionally substituted lower alkenyl, optionally substituted lower alkynyl, optionally substituted cycloalkyl, optionally substituted lower alkanoyl, optionally substituted lower alkoxycarbonyl, optionally substituted aroyl, optionally substituted arylsulfonyl, or optionally substituted lower alkylsulfonyl, and $R^5$ is carboxy, optionally substituted aryl, an optionally substituted aromatic heterocyclic group, optionally substituted lower alkoxycarbonyl, or $-CONR^{10}R^{11}$ (wherein $R^{10}$ and $R^{11}$ have the same meanings as defined above, respectively).

**3.** The tetracyclic compound or a pharmaceutically acceptable salt thereof according to claim 2, wherein $R^5$ is optionally substituted aryl, or an optionally substituted aromatic heterocyclic group.

**4.** The tetracyclic compound or a pharmaceutically acceptable salt thereof according to any one of claims 1 to 3, wherein each of 1, m, and n is 1.

**5.** The tetracyclic compound or a pharmaceutically acceptable salt thereof according to any one of claims 1 to 4, wherein Q is $-CH_2-O-$ or $-O-CH_2-$.

**6.** The tetracyclic compound or a pharmaceutically acceptable salt thereof according to any one of claims 1 to 5, wherein $R^4$ is lower alkyl.

**7.** The tetracyclic compound or a pharmaceutically acceptable salt thereof according to any one of claims 1 to 5, wherein $R^4$ is ethyl.

**8.** The tetracyclic compound or a pharmaceutically acceptable salt thereof according to any one of claims 1, 2, and 4 to 7, wherein $R^5$ is (lower alkylsulfonamido)phenyl.

**9.** The tetracyclic compound or a pharmaceutically acceptable salt thereof according to any one of claims 1, 2, and 4 to 7, wherein $R^5$ is 3-(methanesulfonamido)phenyl.

**10.** The tetracyclic compound or a pharmaceutically acceptable salt thereof according to any one of claims 1, 2, and 4 to 7, wherein $R^5$ is $-CONR^{10}R^{11}$ (wherein $R^{10}$ and $R^{11}$ have the same meanings as defined above).

**11.** The tetracyclic compound or a pharmaceutically acceptable salt thereof according to any one of claims 1 to 10, wherein

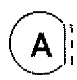

is a monocyclic aromatic heterocycle.

**12.** A tetracyclic compound represented by the formula (Ia) or (Ib):

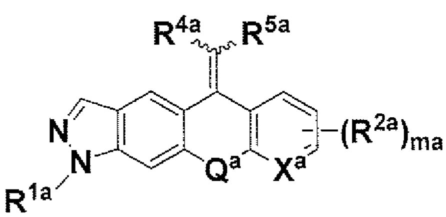 (Ia) 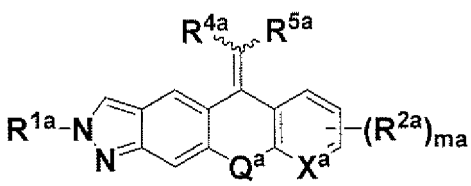 (Ib) ,

(wherein $X^a$, $Q^a$, $R^{1a}$, $R^2$a, $R^{4a}$, and $R^{5a}$ have the same meanings as those of X, Q, $R^1$, $R^2$, $R^4$, and $R^5$ described above, respectively; and ma represents an integer of 1 to 3), or a pharmaceutically acceptable salt thereof.

**13.** The tetracyclic compound or a pharmaceutically acceptable salt thereof according to claim 12, which is represented by the formula (Ia).

**14.** The tetracyclic compound or a pharmaceutically acceptable salt thereof according to claim 12 or 13, wherein $Q^a$ is $-CH_2-O-$ or $-O-CH_2-$.

**15.** The tetracyclic compound or a pharmaceutically acceptable salt thereof according to any one of claims 12 to 14, wherein $R^{4a}$ is lower alkyl.

**16.** The tetracyclic compound or a pharmaceutically acceptable salt thereof according to any one of claims 12 to 14,

wherein $R^{4a}$ is ethyl.

**17.** The tetracyclic compound or a pharmaceutically acceptable salt thereof according to any one of claims 12 to 16, wherein $R^{5a}$ is (lower alkylsulfonamido)phenyl.

**18.** The tetracyclic compound or a pharmaceutically acceptable salt thereof according to any one of claims 12 to 16, wherein $R^{5a}$ is 3-(methanesulfonamido)phenyl.

**19.** The tetracyclic compound or a pharmaceutically acceptable salt thereof according to any one of claims 12 to 16, wherein $R^{5a}$ is $-CONR^{10a}R^{11a}$ (wherein $R^{10a}$ and $R^{11a}$ have the same meanings as those of $R^{10}$ and $R^{11}$ described above, respectively).

**20.** A tetracyclic compound represented by the formula (Ic) or (Id):

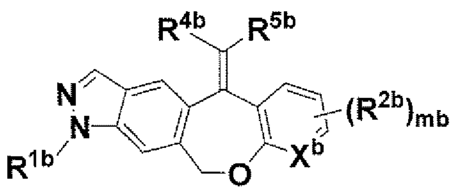 (Ic) 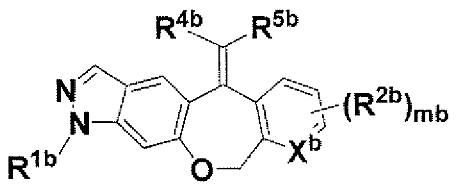 (Id) ,

(wherein $X^b$, $R^{1b}$, $R^{2b}$, $R4^{b}$, and $R^{5b}$ have the same meanings as those of X, $R^1$, $R^2$, $R^4$, and $R^5$ described above, respectively; and mb represents an integer of 1 to 3), or a pharmaceutically acceptable salt thereof.

**21.** The tetracyclic compound or a pharmaceutically acceptable salt thereof according to claim 20, wherein $R^{4b}$ is lower alkyl.

**22.** The tetracyclic compound or a pharmaceutically acceptable salt thereof according to claim 20, wherein $R^{4b}$ is ethyl.

**23.** The tetracyclic compound or a pharmaceutically acceptable salt thereof according to any one of claims 20 to 22, wherein $R^{5b}$ is (lower alkylsulfonamido)phenyl.

**24.** The tetracyclic compound or a pharmaceutically acceptable salt thereof according to any one of claims 20 to 22, wherein $R^{5b}$ is 3-(methanesulfonamido)phenyl.

**25.** The tetracyclic compound or a pharmaceutically acceptable salt thereof according to any one of claims 20 to 22, wherein $R^{5b}$ is $-CONR^{10b}R^{11b}$ (wherein $R^{10b}$ and $R^{11b}$ have the same meanings as those of $R^{10}$ and $R^{11}$ described above, respectively) .

**26.** A pharmaceutical composition comprising, as an active ingredient, the tetracyclic compound or a pharmaceutically acceptable salt thereof according to any one of claims 1 to 25.

**27.** A glucocorticoid receptor modulator comprising, as an active ingredient, the tetracyclic compound or a pharmaceutically acceptable salt thereof according to any one of claims 1 to 25.

**28.** A therapeutic and/or preventive agent for a disease in which glucocorticoid receptors are involved, comprising, as an active ingredient, the tetracyclic compound or a pharmaceutically acceptable salt thereof according to any one of claims 1 to 25.

**29.** A method for modulating glucocorticoid receptors, comprising a step of administering an effective amount of the tetracyclic compound or a pharmaceutically acceptable salt thereof according to any one of claims 1 to 25.

**30.** A method for treating and/or preventing a disease in which glucocorticoid receptors are involved, comprising a step of administering an effective amount of the tetracyclic compound or a pharmaceutically acceptable salt thereof according to any one of claims 1 to 25.

**31.** Use of the tetracyclic compound or a pharmaceutically acceptable salt thereof according to any one of claims 1 to 25 for the manufacture of a glucocorticoid receptor modulator.

**32.** Use of the tetracyclic compound or a pharmaceutically acceptable salt thereof according to any one of claims 1 to 25 for the manufacture of a therapeutic and/or preventive agent for a disease in which glucocorticoid receptors are involved.

**33.** The tetracyclic compound or a pharmaceutically acceptable salt thereof according to any one of claims 1 to 25 for use in modulating glucocorticoid receptors.

**34.** The tetracyclic compound or a pharmaceutically acceptable salt thereof according to any one of claims 1 to 25 for use in treating and/or preventing a disease in which glucocorticoid receptors are involved.

**INTERNATIONAL SEARCH REPORT**

International application No.
PCT/JP2009/062184

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2005-539088 A (Eli Lilly and Co.), 22 December, 2005 (22.12.05), Full text; particularly, Claims; Par. No. [0045]; examples & US 2006/0063759 A1 & US 2009/0149445 A1 & EP 1519915 A2 & WO 2004/052847 A2 & CA 2489276 A & BR 312095 A & HR 20041187 A & NO 20050397 A & PL 374708 A & CN 1665780 A & ZA 200410293 A & CN 101161641 A | 1-28,31-34 |
| A | JP 2006-503107 A (Merck & Co., Inc.), 26 January, 2006 (26.01.06), Full text; particularly, Claims & US 2005/0245588 A1 & EP 1542996 A2 & WO 2004/026248 A2 & CA 2499150 A | 1-28,31-34 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 July, 2009 (24.07.09) | 04 August, 2009 (04.08.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/062184

C (Continuation). DOCUMENTS CONSIDERED TO BE RELEVANT

| Category* | Citation of document, with indication, where appropriate, of the relevant passages | Relevant to claim No. |
|---|---|---|
| A | WO 2008/021926 A2 (BRISTOL-MYERS SQUIBB CO.), 21 February, 2008 (21.02.08), Full text; particularly, Claims & US 2009/0075995 A1 & EP 2049507 A2 & NO 20090564 A & CA 2660318 A & AR 62312 A & CL 23302007 A & KR 10-2009-0038930 A & CL 23302007 A1 | 1-28,31-34 |

Form PCT/ISA/210 (continuation of second sheet) (April 2007)

**INTERNATIONAL SEARCH REPORT**

International application No.
PCT/JP2009/062184

Continuation of A. CLASSIFICATION OF SUBJECT MATTER
(International Patent Classification (IPC))

*C07D491/044*(2006.01)i, *A61K31/4162*(2006.01)i, *A61P1/02*(2006.01)i, *A61P1/04*(2006.01)i, *A61P1/16*(2006.01)i, *A61P1/18*(2006.01)i, *A61P3/10*(2006.01)i, *A61P5/14*(2006.01)i, *A61P5/38*(2006.01)i, *A61P7/06*(2006.01)i, *A61P7/10*(2006.01)i, *A61P9/10*(2006.01)i, *A61P9/14*(2006.01)i, *A61P11/00*(2006.01)i, *A61P11/06*(2006.01)i, *A61P13/12*(2006.01)i, *A61P17/00*(2006.01)i, *A61P17/02*(2006.01)i, *A61P17/04*(2006.01)i, *A61P17/06*(2006.01)i, *A61P17/08*(2006.01)i, *A61P17/14*(2006.01)i, *A61P19/02*(2006.01)i, *A61P19/08*(2006.01)i, *A61P19/10*(2006.01)i, *A61P21/04*(2006.01)i, *A61P25/00*(2006.01)i, *A61P27/02*(2006.01)i, *A61P27/06*(2006.01)i, *A61P27/12*(2006.01)i, *A61P29/00*(2006.01)i, *A61P31/04*(2006.01)i, *A61P31/22*(2006.01)i, *A61P35/00*(2006.01)i, *A61P35/02*(2006.01)i, *A61P37/02*(2006.01)i, *A61P37/06*(2006.01)i, *A61P37/08*(2006.01)i, *A61P43/00*(2006.01)i

(According to International Patent Classification (IPC) or to both national classification and IPC)

Continuation of B. FIELDS SEARCHED
Minimum documentation searched (International Patent Classification (IPC))

C07D491/044, A61K31/4162, A61P1/02, A61P1/04, A61P1/16, A61P1/18, A61P3/10, A61P5/14, A61P5/38, A61P7/06, A61P7/10, A61P9/10, A61P9/14, A61P11/00, A61P11/06, A61P13/12, A61P17/00, A61P17/02, A61P17/04, A61P17/06, A61P17/08, A61P17/14, A61P19/02, A61P19/08, A61P19/10, A61P21/04, A61P25/00, A61P27/02, A61P27/06, A61P27/12, A61P29/00, A61P31/04, A61P31/22, A61P35/00, A61P35/02, A61P37/02, A61P37/06, A61P37/08, A61P43/00

Minimum documentation searched (classification system followed by classification symbols)

**INTERNATIONAL SEARCH REPORT**

International application No.
PCT/JP2009/062184

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 29, 30
because they relate to subject matter not required to be searched by this Authority, namely:
Claims 29 and 30 pertain to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the
☐ The additional search fees were accompanied by the applicant's protest and, where applicable, payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004052847 A
- WO 2005066153 A
- WO 2005066161 A
- WO 2008008882 A
- WO 2008021926 A

### Non-patent literature cited in the description

- **Hatz, HJ.** Glucocoriticoid: Immunologische, Pharmakologie und Therapie richtlinien. 1998
- **T. W. Greene.** Projective Groups in Organic Synthesis. John Wiley & Sons Inc, 1999
- *Chemical & Pharmaceutical Bulletin,* 1991, vol. 39, 2429
- Jikken Kagaku Kouza. Maruzen, 2000, 252
- Synthesis of organic compounds VI, Organic synthesis with the use of metal. Jikken Kagaku Kouza. Maruzen, 2005, 97
- *Org. Synth.,* 1973, vol. 5, 450
- *Synth. Comnun.,* 1997, vol. 27, 1199
- Synthesis of organic compounds I, Hydrocarbons and halides. Jikken Kagaku Kouza. Maruzen, 2005, 298
- Synthesis of organic compounds I, Hydrocarbons and halides. Jikken Kagaku Kouza. Maruzen, 2005, 283
- Synthesis of organic compounds II, Alcohols and amines. Jikken Kagaku Kouza. Maruzen, 2005, 239
- Synthesis of organic compounds I, Hydrocarbons and halides. Jikken Kagaku Kouza. Maruzen, 2005, 341
- *Synth. Commun.,* 1997, vol. 27, 1199
- Synthesis of organic compounds II, Alcohols and amines. Jikken Kagaku Kouza. Maruzen, 2005, 1
- *Mol. Cell Endocrinol.,* 2007, vol. 275 (1-2), 109-117
- *Synth. Commun.,* 1997, vol. 27, 1075-1086